# EUROPEAN PATENT APPLICATION

(11) **EP 4 647 429 A1**
(43) Date of publication of application: **12.11.2025**
(21) Application number: 24738492.8
(22) Date of filing: 03.01.2024
(51) Int. Cl.: C07D 403/04, C07D 403/06, C07D 403/12, C07D 401/14, C07D 237/14, A61K 31/506, A61P 21/00

(54) **PYRIDAZINONE HETEROCYCLIC COMPOUND MYOSIN II INHIBITOR AND USE THEREOF**

(30) Priority: 04.01.2023 CN 202310009260; 24.02.2023 CN 202310161558; 24.03.2023 CN 202310298557; 27.04.2023 CN 202310470296; 23.05.2023 CN 202310584287; 14.06.2023 CN 202310703344; 06.07.2023 CN 202310824314; 26.07.2023 CN 202310924718; 15.08.2023 CN 202311023496; 13.09.2023 CN 202311178238
(71) Applicant: Xizang Haisco Pharmaceutical Co., Ltd., Lhoka, Tibet 856099 (CN)
(72) Inventor: LI, Yao, Tibet 856099 (CN); ZHANG, Haoliang, Tibet 856099 (CN); SHI, Zongjun, Tibet 856099 (CN); WANG, Long, Tibet 856099 (CN); ZHAO, Jingzheng, Tibet 856099 (CN); FU, Chao, Tibet 856099 (CN); GUI, Naicheng, Tibet 856099 (CN); WANG, Jiancheng, Tibet 856099 (CN); LIU, Xin, Tibet 856099 (CN); ZHANG, Chen, Tibet 856099 (CN); YAN, Pangke, Tibet 856099 (CN)
(74) Representative: AWA Denmark A/S
(86) International application number: PCT/CN2024/070392
(87) International publication number: WO 2024/146559

(57) **Abstract**

Disclosed in the present invention are a pyridazinone heterocyclic compound shown in formula (I), a stereoisomer, a deuterated substance, a solvate, a pharmaceutically acceptable salt or a eutectic crystal thereof, a pharmaceutical composition containing same, and a use thereof in the preparation of drugs for treating/preventing Myosin II-mediated diseases. Groups in the formula (I) are as defined in the description.

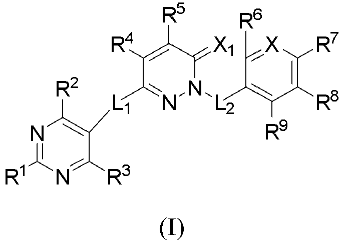

## Description

### Technical Field

The present invention relates to a Myosin II inhibitor, or a stereoisomer, pharmaceutically acceptable salt, solvate, eutectic crystal or deuterated substance thereof, and the use thereof in the preparation of a drug for treating related diseases mediated by Myosin II.

### Background Art

The bone marrow muscle plays two main roles that are vital to the human body: first, muscle contraction, producing a state of movement and maintaining posture; second, skeletal muscle is also the place of glucose, fatty acids and amino acid metabolism. In the daily activities of normal human body, the contraction of bone marrow muscle is closely related to muscle stress, decomposition and remodeling, which are crucial to muscle adaptation. However, in patients with progressive muscular dystrophy, such as Duchenne muscular dystrophy (DMD), the contraction of the muscles leads to successive rounds of amplified sarcolysis that is difficult to repair. As the patient ages, finally these changes will gradually accumulate and develop into a pathological process, which will lead to excessive inflammation, fibrosis and accumulation of fat deposits in the muscles, which will then progress to a sharp decline in body function and eventually lead to death.

DMD is a genetic disorder that affects skeletal muscle. Beck muscular dystrophy (BMD) is a variant of DMD that was first reported by German physician Peter Emil Becker in the 1950s. They are all characterized by progressive muscle degeneration and weakness. Currently, there is still a need for drugs that can treat patients with DMD or BMD.

### Summary of the Invention

The present invention provides a compound of formula (I), formula (I-a), formula (I-b), formula (II), formula (III), formula (IV), formula (V), formula (VA), formula (VI), formula (VII), formula (VIII), or formula (IX), and a stereoisomer, a deuterated substance, a solvate, or a pharmaceutically acceptable salt or a eutectic crystal thereof, wherein the compound has the excellent effects of good activity, excellent physicochemical properties, ease of formulation, excellent pharmacokinetic properties, high bioavailability, and low toxic and side effects.

The compound of formula (I), formula (I-a), formula (I-b), formula (II), formula (II-a), formula (III), formula (III-a), formula (IV), formula (V), formula (VA), formula (VI), formula (VII), formula (VIII), or formula (IX), and a stereoisomer, a deuterated substance, a solvate, or a pharmaceutically acceptable salt or a eutectic crystal thereof, 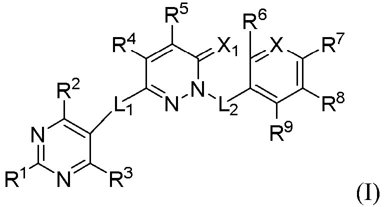 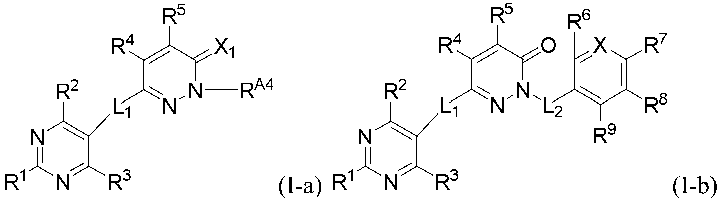 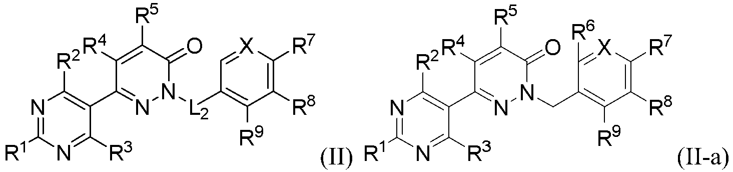 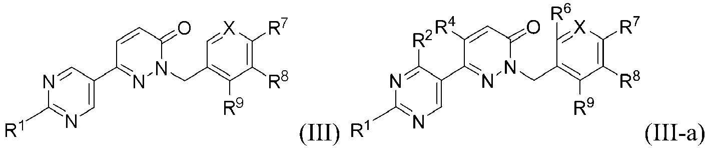 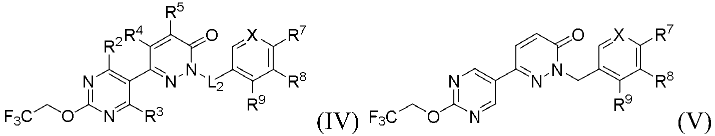 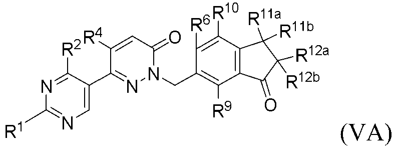 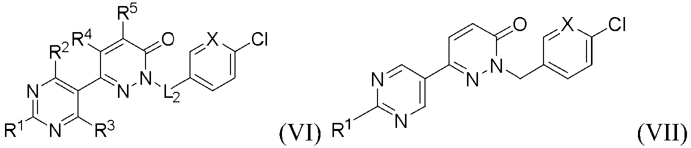 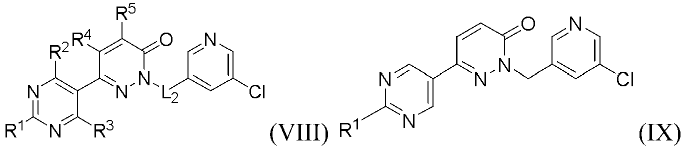 wherein
each of R¹, R² and R³ is independently selected from H, deuterium, R^{A1}, -O-haloC₁₋₄ alkyl, -NH-haloC₁₋₄ alkyl, -OC₁₋₄ alkyl, C₁₋₄ alkoxy, halogen, cyano, nitro, OH, C₁₋₄ alkyl, C₂₋₆ alkenyl, -NH-C₁₋₄ alkyl, -N(C₁₋₄ alkyl)₂, -NH-C₃₋₁₀ cycloalkyl, -NHC(O)C₁₋₄ alkyl, or 4- to 10-membered heterocycloalkyl containing 1 to 3 heteroatoms selected from N, O, and S, or selected from C₅₋₆ alkoxy, and the alkyl, alkoxy, cycloalkyl, or heterocycloalkyl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, C₃₋₁₀ cycloalkyl, 4- to 6-membered heterocycloalkyl containing 1 to 3 heteroatoms selected from N, O, or S, phenyl, and NH₂, provided that R¹, R² and R³ are not simultaneously selected from H;
in some embodiments, each of R¹, R² and R³ is independently selected from H, deuterium, R^{A1}, -O-haloC₁₋₄ alkyl, -NH-haloC₁₋₄ alkyl, -OC₁₋₄ alkyl, C₁₋₄ alkoxy, halogen, cyano, nitro, C₁₋₄ alkyl, C₂₋₆ alkenyl, -NH-C₁₋₄ alkyl, -N(C₁₋₄ alkyl)₂, -NH-C₃₋₁₀ cycloalkyl, -NHC(O)C₁₋₄ alkyl, or 4- to 10-membered heterocycloalkyl containing 1 to 3 heteroatoms selected from N, O, and S, or selected from C₅₋₆ alkoxy, and the alkyl, alkoxy, cycloalkyl, or heterocycloalkyl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, C₃₋₁₀ cycloalkyl, 4- to 6-membered heterocycloalkyl containing 1 to 3 heteroatoms selected from N, O, or S, phenyl, and NH₂, provided that R¹, R² and R³ are not simultaneously selected from H;
in some embodiments, each of R¹, R² and R³ is independently selected from H, deuterium, R^{A1}, -O-haloC₁₋₂ alkyl, -NH-haloC₁₋₂ alkyl, -OC₁₋₂ alkyl, C₁₋₂ alkoxy, halogen, cyano, nitro, OH, C₁₋₂ alkyl, C₂₋₄ alkenyl, -NH-C₁₋₂ alkyl, -N(C₁₋₂ alkyl)₂, -NH-C₃₋₆ monocyclic cycloalkyl, -NH-C₇₋₁₀ bicyclic cycloalkyl, -NHC(O)C₁₋₂ alkyl, 4- to 6-membered monocyclic heterocycloalkyl containing 1 to 3 heteroatoms selected from N, O, or S, or a 7- to 10-membered bicyclic heterocycloalkyl containing 1 to 3 heteroatoms selected from N, O, or S, or selected from C₅₋₆ alkoxy, the alkyl, alkoxy, cycloalkyl, heterocycloalkyl is optionally further substituted with 1, 2 or 3 groups selected from halogen, D, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy, C₃₋₅ monocyclic cycloalkyl, C₇₋₁₀ bicyclic cycloalkyl, 4-, 5-, or 6-membered heterocycloalkyl containing 1, 2, or 3 heteroatoms selected from N, O, or S, phenyl and NH₂;
in some embodiments, R¹ is selected from R^{A1}, or -O-haloC₁₋₄ alkyl; each of R² and R³ is independently selected from H, deuterium, C₂₋₄ alkenyl, C₂₋₄ alkynyl, halogen, cyano, nitro, OH, C₁₋₄ alkyl, haloC₁₋₄ alkyl, or deuterated C₁₋₄ alkyl;
in some embodiments, R¹ is selected from R^{A1}, or -O-haloC₁₋₄ alkyl; each of R² and R³ is independently selected from H, deuterium, C₂₋₄ alkenyl, C₂₋₄ alkynyl, halogen, cyano, nitro, OH, C₁₋₂ alkyl, haloC₁₋₂ alkyl, or deuterated C₁₋₂ alkyl;
in some embodiments, R¹ is selected from -O-haloC₁₋₄ alkyl; each of R² and R³ is independently selected from H, deuterium, C₂₋₄ alkenyl, C₂₋₄ alkynyl, halogen, cyano, nitro, OH, C₁₋₂ alkyl, haloC₁₋₂ alkyl, or deuterated C₁₋₂ alkyl;
In some embodiments, R¹ is selected from -O-haloC₁₋₄ alkyl; each of R² and R³ is independently selected from H;
In some embodiments, R¹ is selected from -O-haloC₁₋₃ alkyl; each of R² and R³ is independently selected from H;
In some embodiments, R³ is selected from H;
In some embodiments, R¹ is selected from R^{A1}; each of R² and R³ is independently selected from H, deuterium, C₂₋₄ alkenyl, C₂₋₄ alkynyl, halogen, cyano, nitro, OH, C₁₋₂ alkyl, haloC₁₋₂ alkyl, or deuterated C₁₋₂ alkyl;
In some embodiments, R¹ is selected from R^{A1}; each of R² and R³ is independently selected from H, deuterium, vinyl, or ethynyl;
In some embodiments, R¹ is selected from 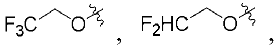 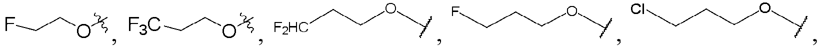 CH₂CH₃, -CH₂CH₂CH₃, 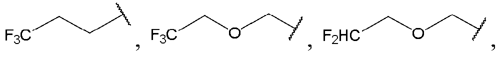 or R^{A1};
R^{A1} is selected from haloC₂₋₆ alkenyl, C₂₋₆ alkynyl, -O-(CH₂)ᵣ-R^{a}, C₃₋₁₀ cycloalkyl, 5- to 10-membered heteroaryl containing 1 to 3 heteroatoms selected from N, O, or S, -C₁₋₄ alkyl-R^{a}, -C₁₋₄ alkyl-OR^{a}, -C₁₋₄ alkyl-NR^{b}R^{a}, -NR^{b}-S(O)₂-R^{a}, -NR^{b}-S(O)₂-NR^{b}R^{a}, -O-NR^{b}R^{a}, -NH-OR^{b}, or -Se-(CH₂)ᵣ-R^{a}, and the alkynyl, heteroaryl, alkyl, or cycloalkyl is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
in some embodiments, R^{A1} is selected from haloC₂₋₆ alkenyl, C₂₋₆ alkynyl, -O-(CH₂)ᵣ-R^{a}, C₃₋₁₀ cycloalkyl, 5- to 10-membered heteroaryl containing 1 to 3 heteroatoms selected from N, O, or S, -C₁₋₄ alkyl-R^{a}, -C₁₋₄ alkyl-OR^{a}, -C₁₋₄ alkyl-NR^{b}R^{a}, -NR^{b}-S(O)₂-R^{a}, -NR^{b}-S(O)₂-NR^{b}R^{a}, -O-NR^{b}R^{a}, -NH-OR^{b}, or -Se-(CH₂)ᵣ-Ra, and the alkynyl, heteroaryl, alkyl, or cycloalkyl is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
in some embodiments, R^{A1} is selected from haloC₂₋₆ alkenyl, C₂₋₆ alkynyl, -O-(CH₂)ᵣ-R^{a}, C₃₋₁₀ cycloalkyl, 5- to 10-membered heteroaryl containing 1 to 3 heteroatoms selected from N, O, or S, -C₁₋₄ alkyl-R^{a}, -C₁₋₄ alkyl-OR^{a}, -C₁₋₄ alkyl-NR^{b}R^{a}, -NR^{b}-S(O)₂-R^{a}, -NR^{b}-S(O)₂-NR^{b}R^{a}, -O-NR^{b}R^{a}, or -NH-OR^{b}, and the alkynyl, heteroaryl, alkyl, or cycloalkyl is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
in some embodiments, R^{A1} is selected from haloC₂₋₆ alkenyl, C₂₋₆ alkynyl, 5-to 10-membered heteroaryl containing 1 to 3 heteroatoms selected from N, O, or S, -C₁₋₄ alkyl-R^{a}, -C₁₋₄ alkyl-OR^{a}, -C₁₋₄ alkyl-NR^{b}R^{a}, -NR^{b}-S(O)₂-R^{a}, or -NR^{b}-S(O)₂-NR^{b}R^{a}, and the alkynyl, heteroaryl, or alkyl is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
In some embodiments, R^{A1} is selected from haloC₂₋₄ alkenyl, C₂₋₄ alkynyl, -O-(CH₂)ᵣ-R^{a}, C₃₋₆ monocyclic cycloalkyl, 5- to 6-membered monocyclic heteroaryl containing 1 to 3 heteroatoms selected from N, O, or S, 8- to 10-membered bicyclic heteroaryl containing 1 to 3 heteroatoms selected from N, O, or S, -C₁₋₂ alkyl-R^{a}, -C₁₋₂ alkyl-OR^{a}, -C₁₋₂ alkyl-NR^{b}R^{a}, -NR^{b}-S(O)₂-R^{a}, -NR^{b}-S(O)₂-NR^{b}R^{a}, - O-NR^{b}R^{a}, -NH-OR^{b}, the alkynyl, heteroaryl, or alkyl is optionally further substituted with 1 to 3 groups selected from halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂; r is selected from 0 or 1;
in some embodiments, R^{A1} is selected from haloC₂₋₄ alkenyl, C₂₋₄ alkynyl, 5-to 6-membered monocyclic heteroaryl containing 1 to 3 heteroatoms selected from N, O, or S, 8- to 10-membered bicyclic heteroaryl containing 1 to 3 heteroatoms selected from N, O, or S, -C₁₋₂ alkyl-R^{a}, -C₁₋₂ alkyl-OR^{a}, -C₁₋₂ alkyl-NR^{b}R^{a}, -NR^{b}-S(O)₂-R^{a}, -NR^{b}-S(O)₂-NR^{b}R^{a}, -O-NR^{b}R^{a}, -NH-OR^{b}, the alkynyl, heteroaryl, or alkyl is optionally further substituted with 1, 2 or 3 groups selected from halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
in some embodiments, R^{A1} is selected from haloC₂₋₄ alkenyl, C₂₋₄ alkynyl, 5-to 6-membered monocyclic heteroaryl containing 1 to 3 heteroatoms selected from N, O, or S, 8- to 10-membered bicyclic heteroaryl containing 1 to 3 heteroatoms selected from N, O, or S, -C1-2 alkyl-R^{a}, -C₁₋₂ alkyl-OR^{a}, -C₁₋₂ alkyl-NR^{b}R^{a}, -NR^{b}-S(O)₂-R^{a}, or -NR^{b}-S(O)₂-NR^{b}R^{a}, and the alkynyl, heteroaryl, or alkyl is optionally further substituted with 1, 2 or 3 groups selected from halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
in some embodiments, RA1 is selected from haloC₂₋₄ alkenyl, C₂₋₄ alkynyl, 5-to 6-membered monocyclic heteroaryl containing 1 to 3 heteroatoms selected from N, O, or S, -C₁₋₂ alkyl-R^{a}, -C₁₋₂ alkyl-OR^{a}, -C₁₋₂ alkyl-NR^{b}R^{a}, -NR^{b}-S(O)₂-R^{a}, or - NR^{b}-S(O)₂-NR^{b}R^{a}, and the alkynyl, heteroaryl, or alkyl is optionally further substituted with 1, 2 or 3 groups selected from halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
in some embodiments, R^{A1} is selected from haloC₂₋₄ alkenyl, C₂₋₄ alkynyl, a 5- or 6-membered monocyclic heteroaryl containing 1, 2, or 3 heteroatoms selected from N, O, or S, -CH₂-R^{a}, -CH₂-OR^{a}, -CH₂-NR^{b}R^{a}, -NR^{b}-S(O)₂-R^{a}, or -NR^{b}-S(O)₂-NR^{b}R^{a}, and the alkynyl, heteroaryl, or CH₂ is optionally further substituted with 1, 2, or 3 groups selected from halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy, and NH₂;
In some embodiments, R^{A1} is selected from C₃₋₆ monocyclic cycloalkyl, 4- to 6- membered monocyclic heteroaryl containing 1 to 3 heteroatoms selected from N, O, or S, 8- to 10- membered benzoC₃₋₆ cycloalkyl containing 1 to 3 heteroatoms selected from N, O, or S, -O-R^{a}, or -O-CH₂-R^{a}, the cycloalkyl, heteroaryl, or benzoC₃₋₆ cycloalkyl is optionally further substituted with 1, 2, or 3 groups selected from halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
In some embodiments, R^{A1} is selected from halovinyl, halopropenyl, ethynyl, propynyl, pyrrolyl, furyl, thienyl, imidazolyl, oxazolyl, thiazolyl, pyrazolyl, isoxazolyl, pyridyl, pyridazinyl, pyrimidinyl, pyrazinyl, -CH₂-R^{a}, -CH₂-OR^{a}, -CH₂-NR^{b}R^{a}, -NR^{b}-S(O)₂-R^{a}, -NR^{b}-S(O)₂-NR^{b}R^{a}, and the ethynyl, propynyl, pyrrolyl, furyl, thienyl, imidazolyl, oxazolyl, thiazolyl, pyrazolyl, isoxazolyl, pyridyl, pyridazinyl, pyrimidinyl, pyrazinyl, or CH₂ is optionally further substituted with 1, 2, or 3 groups selected from F, Cl, deuterium, CN, OH, methyl, ethyl, -CH₂F, - CHF₂, -CF₃, -CH₂CH₂F, -CH₂CHF₂, -CH₂CF₃, -CHFCH₂F, -CHFCHF₂, -CHFCF₃, -CF₂CH₂F, -CF₂CHF₂, -CF₂CF₃, -CH₂D, -CHD₂, -CD₃, -CH₂CH₂D, -CH₂CHD₂, - CH₂CD₃, -CHDCH₂D, -CHDCHD₂, -CHDCD₃, -CD₂CH₂D, -CD₂CHD₂, -CD₂CD₃, methoxy, ethoxy, -OCHF₂, -OCH₂F, -OCF₃, -OCH₂CH₂F, -OCH₂CHF₂, - OCH₂CF₃, -OCHFCH₂F, -OCHFCHF₂, -OCHFCF₃, -OCF₂CH₂F, -OCF₂CHF₂, - OCF₂CF₃, -OCHD₂, -OCH₂D, -OCD₃, -OCH₂CH₂D, -OCH₂CHD₂, -OCH₂CD₃, - OCHDCH₂D, -OCHDCHD₂, -OCHDCD₃, -OCD₂CH₂D, -OCD₂CHD₂, -OCD₂CD₃ and NH₂;
In some embodiments, R^{A1} is selected from 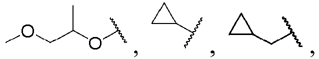 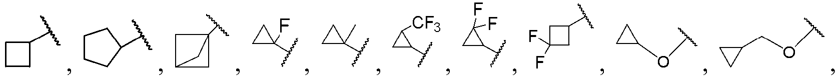 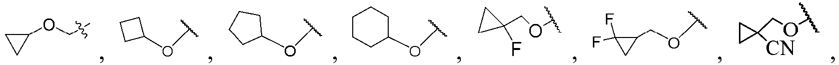 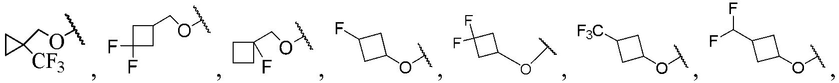 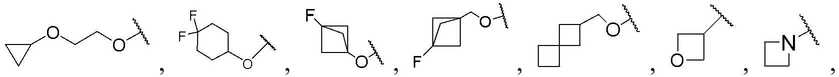 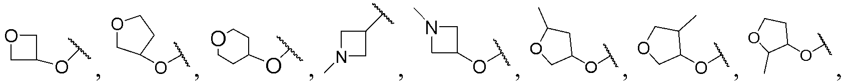 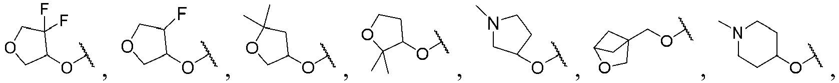 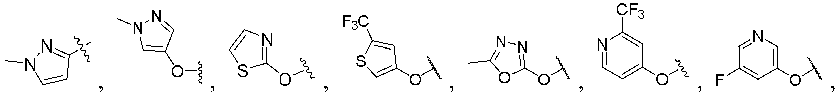 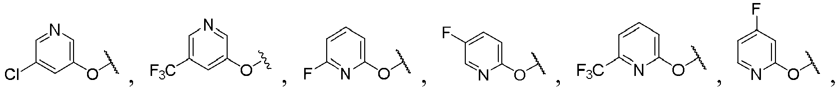 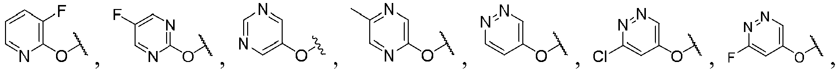 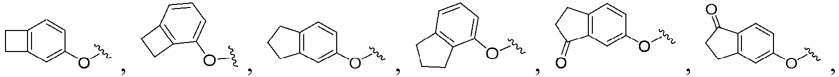 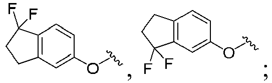
In some embodiments, R^{A1} is selected from 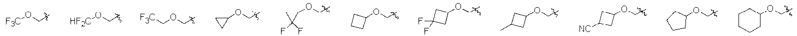 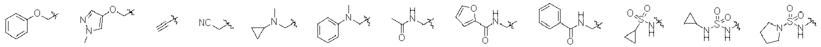 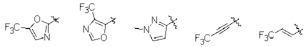 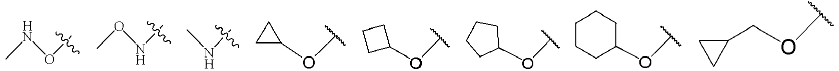 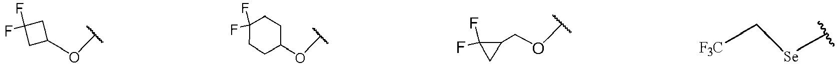 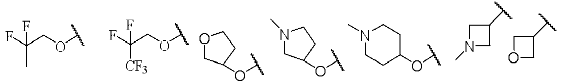 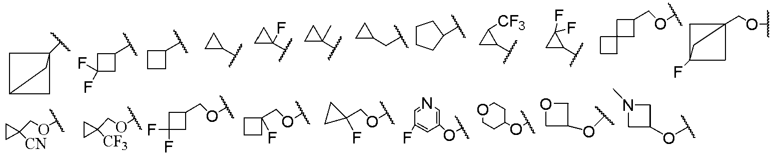 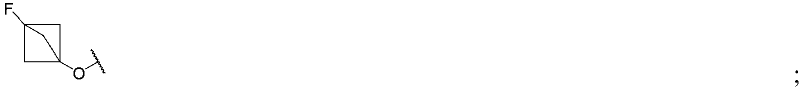
In some embodiments, R^{A1} is selected from 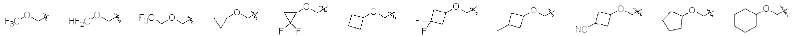 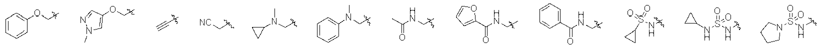 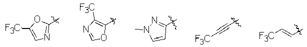 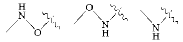 or is selected from 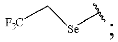
In some embodiments, R^{A1} is selected from 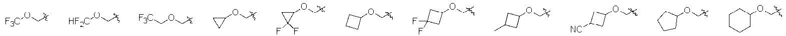 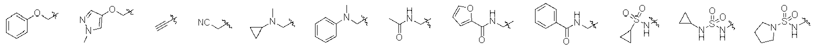 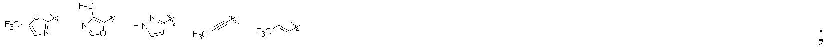
In some embodiments, R^{A1} is selected from 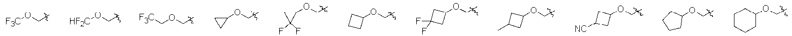 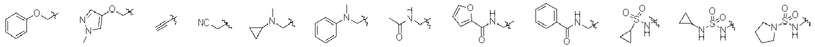 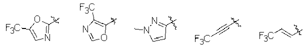 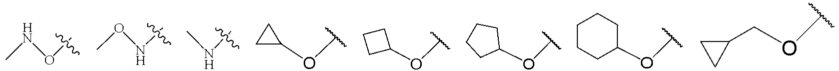 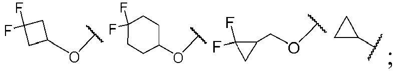
each R^{a} is selected from CN, haloC₁₋₆ alkyl, C₃₋₁₀ cycloalkyl, 4- to 10-membered heterocycloalkyl containing 1 to 3 heteroatoms selected from N, O, or S, 6- to 10-membered aryl, 5- to 10-membered heteroaryl containing 1 to 3 heteroatoms selected from N, O, or S, or -C(O)-R^{a1}, and the alkyl, cycloalkyl, heterocycloalkyl, aryl, or heteroaryl is optionally further substituted with 1 to 3 groups selected from halogen, =O, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
In certain embodiments, each R^{a} is selected from CN, haloC₁₋₄ alkyl, C₃₋₆ monocyclic cycloalkyl, C₇₋₁₀ spirocyclic cycloalkyl, C₄₋₈ bridged cycloalkyl, C₄₋₉ fused cycloalkyl, 4- to 6-membered monocyclic heterocycloalkyl containing 1 to 3 heteroatoms selected from N, O, or S, 7- to 10-membered spirocyclic heterocycloalkyl containing 1 to 3 heteroatoms selected from N, O, or S, 4- to 8-membered bridged heterocycloalkyl containing 1 to 3 heteroatoms selected from N, O, or S, 4- to 6-membered fused heterocycloalkyl containing 1 to 3 heteroatoms selected from N, O, or S, phenyl, 8- to 10- membered aryl, 5- to 6-membered monocyclic heteroaryl containing 1 to 3 heteroatoms selected from N, O, or S, 8-to 10-membered bicyclic heteroaryl containing 1 to 3 heteroatoms selected from N, O, or S, -C(O)-R^{a1}, and the alkyl, cycloalkyl, heterocycloalkyl, aryl, or heteroaryl is optionally further substituted with 1 to 3 groups selected from halogen, =O, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
In certain embodiments, each R^{a} is selected from CN, haloC₁₋₄ alkyl, C₃₋₆ monocyclic cycloalkyl, 4- to 6-membered monocyclic heterocycloalkyl containing 1 to 3 heteroatoms selected from N, O, or S, 7- to 10-membered spirocyclic heterocycloalkyl containing 1 to 3 heteroatoms selected from N, O, or S, 4- to 8-membered bridged heterocycloalkyl containing 1 to 3 heteroatoms selected from N, O, or S, 4- to 6-membered fused heterocycloalkyl containing 1 to 3 heteroatoms selected from N, O, or S, phenyl, 8- to 10- membered aryl, 5- to 6-membered monocyclic heteroaryl containing 1 to 3 heteroatoms selected from N, O, or S, 8-to 10-membered bicyclic heteroaryl containing 1 to 3 heteroatoms selected from N, O, or S, -C(O)-R^{a1}, and the alkyl, cycloalkyl, heterocycloalkyl, aryl, or heteroaryl is optionally further substituted with 1 to 3 groups selected from halogen, =O, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
In certain embodiments, each R^{a} is selected from CN, -CH₂F, -CHF₂, -CF₃, - CH₂CH₂F, -CH₂CHF₂, -CH₂CF₃, -CHFCH₂F, -CHFCHF₂, -CHFCF₃, -CF₂CH₂F, - CF₂CHF₂, -CF₂CF₃, cyclopropyl, cyclobutyl, cyclopentyl, cyclohexyl, azetidinyl, azetanyl, oxetanyl, oxacycloalkyl, piperazinyl, piperidinyl, tetrahydropyranyl, morpholinyl, pyranyl, phenyl, pyrrolyl, furyl, thienyl, imidazolyl, oxazolyl, thiazolyl, pyrazolyl, isoxazolyl, pyridyl, pyridazinyl, pyrimidinyl, pyrazinyl, 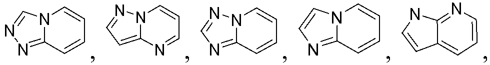 -C(O)-R^{a1}, the above-mentioned groups are optionally further substituted with 1, 2, or 3 groups selected from F, Cl, =O, deuterium, CN, OH, methyl, ethyl, -CH₂F, -CHF₂, -CF₃, -CH₂CH₂F, - CH₂CHF₂, -CH₂CF₃, -CHFCH₂F, -CHFCHF₂, -CHFCF₃, -CF₂CH₂F, -CF₂CHF₂, - CF₂CF₃, -CH₂D, -CHD₂, -CD₃, -CH₂CH₂D, -CH₂CHD₂, -CH₂CD₃, -CHDCH₂D, - CHDCHD₂, -CHDCD₃, -CD₂CH₂D, -CD₂CHD₂, -CD₂CD₃, methoxy, ethoxy, - OCHF₂, -OCH₂F, -OCF₃, -OCH₂CH₂F, -OCH₂CHF₂, -OCH₂CF₃, -OCHFCH₂F, - OCHFCHF₂, -OCHFCF₃, -OCF₂CH₂F, -OCF₂CHF₂, -OCF₂CF₃, -OCHD₂, - OCH₂D, -OCD₃, -OCH₂CH₂D, -OCH₂CHD₂, -OCH₂CD₃, -OCHDCH₂D, - OCHDCHD₂, -OCHDCD₃, -OCD₂CH₂D, -OCD₂CHD₂, -OCD₂CD₃ and NH₂;
each R^{b} is selected from H, deuterium, C₁₋₄ alkyl, C₃₋₆ cycloalkyl, haloC₁₋₄ alkyl, or deuterated C₁₋₄ alkyl; In certain embodiments, each R^{b} is selected from H, deuterium, C₁₋₄ alkyl, or C₃₋₆ cycloalkyl; in certain embodiments, each R^{b} is selected from H, deuterium, C₁₋₂ alkyl, or C₃₋₄ cycloalkyl; In certain embodiments, each R^{b} is selected from H, deuterium, methyl;
R^{a1} is selected from OH, NH₂, -NHC₁₋₄ alkyl, -N(C₁₋₄ alkyl)₂, -NHC₃₋₁₀ cycloalkyl, C₁₋₄ alkoxy, C₁₋₆ alkyl, C₃₋₁₀ cycloalkyl, 6- to 10-membered aryl, 5- to 10-membered heteroaryl containing 1 to 3 heteroatoms selected from N, O, or S; In certain embodiments, R^{a1} is selected from OH, NH₂, -NHC₁₋₂ alkyl, -N(C₁₋₂ alkyl)₂, -NHC₃₋₇ cycloalkyl, C₁₋₂ alkoxy, C₁₋₂ alkyl, C₃₋₇ cycloalkyl, phenyl, 8- to 10-membered bicyclic aryl, 5- to 6-membered heteroaryl containing 1 to 3 heteroatoms selected from N, O, or S, or 8- to 10-membered bicyclic heteroaryl containing 1 to 3 heteroatoms selected from N, O, or S; In certain embodiments, R^{a1} is selected from OH, NH₂, -NHC₁₋₂ alkyl, -N(C₁₋₂ alkyl)₂, -NHC₃₋₄ cycloalkyl, C₁₋₂ alkoxy, C₁₋₂ alkyl, C₃₋₄ cycloalkyl, phenyl, piperazinyl, piperidinyl, tetrahydropyranyl, morpholinyl, pyranyl, phenyl, pyrrolyl, furyl, thienyl, imidazolyl, oxazolyl, thiazolyl, pyrazolyl, isoxazolyl, pyridyl, pyridazinyl, pyrimidinyl, or pyrazinyl; In certain embodiments, R^{a1} is selected from C₁₋₂ alkyl, C₃₋₇ cycloalkyl, phenyl, piperazinyl, piperidinyl, tetrahydropyranyl, morpholinyl, pyranyl, phenyl, pyrrolyl, furyl, thienyl, imidazolyl, oxazolyl, thiazolyl, pyrazolyl, isoxazolyl, pyridyl, pyridazinyl, pyrimidinyl, or pyrazinyl;
X is selected from CR¹⁰ or N; In certain embodiments, X is selected from CH or N; In certain embodiments, X is selected from CH; In certain embodiments, X is selected from N;
X₁ is selected from O, S; in certain embodiments, X₁ is selected from O; in certain embodiments, X₁ is selected from S;
each of R⁴ and R⁵ is independently selected from H, deuterium, C₃₋₁₀ cycloalkyl, C₂₋₆ alkenyl, C₂₋₆ alkynyl, halogen, amino,-NH-C₁₋₄ alkyl, -N(C₁₋₄ alkyl)₂, C₁₋₄ alkyl, haloC₁₋₄ alkyl, 4- to 10-membered heterocycloalkyl containing 1 to 3 heteroatoms selected from N, O, or S, 5- to 10-membered heteroaryl containing 1 to 3 heteroatoms selected from N, O, or S, or 6- to 10-membered aryl; In certain embodiments, each of R⁴ and R⁵ is independently selected from H, deuterium, C₃₋₄ monocyclic cycloalkyl, C₇₋₈ bicyclic cycloalkyl, C₂₋₄ alkenyl, C₂₋₄ alkynyl, F, Cl, amino,-NH-C₁₋₂ alkyl, -N(C₁₋₂ alkyl)₂, C₁₋₂ alkyl, haloC₁₋₂ alkyl, 4-to 6-membered monocyclic heterocycloalkyl containing 1, 2, or 3 heteroatoms selected from N, O, or S, 7- to 10-membered bicyclic heterocycloalkyl containing 1, 2, or 3 heteroatoms selected from N, O, or S, 5- to 6-membered monocyclic heteroaryl containing 1 to 3 heteroatoms selected from N, O, or S, 8- to 10-membered bicyclic heteroaryl containing 1 to 3 heteroatoms selected from N, O, or S, phenyl, or 8- to 10- membered bicyclic aryl; In certain embodiments, each of R⁴ and R⁵ is independently selected from H, deuterium, C₃₋₄ monocyclic cycloalkyl, C₇₋₈ bicyclic cycloalkyl, C₂₋₄ alkenyl, C₂₋₄ alkynyl, F, Cl, amino,-NH-C₁₋₂ alkyl, - N(C₁₋₂ alkyl)₂, C₁₋₂ alkyl, haloC₁₋₂ alkyl, 4- to 6-membered monocyclic heterocycloalkyl containing 1, 2, or 3 heteroatoms selected from N, O, or S; In certain embodiments, each of R⁴ and R⁵ is independently selected from H, deuterium, C₃₋₄ monocyclic cycloalkyl, C₇₋₈ bicyclic cycloalkyl, C₂₋₄ alkenyl, C₂₋₄ alkynyl, or 4- to 5-membered monocyclic heterocycloalkyl containing 1, 2, or 3 heteroatoms selected from N, O, or S; In certain embodiments, each of R⁴ and R⁵ is independently selected from H, deuterium, C₃₋₄ monocyclic cycloalkyl, C₂₋₄ alkenyl, or C₂₋₄ alkynyl; In certain embodiments, each of R⁴ and R⁵ is independently selected from H, deuterium, cyclopropyl, cyclobutyl, vinyl, allyl; In certain embodiments, each of R⁴ and R⁵ is independently selected from H; In certain embodiments, R⁵ is selected from H;
each of R⁶, R⁷, R⁸, R⁹, and R¹⁰ is independently selected from H, deuterium, R^{A2}, halogen, OH, CN, amino, C₁₋₄ alkyl, C₂₋₆ alkenyl, C₂₋₆ alkynyl, C₁₋₄ alkoxy, haloC₁₋₄ alkyl, -NH-C₁₋₄ alkyl, -N(C₁₋₄ alkyl)₂, -S(O)R^{c}, -S(O)²R^{c}, -C(O)R^{c}, - C(O)OR^{c}, -C(O)N(R^{c})₂, or -OC(O)R^{c}, and the alkyl, alkenyl, alkynyl, or alkoxy is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
In certain embodiments, each of R⁶, R⁷, R⁸, R⁹, and R¹⁰ is independently selected from H, deuterium, R^{A2}, F, Cl, OH, CN, amino, C₁₋₂ alkyl, C₂₋₄ alkenyl, C₂₋₄ alkynyl, C₁₋₂ alkoxy, haloC₁₋₂ alkyl, -NH-C₁₋₂ alkyl, -N(C₁₋₂ alkyl)₂, -S(O)R^{c}, - S(O)²R^{c}, -C(O)R^{c}, -C(O)OR^{c}, -C(O)N(R^{c})₂, -OC(O)R^{c}, and the alkyl, alkenyl, alkynyl, alkoxy is optionally further substituted with 1, 2, or 3 groups selected from F, Cl, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
In certain embodiments, each of R⁷ and R⁸ is independently selected from H, deuterium, R^{A2}, F, Cl, OH, CN, amino, C₁₋₂ alkyl, C₂₋₄ alkenyl, C₂₋₄ alkynyl, C₁₋₂ alkoxy, haloC₁₋₂ alkyl, -NH-C₁₋₂ alkyl, -N(C₁₋₂ alkyl)₂, and the alkyl, alkenyl, alkynyl, or alkoxy is optionally further substituted with 1, 2, or 3 groups selected from F, Cl, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂; each of R⁶, R⁹ and R¹⁰ is independently selected from H, deuterium, F, Cl, OH, CN, amino, C₁₋₂ alkyl, C₂₋₄ alkenyl, C₂₋₄ alkynyl, C₁₋₂ alkoxy, haloC₁₋₂ alkyl, -NH-C₁₋₂ alkyl, -N(C₁₋₂ alkyl)₂, and the alkyl, alkenyl, alkynyl, or alkoxy is optionally further substituted with 1, 2, or 3 groups selected from F, Cl, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
In certain embodiments, each of R⁷ and R⁸ is independently selected from H, deuterium, R^{A2}, F, Cl, OH, CN, amino, C₁₋₂ alkyl, C₁₋₂ alkoxy, haloC₁₋₂ alkyl, -NH-C₁₋₂ alkyl, -N(C₁₋₂ alkyl)₂, the alkyl, alkenyl, alkynyl, alkoxy is optionally further substituted with 1, 2, or 3 groups selected from F, Cl, deuterium, CN, OH and NH₂; each of R⁶, R⁹ and R¹⁰ is independently selected from H, deuterium, F, Cl, OH, CN, amino, C₁₋₂ alkyl, the alkyl is optionally further substituted with 1, 2, or 3 groups selected from F, Cl, deuterium, or OH;
In certain embodiments, each R⁷ is independently selected from R^{A2}; each of R⁶, R⁸, R⁹, and R¹⁰ is independently selected from H, deuterium, F, Cl, OH, CN, amino, C₁₋₂ alkyl, the alkyl is optionally further substituted with 1, 2, or 3 groups selected from F, Cl, deuterium, or OH;
In certain embodiments, each R⁸ is independently selected from R^{A2}; each of R⁶, R⁷, R⁹, and R¹⁰ is independently selected from H, deuterium, F, Cl, OH, CN, amino, C₁₋₂ alkyl, the alkyl is optionally further substituted with 1, 2, or 3 groups selected from F, Cl, deuterium, or OH;
In certain embodiments, each R⁷ is independently selected from R^{A2}; each of R⁶, R⁸, R⁹, and R¹⁰ is independently selected from H, deuterium, F, Cl, OH, CN, amino, or C₁₋₂ alkyl;
In certain embodiments, each R⁸ is independently selected from R^{A2}; each of R⁶, R⁷, R⁹, and R¹⁰ is independently selected from H, deuterium, F, Cl, OH, CN, amino, or C₁₋₂ alkyl;
In certain embodiments, each of R⁷ and R⁸ is independently selected from H, deuterium, F, Cl, OH, CN, methyl, or R^{A2}; each of R⁶, R⁹, and R¹⁰ is independently selected from H, deuterium, F, Cl, OH, CN, or methyl;
In certain embodiments, each of R⁶, R⁹, and R¹⁰ is independently selected from H, deuterium, halogen, C₁₋₆ alkyl, or haloC₁₋₆ alkyl; In certain embodiments, each of R⁶, R⁹, and R¹⁰ is independently selected from H, deuterium, halogen, C₁₋₃ alkyl, or haloC₁₋₃ alkyl; In certain embodiments, each of R⁶, R⁹, and R¹⁰ is independently selected from H, deuterium, F, Cl, methyl, trifluoromethyl, or difluoromethyl;
R^{A2} is selected from -(CH₂)ᵣ-(C₃₋₁₀ cycloalkyl), -(CH₂)ᵣ-(4- to 10-membered heterocycloalkyl containing 1 to 3 heteroatoms selected from N, O, S, or Si), or - (CH₂)ᵣ-(5- to 10-membered heteroaryl containing 1 to 3 heteroatoms selected from N, O, or S), -P(O)(R^{c})₂, -Si(R^{c})₃, -SF₅, N₃, B(OH)₂, or -S(O)(=NH)R^{c}, and the cycloalkyl, heterocycloalkyl, or heteroaryl is optionally further substituted with 1 to 3 groups selected from halogen, =O, D, CN, OH, C₁₋₄ alkyl, haloC₁₋₄ alkyl, deuterated C₁₋₄ alkyl, C₁₋₄ alkoxy, haloC₁₋₄ alkoxy, C₃₋₆ cycloalkyl, 4- to 7-membered heterocycloalkyl containing 1 to 3 heteroatoms selected from N, O, or S, deuterated C₁₋₄ alkoxy and NH₂;
in certain embodiments, R^{A2} is selected from -(CH₂)ᵣ-(4- to 10-membered heterocycloalkyl containing 1 to 3 heteroatoms selected from N, O, S, or Si), or - (CH₂)ᵣ-(5- to 10-membered heteroaryl containing 1 to 3 heteroatoms selected from N, O, or S), -P(O)(R^{c})₂, -Si(R^{c})₃, -SF₅, N₃, B(OH)₂, or -S(O)(=NH)R^{c}, and the heterocycloalkyl or heteroaryl is optionally further substituted with 1 to 3 groups selected from halogen, =O, D, CN, OH, C₁₋₄ alkyl, haloC₁₋₄ alkyl, deuterated C₁₋₄ alkyl, C₁₋₄ alkoxy, haloC₁₋₄ alkoxy, C₃₋₆ cycloalkyl, 4- to 7-membered heterocycloalkyl containing 1 to 3 heteroatoms selected from N, O, or S, deuterated C₁₋₄ alkoxy and NH₂;
In certain embodiments, R^{A2} is selected from -(CH₂)ᵣ-(4- to 10-membered heterocycloalkyl containing 1 to 3 heteroatoms selected from N, O, or S), or - (CH₂)ᵣ-(5- to 10-membered heteroaryl containing 1 to 3 heteroatoms selected from N, O, or S), -P(O)(R^{c})₂, -Si(R^{c})₃, -SF₅, -S(O)(=NH)R^{c}, and the heterocycloalkyl, or heterocycloaryl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂; In certain embodiments, R^{A2} is selected from -(CH₂)ᵣ-(4- to 6-membered monocyclic heterocycloalkyl containing 1 to 3 heteroatoms selected from N, O, or S), -(CH₂)ᵣ-(7- to 10-membered spirocyclic heterocycloalkyl containing 1 to 3 heteroatoms selected from N, O, or S), -(CH₂)ᵣ-(5- to 6-membered monocyclic heteroaryl containing 1 to 3 heteroatoms selected from N, O, or S), -(CH₂)ᵣ-(8- to 10-membered bicyclic heteroaryl containing 1 to 3 heteroatoms selected from N, O, or S), -P(O)(R^{c})₂, - Si(R^{c})₃, -SF₅, or -S(O)(=NH)R^{c}, and the heterocycloalkyl or heterocycloaryl is optionally further substituted with 1 to 3 groups selected from halogen, D, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
in certain embodiments, R^{A2} is selected from -(CH₂)ᵣ-(4- to 6-membered monocyclic heterocycloalkyl containing 1 to 3 heteroatoms selected from N, O, or S), -(CH₂)ᵣ-(5- to 6-membered monocyclic heteroaryl containing 1 to 3 heteroatoms selected from N, O, or S), -P(O)(R^{c})₂, -Si(R^{c})₃, -SF₅, -S(O)(=NH)R^{c}, and the heterocycloalkyl, or heterocycloaryl is optionally further substituted with 1 to 3 groups selected from halogen, D, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂; in certain embodiments, R^{A2} is selected from -(CH₂)ᵣ-(4- to 6-membered monocyclic heterocycloalkyl containing 1 to 3 heteroatoms selected from N, O, or S), or -(CH₂)ᵣ-(5- to 6-membered monocyclic heteroaryl containing 1 to 3 heteroatoms selected from N, O, or S), and the heterocycloalkyl, or heteroaryl is optionally further substituted with 1 to 3 groups selected from halogen, D, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
In certain embodiments, R^{A2} is selected from 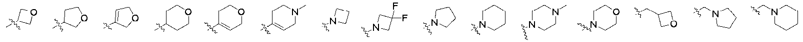 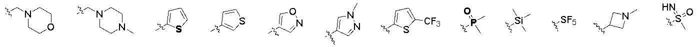 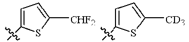 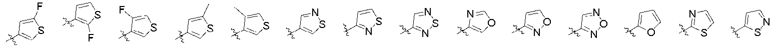 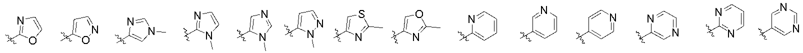 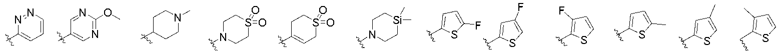 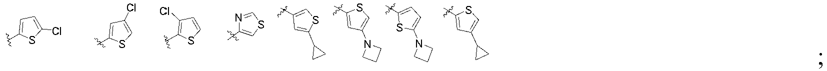
In certain embodiments, R^{A2} is selected from 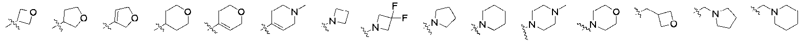 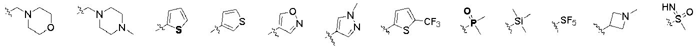 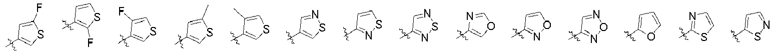 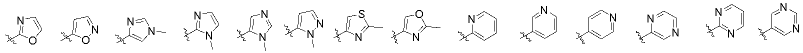 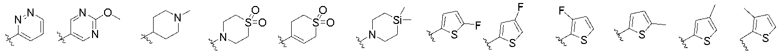 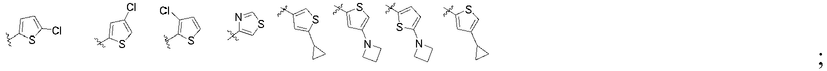
In certain embodiments, R^{A2} is selected from 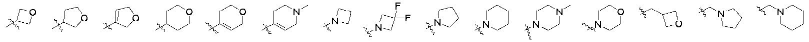 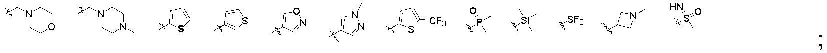
each R^{c} is independently selected from H, OH, C₁₋₄ alkyl, C₃₋₇ cycloalkyl, haloC₁₋₄ alkyl, deuterated C₁₋₄ alkyl, C₁₋₄ alkoxy, haloC₁₋₄ alkoxy, deuterated C₁₋₄ alkoxy, or NH₂; in certain embodiments, each R^{c} is independently selected from C₁₋₂ alkyl, C₃₋₄ cycloalkyl, haloC₁₋₂ alkyl, deuterated C₁₋₂ alkyl, C₁₋₂ alkoxy, haloC₁₋₂ alkoxy, deuterated C₁₋₂ alkoxy or NH₂; in certain embodiments, each R^{c} is independently selected from C₁₋₂ alkyl, or C₃₋₄ cycloalkyl; in certain embodiments, each R^{c} is independently selected from C₁₋₂ alkyl;
each r is independently selected from 0, 1, 2 or 3; in certain embodiments, each r is independently selected from 0 or 1;
L₁ is selected from a bond, O, NH, S, -CD₂-, -CHD-, -CR^{L1}R^{L2}-, C₁₋₄ alkyl, - C(O)-, S(O), or S(O)₂, and the alkyl is optionally further substituted with R^{L1}; in certain embodiments, L₁ is selected from a bond or O; in certain embodiments, L₁ is selected from a bond;
L₂ is selected from O, NH, S, -CH₂-, -CD₂-, -CHD-, -CR^{L1}R^{L2}-, -CHR^{L2}-, - CDR^{L2}-, C₂₋₄ alkyl, -C(O)-, S(O), S(O)₂, the alkyl is optionally further substituted with 1 to 3 R^{L1}; in certain embodiments, L₂ is selected from -CH₂-, -CD₂-, -CHD-, -CR^{L1}R^{L2}-, -CHR^{L2}-, -CDR^{L2}-, C₂₋₄ alkyl, -C(O)-, S(O), S(O)₂, the alkyl is optionally further substituted with 1 to 3 R^{L1}; in certain embodiments, L₂ is selected from -CH₂-, -CR^{L1}R^{L2}-, -CHR^{L2}-, -CDR^{L2}-, or -C(O)-; in certain embodiments, L₂ is selected from -CH₂-; in certain embodiments, L₂ is selected from -CR^{L1}R^{L2}-, -CHR^{L2}-, -CDR^{L2}-, or -C(O)-;
R^{A4} is selected from -CH₂-C₁₋₄ alkyl-C₃₋₆ cycloalkyl, -CH₂-CR^{A4c}=CR^{A4a}R^{A4b}, -CH₂-CH=CH-CH=CR^{A4a}R^{A4b}, -CH(C₁₋₂ alkyl)-CR^{A4c}=CR^{A4a}R^{A4b}, -C(C₁₋₂ alkyl)₂-CR^{A4c}=CR^{A4a}R^{A4b}, L₃-R^{A4'}; in certain embodiments, R^{A4} is selected from -CH₂-C₁₋₄ alkyl-C₃₋₆ cycloalkyl, -CH₂-CH=CR^{A4a}R^{A4b}, -CH₂-CF=CR^{A4a}R^{A4b}, -CH₂-C(CN)=CR^{A4a}R^{A4b}, -CH₂-C(CH₃)=CR^{A4a}R^{A4b}, -CH₂-CH=CH-CH=CR^{A4a}R^{A4b}, - CHCH₃-CR^{A4c}=CR^{A4a}R^{A4b}, -CH(CH₂CH₃)-CR^{A4c}=CR^{A4a}R^{A4b}, -C(CH₃)₂-CR^{A4c}=CR^{A4a}R^{A4b}, -C(CH₂CH₃)₂-CR^{A4c}=CR^{A4a}R^{A4b}, L₃-R^{A4'}; in certain embodiments, R^{A4} is selected from -CH₂-C₁₋₄ alkyl-C₃₋₆ cycloalkyl, -CH₂-CR^{A4c}=CR^{A4a}R^{A4b}, -CH₂-CH=CH-CH=CR^{A4a}R^{A4b}, L₃-R^{A4'}, or is selected from - CHCH₃-CR^{A4c}=CR^{A4a}R^{A4b}; in certain embodiments, R^{A4} is selected from -CH₂-C₁₋₄ alkyl-C₃₋₆ cycloalkyl, -CH₂-CH=CR^{A4a}R^{A4b}, -CH₂-CF=CR^{A4a}R^{A4b}, -CH₂-C(CH₃)=CR^{A4a}R^{A4b}, -CH₂-CH=CH-CH=CR^{A4a}R^{A4b}, L₃-R^{A4'} or is selected from - CHCH₃-CH=CR^{A4a}R^{A4b}; in certain embodiments, R^{A4} is selected from -CH₂-C₁₋₄ alkyl-C₃₋₆ cycloalkyl, -CH₂-CF=CR^{A4a}R^{A4b}, -CH₂-CH=CH-CH=CR^{A4a}R^{A4b}, L₃-R^{A4'} or is selected from -CHCH₃-CH=CR^{A4a}R^{A4b}; in certain embodiments, R^{A4} is selected from -CH₂-C₁₋₄ alkyl-C₃₋₆ cycloalkyl, -CH₂-C(CH₃)=CR^{A4a}R^{A4b}, -CH₂-CH=CH-CH=CR^{A4a}R^{A4b}, L₃-R^{A4'} or is selected from -CHCH₃-CH=CR^{A4a}R^{A4b}; in certain embodiments, R^{A4} is selected from -CH₂-C₁₋₄ alkyl-C₃₋₆ cycloalkyl, -CH₂-CH=CR^{A4a}R^{A4b}, -CH₂-CH=CH-CH=CR^{A4a}R^{A4b}, L₃-R^{A4'} or is selected from - CHCH₃-CH=CR^{A4a}R^{A4b};
in certain embodiments, R^{A4} is selected from -CH₂-C₁₋₄ alkyl-C₃₋₆ cycloalkyl, -CH₂-CF=CR^{A4a}R^{A4b}, L₃-R^{A4'} or is selected from -CHCH₃-CH=CR^{A4a}R^{A4b}; in certain embodiments, R^{A4} is selected from -CH₂-C₁₋₄ alkyl-C₃₋₆ cycloalkyl, -CH₂-C(CH₃)=CR^{A4a}R^{A4b}, L₃-R^{A4'} or is selected from -CHCH₃-CH=CR^{A4a}R^{A4b}; in certain embodiments, R^{A4} is selected from -CH₂-C₁₋₄ alkyl-C₃₋₆ cycloalkyl, -CH₂-CH=CR^{A4a}R^{A4b}, L₃-R^{A4'} or is selected from -CHCH₃-CH=CR^{A4a}R^{A4b}; in certain embodiments, R^{A4} is selected from -CH₂-C₁₋₄ alkyl-C₃₋₆ cycloalkyl or is selected from -CHCH₃-CH=CR^{A4a}R^{A4b}; in certain embodiments, R^{A4} is selected from 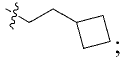 in certain embodiments, R^{A4} is selected from -CH₂-CH=CR^{A4a}R^{A4b}, L₃-R^{A4'} or is selected from -CHCH₃-CH=CR^{A4a}R^{A4b}; in certain embodiments, R^{A4} is selected from 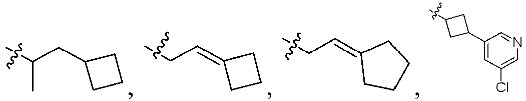 or is selected from 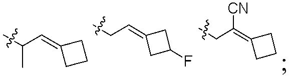 in certain embodiments, R^{A4} is selected from 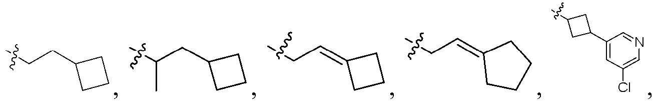 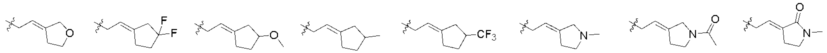 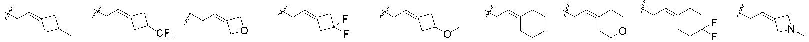 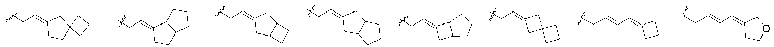 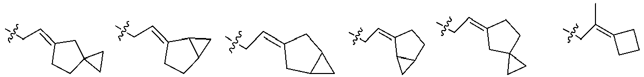 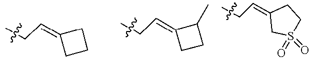 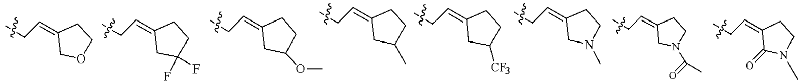 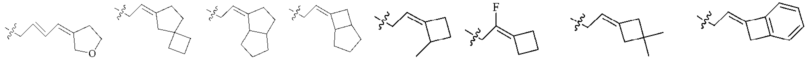 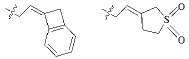 or is selected from 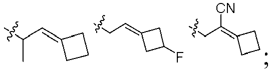
In certain embodiments, R^{A4} is selected from 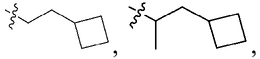 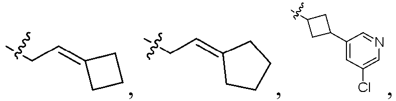 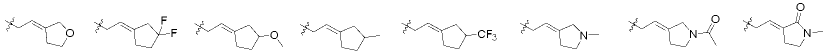 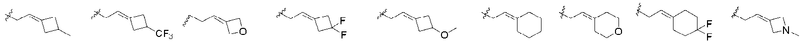 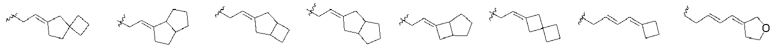 or is selected from 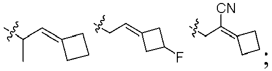
R^{A4c} is selected from H, halogen, deuterium, CN, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂; in certain embodiments, R^{A4c} is selected from H, halogen, deuterium, C₁₋₄ alkyl, haloC₁₋₄ alkyl, or C₁₋₄ alkoxy; in certain embodiments, R^{A4c} is selected from H, halogen, or C₁₋₄ alkyl; in certain embodiments, R^{A4c} is selected from H;
R^{A4a} and R^{A4b} together with the carbon atom to which they are attached form a 3- to 6- membered monocyclic carbocyclic ring, a C₇₋₁₀ spirocyclic carbocyclic ring, a C₇₋₁₀ bridged carbocyclic ring or a C₇₋₁₀ fused carbocyclic ring, or a 4- to 7-membered heterocyclic ring containing 1 to 3 heteroatoms selected from N, O, or S, or a 6-membered fused carbocyclic ring, and the carbocyclic ring or heterocyclic ring is optionally substituted with 1 to 3 groups selected from halogen, =O, deuterium, CN, OH, C₁₋₄ alkyl, haloC₁₋₄ alkyl, deuterated C₁₋₄ alkyl, -COC₁₋₄ alkyl, C₁₋₄ alkoxy, haloC₁₋₄ alkoxy, deuterated C₁₋₄ alkoxy and NH₂; the carbocyclic ring includes cycloalkyl and aryl, and the heterocyclic ring includes heterocycloalkyl and heteroaryl;
in certain embodiments, R^{A4a} and R^{A4b} together with the carbon atom to which they are attached form a 3- to 6-membered carbocyclic ring; in certain embodiments, R^{A4a} and R^{A4b} together with the carbon atom to which they are attached form a 3-, 4-, 5- or 6-membered carbocyclic ring; in certain embodiments, R^{A4a} and R^{A4b} together with the carbon atom to which they are attached form a 3-, 4-, 5-, or 6- membered cycloalkyl; in certain embodiments, R^{A4a} and R^{A4b} together with the carbon atom to which they are attached form a 4-, 5-, or 6- membered cycloalkyl; in certain embodiments, R^{A4a} and R^{A4b} together with the carbon atoms to which they are attached form a 4-, 5-, or 6-membered monocyclic cycloalkyl, a C₇₋₁₀ spirocyclic carbocyclic ring, a C₇₋₁₀ fused carbocyclic ring, a 6-membered fused carbocyclic ring, a 5- or 6-membered heterocyclic cycloalkyl containing 1 to 3 heteroatoms selected from N, O, or S, and the cycloalkyl or heterocycloalkyl is optionally substituted with 1 to 3 groups selected from halogen, =O, deuterium, CN, OH, C₁₋₂ alkyl, haloC₁₋₂ alkyl, deuterated C₁₋₂ alkyl, -COC₁₋₂ alkyl, C₁₋₂ alkoxy, haloC₁₋₂ alkoxy, deuterated C₁₋₂ alkoxy and NH₂;
in certain embodiments, R^{A4a} and R^{A4b} together with the carbon atoms to which they are attached form a 4-, 5-, or 6-membered monocyclic cycloalkyl, a C₇₋₁₀ spirocyclic carbocyclic ring, a C₇₋₁₀ fused carbocyclic ring, a 5- or 6-membered heterocyclic cycloalkyl containing 1 to 3 heteroatoms selected from N, O, or S, and the cycloalkyl or heterocycloalkyl is optionally substituted with 1 to 3 groups selected from halogen, =O, deuterium, CN, OH, C₁₋₂ alkyl, haloC₁₋₂ alkyl, deuterated C₁₋₂ alkyl, -COC₁₋₂ alkyl, C₁₋₂ alkoxy, haloC₁₋₂ alkoxy, deuterated C₁₋₂ alkoxy and NH₂;
L₃ is selected from C₃₋₆ cycloalkyl, or -CH(C₁₋₂ alkyl)-C₁₋₄ alkyl, and the cycloalkyl is optionally substituted with 1 to 3 R^{L3};
in certain embodiments, L₃ is selected from C₃₋₆ cycloalkyl, -CH(C₁₋₂ alkyl)-C₁₋₄ alkyl; in certain embodiments, L₃ is selected from C₃₋₆ cycloalkyl, -CH(C₁₋₂ alkyl)-C₁₋₂ alkyl; in certain embodiments, L₃ is selected from cyclobutyl, cyclopentyl, cyclohexyl, or -CH(CH₃)-CH₂-*, wherein * represents an attachment site with R^{4A'};
each R^{L3} is independently selected from halogen, =O, deuterium, CN, OH, C₁₋₄ alkyl, haloC₁₋₄ alkyl, deuterated C₁₋₄ alkyl, -COC₁₋₄ alkyl, C₁₋₄ alkoxy, haloC₁₋₄ alkoxy, deuterated C₁₋₄ alkoxy and NH₂;
alternatively, two R^{L3} on adjacent ring atoms together with the atoms to which they are attached form a 5- to 7-membered carbocyclic ring or a 5- to 7-membered heterocyclic ring containing 1 to 3 heteroatoms selected from N, O, or S; in certain embodiments, C₄ cycloalkyl or C₅ cycloalkyl is formed; in certain embodiments, heteroaryl, such as pyrazolyl, imidazolyl, thiazolyl, thienyl, oxazolyl, or furyl, is formed, or 5-membered heterocycloalkyl containing 1 to 2 N, O, or S heteroatoms is formed; in certain embodiments, the carbocyclic or heterocyclic ring is optionally substituted;
R^{A4'} is selected from H, C₃₋₆ cycloalkyl, a 5- to 10-membered heteroaryl containing 1 to 3 heteroatoms selected from N, O, or S, or a 4- to 7-membered heterocycloalkyl containing 1 to 3 heteroatoms selected from N, O, or S, and the cycloalkyl, heteroaryl and heterocycloalkyl are optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂; in certain embodiments, R^{A4'} is selected from C₃₋₆ cycloalkyl, or a 5- to 10-membered heteroaryl containing 1 to 3 heteroatoms selected from N, O, or S, and the cycloalkyl or heteroaryl is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂; in certain embodiments, R^{A4'} is selected from C_{4, 5, 6} cycloalkyl, a 5- to 6-membered monocyclic heteroaryl containing 1, 2, or 3 heteroatoms selected from N, O, or S, or a 8- to 10-membered bicyclic heteroaryl containing 1, 2, or 3 heteroatoms selected from N, O, or S, and the cycloalkyl or heteroaryl is optionally further substituted with 1, 2, or 3 groups selected from F, Cl, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂; in certain embodiments, R^{A4'} is selected from C_{4, 5} cycloalkyl, or a 5- to 6-membered monocyclic heteroaryl containing 1, 2, or 3 heteroatoms selected from N, O, or S, and the heteroaryl is optionally further substituted with 1, 2, or 3 groups selected from F, Cl, deuterium, CN, OH, C₁₋₂ alkyl, haloC₁₋₂ alkyl, deuterated C₁₋₂ alkyl and NH₂;
each of R^{L1} and R^{L2} is independently selected from halogen, OH, CN, amino, C₁₋₄ alkyl, C₁₋₄ alkoxy, C₃₋₆ cycloalkyl, or 4- to 6-membered heterocycloalkyl containing 1 to 3 heteroatoms selected from N, O, or S; in certain embodiments, each of R^{L1} and R^{L2} is independently selected from F, Cl, OH, CN, amino, C₁₋₂ alkyl, C₁₋₂ alkoxy, C₃₋₄ cycloalkyl, or 4,5-membered heterocycloalkyl containing 1 to 3 heteroatoms selected from N, O, or S; in certain embodiments, each of R^{L1} and R^{L2} is independently selected from F, Cl, OH, CN, amino, C₁₋₂ alkyl, or C₁₋₂ alkoxy; in certain embodiments, each of R^{L1} and R^{L2} is independently selected from F, Cl, OH, CN, C₁₋₂ alkyl, or C₁₋₂ alkoxy; in certain embodiments, each of R^{L1} and R^{L2} is independently selected from F or C₁₋₂ alkyl;
alternatively, R², R⁴ and L₁ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3}; in certain embodiments, R², R⁴ and L₁ together with the atoms to which they are attached form a 6- to 7-membered cycloalkyl or phenyl, and the cycloalkyl is optionally further substituted with 1 to 3 groups selected from R^{A3}; in certain embodiments, R², R⁴ and L₁ together with the atoms to which they are attached form cyclohexyl or phenyl, and the cyclohexyl is optionally further substituted with 1 to 3 groups selected from R^{A3}; in certain embodiments, R², R⁴, L₁ together with the atoms to which they are attached form cyclohexyl or phenyl; in certain embodiments, R², R⁴ together with the atoms to which they are attached form C₅₋₇ cycloalkyl; in certain embodiments, R², R⁴ together with the atoms to which they are attached form cyclopentyl, cyclohexyl, or cycloheptyl;
alternatively, R⁴, R⁵ together with the atoms to which they are attached form a 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally substituted with 1 to 3 groups selected from R^{A3}; in certain embodiments, R⁴, R⁵ together with the atoms to which they are attached form phenyl, 5- to 6-membered cycloalkyl, 5- to 6-membered heteroaryl containing 1 to 3 heteroatoms selected from N, O, or S, and the cycloalkyl is optionally substituted with 1 to 3 groups selected from R^{A3}; in certain embodiments, R⁴, R⁵ together with the atoms to which they are attached form phenyl, cyclopentyl, cyclohexyl, pyrrolyl, furyl, thienyl, imidazolyl, oxazolyl, thiazolyl, pyrazolyl, isoxazolyl, pyridyl, pyridazinyl, pyrimidinyl, or pyrazinyl;
alternatively, R^{L1} and R⁹ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3}; in certain embodiments, R^{L1} and R⁹ together with the atoms to which they are attached form 5- to 6-membered cycloalkyl, and the cycloalkyl is optionally further substituted with 1 to 3 groups selected from R^{A3}; in certain embodiments, R^{L1} and R⁹ together with the atoms to which they are attached form cyclopentyl or cyclohexyl;
alternatively, R^{L2} and R⁹ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3}; in certain embodiments, R^{L2} and R⁹ together with the atoms to which they are attached form 5- to 6-membered cycloalkyl, and the cycloalkyl is optionally further substituted with 1 to 3 groups selected from R^{A3}; in certain embodiments, R^{L2} and R⁹ together with the atoms to which they are attached form cyclopentyl or cyclohexyl;
each R^{A3} is independently selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂ group substitution; in certain embodiments, each R^{A3} is independently selected from =O, F, Cl, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂; in certain embodiments, each R^{A3} is independently selected from =O, F, Cl, deuterium, CN, OH, methyl, ethyl, -CH₂F, -CHF₂, -CF₃, -CH₂CH₂F, - CH₂CHF₂, -CH₂CF₃, -CHFCH₂F, -CHFCHF₂, -CHFCF₃, -CF₂CH₂F, -CF₂CHF₂, - CF₂CF₃, -CH₂D, -CHD₂, -CD₃, -CH₂CH₂D, -CH₂CHD₂, -CH₂CD₃, -CHDCH₂D, - CHDCHD₂, -CHDCD₃, -CD₂CH₂D, -CD₂CHD₂, -CD₂CD₃, methoxy, ethoxy, - OCHF₂, -OCH₂F, -OCF₃, -OCH₂CH₂F, -OCH₂CHF₂, -OCH₂CF₃, -OCHFCH₂F, - OCHFCHF₂, -OCHFCF₃, -OCF₂CH₂F, -OCF₂CHF₂, -OCF₂CF₃, -OCHD₂, - OCH₂D, -OCD₃, -OCH₂CH₂D, -OCH₂CHD₂, -OCH₂CD₃, -OCHDCH₂D, - OCHDCHD₂, -OCHDCD₃, -OCD₂CH₂D, -OCD₂CHD₂, -OCD₂CD₃ and NH₂;
each of R^{11a}, R^{11b}, R^{12a}, and R^{12b} is independently selected from H, deuterium, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, or C₃₋₆ cycloalkyl; alternatively, any two of R^{11a}, R^{11b}, R^{12a}, and R^{12b} together with the atoms to which they are attached form C₃₋₆ cycloalkyl, which is optionally further substituted with 1 to 3 groups selected from =O, 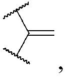 halogen, deuterium, CN, OH, C₁₋₃ alkyl, haloC₁₋₃ alkyl, deuterated C₁₋₃ alkyl, C₁₋₃ alkoxy, haloC₁₋₃ alkoxy, or deuterated C₁₋₃ alkoxy;
in certain embodiments, each of R^{11a}, R^{11b}, R^{12a}, and R^{12b} is independently selected from H, deuterium, OH, C₁₋₃ alkyl, or haloC₁₋₃ alkyl; alternatively, R^{11a} and R^{11b}, or R^{12a} and R^{12b} together with the atoms to which they are attached form C₃₋₆ cycloalkyl;
in certain embodiments, each of R^{11a}, R^{11b}, R^{12a}, and R^{12b} is independently selected from H, deuterium, OH, C₁₋₃ alkyl, or haloC₁₋₃ alkyl; alternatively, R^{11a} and R^{11b}, or R^{12a} and R^{12b} together with the atoms to which they are attached form cyclopropyl;
provided that: one of the combinations of R⁶ and R¹⁰, R¹⁰ and R⁷, R⁷ and R⁸, and R⁸ and R⁹ together with the atoms to which they are attached form a 4- to 8-membered carbocyclic ring or 5- to 7-membered heterocycloalkyl containing 1 to 3 heteroatoms selected from N, O, S, B, or Si, and the carbocyclic ring or heterocycloalkyl is optionally further substituted with 1 to 3 groups selected from =O, 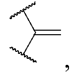 halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, 5- to 7-membered heterocycloalkyl containing 1 to 3 heteroatoms selected from N, O, S, B, or Si, 5-to 7-membered heteroaryl containing 1 to 3 heteroatoms selected from N, O, S, B, or Si and NH₂;
In certain embodiments, the compounds of the present invention satisfy at least one of the following conditions, the compounds of the present invention satisfy one, two, three or four of the following conditions:
   (1). at least one group of R¹, R² and R³ is selected from R^{A1};
   (2). at least one group of R⁴ and R⁵ is selected from C₃₋₁₀ cycloalkyl, C₂₋₆ alkenyl, or C₂₋₆ alkynyl;
   (3). R⁴, R⁵ together with the atoms to which they are attached form 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3}, and R¹ is selected from R^{A1} or -O-haloC₁₋₄ alkyl;
   (4). at least one group of R⁶, R⁷, R⁸, R⁹, and R¹⁰ is selected from R^{A2}; or one of the combinations of R⁶ and R¹⁰, R¹⁰ and R⁷, R⁷ and R⁸, and R⁸ and R⁹ together with the atoms to which they are attached form 4- to 7-membered carbocyclic ring or 5- to 7-membered heterocycloalkyl containing 1 to 3 heteroatoms selected from N, O, S, or B or 8 membered carbocyclic ring or 5- to 7-membered heterocycloalkyl containing 1 Si atom, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
   (5). R², R⁴, L₁ together with the atoms to which they are attached form 5-to 7-membered carbocyclic ring or 5- to 7-membered heterocyclic ring containing 1 to 3 heteroatoms selected from N, O, or S, and the carbocyclic ring or heterocyclic ring is optionally further substituted with 1 to 3 groups selected from R^{A3}, and R¹ is not heterocycloalkyl;
   (6). L₁ is selected from O, NH, S, -CD₂-, -CHD-, -CR^{L1}R^{L2}-, C₁₋₄ alkyl, - C(O)-, S(O), or S(O)₂, and the alkyl is optionally further substituted with R^{L1};
   (7). L₂ is selected from O, NH, S, -CR^{L1}R^{L2}-, -CHR^{L2}-, -CDR^{L2}-, -C(O)-, S(O), or S(O)₂;
   (8). R^{A4} is selected from -CH₂-CR^{A4c}=CR^{A4a}R^{A4b}, -CH₂-CH=CH-CH=CR^{A4a}R^{A4b}, or L₃-R^{A4'} or is selected from -CH(C₁₋₂ alkyl)-CR^{A4c}=CR^{A4a}R^{A4b}, or -C(C₁₋₂ alkyl)₂-CR^{A4c}=CR^{A4a}R^{A4b};
   (9). X₁ is selected from S;
   (10). R^{L2} and R⁹ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
in certain embodiments, the compounds of the present invention satisfy at least one of the following conditions, in certain embodiments, the compounds of the present invention satisfy one, two, three or four of the following conditions:
   (1). at least one group of R¹, R² and R³ is selected from R^{A1};
   (2). at least one group of R⁴ and R⁵ is selected from C₃₋₁₀ cycloalkyl, C₂₋₆ alkenyl, or C₂₋₆ alkynyl;
   (3). R⁴, R⁵ together with the atoms to which they are attached form 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3}, and R¹ is selected from R^{A1} or -O-haloC₁₋₄ alkyl;
   (4). at least one group of R⁶, R⁷, R⁸, R⁹, and R¹⁰ is selected from R^{A2};
   (5). R², R⁴, L₁ together with the atoms to which they are attached form 5-to 7-membered carbocyclic ring or 5- to 7-membered heterocyclic ring containing 1 to 3 heteroatoms selected from N, O, or S, and the carbocyclic ring or heterocyclic ring is optionally further substituted with 1 to 3 groups selected from R^{A3}, and R¹ is not heterocycloalkyl;
   (6). L₁ is selected from O, NH, S, -CD₂-, -CHD-, -CR^{L1}R^{L2}-, C₁₋₄ alkyl, - C(O)-, S(O), or S(O)₂, and the alkyl is optionally further substituted with R^{L1};
   (7). L₂ is selected from O, NH, S, -CR^{L1}R^{L2}-, -CHR^{L2}-, -CDR^{L2}-, -C(O)-, C₂₋₄ alkyl, S(O), or S(O)₂, and the alkyl is substituted with 1 to 3 R^{L1};
   (8). R^{A4} is selected from -CH₂-CR^{A4c}=CR^{A4a}R^{A4b}, or L₃-R^{A4'} or is selected from -CH(C₁₋₂ alkyl)-CR^{A4c}=CR^{A4a}R^{A4b}, or -C(C₁₋₂ alkyl)₂-CR^{A4c}=CR^{A4a}R^{A4b};
   (9). X₁ is selected from S.

The compounds of the present invention satisfy at least one of the following conditions, in certain embodiments, the compounds of the present invention satisfy one, two, three or four of the following conditions:
(1). at least one group of R¹, R² and R³ is selected from R^{A1};
(2). at least one group of R⁴ and R⁵ is selected from C₃₋₁₀ cycloalkyl, C₂₋₆ alkenyl, or C₂₋₆ alkynyl;
(3). R⁴, R⁵ together with the atoms to which they are attached form 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3}, and R¹ is selected from R^{A1} or -O-haloC₁₋₄ alkyl;
(4). at least one group of R⁶, R⁷, R⁸, R⁹, and R¹⁰ is selected from R^{A2}; or one of the combinations of R⁶ and R¹⁰, R¹⁰ and R⁷, R⁷ and R⁸, and R⁸ and R⁹ together with the atoms to which they are attached form 4- to 7-membered carbocyclic ring or 5- to 7-membered heterocycloalkyl containing 1 to 3 heteroatoms selected from N, O, S, or B or 8 membered carbocyclic ring or 5- to 7-membered heterocycloalkyl containing 1 Si atom, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
(5). R², R⁴, L₁ together with the atoms to which they are attached form 5-to 7-membered carbocyclic ring or 5- to 7-membered heterocyclic ring containing 1 to 3 heteroatoms selected from N, O, or S, and the carbocyclic ring or heterocyclic ring is optionally further substituted with 1 to 3 groups selected from R^{A3}, and R¹ is not heterocycloalkyl;
(6). L₁ is selected from O, NH, S, -CD₂-, -CHD-, -CR^{L1}R^{L2}-, C₁₋₄ alkyl, - C(O)-, S(O), or S(O)₂, and the alkyl is optionally further substituted with R^{L1};
(7). L₂ is selected from O, NH, S, -CR^{L1}R^{L2}-, -CHR^{L2}-, -CDR^{L2}-, -C(O)-, S(O), or S(O)₂;
(8). R^{A4} is selected from -CH₂-CH=CR^{A4a}R^{A4b}, -CH₂-CH=CH-CH=CR^{A4a}R^{A4b}, or L₃-R^{A4'} or selected from -CH(C₁₋₂ alkyl)-CR^{A4c}=CR^{A4a}R^{A4b} or - C(C₁₋₂ alkyl)₂-CR^{A4c}=CR^{A4a}R^{A4b};
(9). X₁ is selected from S;
(10). R^{L2} and R⁹ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};

in certain embodiments, the compounds of the present invention satisfy at least one of the following conditions, in certain embodiments, the compounds of the present invention satisfy one, two, three or four of the following conditions:
   (1). at least one group of R¹, R² and R³ is selected from R^{A1};
   (2). at least one group of R⁴ and R⁵ is selected from C₃₋₁₀ cycloalkyl, C₂₋₆ alkenyl, or C₂₋₆ alkynyl;
   (3). R⁴, R⁵ together with the atoms to which they are attached form 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3}, and R¹ is selected from R^{A1} or -O-haloC₁₋₄ alkyl;
   (4). at least one group of R⁶, R⁷, R⁸, R⁹, and R¹⁰ is selected from R^{A2};
   (5). R², R⁴, L₁ together with the atoms to which they are attached form 5-to 7-membered carbocyclic ring or 5- to 7-membered heterocyclic ring containing 1 to 3 heteroatoms selected from N, O, or S, and the carbocyclic ring or heterocyclic ring is optionally further substituted with 1 to 3 groups selected from R^{A3}, and R¹ is not heterocycloalkyl;
   (6). L₁ is selected from O, NH, S, -CD₂-, -CHD-, -CR^{L1}R^{L2}-, C₁₋₄ alkyl, - C(O)-, S(O), or S(O)₂, and the alkyl is optionally further substituted with R^{L1};
   (7). L₂ is selected from O, NH, S, -CR^{L1}R^{L2}-, -CHR^{L2}-, -CDR^{L2}-, -C(O)-, C₂₋₄ alkyl, S(O), or S(O)₂, and the alkyl is substituted with 1 to 3 R^{L1};
   (8). R^{A4} is selected from -CH₂-CH=CR^{A4a}R^{A4b} or L₃-R^{A4'} or is selected from -CH(C₁₋₂ alkyl)-CR^{A4c}=CR^{A4a}R^{A4b}, or -C(C₁₋₂ alkyl)₂-CR^{A4c}-CR^{A4a}R^{A4b};
   (9). X₁ is selected from S;
in certain embodiments, the compounds of the present invention satisfy at least one of the following conditions, in certain embodiments, the compounds of the present invention satisfy one, two, three or four of the following conditions:
   (1). at least one group of R¹, R² and R³ is selected from R^{A1};
   (2). at least one group of R⁴ and R⁵ is selected from C₃₋₇ cycloalkyl, C₂₋₄ alkenyl, or C₂₋₄ alkynyl;
   (3). at least one group of R⁷ and R⁸ is selected from R^{A2};
   (4). L₂ is selected from -CR^{L1}R^{L2}-, -CHR^{L2}-, -CDR^{L2}-, or -C(O)-;
   (5). one of the combinations of R⁷ and R⁸, and R⁸ and R⁹ together with the atoms to which they are attached form 4- to 7-membered carbocyclic ring, 5- to 7-membered heterocycloalkyl containing 1 to 3 heteroatoms selected from N, O, S, or B or 8 membered carbocyclic ring or 5- to 7-membered heterocycloalkyl containing 1 Si atom, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
in certain embodiments, the compounds of the present invention satisfy at least one of the following conditions, in certain embodiments, the compounds of the present invention satisfy one, two, three or four of the following conditions:
   (1). at least one group of R¹, R² and R³ is selected from R^{A1};
   (2). at least one group of R⁷ and R³ is selected from R^{A2};
   (3). one of the combinations of R⁷ and R⁸, and R⁸ and R⁹ together with the atoms to which they are attached form 4- to 7-membered carbocyclic ring, 5- to 7-membered heterocycloalkyl containing 1 to 3 heteroatoms selected from N, O, S, or B or 8 membered carbocyclic ring or 5- to 7-membered heterocycloalkyl containing 1 Si atom, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
in certain embodiments, the compounds of the present invention satisfy at least one of the following conditions, in certain embodiments, the compounds of the present invention satisfy one, two, three or four of the following conditions:
   (1). at least one group of R⁷ and R⁸ is selected from R^{A2};
   (2). at least one group of R⁴ and R⁵ is selected from C₃₋₁₀ cycloalkyl, C₂₋₆ alkenyl, or C₂₋₆ alkynyl;
   (3). R⁴, R⁵ together with the atoms to which they are attached form a 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
   (4). R², R⁴ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
   (5). L₂ is selected from -CR^{L1}R^{L2}-, -CHR^{L2}-, -CDR^{L2}-, -C(O)-, or C₂₋₄ alkyl, and the alkyl is substituted with 1 to 3 R^{L1};
   (6). one of the combinations of R⁷ and R⁸, and R⁸ and R⁹ together with the atoms to which they are attached form 4- to 7-membered carbocyclic ring, 5- to 7-membered heterocycloalkyl containing 1 to 3 heteroatoms selected from N, O, S, or B or 8 membered carbocyclic ring or 5- to 7-membered heterocycloalkyl containing 1 Si atom, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
in certain embodiments, the compounds of the present invention satisfy at least one of the following conditions, in certain embodiments, the compounds of the present invention satisfy one, two, three or four of the following conditions:
   (1). R¹ is selected from R^{A1};
   (2). at least one group of R⁴ and R⁵ is selected from cyclopropyl, vinyl, or ethynyl;
   (3). R⁴, R⁵ together with the atoms to which they are attached form phenyl, cyclohexyl, cyclopentyl, thienyl, furyl, pyrrolyl, pyrazolyl, or isoxazolyl;
   (4). at least one group of R⁷ and R⁸ is selected from R^{A2};
   (5). R², R⁴, L₁ together with the atoms to which they are attached form cyclohexyl or phenyl;
   (6). L₁ is selected from O;
   (7). L₂ is selected from -C(O)-, -CHF-, -CF₂-, or -C(CH₃)₂-;
   (8). one of the combinations of R⁷ and R⁸, and R⁸ and R⁹ together with the atoms to which they are attached form 4- to 7-membered carbocyclic ring or 8 membered carbocyclic ring or 5- to 7-membered heterocycloalkyl containing 1 Si atom, and the carbocyclic ring or heterocycloalkyl is optionally further substituted with 1 to 3 groups selected from =O, F, Cl, deuterium, OH, C₁₋₂ alkyl, or haloC₁₋₂ alkyl;
in certain embodiments, one of the combinations of R⁶ and R¹⁰, R¹⁰ and R⁷, R⁷ and R⁸, and R⁸ and R⁹ together with the atoms to which they are attached form C₄₋₈ cycloalkyl or 5- to 7-membered heterocycloalkyl containing 1 to 3 heteroatoms selected from N, O, S, B, or Si, and the cycloalkyl or heterocycloalkyl is optionally further substituted with 1 to 3 groups selected from =O, 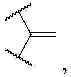 halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, 5- to 7-membered heterocycloalkyl containing 1 to 3 heteroatoms selected from N, O, S, B, or Si, 5-to 7-membered heteroaryl containing 1 to 3 heteroatoms selected from N, O, S, B, or Si and NH₂;
in certain embodiments, one of the combinations of R⁶ and R¹⁰, R¹⁰ and R⁷, R⁷ and R⁸, and R⁸ and R⁹ together with the atoms to which they are attached form C₄₋₆ monocyclic cycloalkyl, C₆₋₈ bicyclic bridged cycloalkyl, C₆₋₈ bicyclic fused cycloalkyl, C₆₋₈ bicyclic spiro cycloalkyl, or 5- to 7-membered heterocycloalkyl containing 1 Si heteroatom, and the cycloalkyl or heterocycloalkyl is optionally further substituted with 1 to 3 groups selected from =O, 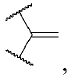 halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
in certain embodiments, one of the combinations of R⁶ and R¹⁰, R¹⁰ and R⁷, R⁷ and R⁸, and R⁸ and R⁹ together with the atoms to which they are attached form C₄₋₆ monocyclic cycloalkyl, and the cycloalkyl is optionally further substituted with 1 to 3 groups selected from =O, 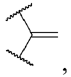 halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
in certain embodiments, R⁷ and R⁸ together with the atoms to which they are attached form C₄₋₈ cycloalkyl, and the cycloalkyl is optionally further substituted with 1 to 3 groups selected from =O, 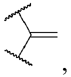 halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, 5- to 7-membered heterocycloalkyl containing 1 to 3 heteroatoms selected from N, O, S, B, or Si, 5- to 7-membered heteroaryl containing 1 to 3 heteroatoms selected from N, O, S, B, or Si and NH₂;
in certain embodiments, Rand R⁸ together with the atoms to which they are attached form C₄₋₆ monocyclic cycloalkyl, C₆₋₈ bicyclic bridged cycloalkyl, C₆₋₈ bicyclic fused cycloalkyl, or C₆₋₈ bicyclic spiro cycloalkyl, and the cycloalkyl is optionally further substituted with 1 to 3 groups selected from =O, 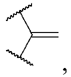 halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
in certain embodiments, Rand R⁸ together with the atoms to which they are attached form C₄₋₆ monocyclic cycloalkyl, and the cycloalkyl is optionally further substituted with 1 to 3 groups selected from =O, 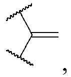 halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
in certain embodiments, one of the combinations of R⁶ and R¹⁰, R¹⁰ and R⁷, R⁷ and R⁸, and R⁸ and R⁹ together with the atoms to which they are attached form cyclobutyl, cyclopentyl, cyclohexyl, cyclobutenyl, cyclopentenyl, cyclohexenyl, 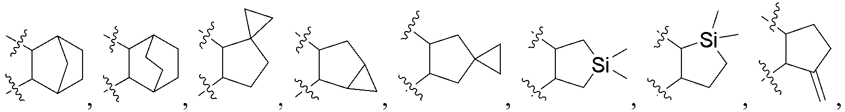 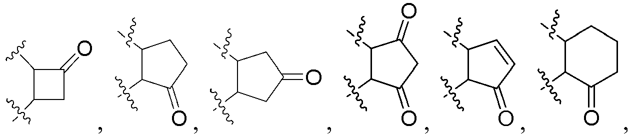 which is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
in certain embodiments, R⁷ and R⁸ together with the atoms to which they are attached form cyclobutyl, cyclopentyl, cyclohexyl, cyclobutenyl, cyclopentenyl, cyclohexenyl, 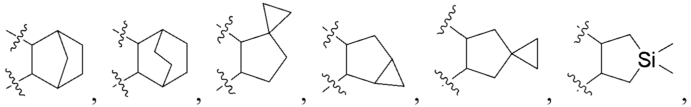 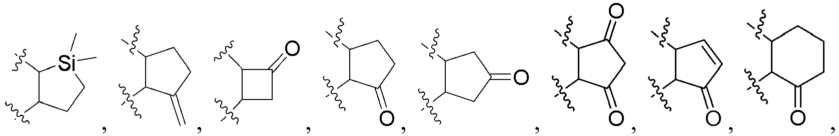 which is optionally further substituted with 1 to 3 groups selected from halogen, deuterium, CN, OH, C₁₋₃ alkyl, haloC₁₋₃ alkyl, deuterated C₁₋₃ alkyl, C₁₋₃ alkoxy, haloC₁₋₃ alkoxy, deuterated C₁₋₃ alkoxy and NH₂.

Specifically, in the first technical solution, the present invention provides a compound represented by formula (I) or (I-a), a stereoisomer, deuterated substance, solvate, or pharmaceutically acceptable salt thereof, 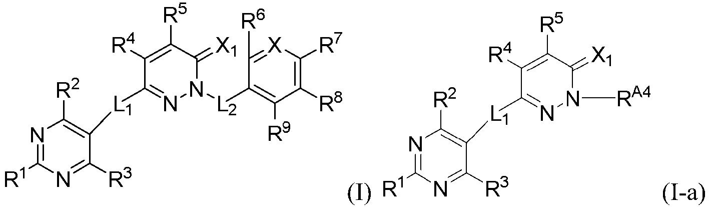 wherein
each of R¹, R² and R³ is independently selected from H, deuterium, R^{A1}, -O-haloC₁₋₄ alkyl, -NH-haloC₁₋₄ alkyl, -OC₁₋₄ alkyl, C₁₋₄ alkoxy, C₅₋₆ alkoxy, halogen, cyano, nitro, C₁₋₄ alkyl, C₂₋₆ alkenyl, -NH-C₁₋₄ alkyl, -N(C₁₋₄ alkyl)₂, -NH-C₃₋₁₀ cycloalkyl, -NHC(O)C₁₋₄ alkyl, or 4- to 10-membered heterocycloalkyl containing 1 to 3 heteroatoms selected from N, O, and S, and the alkyl, alkoxy, cycloalkyl, or heterocycloalkyl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, C₃₋₁₀ cycloalkyl, 4- to 6-membered heterocycloalkyl containing 1 to 3 heteroatoms selected from N, O, or S, phenyl, and NH₂, provided that R¹, R² and R³ are not simultaneously selected from H;
R^{A1} is selected from haloC₂₋₆ alkenyl, C₂₋₆ alkynyl, -O-(CH₂)ᵣ-R^{a}, C₃₋₁₀ cycloalkyl, 5- to 10-membered heteroaryl containing 1 to 3 heteroatoms selected from N, O, or S, -C₁₋₄ alkyl-R^{a}, -C₁₋₄ alkyl-OR^{a}, -C₁₋₄ alkyl-NR^{b}R^{a}, -NR^{b}-S(O)₂-R^{a}, -NR^{b}-S(O)₂-NR^{b}R^{a}, -O-NR^{b}R^{a}, -NH-OR^{b}, or -Se-(CH₂)ᵣ-R^{a}, and the alkynyl, heteroaryl, alkyl, or cycloalkyl is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{a} is selected from CN, haloC₁₋₆ alkyl, C₃₋₁₀ cycloalkyl, 4- to 10-membered heterocycloalkyl containing 1 to 3 heteroatoms selected from N, O, or S, 6- to 10-membered aryl, 5- to 10-membered heteroaryl containing 1 to 3 heteroatoms selected from N, O, or S, or -C(O)-R^{a1}, and the alkyl, cycloalkyl, heterocycloalkyl, aryl, or heteroaryl is optionally further substituted with 1 to 3 groups selected from halogen, =O, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{b} is selected from H, deuterium, C₁₋₄ alkyl, C₃₋₆ cycloalkyl, haloC₁₋₄ alkyl, or deuterated C₁₋₄ alkyl;
R^{a1} is selected from OH, NH₂, -NHC₁₋₄ alkyl, -N(C₁₋₄ alkyl)₂, -NHC₃₋₁₀ cycloalkyl, C₁₋₄ alkoxy, C₁₋₆ alkyl, C₃₋₁₀ cycloalkyl, 6- to 10-membered aryl, 5- to 10-membered heteroaryl containing 1 to 3 heteroatoms selected from N, O, or S;
X is selected from CR¹⁰ or N;
X₁ is selected from O or S;
each of R⁴ and R⁵ is independently selected from H, deuterium, C₃₋₁₀ cycloalkyl, C₂₋₆ alkenyl, C₂₋₆ alkynyl, halogen, amino,-NH-C₁₋₄ alkyl, -N(C₁₋₄ alkyl)₂, C₁₋₄ alkyl, haloC₁₋₄ alkyl, 4- to 10-membered heterocycloalkyl containing 1 to 3 heteroatoms selected from N, O, or S, 5- to 10-membered heteroaryl containing 1 to 3 heteroatoms selected from N, O, or S, or 6- to 10-membered aryl;
each of R⁶, R⁷, R⁸, R⁹, and R¹⁰ is independently selected from H, deuterium, R^{A2}, halogen, OH, CN, amino, C₁₋₄ alkyl, C₂₋₆ alkenyl, C₂₋₆ alkynyl, C₁₋₄ alkoxy, haloC₁₋₄ alkyl, -NH-C₁₋₄ alkyl, -N(C₁₋₄ alkyl)₂, -S(O)R^{c}, -S(O)²R^{c}, -C(O)R^{c}, - C(O)OR^{c}, -C(O)N(R^{c})₂, or -OC(O)R^{c}, and the alkyl, alkenyl, alkynyl, or alkoxy is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
R^{A2} is selected from -(CH₂)ᵣ-(4- to 10-membered heterocycloalkyl containing 1 to 3 heteroatoms selected from N, O, S, or Si), or -(CH₂)ᵣ-(5- to 10-membered heteroaryl containing 1 to 3 heteroatoms selected from N, O, or S), -P(O)(R^{c})₂, - Si(R^{c})₃, -SF₅, N₃, B(OH)₂, or -S(O)(=NH)R^{c}, and the heterocycloalkyl or heteroaryl is optionally further substituted with 1 to 3 groups selected from halogen, =O, D, CN, OH, C₁₋₄ alkyl, haloC₁₋₄ alkyl, deuterated C₁₋₄ alkyl, C₁₋₄ alkoxy, haloC₁₋₄ alkoxy, C₃₋₆ cycloalkyl, 4- to 7-membered heterocycloalkyl containing 1 to 3 heteroatoms selected from N, O, or S, deuterated C₁₋₄ alkoxy and NH₂;
each R^{c} is independently selected from H, OH, C₁₋₄ alkyl, C₃₋₇ cycloalkyl, haloC₁₋₄ alkyl, deuterated C₁₋₄ alkyl, C₁₋₄ alkoxy, haloC₁₋₄ alkoxy, deuterated C₁₋₄ alkoxy, or NH₂;
each r is independently selected from 0, 1, 2 or 3;
L₁ is selected from a bond, O, NH, S, -CD₂-, -CHD-, -CR^{L1}R^{L2}-, C₁₋₄ alkyl, - C(O)-, S(O), or S(O)₂, and the alkyl is optionally further substituted with R^{L1};
L₂ is selected from O, NH, S, -CH₂-, -CD₂-, -CHD-, -CR^{L1}R^{L2}-, -CHR^{L2}-, - CDR^{L2}-, C₂₋₄ alkyl, -C(O)-, S(O), S(O)₂, the alkyl is optionally further substituted with 1 to 3 R^{L1};
R^{A4} is selected from -CH₂-C₁₋₄ alkyl-C₃₋₆ cycloalkyl, -CH₂-CR^{A4c}=CR^{A4a}R^{A4b}, -CH₂-CH=CH-CH=CR^{A4a}R^{A4b}, -CH(C₁₋₂ alkyl)-CR^{A4c}=CR^{A4a}R^{A4b}, -C(C₁₋₂ alkyl)₂-CR^{A4c}=CR^{A4a}R^{A4b}, or L₃-R^{A4'};
R^{A4c} is selected from H, halogen, deuterium, CN, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
R^{A4a} and R^{A4b} together with the carbon atom to which they are attached form a 3- to 6- membered monocyclic carbocyclic ring, a C₇₋₁₀ spirocyclic carbocyclic ring, a C₇₋₁₀ bridged carbocyclic ring, a C₇₋₁₀ fused carbocyclic ring, a 6-membered fused carbocyclic ring, or a 4- to 7-membered heterocyclic ring containing 1 to 3 heteroatoms selected from N, O, or S, and the carbocyclic ring or heterocyclic ring is optionally substituted with 1 to 3 groups selected from halogen, =O, deuterium, CN, OH, C₁₋₄ alkyl, haloC₁₋₄ alkyl, deuterated C₁₋₄ alkyl, -COC₁₋₄ alkyl, C₁₋₄ alkoxy, haloC₁₋₄ alkoxy, deuterated C₁₋₄ alkoxy and NH₂; L₃ is selected from C₃₋₆ cycloalkyl, or -CH(C₁₋₂ alkyl)-C₁₋₄ alkyl, and the cycloalkyl is optionally substituted with 1 to 3 R^{L3};
each R^{L3} is independently selected from halogen, =O, deuterium, CN, OH, C₁₋₄ alkyl, haloC₁₋₄ alkyl, deuterated C₁₋₄ alkyl, -COC₁₋₄ alkyl, C₁₋₄ alkoxy, haloC₁₋₄ alkoxy, deuterated C₁₋₄ alkoxy and NH₂;
alternatively, two R^{L3} on adjacent ring atoms together with the atoms to which they are attached form a 5- to 7-membered carbocyclic ring or a 5- to 7-membered heterocyclic ring containing 1 to 3 heteroatoms selected from N, O, or S;
R^{A4'} is selected from H, C₃₋₆ cycloalkyl, a 5- to 10-membered heteroaryl containing 1 to 3 heteroatoms selected from N, O, or S, or a 4- to 7-membered heterocycloalkyl containing 1 to 3 heteroatoms selected from N, O, or S, and the cycloalkyl, heteroaryl and heterocycloalkyl are optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each of R^{L1} and R^{L2} is independently selected from halogen, OH, CN, amino, C₁₋₄ alkyl, C₁₋₄ alkoxy, C₃₋₆ cycloalkyl, or 4- to 6-membered heterocycloalkyl containing 1 to 3 heteroatoms selected from N, O, or S;
alternatively, R², R⁴ and L₁ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
alternatively, R⁴, R⁵ together with the atoms to which they are attached form a 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally substituted with 1 to 3 groups selected from R^{A3};
alternatively, R^{L2} and R⁹ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
alternatively, one of the combinations of R⁶ and R¹⁰, R¹⁰ and R⁷, R⁷ and R⁸, and R⁸ and R⁹ together with the atoms to which they are attached form 4- to 7-membered carbocyclic ring, 5- to 7-membered heterocycloalkyl containing 1 to 3 heteroatoms selected from N, O, S, or B or 8 membered carbocyclic ring or 5- to 7-membered heterocycloalkyl containing 1 Si atom, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{A3} is independently selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂ group substitution;
provided that the compound satisfies at least one of the following conditions:
   (1). at least one group of R¹, R² and R³ is selected from R^{A1};
   (2). at least one group of R⁴ and R⁵ is selected from C₃₋₁₀ cycloalkyl, C₂₋₆ alkenyl, or C₂₋₆ alkynyl;
   (3). R⁴, R⁵ together with the atoms to which they are attached form 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3}, and R¹ is selected from R^{A1} or -O-haloC₁₋₄ alkyl;
   (4). at least one group of R⁶, R⁷, R⁸, R⁹, and R¹⁰ is selected from R^{A2}; alternatively, one of the combinations of R⁶ and R¹⁰, R¹⁰ and R⁷, R⁷ and R⁸, and R⁸ and R⁹ together with the atoms to which they are attached form 4- to 7-membered carbocyclic ring, 5- to 7-membered heterocycloalkyl containing 1 to 3 heteroatoms selected from N, O, S, or B or 8 membered carbocyclic ring or 5- to 7-membered heterocycloalkyl containing 1 Si atom, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
   (5). R², R⁴, L₁ together with the atoms to which they are attached form 5-to 7-membered carbocyclic ring or 5- to 7-membered heterocyclic ring containing 1 to 3 heteroatoms selected from N, O, or S, and the carbocyclic ring or heterocyclic ring is optionally further substituted with 1 to 3 groups selected from R^{A3}, and R¹ is not heterocycloalkyl;
   (6). L₁ is selected from O, NH, S, -CD₂-, -CHD-, -CR^{L1}R^{L2}-, C₁₋₄ alkyl, - C(O)-, S(O), or S(O)₂, and the alkyl is optionally further substituted with R^{L1};
   (7). L₂ is selected from O, NH, S, -CR^{L1}R^{L2}-, -CHR^{L2}-, -CDR^{L2}-, -C(O)-, S(O), or S(O)₂;
   (8). R^{A4} is selected from -CH₂-CR^{A4c}=CR^{A4a}R^{A4b}, -CH₂-CH=CH-CH=CR^{A4a}R^{A4b}, L₃-R^{A4'}, -CH(C₁₋₂ alkyl)-CR^{A4c}=CR^{A4a}R^{A4b}, or -C(C₁₋₂ alkyl)₂-CR^{A4c}=CR^{A4a}R^{A4b};
   (9). X₁ is selected from S;
   (10). R^{L2} and R⁹ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3},

As an alternative to the first technical solution of the present invention, a compound represented by formula (I) or (I-a), a stereoisomer, deuterated substance, solvate, or pharmaceutically acceptable salt thereof is provided, 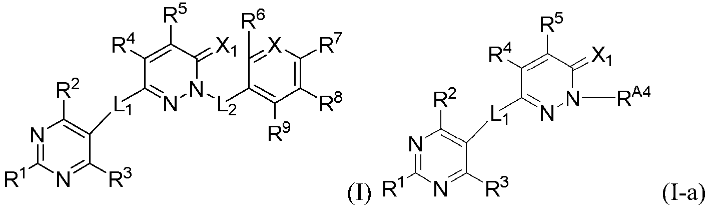
wherein, each of R¹, R² and R³ is independently selected from H, deuterium, R^{A1}, -O-haloC₁₋₄ alkyl, -NH-haloC₁₋₄ alkyl, -OC₁₋₄ alkyl, C₁₋₄ alkoxy, halogen, cyano, nitro, C₁₋₄ alkyl, C₂₋₆ alkenyl, -NH-C₁₋₄ alkyl, -N(C₁₋₄ alkyl)₂, -NH-C₃₋₁₀ cycloalkyl, -NHC(O)C₁₋₄ alkyl, or 4- to 10-membered heterocycloalkyl containing 1 to 3 heteroatoms selected from N, O, and S, or selected from C₅₋₆ alkoxy, and the alkyl, alkoxy, cycloalkyl, or heterocycloalkyl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, C₃₋₁₀ cycloalkyl, 4- to 6-membered heterocycloalkyl containing 1 to 3 heteroatoms selected from N, O, or S, phenyl, and NH₂, provided that R¹, R² and R³ are not simultaneously selected from H;
R^{A1} is selected from haloC₂₋₆ alkenyl, C₂₋₆ alkynyl, 5- to 10-membered heteroaryl containing 1 to 3 heteroatoms selected from N, O, or S, -C₁₋₄ alkyl-R^{a}, - C₁₋₄ alkyl-OR^{a}, -C₁₋₄ alkyl-NR^{b}R^{a}, -NR^{b}-S(O)₂-R^{a}, or -NR^{b}-S(O)₂-NR^{b}R^{a}, or R^{A1} is selected from -O-NR^{b}R^{a} or -NH-OR^{b}, and the alkynyl, heteroaryl, or alkyl is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{a} is selected from CN, haloC₁₋₆ alkyl, C₃₋₁₀ cycloalkyl, 4- to 10-membered heterocycloalkyl containing 1 to 3 heteroatoms selected from N, O, or S, 6- to 10-membered aryl, 5- to 10-membered heteroaryl containing 1 to 3 heteroatoms selected from N, O, or S, or -C(O)-R^{a1}, and the alkyl, cycloalkyl, heterocycloalkyl, aryl, or heteroaryl is optionally further substituted with 1 to 3 groups selected from halogen, =O, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{b} is selected from H, deuterium, C₁₋₄ alkyl, C₃₋₆ cycloalkyl, haloC₁₋₄ alkyl, or deuterated C₁₋₄ alkyl;
R^{a1} is selected from OH, NH₂, -NHC₁₋₄ alkyl, -N(C₁₋₄ alkyl)₂, -NHC₃₋₁₀ cycloalkyl, C₁₋₄ alkoxy, C₁₋₆ alkyl, C₃₋₁₀ cycloalkyl, 6- to 10-membered aryl, 5- to 10-membered heteroaryl containing 1 to 3 heteroatoms selected from N, O, or S;
X is selected from CR¹⁰ or N;
X₁ is selected from O or S;
each of R⁴ and R⁵ is independently selected from H, deuterium, C₃₋₁₀ cycloalkyl, C₂₋₆ alkenyl, C₂₋₆ alkynyl, halogen, amino,-NH-C₁₋₄ alkyl, -N(C₁₋₄ alkyl)₂, C₁₋₄ alkyl, haloC₁₋₄ alkyl, 4- to 10-membered heterocycloalkyl containing 1 to 3 heteroatoms selected from N, O, or S, 5- to 10-membered heteroaryl containing 1 to 3 heteroatoms selected from N, O, or S, or 6- to 10-membered aryl;
each of R⁶, R⁷, R⁸, R⁹, and R¹⁰ is independently selected from H, deuterium, R^{A2}, halogen, OH, CN, amino, C₁₋₄ alkyl, C₂₋₆ alkenyl, C₂₋₆ alkynyl, C₁₋₄ alkoxy, haloC₁₋₄ alkyl, -NH-C₁₋₄ alkyl, -N(C₁₋₄ alkyl)₂, -S(O)R^{c}, -S(O)²R^{c}, -C(O)R^{c}, - C(O)OR^{c}, -C(O)N(R^{c})₂, or -OC(O)R^{c}, and the alkyl, alkenyl, alkynyl, or alkoxy is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
R^{A2} is selected from -(CH₂)ᵣ-(4- to 10-membered heterocycloalkyl containing 1 to 3 heteroatoms selected from N, O, S, or Si), or -(CH₂)ᵣ-(5- to 10-membered heteroaryl containing 1 to 3 heteroatoms selected from N, O, or S), -P(O)(R^{c})₂, - Si(R^{c})₃, -SF₅, N₃, B(OH)₂, or -S(O)(=NH)R^{c}, and the heterocycloalkyl or heteroaryl is optionally further substituted with 1 to 3 groups selected from halogen, =O, D, CN, OH, C₁₋₄ alkyl, haloC₁₋₄ alkyl, deuterated C₁₋₄ alkyl, C₁₋₄ alkoxy, haloC₁₋₄ alkoxy, C₃₋₆ cycloalkyl, 4- to 7-membered heterocycloalkyl containing 1 to 3 heteroatoms selected from N, O, or S, deuterated C₁₋₄ alkoxy and NH₂;
each R^{c} is independently selected from H, OH, C₁₋₄ alkyl, C₃₋₇ cycloalkyl, haloC₁₋₄ alkyl, deuterated C₁₋₄ alkyl, C₁₋₄ alkoxy, haloC₁₋₄ alkoxy, deuterated C₁₋₄ alkoxy, or NH₂;
each r is independently selected from 0, 1, 2 or 3;
L₁ is selected from a bond, O, NH, S, -CD₂-, -CHD-, -CR^{L1}R^{L2}-, C₁₋₄ alkyl, - C(O)-, S(O), or S(O)₂, and the alkyl is optionally further substituted with R^{L1};
L₂ is selected from O, NH, S, -CH₂-, -CD₂-, -CHD-, -CR^{L1}R^{L2}-, -CHR^{L2}-, - CDR^{L2}-, C₂₋₄ alkyl, -C(O)-, S(O), S(O)₂, the alkyl is optionally further substituted with 1 to 3 R^{L1};
R^{A4} is selected from -CH₂-C₁₋₄ alkyl-C₃₋₆ cycloalkyl, -CH₂-CR^{A4c}=CR^{A4a}R^{A4b}, -CH₂-CH=CH-CH=CR^{A4a}R^{A4b}, L₃-R^{A4'} or is selected from -CH(C₁₋₂ alkyl)-CR^{A4c}=CR^{A4a}R^{A4b}, -C(C₁₋₂ alkyl)₂-CR^{A4c}=CR^{A4a}R^{A4b}; or R^{A4} is selected from - CH₂-C₁₋₄ alkyl-C₃₋₆ cycloalkyl, -CH₂-CF=CR^{A4a}R^{A4b}, -CH₂-C(CH₃)=CR^{A4a}R^{A4b}, - CH₂-CH=CR^{A4a}R^{A4b}, -CH₂-CH=CH-CH=CR^{A4a}R^{A4b}, L₃-R^{A4'} or is selected from - CHCH₃-CH=CR^{A4a}R^{A4b}, -C(C₁₋₂ alkyl)₂-CH=CR^{A4a}R^{A4b}, -CH₂-C(CN)=CR^{A4a}R^{A4b}; or R^{A4} is selected from -CH₂-C₁₋₄ alkyl-C₃₋₆ cycloalkyl, -CH₂-CH=CR^{A4a}R^{A4b}, - CH₂-CH=CH-CH=CR^{A4a}R^{A4b}, L₃-R^{A4'} or is selected from -CHCH₃-CH=CR^{A4a}R^{A4b};
R^{A4c} is selected from H, halogen, deuterium, CN, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
R^{A4a} and R^{A4b} together with the carbon atom to which they are attached form a 3- to 6- membered monocyclic carbocyclic ring, a C₇₋₁₀ spirocyclic carbocyclic ring, a C₇₋₁₀ bridged carbocyclic ring, a C₇₋₁₀ fused carbocyclic ring, or a 4- to 7-membered heterocyclic ring containing 1 to 3 heteroatoms selected from N, O, or S, or a 6-membered fused carbocyclic ring, and the carbocyclic ring or heterocyclic ring is optionally substituted with 1 to 3 groups selected from halogen, =O, deuterium, CN, OH, C₁₋₄ alkyl, haloC₁₋₄ alkyl, deuterated C₁₋₄ alkyl, -COC₁₋₄ alkyl, C₁₋₄ alkoxy, haloC₁₋₄ alkoxy, deuterated C₁₋₄ alkoxy and NH₂;
L₃ is selected from C₃₋₆ cycloalkyl, or -CH(C₁₋₂ alkyl)-C₁₋₄ alkyl, and the cycloalkyl is optionally substituted with 1 to 3 R^{L3};
each R^{L3} is independently selected from halogen, =O, deuterium, CN, OH, C₁₋₄ alkyl, haloC₁₋₄ alkyl, deuterated C₁₋₄ alkyl, -COC₁₋₄ alkyl, C₁₋₄ alkoxy, haloC₁₋₄ alkoxy, deuterated C₁₋₄ alkoxy and NH₂;
alternatively, two R^{L3} on adjacent ring atoms together with the atoms to which they are attached form a 5- to 7-membered carbocyclic ring or a 5- to 7-membered heterocyclic ring containing 1 to 3 heteroatoms selected from N, O, or S;
R^{A4'} is selected from H, C₃₋₆ cycloalkyl, a 5- to 10-membered heteroaryl containing 1 to 3 heteroatoms selected from N, O, or S, or a 4- to 7-membered heterocycloalkyl containing 1 to 3 heteroatoms selected from N, O, or S, and the cycloalkyl, heteroaryl and heterocycloalkyl are optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each of R^{L1} and R^{L2} is independently selected from halogen, OH, CN, amino, C₁₋₄ alkyl, C₁₋₄ alkoxy, C₃₋₆ cycloalkyl, or 4- to 6-membered heterocycloalkyl containing 1 to 3 heteroatoms selected from N, O, or S;
alternatively, R², R⁴ and L₁ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
alternatively, R⁴, R⁵ together with the atoms to which they are attached form a 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally substituted with 1 to 3 groups selected from R^{A3};
alternatively, R^{L2} and R⁹ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
alternatively, one of the combinations of R⁶ and R¹⁰, R¹⁰ and R⁷, R⁷ and R⁸, and R⁸ and R⁹ together with the atoms to which they are attached form 4- to 7-membered carbocyclic ring, 5- to 7-membered heterocycloalkyl containing 1 to 3 heteroatoms selected from N, O, S, or B or 8 membered carbocyclic ring, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{A3} is independently selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂ group substitution;
provided that the compound satisfies at least one of the following conditions:
   (1). at least one group of R¹, R² and R³ is selected from R^{A1};
   (2). at least one group of R⁴ and R⁵ is selected from C₃₋₁₀ cycloalkyl, C₂₋₆ alkenyl, or C₂₋₆ alkynyl;
   (3). R⁴, R⁵ together with the atoms to which they are attached form 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3}, and R¹ is selected from R^{A1} or -O-haloC₁₋₄ alkyl;
   (4). at least one group of R⁶, R⁷, R⁸, R⁹, and R¹⁰ is selected from R^{A2}; alternatively, one of the combinations of R⁶ and R¹⁰, R¹⁰ and R⁷, R⁷ and R⁸, and R⁸ and R⁹ together with the atoms to which they are attached form 4- to 7-membered carbocyclic ring, 5- to 7-membered heterocycloalkyl containing 1 to 3 heteroatoms selected from N, O, S, or B, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
   (5). R², R⁴, L₁ together with the atoms to which they are attached form 5-to 7-membered carbocyclic ring or 5- to 7-membered heterocyclic ring containing 1 to 3 heteroatoms selected from N, O, or S, and the carbocyclic ring or heterocyclic ring is optionally further substituted with 1 to 3 groups selected from R^{A3}, and R¹ is not heterocycloalkyl;
   (6). L₁ is selected from O, NH, S, -CD₂-, -CHD-, -CR^{L1}R^{L2}-, C₁₋₄ alkyl, - C(O)-, S(O), or S(O)₂, and the alkyl is optionally further substituted with R^{L1};
   (7). L₂ is selected from O, NH, S, -CR^{L1}R^{L2}-, -CHR^{L2}-, -CDR^{L2}-, -C(O)-, S(O), or S(O)₂;
   (8). R^{A4} is selected from -CH₂-CF=CR^{A4a}R^{A4b}, -CH₂-CH=CH-CH=CR^{A4a}R^{A4b}, L₃-R^{A4'} or is selected from -CHCH₃-CH=CR^{A4a}R^{A4b}, -CH₂-C(CN)=CR^{A4a}R^{A4b}, -CH(CH₂CH₃)-CH=CR^{A4a}R^{A4b}, -C(CH₃)₂-CH=CR^{A4a}R^{A4b}, - C(CH₂CH₃)₂-CH=CR^{A4a}R^{A4b}; or R^{A4} is selected from -CH₂-C(CH₃)=CR^{A4a}R^{A4b}, - CH₂-CH=CH-CH=CR^{A4a}R^{A4b}, L₃-R^{A4'} or is selected from -CHCH₃-CH=CR^{A4a}R^{A4b}; or R^{A4} is selected from -CH₂-CH=CR^{A4a}R^{A4b}, -CH₂-CH=CH-CH=CR^{A4a}R^{A4b}, or L₃-R^{A4'} or is selected from -CHCH₃-CH=CR^{A4a}R^{A4b};
   (9). X₁ is selected from S;
   (10). R^{L2} and R⁹ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3},

As an alternative to the first technical solution of the present invention, a compound represented by formula (I) or (I-a), a stereoisomer, deuterated substance, solvate, or pharmaceutically acceptable salt thereof is provided, 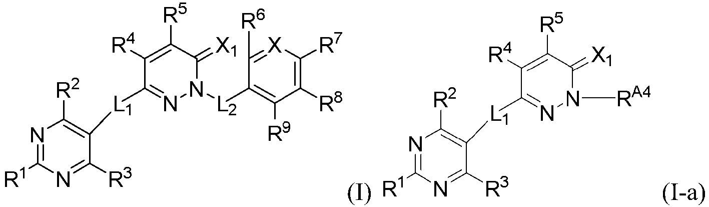 wherein
each of R¹, R² and R³ is independently selected from H, deuterium, R^{A1}, -O-haloC₁₋₄ alkyl, -NH-haloC₁₋₄ alkyl, -OC₁₋₄ alkyl, C₁₋₄ alkoxy, halogen, cyano, nitro, OH, C₁₋₄ alkyl, C₂₋₆ alkenyl, -NH-C₁₋₄ alkyl, -N(C₁₋₄ alkyl)₂, -NH-C₃₋₁₀ cycloalkyl, -NHC(O)C₁₋₄ alkyl, or 4- to 10-membered heterocycloalkyl containing 1 to 3 heteroatoms selected from N, O, and S, or selected from C₅₋₆ alkoxy, and the alkyl, alkoxy, cycloalkyl, or heterocycloalkyl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, C₃₋₁₀ cycloalkyl, 4- to 6-membered heterocycloalkyl containing 1 to 3 heteroatoms selected from N, O, or S, phenyl, and NH₂, provided that R¹, R² and R³ are not simultaneously selected from H;
R^{A1} is selected from haloC₂₋₆ alkenyl, C₂₋₆ alkynyl, 5- to 10-membered heteroaryl containing 1 to 3 heteroatoms selected from N, O, or S, -C₁₋₄ alkyl-Ra, - C₁₋₄ alkyl-ORa, -C₁₋₄ alkyl-NR^{b}R^{a}, -NR^{b}-S(O)₂-R^{a}, or -NR^{b}-S(O)₂-NR^{b}R^{a}, or R^{A1} is selected from -O-NR^{b}R^{a} or -NH-OR^{b}, and the alkynyl, heteroaryl, or alkyl is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{a} is selected from CN, haloC₁₋₆ alkyl, C₃₋₁₀ cycloalkyl, 4- to 10-membered heterocycloalkyl containing 1 to 3 heteroatoms selected from N, O, or S, 6- to 10-membered aryl, 5- to 10-membered heteroaryl containing 1 to 3 heteroatoms selected from N, O, or S, or -C(O)-R^{a1}, and the alkyl, cycloalkyl, heterocycloalkyl, aryl, or heteroaryl is optionally further substituted with 1 to 3 groups selected from halogen, =O, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{b} is selected from H, deuterium, C₁₋₄ alkyl, C₃₋₆ cycloalkyl, haloC₁₋₄ alkyl, or deuterated C₁₋₄ alkyl;
R^{a1} is selected from OH, NH₂, -NHC₁₋₄ alkyl, -N(C₁₋₄ alkyl)₂, -NHC₃₋₁₀ cycloalkyl, C₁₋₄ alkoxy, C₁₋₆ alkyl, C₃₋₁₀ cycloalkyl, 6- to 10-membered aryl, 5- to 10-membered heteroaryl containing 1 to 3 heteroatoms selected from N, O, or S;
X is selected from CR¹⁰ or N;
X₁ is selected from O or S;
each of R⁴ and R⁵ is independently selected from H, deuterium, C₃₋₁₀ cycloalkyl, C₂₋₆ alkenyl, C₂₋₆ alkynyl, halogen, amino,-NH-C₁₋₄ alkyl, -N(C₁₋₄ alkyl)₂, C₁₋₄ alkyl, haloC₁₋₄ alkyl, 4- to 10-membered heterocycloalkyl containing 1 to 3 heteroatoms selected from N, O, or S, 5- to 10-membered heteroaryl containing 1 to 3 heteroatoms selected from N, O, or S, or 6- to 10-membered aryl;
each of R⁶, R⁷, R⁸, R⁹, and R¹⁰ is independently selected from H, deuterium, R^{A2}, halogen, OH, CN, amino, C₁₋₄ alkyl, C₂₋₆ alkenyl, C₂₋₆ alkynyl, C₁₋₄ alkoxy, haloC₁₋₄ alkyl, -NH-C₁₋₄ alkyl, -N(C₁₋₄ alkyl)₂, -S(O)R^{c}, -S(O)²R^{c}, -C(O)R^{c}, - C(O)OR^{c}, -C(O)N(R^{c})₂, or -OC(O)R^{c}, and the alkyl, alkenyl, alkynyl, or alkoxy is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
or as an alternative, R⁷ and R⁸ together with the atoms to which they are attached form 4- to 7-membered carbocyclic ring or 5- to 7-membered heterocycloalkyl containing 1 to 3 heteroatoms selected from N, O, S, or B, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
R^{A2} is selected from -(CH₂)ᵣ-(4- to 10-membered heterocycloalkyl containing 1 to 3 heteroatoms selected from N, O, or S), or -(CH₂)ᵣ-(5- to 10-membered heteroaryl containing 1 to 3 heteroatoms selected from N, O, or S), -P(O)(R^{c})₂, - Si(R^{c})₃, -SF₅, -S(O)(=NH)R^{c}, and the heterocycloalkyl or heterocycloaryl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{c} is independently selected from H, OH, C₁₋₄ alkyl, C₃₋₇ cycloalkyl, haloC₁₋₄ alkyl, deuterated C₁₋₄ alkyl, C₁₋₄ alkoxy, haloC₁₋₄ alkoxy, deuterated C₁₋₄ alkoxy, or NH₂;
each r is independently selected from 0, 1, 2 or 3;
L₁ is selected from a bond, O, NH, S, -CD₂-, -CHD-, -CR^{L1}R^{L2}-, C₁₋₄ alkyl, - C(O)-, S(O), or S(O)₂, and the alkyl is optionally further substituted with R^{L1};
L₂ is selected from O, NH, S, -CH₂-, -CD₂-, -CHD-, -CR^{L1}R^{L2}-, -CHR^{L2}-, - CDR^{L2}-, C₂₋₄ alkyl, -C(O)-, S(O), S(O)₂, the alkyl is optionally further substituted with 1 to 3 R^{L1};
R^{A4} is selected from -CH₂-C₁₋₄ alkyl-C₃₋₆ cycloalkyl, -CH₂-CH=CR^{A4a}R^{A4b}, L₃-R^{A4'} or is selected from -CH(C₁₋₂ alkyl)-CR^{A4c}=CR^{A4a}R^{A4b}, -C(C₁₋₂ alkyl)₂-CR^{A4c}=CR^{A4a}R^{A4b};
R^{A4a}, R^{A4b} and the carbon atoms to which they are attached form 3- to 6-membered carbocyclic ring;
L₃ is selected from C₃₋₆ cycloalkyl, or -CH(C₁₋₂ alkyl)-C₁₋₄ alkyl;
R^{A4'} is selected from C₃₋₆ cycloalkyl, or a 5- to 10-membered heteroaryl containing 1 to 3 heteroatoms selected from N, O, or S, and the cycloalkyl or heteroaryl is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each of R^{L1} and R^{L2} is independently selected from halogen, OH, CN, amino, C₁₋₄ alkyl, C₁₋₄ alkoxy, C₃₋₆ cycloalkyl, or 4- to 6-membered heterocycloalkyl containing 1 to 3 heteroatoms selected from N, O, or S;
alternatively, R², R⁴ and L₁ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
alternatively, R⁴, R⁵ together with the atoms to which they are attached form a 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally substituted with 1 to 3 groups selected from R^{A3};
alternatively, R^{L1} and R⁹ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
each R^{A3} is independently selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂ group substitution;
provided that the compound satisfies one of the following conditions:
   (1). at least one group of R¹, R² and R³ is selected from R^{A1};
   (2). at least one group of R⁴ and R⁵ is selected from C₃₋₁₀ cycloalkyl, C₂₋₆ alkenyl, or C₂₋₆ alkynyl;
   (3). R⁴, R⁵ together with the atoms to which they are attached form 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3}, and R¹ is selected from R^{A1} or -O-haloC₁₋₄ alkyl;
   (4). at least one group of R⁶, R⁷, R⁸, R⁹, and R¹⁰ is selected from R^{A2};
   (5). R², R⁴, L₁ together with the atoms to which they are attached form 5-to 7-membered carbocyclic ring or 5- to 7-membered heterocyclic ring containing 1 to 3 heteroatoms selected from N, O, or S, and the carbocyclic ring or heterocyclic ring is optionally further substituted with 1 to 3 groups selected from R^{A3}, and R¹ is not heterocycloalkyl;
   (6). L₁ is selected from O, NH, S, -CD₂-, -CHD-, -CR^{L1}R^{L2}-, C₁₋₄ alkyl, - C(O)-, S(O), or S(O)₂, and the alkyl is optionally further substituted with R^{L1};
   (7). L₂ is selected from O, NH, S, -CR^{L1}R^{L2}-, -CHR^{L2}-, -CDR^{L2}-, -C(O)-, C₂₋₄ alkyl, S(O), or S(O)₂, and the alkyl is substituted with 1 to 3 R^{L1};
   (8). R^{A4} is selected from -CH₂-CH=CR^{A4a}R^{A4b} or L₃-R^{A4'} or is selected from -CH(C₁₋₂ alkyl)-CR^{A4c}=CR^{A4a}R^{A4b}, or -C(C₁₋₂ alkyl)₂-CR^{A4c}=CR^{A4a}R^{A4b};
   (9). X₁ is selected from S.

A second technical solution of the present invention provides a compound represented by formula (I-b), a stereoisomer, deuterated substance, solvate, deuterated substance, or pharmaceutically acceptable salt thereof, 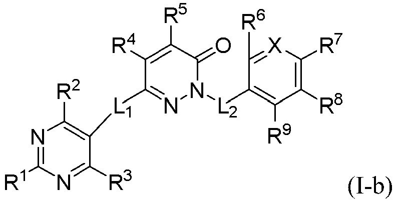 wherein
each of R¹, R² and R³ is independently selected from H, deuterium, R^{A1}, -O-haloC₁₋₄ alkyl, -NH-haloC₁₋₄ alkyl, -OC₁₋₄ alkyl, C₁₋₄ alkoxy, halogen, cyano, nitro, OH, C₁₋₄ alkyl, C₂₋₆ alkenyl, -NH-C₁₋₄ alkyl, -N(C₁₋₄ alkyl)₂, -NH-C₃₋₁₀ cycloalkyl, -NHC(O)C₁₋₄ alkyl, or 4- to 10-membered heterocycloalkyl containing 1 to 3 heteroatoms selected from N, O, and S, or selected from C₅₋₆ alkoxy, and the alkyl, alkoxy, cycloalkyl, or heterocycloalkyl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, C₃₋₁₀ cycloalkyl, 4- to 6-membered heterocycloalkyl containing 1 to 3 heteroatoms selected from N, O, or S, phenyl, and NH₂, provided that R¹, R² and R³ are not simultaneously selected from H;
R^{A1} is selected from haloC₂₋₆ alkenyl, C₂₋₆ alkynyl, 5- to 10-membered heteroaryl containing 1 to 3 heteroatoms selected from N, O, or S, -C₁₋₄ alkyl-R^{a}, - C₁₋₄ alkyl-OR^{a}, -C₁₋₄ alkyl-NR^{b}R^{a}, -NR^{b}-S(O)₂-R^{a}, or -NR^{b}-S(O)₂-NR^{b}R^{a}, and the alkynyl, heteroaryl, or alkyl is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{a} is selected from CN, haloC₁₋₆ alkyl, C₃₋₁₀ cycloalkyl, 4- to 10-membered heterocycloalkyl containing 1 to 3 heteroatoms selected from N, O, or S, 6- to 10-membered aryl, 5- to 10-membered heteroaryl containing 1 to 3 heteroatoms selected from N, O, or S, or -C(O)-R^{a1}, and the alkyl, cycloalkyl, heterocycloalkyl, aryl, or heteroaryl is optionally further substituted with 1 to 3 groups selected from halogen, =O, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{b} is selected from H, deuterium, C₁₋₄ alkyl, C₃₋₆ cycloalkyl, haloC₁₋₄ alkyl, or deuterated C₁₋₄ alkyl;
R^{a1} is selected from OH, NH₂, -NHC₁₋₄ alkyl, -N(C₁₋₄ alkyl)₂, -NHC₃₋₁₀ cycloalkyl, C₁₋₄ alkoxy, C₁₋₆ alkyl, C₃₋₁₀ cycloalkyl, 6- to 10-membered aryl, 5- to 10-membered heteroaryl containing 1 to 3 heteroatoms selected from N, O, or S;
X is selected from CR¹⁰ or N;
each of R⁴ and R⁵ is independently selected from H, deuterium, C₃₋₁₀ cycloalkyl, C₂₋₆ alkenyl, C₂₋₆ alkynyl, halogen, amino,-NH-C₁₋₄ alkyl, -N(C₁₋₄ alkyl)₂, C₁₋₄ alkyl, haloC₁₋₄ alkyl, 4- to 10-membered heterocycloalkyl containing 1 to 3 heteroatoms selected from N, O, or S, 5- to 10-membered heteroaryl containing 1 to 3 heteroatoms selected from N, O, or S, or 6- to 10-membered aryl;
each of R⁶, R⁷, R⁸, R⁹, and R¹⁰ is independently selected from H, deuterium, R^{A2}, halogen, OH, CN, amino, C₁₋₄ alkyl, C₂₋₆ alkenyl, C₂₋₆ alkynyl, C₁₋₄ alkoxy, haloC₁₋₄ alkyl, -NH-C₁₋₄ alkyl, -N(C₁₋₄ alkyl)₂, -S(O)R^{c}, -S(O)²R^{c}, -C(O)R^{c}, - C(O)OR^{c}, -C(O)N(R^{c})₂, or -OC(O)R^{c}, and the alkyl, alkenyl, alkynyl, or alkoxy is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
or as an alternative, R⁷ and R⁸ together with the atoms to which they are attached form 4- to 7-membered carbocyclic ring, 5- to 7-membered heterocycloalkyl containing 1 to 3 heteroatoms selected from N, O, S, or B or 8 membered carbocyclic ring or 5- to 7-membered heterocycloalkyl containing 1 Si atom, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
R^{A2} is selected from -(CH₂)ᵣ-(4- to 10-membered heterocycloalkyl containing 1 to 3 heteroatoms selected from N, O, or S), or -(CH₂)ᵣ-(5- to 10-membered heteroaryl containing 1 to 3 heteroatoms selected from N, O, or S), -P(O)(R^{c})₂, - Si(R^{c})₃, -SF₅, -S(O)(=NH)R^{c}, and the heterocycloalkyl or heterocycloaryl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{c} is independently selected from H, OH, C₁₋₄ alkyl, C₃₋₇ cycloalkyl, haloC₁₋₄ alkyl, deuterated C₁₋₄ alkyl, C₁₋₄ alkoxy, haloC₁₋₄ alkoxy, deuterated C₁₋₄ alkoxy, or NH₂;
each r is independently selected from 0, 1, 2 or 3;
L₁ is selected from a bond, O, NH, S, -CD₂-, -CHD-, -CR^{L1}R^{L2}-, C₁₋₄ alkyl, - C(O)-, S(O), or S(O)₂, and the alkyl is optionally further substituted with R^{L1};
L₂ is selected from O, NH, S, -CH₂-, -CD₂-, -CHD-, -CR^{L1}R^{L2}-, -CHR^{L2}-, - CDR^{L2}-, C₂₋₄ alkyl, -C(O)-, S(O), S(O)₂, the alkyl is optionally further substituted with 1 to 3 R^{L1};
each of R^{L1} and R^{L2} is independently selected from halogen, OH, CN, amino, C₁₋₄ alkyl, C₁₋₄ alkoxy, C₃₋₆ cycloalkyl, or 4- to 6-membered heterocycloalkyl containing 1 to 3 heteroatoms selected from N, O, or S;
alternatively, R², R⁴ and L₁ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
alternatively, R⁴, R⁵ together with the atoms to which they are attached form a 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally substituted with 1 to 3 groups selected from R^{A3};
alternatively, R^{L1} and R⁹ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
each R^{A3} is independently selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂ group substitution;
provided that the compound satisfies one of the following conditions:
   (1). at least one group of R¹, R² and R³ is selected from R^{A1};
   (2). at least one group of R⁴ and R⁵ is selected from C₃₋₁₀ cycloalkyl, C₂₋₆ alkenyl, or C₂₋₆ alkynyl;
   (3). R⁴, R⁵ together with the atoms to which they are attached form 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3}, and R¹ is selected from R^{A1} or -O-haloC₁₋₄ alkyl;
   (4). at least one group of R⁶, R⁷, R⁸, R⁹, and R¹⁰ is selected from R^{A2};
   (5). R², R⁴, L₁ together with the atoms to which they are attached form 5-to 7-membered carbocyclic ring or 5- to 7-membered heterocyclic ring containing 1 to 3 heteroatoms selected from N, O, or S, and the carbocyclic ring or heterocyclic ring is optionally further substituted with 1 to 3 groups selected from R^{A3}, and R¹ is not heterocycloalkyl;
   (6). L₁ is selected from O, NH, S, -CD₂-, -CHD-, -CR^{L1}R^{L2}-, C₁₋₄ alkyl, - C(O)-, S(O), or S(O)₂, and the alkyl is optionally further substituted with R^{L1};
   (7). L₂ is selected from O, NH, S, -CR^{L1}R^{L2}-, -CHR^{L2}-, -CDR^{L2}-, -C(O)-, C₂₋₄ alkyl, S(O), or S(O)₂, and the alkyl is substituted with 1 to 3 R^{L1};
other group definitions are consistent with any of the above technical solutions.

A third technical solution of the present invention is a compound of the present invention, and a stereoisomer, deuterated substance, solvate, or pharmaceutically acceptable salt thereof, having the structure of formula (II): 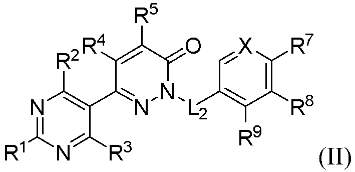
L₂ is selected from -CH₂-, -CR^{L1}R^{L2}-, -CHR^{L2}-, -CDR^{L2}-, or -C(O)-;
X is selected from CR¹⁰ or N;
each of R⁴ and R⁵ is independently selected from H, deuterium, C₃₋₇ cycloalkyl, C₂₋₄ alkenyl, C₂₋₄ alkynyl, F, Cl, amino,-NH-C₁₋₂ alkyl, -N(C₁₋₂ alkyl)₂, C₁₋₂ alkyl, or 4- to 7-membered heterocycloalkyl containing 1 to 3 heteroatoms selected from N, O, or S;
each of R⁷ and R⁸ is independently selected from H, deuterium, R^{A2}, F, Cl, OH, CN, amino, C₁₋₄ alkyl, C₂₋₄ alkenyl, C₂₋₄ alkynyl, C₁₋₄ alkoxy, haloC₁₋₄ alkyl, - NH-C₁₋₂ alkyl, -N(C₁₋₂ alkyl)₂, -S(O)R^{c}, -S(O)²R^{c}, -C(O)R^{c}, -C(O)OR^{c}, - C(O)N(R^{c})₂, or -OC(O)R^{c};
each of R⁹ and R¹⁰ is independently selected from H, deuterium, F, Cl, OH, CN, amino, or C₁₋₂ alkyl;
R^{A2} is selected from -(CH₂)ᵣ-(4- to 6-membered monocyclic heterocycloalkyl containing 1 to 3 heteroatoms selected from N, O, or S), -(CH₂)ᵣ-(7- to 10-membered spirocyclic heterocycloalkyl containing 1 to 3 heteroatoms selected from N, O, or S), -(CH₂)ᵣ-(5- to 6-membered monocyclic heteroaryl containing 1 to 3 heteroatoms selected from N, O, or S), -(CH₂)ᵣ-(8- to 10-membered bicyclic heteroaryl containing 1 to 3 heteroatoms selected from N, O, or S), -P(O)(R^{c})₂, - Si(R^{c})₃, -SF₅, or -S(O)(=NH)R^{c}, and the heterocycloalkyl or heteroaryl is optionally further substituted with 1 to 3 groups selected from halogen, D, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
alternatively, one of the combinations of R⁷ and R⁸, and R⁸ and R⁹ together with the atoms to which they are attached form 4- to 7-membered carbocyclic ring, 5- to 7-membered heterocycloalkyl containing 1 to 3 heteroatoms selected from N, O, S, or B or 8 membered carbocyclic ring or 5- to 7-membered heterocycloalkyl containing 1 Si atom, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
provided that the compound satisfies at least one of the following conditions:
   (1). at least one group of R¹, R² and R³ is selected from R^{A1};
   (2). at least one group of R⁴ and R⁵ is selected from C₃₋₇ cycloalkyl, C₂₋₄ alkenyl, or C₂₋₄ alkynyl;
   (3). at least one group of R⁷ and R⁸ is selected from R^{A2};
   (4). L₂ is selected from -CR^{L1}R^{L2}-, -CHR^{L2}-, -CDR^{L2}-, or -C(O)-;
   (5). one of the combinations of R⁷ and R⁸, and R⁸ and R⁹ together with the atoms to which they are attached form 4- to 7-membered carbocyclic ring, 5- to 7-membered heterocycloalkyl containing 1 to 3 heteroatoms selected from N, O, S, or B or 8 membered carbocyclic ring, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
Other group definitions are consistent with any of the above technical solutions.

As an alternative to the third technical solution of the present invention, a compound of the present invention, and a stereoisomer, deuterated substance, solvate, or pharmaceutically acceptable salt thereof, has the structure of formula (II): 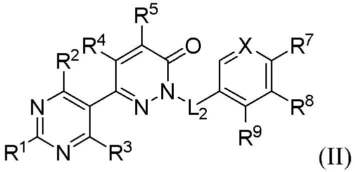
L₂ is selected from -CH₂-, -CR^{L1}R^{L2}-, -CHR^{L2}- , -CDR^{L2}-, or -C(O)-;
X is selected from CR¹⁰ or N;
each of R⁴ and R⁵ is independently selected from H, deuterium, C₃₋₇ cycloalkyl, C₂₋₄ alkenyl, C₂₋₄ alkynyl, F, Cl, amino,-NH-C₁₋₂ alkyl, -N(C₁₋₂ alkyl)₂, C₁₋₂ alkyl, or 4- to 7-membered heterocycloalkyl containing 1 to 3 heteroatoms selected from N, O, or S;
each of R⁷ and R⁸ is independently selected from H, deuterium, R^{A2}, F, Cl, OH, CN, amino, C₁₋₄ alkyl, C₂₋₄ alkenyl, C₂₋₄ alkynyl, C₁₋₄ alkoxy, haloC₁₋₄ alkyl, - NH-C₁₋₂ alkyl, -N(C₁₋₂ alkyl)₂, -S(O)R^{c}, -S(O)²R^{c}, -C(O)R^{c}, -C(O)OR^{c}, - C(O)N(R^{c})₂, or -OC(O)R^{c};
or as an alternative, R⁷ and R⁸ together with the atoms to which they are attached form 4- to 7-membered carbocyclic ring or 5- to 7-membered heterocycloalkyl containing 1 to 3 heteroatoms selected from N, O, S, or B or 8 membered carbocyclic ring, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each of R⁹ and R¹⁰ is independently selected from H, deuterium, F, Cl, OH, CN, amino, or C₁₋₂ alkyl;
R^{A2} is selected from -(CH₂)ᵣ-(4- to 6-membered monocyclic heterocycloalkyl containing 1 to 3 heteroatoms selected from N, O, or S), -(CH₂)ᵣ-(7- to 10-membered spirocyclic heterocycloalkyl containing 1 to 3 heteroatoms selected from N, O, or S), -(CH₂)ᵣ-(5- to 6-membered monocyclic heteroaryl containing 1 to 3 heteroatoms selected from N, O, or S), -(CH₂)ᵣ-(8- to 10-membered bicyclic heteroaryl containing 1 to 3 heteroatoms selected from N, O, or S), -P(O)(R^{c})₂, - Si(R^{c})₃, -SF₅, or -S(O)(=NH)R^{c}, and the heterocycloalkyl or heterocycloaryl is optionally further substituted with 1 to 3 groups selected from halogen, D, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
provided that the compound satisfies one of the following conditions:
   (1). at least one group of R¹, R² and R³ is selected from R^{A1};
   (2). at least one group of R⁴ and R⁵ is selected from C₃₋₇ cycloalkyl, C₂₋₄ alkenyl, or C₂₋₄ alkynyl;
   (3). at least one group of R⁷ and R⁸ is selected from R^{A2};
   (4). L₂ is selected from -CR^{L1}R^{L2}-, -CHR^{L2}-, -CDR^{L2}-, or -C(O)-;
other group definitions are consistent with any of the above technical solutions.

A fourth technical solution of the present invention relates to a compound of formula (I), (II), or (I-b), a stereoisomer, a deuterated substance, a solvate, or a pharmaceutically acceptable salt or a eutectic crystal thereof, wherein R², R⁴, L₁ together with the atoms to which they are attached form 6- to 7- membered cycloalkyl, phenyl, and the cycloalkyl is optionally further substituted with 1 to 3 groups selected from R^{A3}, and R¹ is not heterocycloalkyl; other group definitions are consistent with any of the above technical solutions.

A fifth technical solution of the present invention is a compound of the present invention, a stereoisomer, a deuterated substance, a solvate, or a pharmaceutically acceptable salt or a eutectic crystal thereof, wherein R⁴, R⁵ together with the atoms to which they are attached form phenyl, 5- to 6-membered cycloalkyl, or 5- to 6-membered heteroaryl containing 1 to 3 heteroatoms selected from N, O, or S, and the cycloalkyl is optionally substituted with 1 to 3 groups selected from R^{A3}, and R¹ is selected from R^{A1} or -O-haloC₁₋₄ alkyl; other group definitions are consistent with any of the above technical solutions.

A sixth technical solution of the present invention is a compound of the present invention, a stereoisomer, a deuterated substance, a solvate, or a pharmaceutically acceptable salt or a eutectic crystal thereof, wherein R^{L1} and R⁹ together with the atoms to which they are attached form 5- to 6-membered cycloalkyl, and the cycloalkyl is optionally further substituted with 1 to 3 groups selected from R^{A3}; other group definitions are consistent with any of the above technical solutions.

A seventh technical solution of the present invention is a compound of the present invention, a stereoisomer, a deuterated substance, a solvate, or a pharmaceutically acceptable salt or a eutectic crystal thereof, wherein each R^{A3} is independently selected from =O, halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂; other group definitions are consistent with any of the above technical solutions.

An eighth technical solution of the present invention is a compound of the present invention, a stereoisomer, a deuterated substance, a solvate, or a pharmaceutically acceptable salt or a eutectic crystal thereof, wherein,
R², R⁴, L₁ together with the atoms to which they are attached form 6- to 7-membered cycloalkyl, phenyl or 5- to 7-membered cycloalkyl, and the cycloalkyl is optionally further substituted with 1 to 3 groups selected from R^{A3}, and R¹ is not heterocycloalkyl; or
R⁴, R⁵ together with the atoms to which they are attached form phenyl, 5- to 6-membered cycloalkyl, or 5- to 6-membered heteroaryl containing 1 to 3 heteroatoms selected from N, O, or S, and the cycloalkyl is optionally substituted with 1-3 groups selected from R^{A3}, and R¹ is selected from R^{A1} or -O-haloC₁₋₄ alkyl; or
R^{L1} and R⁹ together with the atoms to which they are attached form 5- to 6-membered cycloalkyl, and the cycloalkyl is optionally further substituted with 1 to 3 groups selected from R^{A3};
each R^{A3} is independently selected from =O, halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
other group definitions are consistent with any of the above technical solutions.

A ninth technical solution of the present invention is a compound of the present invention, a stereoisomer, a deuterated substance, a solvate, or a pharmaceutically acceptable salt or a eutectic crystal thereof,
R¹ is selected from R^{A1} or -O-haloC₁₋₄ alkyl, and the alkyl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each of R² and R³ is independently selected from H, deuterium, C₂₋₄ alkenyl, C₂₋₄ alkynyl, halogen, cyano, nitro, OH, C₁₋₄ alkyl, haloC₁₋₄ alkyl, or deuterated C₁₋₄ alkyl;
R^{A1} is selected from haloC₂₋₄ alkenyl, C₂₋₄ alkynyl, -O-(CH₂)ᵣ-R^{a}, C₃₋₆ monocyclic cycloalkyl, 5- to 6-membered monocyclic heteroaryl containing 1 to 3 heteroatoms selected from N, O, or S, 8- to 10-membered bicyclic heteroaryl containing 1 to 3 heteroatoms selected from N, O, or S, -C₁₋₂ alkyl-R^{a}, -C₁₋₂ alkyl-OR^{a}, -C₁₋₂ alkyl-NR^{b}R^{a}, -NR^{b}-S(O)₂-R^{a}, -NR^{b}-S(O)₂-NR^{b}R^{a}, -O-NR^{b}R^{a}, -NH-OR^{b}, or -Se-(CH₂)ᵣ-Ra, and the alkynyl, heteroaryl, or alkyl is optionally further substituted with 1 to 3 groups selected from halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
r is selected from 0 or 1;
each R^{a} is selected from CN, haloC₁₋₄ alkyl, C₃₋₆ monocyclic cycloalkyl, C₇₋₁₀ spirocyclic cycloalkyl, C₄₋₈ bridged cycloalkyl, C₄₋₉ fused cycloalkyl, 4- to 6-membered monocyclic heterocycloalkyl containing 1 to 3 heteroatoms selected from N, O, or S, 7- to 10-membered spirocyclic heterocycloalkyl containing 1 to 3 heteroatoms selected from N, O, or S, 4- to 8-membered bridged heterocycloalkyl containing 1 to 3 heteroatoms selected from N, O, or S, 4- to 6-membered fused heterocycloalkyl containing 1 to 3 heteroatoms selected from N, O, or S, phenyl, 8-to 10- membered aryl, 5- to 6-membered monocyclic heteroaryl containing 1 to 3 heteroatoms selected from N, O, or S, 8- to 10-membered bicyclic heteroaryl containing 1 to 3 heteroatoms selected from N, O, or S, or -C(O)-R^{a1}, and the alkyl, cycloalkyl, heterocycloalkyl, aryl, or heteroaryl is optionally further substituted with 1 to 3 groups selected from halogen, =O, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
each R^{b} is selected from H, deuterium, C₁₋₂ alkyl, or C₃₋₄ cycloalkyl;
other group definitions are consistent with any of the above technical solutions.

A tenth technical solution of the present invention is a compound of the present invention, a stereoisomer, a deuterated substance, a solvate, or a pharmaceutically acceptable salt or a eutectic crystal thereof,
R¹ is selected from R^{A1} or -O-haloC₁₋₄ alkyl, and the alkyl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each of R² and R³ is independently selected from H, deuterium, C₂₋₄ alkenyl, C₂₋₄ alkynyl, halogen, cyano, nitro, OH, C₁₋₄ alkyl, haloC₁₋₄ alkyl, or deuterated C₁₋₄ alkyl;
R^{A1} is selected from haloC₂₋₄ alkenyl, C₂₋₄ alkynyl, 5- to 6-membered monocyclic heteroaryl containing 1 to 3 heteroatoms selected from N, O, or S, 8-to 10-membered bicyclic heteroaryl containing 1 to 3 heteroatoms selected from N, O, or S, -C₁₋₂ alkyl-R^{a}, -C₁₋₂ alkyl-OR^{a}, -C₁₋₂ alkyl-NR^{b}R^{a}, -NR^{b}-S(O)₂-R^{a}, -NR^{b}-S(O)₂-NR^{b}R^{a}, or selected from -O-NR^{b}R^{a}, -NH-OR^{b}, or selected from -Se-(CH₂)ᵣ-Ra, and the alkynyl, heteroaryl, or alkyl is optionally further substituted with 1 to 3 groups selected from halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
each R^{a} is selected from CN, haloC₁₋₄ alkyl, C₃₋₆ monocyclic cycloalkyl, C₇₋₁₀ spirocyclic cycloalkyl, C₄₋₈ bridged cycloalkyl, C₄₋₉ fused cycloalkyl, 4- to 6-membered monocyclic heterocycloalkyl containing 1 to 3 heteroatoms selected from N, O, or S, 7- to 10-membered spirocyclic heterocycloalkyl containing 1 to 3 heteroatoms selected from N, O, or S, 4- to 8-membered bridged heterocycloalkyl containing 1 to 3 heteroatoms selected from N, O, or S, 4- to 6-membered fused heterocycloalkyl containing 1 to 3 heteroatoms selected from N, O, or S, phenyl, 8-to 10- membered aryl, 5- to 6-membered monocyclic heteroaryl containing 1 to 3 heteroatoms selected from N, O, or S, 8- to 10-membered bicyclic heteroaryl containing 1 to 3 heteroatoms selected from N, O, or S, or -C(O)-R^{a1}, and the alkyl, cycloalkyl, heterocycloalkyl, aryl, or heteroaryl is optionally further substituted with 1 to 3 groups selected from halogen, =O, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
each R^{b} is selected from H, deuterium, C₁₋₂ alkyl, or C₃₋₄ cycloalkyl;
other group definitions are consistent with any of the above technical solutions.

An eleventh technical solution of the present invention is a compound of the present invention, and a stereoisomer, deuterated substance, solvate, or pharmaceutically acceptable salt thereof, having the structure of formula (III): 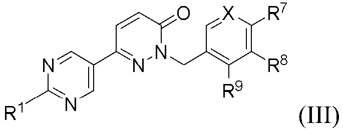
X is selected from CR¹⁰ or N;
each of R⁹ and R¹⁰ is independently selected from H, deuterium, F, Cl, OH, CN, amino, or C₁₋₂ alkyl;
provided that the compound satisfies one of the following conditions:
   (1). at least one group of R¹, R² and R³ is selected from R^{A1};
   (2). at least one group of R⁷ and R⁸ is selected from R^{A2};
   (3). one of the combinations of R⁷ and R⁸, and R⁸ and R⁹ together with the atoms to which they are attached form 4- to 7-membered carbocyclic ring, 5- to 7-membered heterocycloalkyl containing 1 to 3 heteroatoms selected from N, O, S, or B or 8 membered carbocyclic ring or 5- to 7-membered heterocycloalkyl containing 1 Si atom, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
other group definitions are consistent with any of the above technical solutions.

A twelfth technical solution of the present invention is a compound of the present invention, and a stereoisomer, deuterated substance, solvate, or pharmaceutically acceptable salt thereof, having the structure of formula (III): 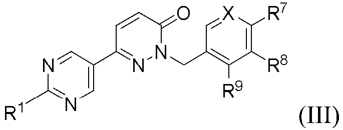
X is selected from CR¹⁰ or N;
each of R⁹ and R¹⁰ is independently selected from H, deuterium, F, Cl, OH, CN, amino, or C₁₋₂ alkyl;
provided that the compound satisfies one of the following conditions:
   (1). at least one group of R¹, R² and R³ is selected from R^{A1};
   (2). at least one group of R⁷ and R⁸ is selected from R^{A2};
other group definitions are consistent with any of the above technical solutions.

A thirteenth technical solution of the present invention is a compound of the present invention, and a stereoisomer, deuterated substance, solvate, or pharmaceutically acceptable salt thereof, having the structure of formula (IV) or (V): 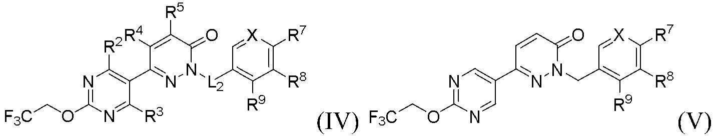
X is selected from CR¹⁰ or N;
each of R⁹ and R¹⁰ is independently selected from H, deuterium, F, Cl, OH, CN, amino, or C₁₋₂ alkyl;
L₂ is selected from -CH₂-, -CD₂-, -CHD-, -CR^{L1}R^{L2}-, -CHR^{L2}- , -CDR^{L2}-, C₂₋₄ alkyl, or -C(O)-, and the alkyl is optionally further substituted with 1 to 3 R^{L1};
provided that the compound satisfies one of the following conditions:
   (1). at least one group of R⁷ and R⁸ is selected from R^{A2};
   (2). at least one group of R⁴ and R⁵ is selected from C₃₋₁₀ cycloalkyl, C₂₋₆ alkenyl, or C₂₋₆ alkynyl;
   (3). R⁴, R⁵ together with the atoms to which they are attached form a 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
   (4). R², R⁴ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
   (5). L₂ is selected from -CR^{L1}R^{L2}-, -CHR^{L2}-, -CDR^{L2}-, -C(O)-, or C₂₋₄ alkyl, and the alkyl is substituted with 1 to 3 R^{L1};
   (6). one of the combinations of R⁷ and R⁸, and R⁸ and R⁹ together with the atoms to which they are attached form 4- to 7-membered carbocyclic ring, 5- to 7-membered heterocycloalkyl containing 1 to 3 heteroatoms selected from N, O, S, or B or 8 membered carbocyclic ring or 5- to 7-membered heterocycloalkyl containing 1 Si atom, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
other group definitions are consistent with any of the above technical solutions.

A fourteenth technical solution of the present invention is a compound of the present invention, and a stereoisomer, deuterated substance, solvate, or pharmaceutically acceptable salt thereof, having the structure of formula (IV) or (V): 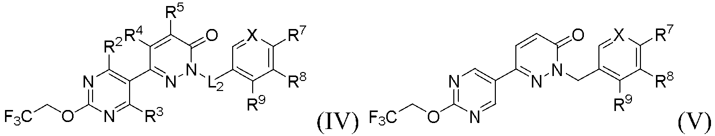
X is selected from CR¹⁰ or N;
each of R⁹ and R¹⁰ is independently selected from H, deuterium, F, Cl, OH, CN, amino, or C₁₋₂ alkyl;
L₂ is selected from -CH₂-, -CD₂-, -CHD-, -CR^{L1}R^{L2}-, -CHR^{L2}- , -CDR^{L2}-, C₂₋₄ alkyl, or -C(O)-, and the alkyl is optionally further substituted with 1 to 3 R^{L1};
provided that the compound satisfies one of the following conditions:
   (1). at least one group of R⁷ and R⁸ is selected from R^{A2};
   (2). at least one group of R⁴ and R⁵ is selected from C₃₋₁₀ cycloalkyl, C₂₋₆ alkenyl, or C₂₋₆ alkynyl;
   (3). R⁴, R⁵ together with the atoms to which they are attached form a 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
   (4). R², R⁴ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
   (5). L₂ is selected from -CR^{L1}R^{L2}-, -CHR^{L2}- , -CDR^{L2}-, -C(O)-, or C₂₋₄ alkyl, and the alkyl is substituted with 1 to 3 R^{L1};
other group definitions are consistent with any of the above technical solutions.

A fifteenth technical solution of the present invention is a compound of the present invention, and a stereoisomer, deuterated substance, solvate, or pharmaceutically acceptable salt thereof, having the structure of formula (VI), (VII), (VIII), or (IX): 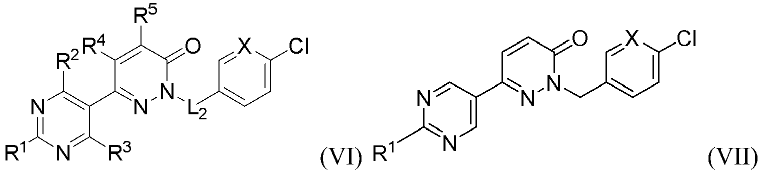 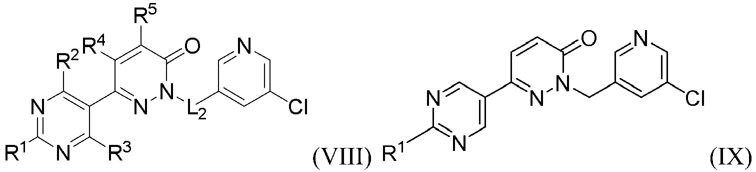
X is selected from CR¹⁰ or N;
L₂ is selected from -CH₂-, -CD₂-, -CHD-, -CR^{L1}R^{L2}-, -CHR^{L2}- , -CDR^{L2}-, C₂₋₄ alkyl, or -C(O)-, and the alkyl is optionally further substituted with 1 to 3 R^{L1};
each of R⁹ and R¹⁰ is independently selected from H, deuterium, OH, CN, amino, C₁₋₄ alkyl, or haloC₁₋₄ alkyl;
R¹ is selected from R^{A1};
each of R² and R³ is independently selected from H, deuterium, C₂₋₄ alkenyl, C₂₋₄ alkynyl, halogen, cyano, nitro, OH, C₁₋₄ alkyl, haloC₁₋₄ alkyl, or deuterated C₁₋₄ alkyl;
other group definitions are consistent with any of the above technical solutions.

A sixteenth technical solution of the present invention relates to a compound of formula (I-a) of the present invention, a stereoisomer, a deuterated substance, a solvate, or a pharmaceutically acceptable salt or a eutectic crystal thereof, wherein
R^{A4} is selected from -CH₂-CF=CR^{A4a}R^{A4b}, -CH₂-CH=CH-CH=CR^{A4a}R^{A4b}, L₃-R^{A4'}, -CH(C₁₋₂ alkyl)-CR^{A4c}=CR^{A4a}R^{A4b}, -C(C₁₋₂ alkyl)₂-CR^{A4c}=CR^{A4a}R^{A4b}, -CH₂-C(CN)=CR^{A4a}R^{A4b}; or R^{A4} is selected from -CH₂-C(CH₃)=CR^{A4a}R^{A4b}, -CH₂-CH=CH-CH=CR^{A4a}R^{A4b}, L₃-R^{A4'}, -CH(C₁₋₂ alkyl)-CR^{A4c}=CR^{A4a}R^{A4b}, -C(C₁₋₂ alkyl)₂-CR^{A4c}=CR^{A4a}R^{A4b}, -CH₂-C(CN)=CR^{A4a}R^{A4b}; or R^{A4} is selected from -CH₂-CH=CR^{A4a}R^{A4b}, -CH₂-CH=CH-CH=CR^{A4a}R^{A4b}, L₃-R^{A4'}, -CH(C₁₋₂ alkyl)-CR^{A4c}=CR^{A4a}R^{A4b}, -C(C₁₋₂ alkyl)₂-CR^{A4c}=CR^{A4a}R^{A4b}, -CH₂-C(CN)=CR^{A4a}R^{A4b};
R^{A4a} and R^{A4b} together with the carbon atoms to which they are attached form a 4-, 5-, or 6-membered monocyclic cycloalkyl, a C₇₋₁₀ spirocyclic carbocyclic ring, a C₇₋₁₀ fused carbocyclic ring, a 5- or 6-membered heterocyclic cycloalkyl containing 1 to 3 heteroatoms selected from N, O, or S, or a 6-membered fused carbocyclic ring, and the cycloalkyl or heterocycloalkyl is optionally substituted with 1 to 3 groups selected from halogen, =O, deuterium, CN, OH, C₁₋₂ alkyl, haloC₁₋₂ alkyl, deuterated C₁₋₂ alkyl, -COC₁₋₂ alkyl, C₁₋₂ alkoxy, haloC₁₋₂ alkoxy, deuterated C₁₋₂ alkoxy and NH₂;
L₃ is selected from C₃₋₆ cycloalkyl, or -CH(C₁₋₂ alkyl)-C₁₋₂ alkyl;
R^{A4'} is selected from C₃₋₆ cycloalkyl, or a 5- to 10-membered heteroaryl containing 1 to 3 heteroatoms selected from N, O, or S, and the cycloalkyl or heteroaryl is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
and the definitions of other groups are consistent with those of any one of the foregoing technical solutions.

A seventeenth technical solution of the present invention relates to a compound of formula (I-a) of the present invention, a stereoisomer, a deuterated substance, a solvate, or a pharmaceutically acceptable salt or a eutectic crystal thereof, wherein
R^{A4} is selected from -CH₂-CF=CR^{A4a}R^{A4b}, -CH₂-CH=CH-CH=CR^{A4a}R^{A4b}, L₃-R^{A4'}; or R^{A4} is selected from -CH₂-C(CH₃)=CR^{A4a}R^{A4b}, -CH₂-CH=CH-CH=CR^{A4a}R^{A4b}, L₃-R^{A4'}; or R^{A4} is selected from -CH₂-CH=CR^{A4a}R^{A4b}, -CH₂-CH=CH-CH=CR^{A4a}R^{A4b}, L₃-R^{A4'};
R^{A4a} and R^{A4b} together with the carbon atoms to which they are attached form a 4-, 5-, or 6-membered monocyclic cycloalkyl, a C₇₋₁₀ spirocyclic carbocyclic ring, a C₇₋₁₀ fused carbocyclic ring, a 5- or 6-membered heterocyclic cycloalkyl containing 1 to 3 heteroatoms selected from N, O, or S, or a 6-membered fused carbocyclic ring, and the cycloalkyl or heterocycloalkyl is optionally substituted with 1 to 3 groups selected from halogen, =O, deuterium, CN, OH, C₁₋₂ alkyl, haloC₁₋₂ alkyl, deuterated C₁₋₂ alkyl, -COC₁₋₂ alkyl, C₁₋₂ alkoxy, haloC₁₋₂ alkoxy, deuterated C₁₋₂ alkoxy and NH₂;
L₃ is selected from C₃₋₆ cycloalkyl, or -CH(C₁₋₂ alkyl)-C₁₋₂ alkyl;
R^{A4'} is selected from C₃₋₆ cycloalkyl, or a 5- to 10-membered heteroaryl containing 1 to 3 heteroatoms selected from N, O, or S, and the cycloalkyl or heteroaryl is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
and the definitions of other groups are consistent with those of any one of the foregoing technical solutions.

An eighteenth technical solution of the present invention relates to a compound of formula (I-a) of the present invention, a stereoisomer, a deuterated substance, a solvate, or a pharmaceutically acceptable salt or a eutectic crystal thereof, wherein
R^{A4} is selected from -CH₂-CH₂-C₃₋₆ cycloalkyl, -CH₂-CF=CR^{A4a}R^{A4b}, L₃-R^{A4'}, -CH(C₁₋₂ alkyl)-CR^{A4c}=CR^{A4a}R^{A4b}, -C(C₁₋₂ alkyl)₂-CR^{A4c}=CR^{A4a}R^{A4b}, -CH₂-C(CN)=CR^{A4a}R^{A4b}; or R^{A4} is selected from -CH₂-CH₂-C₃₋₆ cycloalkyl, -CH₂-C(CH₃)=CR^{A4a}R^{A4b}, L₃-R^{A4'}, -CH(C₁₋₂ alkyl)-CR^{A4c}=CR^{A4a}R^{A4b}, -C(C₁₋₂ alkyl)₂-CR^{A4c}=CR^{A4a}R^{A4b}, -CH₂-C(CN)=CR^{A4a}R^{A4b}; or R^{A4} is selected from -CH₂-CH₂-C₃₋₆ cycloalkyl, -CH₂-CH=CR^{A4a}R^{A4b}, L₃-R^{A4}, -CH(C₁₋₂ alkyl)-CR^{A4c}=CR^{A4a}R^{A4b}, - C(C₁₋₂ alkyl)₂-CR^{A4c}=CR^{A4a}R^{A4b}, -CH₂-C(CN)=CR^{A4a}R^{A4b};
R^{A4a}, R^{A4b} and the carbon atoms to which they are attached form 4-, 5-, 6-membered cycloalkyl;
L₃ is selected from C₃₋₆ cycloalkyl, or -CH(C₁₋₂ alkyl)-C₁₋₂ alkyl;
R^{A4'} is selected from C₃₋₆ cycloalkyl, or a 5- to 10-membered heteroaryl containing 1 to 3 heteroatoms selected from N, O, or S, and the cycloalkyl or heteroaryl is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
provided that, the compound satisfies the following: R^{A4} is selected from - CH₂-CF=CR^{A4a}R^{A4b}, or L₃-R^{A4'}; or satisfies the following: R^{A4} is selected from - CH₂-C(CH₃)=CR^{A4a}R^{A4b}, or L₃-R^{A4'}; or satisfies the following: R^{A4} is selected from -CH₂-CH=CR^{A4a}R^{A4b}, or L₃-R^{A4'};
and the definitions of other groups are consistent with those of any one of the foregoing technical solutions.

A nineteenth technical solution of the present invention relates to a compound of formula (I-a) of the present invention, a stereoisomer, a deuterated substance, a solvate, or a pharmaceutically acceptable salt or a eutectic crystal thereof, wherein
R^{A4} is selected from -CH₂-CH₂-C₃₋₆ cycloalkyl, -CH₂-CF=CR^{A4a}R^{A4b}, L₃-R^{A4'}; or R^{A4} is selected from -CH₂-CH₂-C₃₋₆ cycloalkyl, -CH₂-C(CH₃)=CR^{A4a}R^{A4b}, L₃-R^{A4'}; or R^{A4} is selected from -CH₂-CH₂-C₃₋₆ cycloalkyl, -CH₂-CH=CR^{A4a}R^{A4b}, L₃-R^{A4};
R^{A4a}, R^{A4b} and the carbon atoms to which they are attached form 4-, 5-, 6-membered cycloalkyl;
L₃ is selected from C₃₋₆ cycloalkyl, or -CH(C₁₋₂ alkyl)-C₁₋₂ alkyl;
R^{A4'} is selected from C₃₋₆ cycloalkyl, or a 5- to 10-membered heteroaryl containing 1 to 3 heteroatoms selected from N, O, or S, and the cycloalkyl or heteroaryl is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
provided that, the compound satisfies the following: R^{A4} is selected from - CH₂-CF=CR^{A4a}R^{A4b}, or L₃-R^{A4'}; or satisfies the following: R^{A4} is selected from - CH₂-C(CH₃)=CR^{A4a}R^{A4b}, or L₃-R^{A4'}; or satisfies the following: R^{A4} is selected from -CH₂-CH=CR^{A4a}R^{A4b}, or L₃-R^{A4'};
and the definitions of other groups are consistent with those of any one of the foregoing technical solutions.

A twentieth technical solution of the present invention relates to a compound of formula (I-a) of the present invention, a stereoisomer, a deuterated substance, a solvate, or a pharmaceutically acceptable salt or a eutectic crystal thereof, wherein
R¹ is selected from R^{A1}, -O-haloC₁₋₂ alkyl, -NH-haloC₁₋₂ alkyl, -OC₁₋₂ alkyl, C₁₋₂ alkoxy, halogen, cyano, nitro, OH, C₁₋₂ alkyl, C₂₋₄ alkenyl, -NH-C₁₋₂ alkyl, - N(C₁₋₂ alkyl)₂, -NH-C₃₋₁₀ cycloalkyl, -NHC(O)C₁₋₂ alkyl, or 4- to 10-membered heterocycloalkyl containing 1 to 3 heteroatoms selected from N, O, and S, and the alkyl, alkoxy, cycloalkyl, or heterocycloalkyl is optionally further substituted with 1 to 3 groups selected from halogen, D, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy, C₃₋₈ cycloalkyl, 4- to 6-membered heterocycloalkyl containing 1 to 3 heteroatoms selected from N, O, or S, phenyl, and NH₂;
each of R² and R³ is independently selected from H, deuterium, C₁₋₄ alkoxy, halogen, cyano, nitro, OH, C₁₋₂ alkyl, C₂₋₄ alkenyl, -NH-C₁₋₂ alkyl, or -N(C₁₋₂ alkyl)₂, and the alkyl or alkoxy is optionally further substituted with 1 to 3 groups selected from halogen, D, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy, and NH₂;
X₁ is selected from O or S;
each of R⁴ and R⁵ is independently selected from H, deuterium, C₃₋₇ cycloalkyl, C₂₋₄ alkenyl, C₂₋₄ alkynyl, F, Cl, amino,-NH-C₁₋₂ alkyl, -N(C₁₋₂ alkyl)₂, C₁₋₂ alkyl, or 4- to 7-membered heterocycloalkyl containing 1 to 3 heteroatoms selected from N, O, or S;
L₁ is selected from a bond;
other group definitions are consistent with any of the above technical solutions.

A twenty-first technical solution of the present invention relates to a compound of formula (I-a) of the present invention, a stereoisomer, a deuterated substance, a solvate, or a pharmaceutically acceptable salt or a eutectic crystal thereof, wherein
R^{A4} is selected from 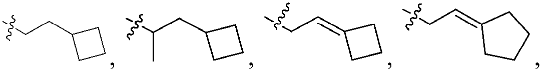 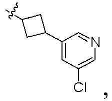 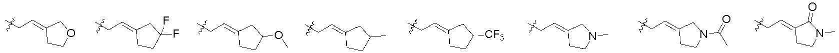 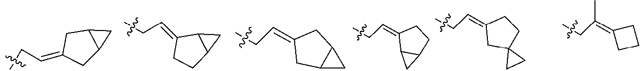 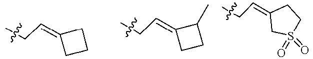 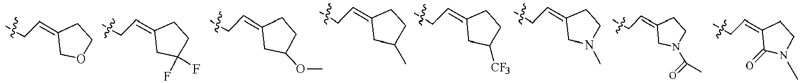 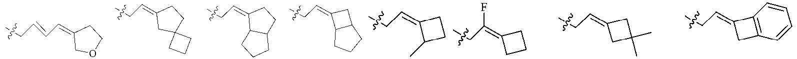 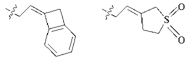 or is selected from 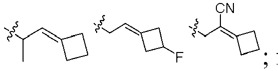 further, R^{A4} is selected from 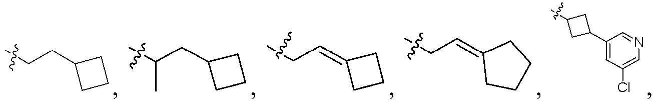 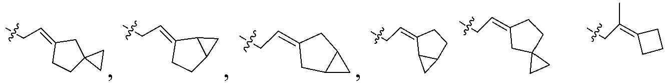 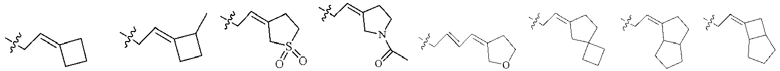 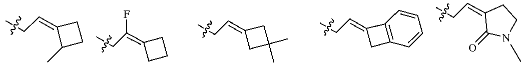 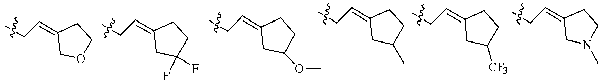 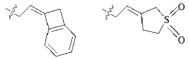 or is selected from 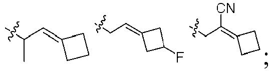
and the definitions of other groups are consistent with those of any one of the foregoing technical solutions.

A twenty-second technical solution of the present invention relates to a compound of formula (I-a) of the present invention, a stereoisomer, a deuterated substance, a solvate, or a pharmaceutically acceptable salt or a eutectic crystal thereof, wherein
R^{A4} is selected from 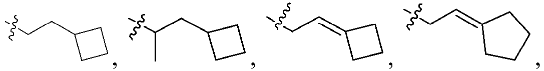 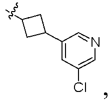 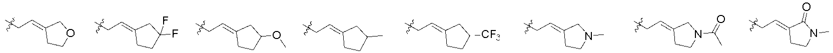 or is selected from 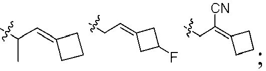 further, R^{A4} is selected from 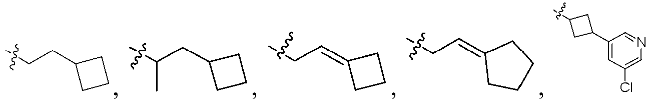 or is selected from 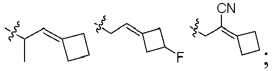
and the definitions of other groups are consistent with those of any one of the foregoing technical solutions.

A twenty-third technical solution of the present invention is a compound of the present invention, a stereoisomer, a deuterated substance, a solvate, or a pharmaceutically acceptable salt or a eutectic crystal thereof, wherein
L₁ is selected from a bond, or O;
L₂ is selected from -CH₂-, -C(O)-, -CHF-, -CF₂-, or C(CH₃)₂-;
each of R^{L1} and R^{L2} is independently selected from H, deuterium, or F;
each of R⁶, R⁹, and R¹⁰ is independently selected from H, deuterium, F, Cl, OH, CN, or methyl;
each of R⁷ and R⁸ is independently selected from H, deuterium, F, Cl, OH, CN, methyl, or R^{A2};
R^{A2} is selected from 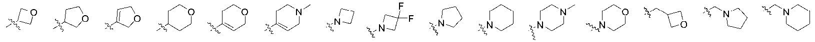 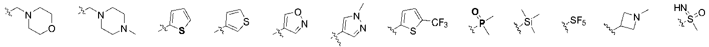 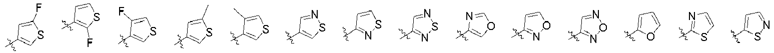 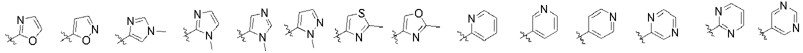 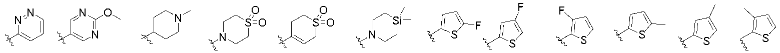 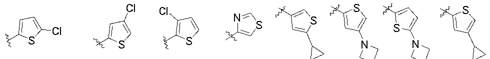 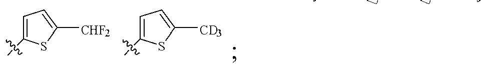
each of R² and R³ is independently selected from H, deuterium, vinyl, or ethynyl;
each of R⁴ and R⁵ is independently selected from H, deuterium, cyclopropyl, vinyl, or ethynyl;
alternatively, R⁴, R⁵ together with the atoms to which they are attached form phenyl, cyclohexyl, cyclopentyl, thienyl, furyl, pyrrolyl, pyrazolyl, or isoxazolyl;
alternatively, R², R⁴, L₁ together with the atoms to which they are attached form cyclohexyl or phenyl;
alternatively, R^{L1} and R⁹ together with the atoms to which they are attached form cyclopentyl or cyclohexyl;
alternatively, R⁷ and R⁸ together with the atoms to which they are attached form 4- to 7-membered carbocyclic ring or 5- to 7-membered heterocycloalkyl containing 1 to 3 heteroatoms selected from N, O, S, or B or 8 membered carbocyclic ring, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
R¹ is selected from 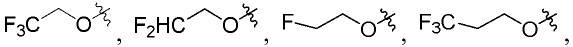 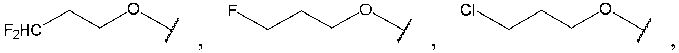 -CH₂CH₃, - CH₂CH₂CH₃, 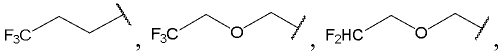 R^{A1};
R^{A1} is selected from 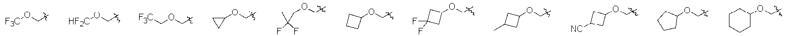 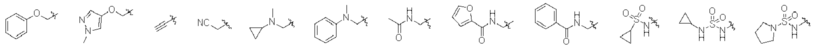 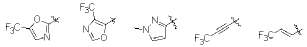 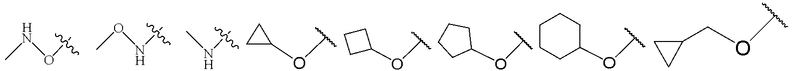 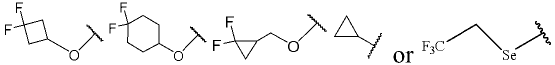 or 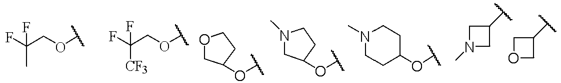 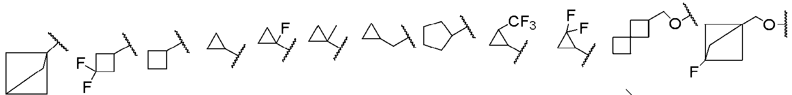 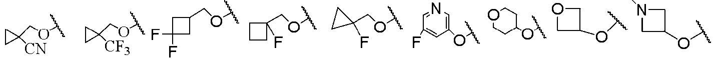 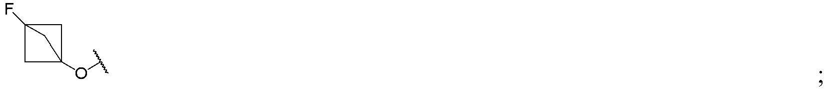
provided that the compound satisfies one of the following conditions:
   (1). R¹ is selected from R^{A1}
   (2). at least one group of R⁴ and R⁵ is selected from cyclopropyl, vinyl, or ethynyl;
   (3). R⁴, R⁵ together with the atoms to which they are attached form phenyl, cyclohexyl, cyclopentyl, thienyl, furyl, pyrrolyl, pyrazolyl, or isoxazolyl;
   (4). at least one group of R⁷ and R⁸ is selected from R^{A2};
   (5). R², R⁴, L₁ together with the atoms to which they are attached form cyclohexyl or phenyl;
   (6). L₁ is selected from O;
   (7). L₂ is selected from -C(O)-, -CHF-, -CF₂-, or -C(CH₃)₂-;
   (8). one of the combinations of R⁷ and R⁸, and R⁸ and R⁹ together with the atoms to which they are attached form 4- to 7-membered carbocyclic ring or 8 membered carbocyclic ring or 5- to 7-membered heterocycloalkyl containing 1 Si atom, and the carbocyclic ring or heterocycloalkyl is optionally further substituted with 1 to 3 groups selected from =O, F, Cl, deuterium, OH, C₁₋₂ alkyl, or haloC₁₋₂ alkyl;
and the definitions of other groups are consistent with those of any one of the foregoing technical solutions.

A twenty-fourth technical solution of the present invention is a compound of the present invention, a stereoisomer, a deuterated substance, a solvate, or a pharmaceutically acceptable salt or a eutectic crystal thereof, wherein
L₁ is selected from a bond, or O;
L₂ is selected from -CH₂-, -C(O)-, -CHF-, -CF₂-, or C(CH₃)₂-;
each of R^{L1} and R^{L2} is independently selected from H, deuterium, or F;
each of R⁶, R⁹, and R¹⁰ is independently selected from H, deuterium, F, Cl, OH, CN, or methyl;
each of R⁷ and R⁸ is independently selected from H, deuterium, F, Cl, OH, CN, methyl, or R^{A2};
or as an alternative, R⁷ and R⁸ together with the atoms to which they are attached form 4- to 7-membered carbocyclic ring, 5- to 7-membered heterocycloalkyl containing 1 to 3 heteroatoms selected from N, O, S, or B or 8 membered carbocyclic ring or 5- to 7-membered heterocycloalkyl containing 1 Si atom, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
R^{A2} is selected from 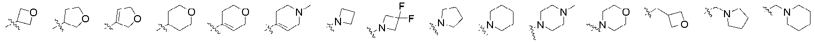 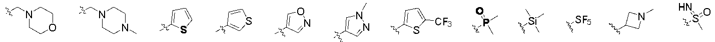 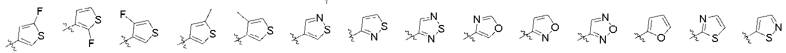 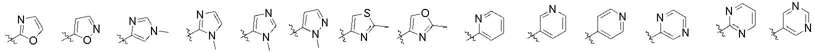 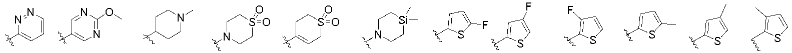 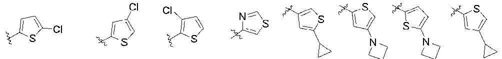 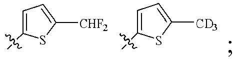
each of R² and R³ is independently selected from H, deuterium, vinyl, or ethynyl;
each of R⁴ and R⁵ is independently selected from H, deuterium, cyclopropyl, vinyl, or ethynyl;
alternatively, R⁴, R⁵ together with the atoms to which they are attached form phenyl, cyclohexyl, cyclopentyl, thienyl, furyl, pyrrolyl, pyrazolyl, or isoxazolyl;
alternatively, R², R⁴, L₁ together with the atoms to which they are attached form cyclohexyl or phenyl;
alternatively, R^{L1} and R⁹ together with the atoms to which they are attached form cyclopentyl or cyclohexyl;
R¹ is selected from 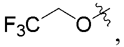 or R^{A1}; or R¹ is selected from 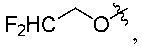 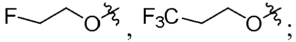
R^{A1} is selected from 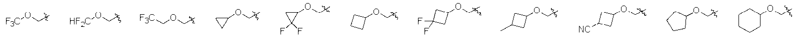 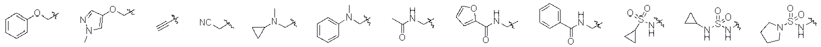 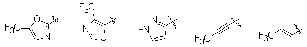 or is selected from 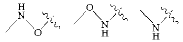 or is selected from 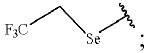
provided that the compound satisfies one of the following conditions:
   (1). R¹ is selected from R^{A1}
   (2). at least one group of R⁴ and R⁵ is selected from cyclopropyl, vinyl, or ethynyl;
   (3). R⁴, R⁵ together with the atoms to which they are attached form phenyl, cyclohexyl, cyclopentyl, thienyl, furyl, pyrrolyl, pyrazolyl, or isoxazolyl;
   (4). at least one group of R⁷ and R⁸ is selected from R^{A2};
   (5). R², R⁴, L₁ together with the atoms to which they are attached form cyclohexyl or phenyl;
   (6). L₁ is selected from O;
   (7). (7) L₂ is selected from -C(O)-, -CHF-, -CF₂-, or C(CH₃)₂-.

A twenty-fifth technical solution of the present invention is a compound of the present invention, a stereoisomer, deuterated substance, solvate, or
pharmaceutically acceptable salt or eutectic crystal thereof, wherein
L₁ is selected from a bond, or O;
L₂ is selected from -CH₂-, -C(O)-, -CHF-, -CF₂-, or C(CH₃)₂-;
each of R^{L1} and R^{L2} is independently selected from H, deuterium, or F;
each of R⁶, R⁹, and R¹⁰ is independently selected from H, deuterium, F, Cl, OH, CN, or methyl;
each of R⁷ and R⁸ is independently selected from H, deuterium, F, Cl, OH, CN, methyl, or R^{A2};
or as an alternative, R⁷ and R⁸ together with the atoms to which they are attached form 4- to 7-membered carbocyclic ring, 5- to 7-membered heterocycloalkyl containing 1 to 3 heteroatoms selected from N, O, S, or B or 8 membered carbocyclic ring or 5- to 7-membered heterocycloalkyl containing 1 Si atom, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
R^{A2} is selected from 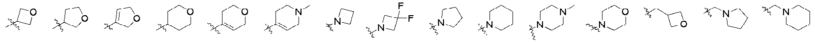 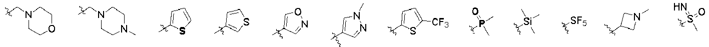 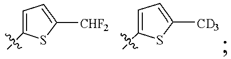
each of R² and R³ is independently selected from H, deuterium, vinyl, or ethynyl;
each of R⁴ and R⁵ is independently selected from H, deuterium, cyclopropyl, vinyl, or ethynyl;
alternatively, R⁴, R⁵ together with the atoms to which they are attached form phenyl, cyclohexyl, cyclopentyl, thienyl, furyl, pyrrolyl, pyrazolyl, or isoxazolyl;
alternatively, R², R⁴, L₁ together with the atoms to which they are attached form cyclohexyl or phenyl;
alternatively, R^{L1} and R⁹ together with the atoms to which they are attached form cyclopentyl or cyclohexyl;
R¹ is selected from 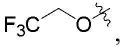 or R^{A1}; or R¹ is selected from 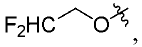 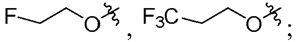
R^{A1} is selected from 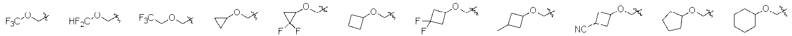 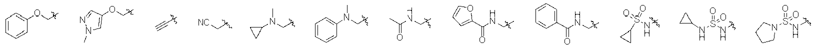 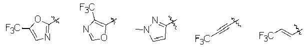 or is selected from 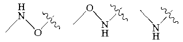 or is selected from 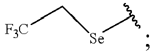
provided that the compound satisfies one of the following conditions:
   (1). R¹ is selected from R^{A1}
   (2). at least one group of R⁴ and R⁵ is selected from cyclopropyl, vinyl, or ethynyl;
   (3). R⁴, R⁵ together with the atoms to which they are attached form phenyl, cyclohexyl, cyclopentyl, thienyl, furyl, pyrrolyl, pyrazolyl, or isoxazolyl;
   (4). at least one group of R⁷ and R⁸ is selected from R^{A2};
   (5). R², R⁴, L₁ together with the atoms to which they are attached form cyclohexyl or phenyl;
   (6). L₁ is selected from O;
   (7). L₂ is selected from -C(O)-, -CHF-, -CF₂-, or C(CH₃)₂-.

A twenty-sixth technical solution of the present invention is a compound of the present invention, a stereoisomer, a deuterated substance, a solvate, or a pharmaceutically acceptable salt or a eutectic crystal thereof, wherein
L₁ is selected from a bond, or O;
L₂ is selected from -CH₂-, -C(O)-, -CHF-, -CF₂-, or C(CH₃)₂-;
each of R^{L1} and R^{L2} is independently selected from H, deuterium, or F;
each of R⁶, R⁹, and R¹⁰ is independently selected from H, deuterium, F, Cl, OH, CN, or methyl;
each of R⁷ and R⁸ is independently selected from H, deuterium, F, Cl, OH, CN, methyl, or R^{A2};
or as an alternative, R⁷ and R⁸ together with the atoms to which they are attached form 4- to 7-membered carbocyclic ring, 5- to 7-membered heterocycloalkyl containing 1 to 3 heteroatoms selected from N, O, S, or B or 8 membered carbocyclic ring or 5- to 7-membered heterocycloalkyl containing 1 Si atom, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
R^{A2} is selected from 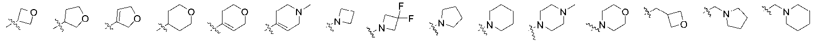 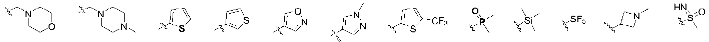 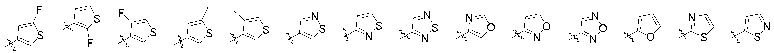 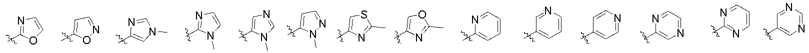 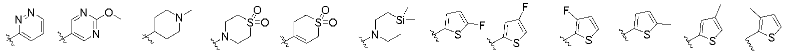 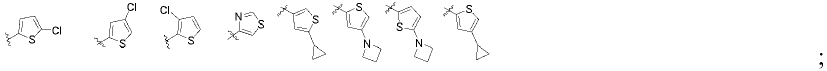
each of R² and R³ is independently selected from H, deuterium, vinyl, or ethynyl;
each of R⁴ and R⁵ is independently selected from H, deuterium, cyclopropyl, vinyl, or ethynyl;
alternatively, R⁴, R⁵ together with the atoms to which they are attached form phenyl, cyclohexyl, cyclopentyl, thienyl, furyl, pyrrolyl, pyrazolyl, or isoxazolyl;
alternatively, R², R⁴, L₁ together with the atoms to which they are attached form cyclohexyl or phenyl;
alternatively, R^{L1} and R⁹ together with the atoms to which they are attached form cyclopentyl or cyclohexyl;
R¹ is selected from 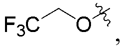 or R^{A1}; or R¹ is selected from 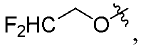 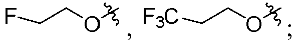
R^{A1} is selected from 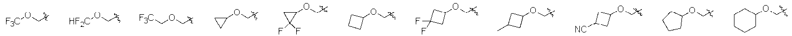 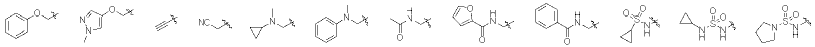 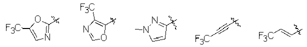 or is selected from 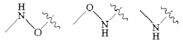 or is selected from 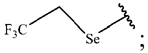
provided that the compound satisfies one of the following conditions:
   (1). R¹ is selected from R^{A1}
   (2). at least one group of R⁴ and R⁵ is selected from cyclopropyl, vinyl, or ethynyl;
   (3). R⁴, R⁵ together with the atoms to which they are attached form phenyl, cyclohexyl, cyclopentyl, thienyl, furyl, pyrrolyl, pyrazolyl, or isoxazolyl;
   (4). at least one group of R⁷ and R⁸ is selected from R^{A2};
   (5). R², R⁴, L₁ together with the atoms to which they are attached form cyclohexyl or phenyl;
   (6). L₁ is selected from O;
   (7). (7) L₂ is selected from -C(O)-, -CHF-, -CF₂-, or C(CH₃)₂-.

A twenty-seventh technical solution of the present invention is a compound of the present invention, a stereoisomer, a deuterated substance, a solvate, or a pharmaceutically acceptable salt or a eutectic crystal thereof, wherein
L₁ is selected from a bond, or O;
L₂ is selected from -CH₂-, -C(O)-, -CHF-, -CF₂-, or C(CH₃)₂-;
each of R^{L1} and R^{L2} is independently selected from H, deuterium, or F;
each of R⁶, R⁹, and R¹⁰ is independently selected from H, deuterium, F, Cl, OH, CN, or methyl;
each of R⁷ and R⁸ is independently selected from H, deuterium, F, Cl, OH, CN, methyl, or R^{A2};
or as an alternative, R⁷ and R⁸ together with the atoms to which they are attached form 4- to 7-membered carbocyclic ring, 5- to 7-membered heterocycloalkyl containing 1 to 3 heteroatoms selected from N, O, S, or B or 8 membered carbocyclic ring or 5- to 7-membered heterocycloalkyl containing 1 Si atom, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
R^{A2} is selected from 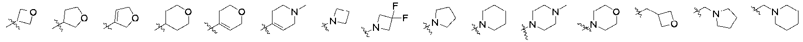 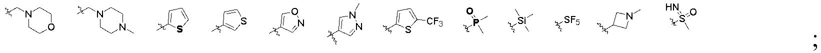
each of R² and R³ is independently selected from H, deuterium, vinyl, or ethynyl;
each of R⁴ and R⁵ is independently selected from H, deuterium, cyclopropyl, vinyl, or ethynyl;
alternatively, R⁴, R⁵ together with the atoms to which they are attached form phenyl, cyclohexyl, cyclopentyl, thienyl, furyl, pyrrolyl, pyrazolyl, or isoxazolyl;
alternatively, R², R⁴, L₁ together with the atoms to which they are attached form cyclohexyl or phenyl;
alternatively, R^{L1} and R⁹ together with the atoms to which they are attached form cyclopentyl or cyclohexyl;
R¹ is selected from 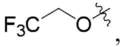 or R^{A1}; or R¹ is selected from 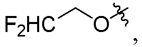 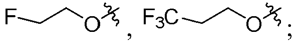
R^{A1} is selected from 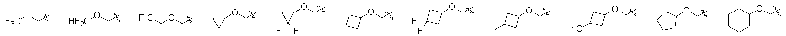 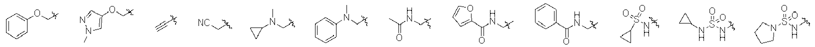 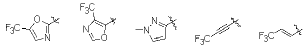 or is selected from 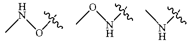 or is selected from 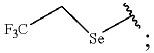
provided that the compound satisfies one of the following conditions:
   (1). R¹ is selected from R^{A1}
   (2). at least one group of R⁴ and R⁵ is selected from cyclopropyl, vinyl, or ethynyl;
   (3). R⁴, R⁵ together with the atoms to which they are attached form phenyl, cyclohexyl, cyclopentyl, thienyl, furyl, pyrrolyl, pyrazolyl, or isoxazolyl;
   (4). at least one group of R⁷ and R⁸ is selected from R^{A2},
   (5). R², R⁴, L₁ together with the atoms to which they are attached form cyclohexyl or phenyl;
   (6). L₁ is selected from O;
   (7). L₂ is selected from -C(O)-, -CHF-, -CF₂-, or C(CH₃)₂-.

A twenty-eighth technical solution of the present invention relates to a compound of formula (I-a), a stereoisomer, a deuterated substance, a solvate, or a pharmaceutically acceptable salt or a eutectic crystal thereof, wherein
L₁ is selected from a bond, or O;
each of R² and R³ is independently selected from H, deuterium, vinyl, or ethynyl;
each of R⁴ and R⁵ is independently selected from H, deuterium, cyclopropyl, vinyl, or ethynyl;
alternatively, R⁴, R⁵ together with the atoms to which they are attached form phenyl, cyclohexyl, cyclopentyl, thienyl, furyl, pyrrolyl, pyrazolyl, or isoxazolyl;
R¹ is selected from 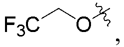 or R^{A1}; or R¹ is selected from 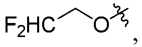 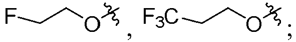
R^{A1} is selected from 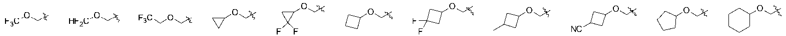 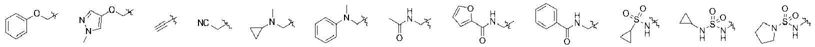 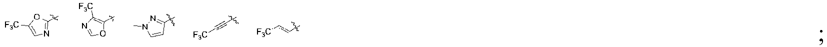
X₁ is selected from O or S;
R^{A4} is selected from 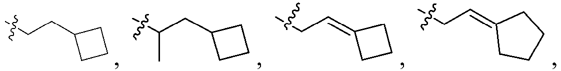 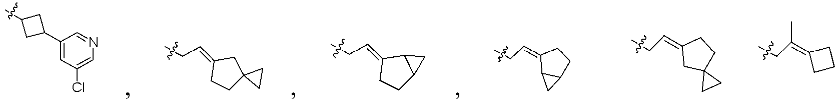 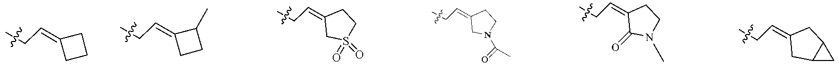 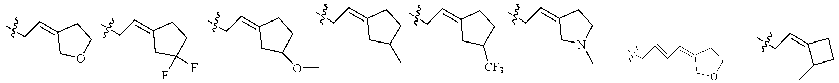 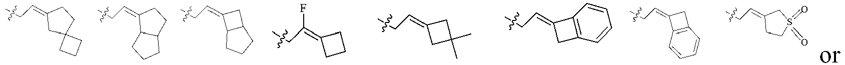 selected from

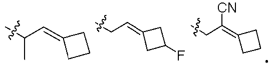

As an alternative technical solution, the compound represented by formula (I) or (I-a), a stereoisomer, deuterated substance, solvate, or pharmaceutically acceptable salt thereof, wherein: R¹ is -O-haloC₁₋₄ alkyl, R² and R³ are H, X1 is S or at least one of R⁶, R⁷, R⁸, and R⁹ is R^{A2}, R^{A2} is selected from -(4- to 10-membered heterocycloalkyl containing 1 to 3 heteroatoms selected from N, O, S, or Si), or -(5- to 10-membered heteroaryl containing 1 to 3 heteroatoms selected from N, O, or S), -P(O)(R^{c})₂, -Si(R^{c})₃, -SF₅, B(OH)₂, or -S(O)(=NH)R^{c}, and the heterocycloalkyl or heteroaryl is optionally further substituted with 1 to 3 groups selected from halogen, =O, D, CN, OH, C₁₋₄ alkyl, haloC₁₋₄ alkyl, deuterated C₁₋₄ alkyl, C₁₋₄ alkoxy, haloC₁₋₄ alkoxy, C₃₋₆ cycloalkyl, 4- to 7-membered heterocycloalkyl containing 1 to 3 heteroatoms selected from N, O, or S, deuterated C₁₋₄ alkoxy and NH₂;
alternatively, R¹ is R^{A1}, R² and R³ are H, X1 is O, R^{A1} is C₂₋₆ alkynyl, -C₁₋₄ alkyl-OR^{a}, -NR^{b}-S(O)₂-R^{a}, each R^{a} is selected from C₃₋₁₀ cycloalkyl or 4- to 10-membered heterocycloalkyl containing 1 to 3 heteroatoms selected from N, O, and S, and the cycloalkyl and heterocycloalkyl are optionally further substituted with 1 to 3 groups selected from halogen, =O, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂; and the rest of the groups are as described in any of the above technical solutions.

A twenty-ninth technical solution of the present invention relates to a compound of formula (I-a), a stereoisomer, deuterated substance, solvate,
or pharmaceutically acceptable salt or eutectic crystal thereof, wherein
L₁ is selected from a bond, or O;
each of R² and R³ is independently selected from H, deuterium, vinyl, or ethynyl;
each of R⁴ and R⁵ is independently selected from H, deuterium, cyclopropyl, vinyl, or ethynyl;
alternatively, R⁴, R⁵ together with the atoms to which they are attached form phenyl, cyclohexyl, cyclopentyl, thienyl, furyl, pyrrolyl, pyrazolyl, or isoxazolyl;
R¹ is selected from 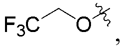 or R^{A1}; or R¹ is selected from 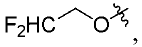 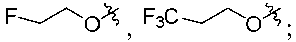
R^{A1} is selected from 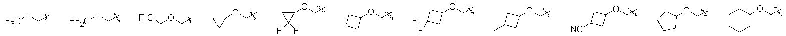 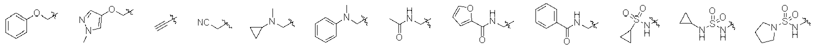 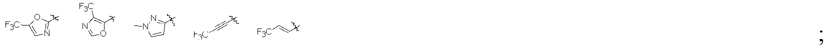
X₁ is selected from O or S;
R^{A4} is selected from 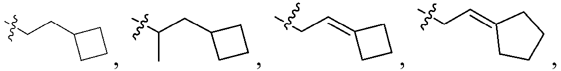 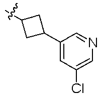 or is selected from

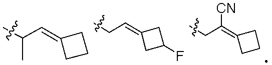

As an alternative technical solution, the compound represented by formula (I) or (I-a), a stereoisomer, deuterated substance, solvate, or pharmaceutically acceptable salt thereof, wherein: R¹ is -O-haloC₁₋₄ alkyl, R² and R³ are H, X1 is S or at least one of R⁶, R⁷, R⁸, and R⁹ is R^{A2}, R^{A2} is selected from -(4- to 10-membered heterocycloalkyl containing 1 to 3 heteroatoms selected from N, O, S, or Si), or -(5- to 10-membered heteroaryl containing 1 to 3 heteroatoms selected from N, O, or S), -P(O)(R^{c})₂, -Si(R^{c})₃, -SF₅, B(OH)₂, or -S(O)(=NH)R^{c}, and the heterocycloalkyl or heteroaryl is optionally further substituted with 1 to 3 groups selected from halogen, =O, D, CN, OH, C₁₋₄ alkyl, haloC₁₋₄ alkyl, deuterated C₁₋₄ alkyl, C₁₋₄ alkoxy, haloC₁₋₄ alkoxy, C₃₋₆ cycloalkyl, 4- to 7-membered heterocycloalkyl containing 1 to 3 heteroatoms selected from N, O, or S, deuterated C₁₋₄ alkoxy and NH₂;
alternatively, R¹ is R^{A1}, R² and R³ are H, X₁ is O, R_{A1} is C₂₋₆ alkynyl, -C₁₋₄ alkyl-OR^{a}, -NR^{b}-S(O)₂-R^{a}, each R^{a} is selected from C₃₋₁₀ cycloalkyl or 4- to 10-membered heterocycloalkyl containing 1 to 3 heteroatoms selected from N, O, and S, and the cycloalkyl and heterocycloalkyl are optionally further substituted with 1 to 3 groups selected from halogen, =O, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂; and the rest of the groups are as described in any of the above technical solutions.

A thirtieth technical solution of the present invention is a compound of the present invention, and a stereoisomer, deuterated substance, solvate, or pharmaceutically
acceptable salt thereof, having the structure of formula (I): 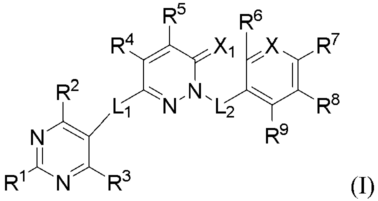
wherein
each of R¹, R² and R³ is independently selected from H, deuterium, R^{A1}, -O-haloC₁₋₄ alkyl, -NH-haloC₁₋₄ alkyl, -OC₁₋₄ alkyl, C₁₋₄ alkoxy, C₅₋₆ alkoxy, halogen, cyano, nitro, C₁₋₄ alkyl, C₂₋₆ alkenyl, -NH-C₁₋₄ alkyl, -N(C₁₋₄ alkyl)₂, -NH-C₃₋₁₀ cycloalkyl, -NHC(O)C₁₋₄ alkyl, or 4- to 10-membered heterocycloalkyl containing 1 to 3 heteroatoms selected from N, O, and S, and the alkyl, alkoxy, cycloalkyl, or heterocycloalkyl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, C₃₋₁₀ cycloalkyl, 4- to 6-membered heterocycloalkyl containing 1 to 3 heteroatoms selected from N, O, or S, phenyl, and NH₂, provided that R¹, R² and R³ are not simultaneously selected from H;
R^{A1} is selected from haloC₂₋₆ alkenyl, C₂₋₆ alkynyl, -O-(CH₂)ᵣ-R^{a}, C₃₋₁₀ cycloalkyl, 5- to 10-membered heteroaryl containing 1 to 3 heteroatoms selected from N, O, or S, -C₁₋₄ alkyl-R^{a}, -C₁₋₄ alkyl-OR^{a}, -C₁₋₄ alkyl-NR^{b}R^{a}, -NR^{b}-S(O)₂-R^{a}, -NR^{b}-S(O)₂-NR^{b}R^{a}, -O-NR^{b}R^{a}, -NH-OR^{b}, or -Se-(CH₂)ᵣ-R^{a}, and the alkynyl, heteroaryl or alkyl is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{a} is selected from CN, haloC₁₋₆ alkyl, C₃₋₁₀ cycloalkyl, 4- to 10-membered heterocycloalkyl containing 1 to 3 heteroatoms selected from N, O, or S, 6- to 10-membered aryl, 5- to 10-membered heteroaryl containing 1 to 3 heteroatoms selected from N, O, or S, or -C(O)-R^{a1}, and the alkyl, cycloalkyl, heterocycloalkyl, aryl, or heteroaryl is optionally further substituted with 1 to 3 groups selected from halogen, =O, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{b} is selected from H, deuterium, C₁₋₄ alkyl, C₃₋₆ cycloalkyl, haloC₁₋₄ alkyl, or deuterated C₁₋₄ alkyl;
R^{a1} is selected from OH, NH₂, -NHC₁₋₄ alkyl, -N(C₁₋₄ alkyl)₂, -NHC₃₋₁₀ cycloalkyl, C₁₋₄ alkoxy, C₁₋₆ alkyl, C₃₋₁₀ cycloalkyl, 6- to 10-membered aryl, 5- to 10-membered heteroaryl containing 1 to 3 heteroatoms selected from N, O, or S;
X is selected from CR¹⁰ or N;
X₁ is selected from O or S;
each of R⁴ and R⁵ is independently selected from H, deuterium, C₃₋₁₀ cycloalkyl, C₂₋₆ alkenyl, C₂₋₆ alkynyl, halogen, amino,-NH-C₁₋₄ alkyl, -N(C₁₋₄ alkyl)₂, C₁₋₄ alkyl, haloC₁₋₄ alkyl, 4- to 10-membered heterocycloalkyl containing 1 to 3 heteroatoms selected from N, O, or S, 5- to 10-membered heteroaryl containing 1 to 3 heteroatoms selected from N, O, or S, or 6- to 10-membered aryl;
each of R⁶, R⁷, R⁸, R⁹, and R¹⁰ is independently selected from H, deuterium, R^{A2}, halogen, OH, CN, amino, C₁₋₄ alkyl, C₂₋₆ alkenyl, C₂₋₆ alkynyl, C₁₋₄ alkoxy, haloC₁₋₄ alkyl, -NH-C₁₋₄ alkyl, -N(C₁₋₄ alkyl)₂, -S(O)R^{c}, -S(O)²R^{c}, -C(O)R^{c}, - C(O)OR^{c}, -C(O)N(R^{c})₂, or -OC(O)R^{c}, and the alkyl, alkenyl, alkynyl, or alkoxy is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂; provided that, at least one of the combinations of R⁷ and R⁸, and R⁸ and R⁹ together with the atoms to which they are attached form 4- to 8-membered carbocyclic ring, or 5- to 7-membered heterocycloalkyl containing 1 to 3 heteroatoms selected from N, O, S, B, or Si, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
R^{A2} is selected from -(CH₂)ᵣ-(4- to 10-membered heterocycloalkyl containing 1 to 3 heteroatoms selected from N, O, S, or Si), or -(CH₂)ᵣ-(5- to 10-membered heteroaryl containing 1 to 3 heteroatoms selected from N, O, or S), -P(O)(R^{c})₂, - Si(R^{c})₃, -SF₅, N₃, B(OH)₂, or -S(O)(=NH)R^{c}, and the heterocycloalkyl or heteroaryl is optionally further substituted with 1 to 3 groups selected from halogen, =O, D, CN, OH, C₁₋₄ alkyl, haloC₁₋₄ alkyl, deuterated C₁₋₄ alkyl, C₁₋₄ alkoxy, haloC₁₋₄ alkoxy, C₃₋₆ cycloalkyl, 4- to 7-membered heterocycloalkyl containing 1 to 3 heteroatoms selected from N, O, or S, deuterated C₁₋₄ alkoxy and NH₂;
each R^{c} is independently selected from H, OH, C₁₋₄ alkyl, C₃₋₇ cycloalkyl, haloC₁₋₄ alkyl, deuterated C₁₋₄ alkyl, C₁₋₄ alkoxy, haloC₁₋₄ alkoxy, deuterated C₁₋₄ alkoxy, or NH₂;
each r is independently selected from 0, 1, 2 or 3;
L₁ is selected from a bond, O, NH, S, -CD₂-, -CHD-, -CR^{L1}R^{L2}-, C₁₋₄ alkyl, - C(O)-, S(O), or S(O)₂, and the alkyl is optionally further substituted with R^{L1};
L₂ is selected from O, NH, S, -CH₂-, -CD₂-, -CHD-, -CR^{L1}R^{L2}-, -CHR^{L2}-, - CDR^{L2}-, C₂₋₄ alkyl, -C(O)-, S(O), S(O)₂, the alkyl is optionally further substituted with 1 to 3 R^{L1};
each of R^{L1} and R^{L2} is independently selected from halogen, OH, CN, amino, C₁₋₄ alkyl, C₁₋₄ alkoxy, C₃₋₆ cycloalkyl, or 4- to 6-membered heterocycloalkyl containing 1 to 3 heteroatoms selected from N, O, or S;
alternatively, R², R⁴ and L₁ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
alternatively, R⁴, R⁵ together with the atoms to which they are attached form a 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally substituted with 1 to 3 groups selected from R^{A3};
alternatively, R^{L2} and R⁹ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
alternatively, R⁶ and R¹⁰ together with the atoms to which they are attached form 4- to 7-membered carbocyclic ring, 5- to 7-membered heterocycloalkyl containing 1 to 3 heteroatoms selected from N, O, S, or B or 8 membered carbocyclic ring or 5- to 7-membered heterocycloalkyl containing 1 Si atom, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{A3} is independently selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂ group substitution.

A thirty-first technical solution of the present invention is a compound of the present invention, a stereoisomer, a deuterated substance, a solvate, or a pharmaceutically acceptable salt or a eutectic crystal thereof, having the structure of formula (I): 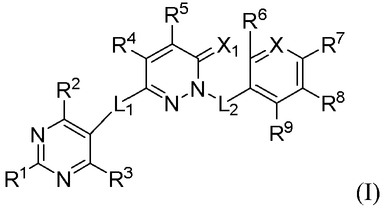 wherein
each of R¹, R² and R³ is independently selected from H, deuterium, R^{A1}, -O-haloC₁₋₄ alkyl, -NH-haloC₁₋₄ alkyl, -OC₁₋₄ alkyl, C₁₋₆ alkoxy, halogen, cyano, nitro, C₁₋₄ alkyl, -NH-C₁₋₄ alkyl, -N(C₁₋₄ alkyl)₂, -NH-C₃₋₁₀ cycloalkyl, -NHC(O)C₁₋₄ alkyl, or 4- to 10-membered heterocycloalkyl containing 1 to 3 heteroatoms selected from N, O, and S, and the alkyl, alkoxy, cycloalkyl, or heterocycloalkyl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, C₃₋₁₀ cycloalkyl, 4- to 6-membered heterocycloalkyl containing 1 to 3 heteroatoms selected from N, O, or S, phenyl, and NH₂, provided that R¹, R² and R³ are not simultaneously selected from H;
R^{A1} is selected from haloC₂₋₆ alkenyl, C₂₋₆ alkynyl, -O-(CH₂)ᵣ-R^{a}, C₃₋₁₀ cycloalkyl, 5- to 10-membered heteroaryl containing 1 to 3 heteroatoms selected from N, O, or S, -C₁₋₄ alkyl-R^{a}, -C₁₋₄ alkyl-OR^{a}, -C₁₋₄ alkyl-NR^{b}R^{a}, -NR^{b}-S(O)₂-R^{a}, -NR^{b}-S(O)₂-NR^{b}R^{a}, -O-NR^{b}R^{a}, -NH-OR^{b}, or -Se-(CH₂)ᵣ-R^{a}, and the alkynyl, heteroaryl, alkyl, or cycloalkyl is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{a} is selected from CN, haloC₁₋₆ alkyl, C₃₋₁₀ cycloalkyl, 4- to 10-membered heterocycloalkyl containing 1 to 3 heteroatoms selected from N, O, or S, 6- to 10-membered aryl, 5- to 10-membered heteroaryl containing 1 to 3 heteroatoms selected from N, O, or S, or -C(O)-R^{a1}, and the alkyl, cycloalkyl, heterocycloalkyl, aryl, or heteroaryl is optionally further substituted with 1 to 3 groups selected from halogen, =O, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{b} is selected from H, deuterium, C₁₋₄ alkyl, C₃₋₆ cycloalkyl, haloC₁₋₄ alkyl, or deuterated C₁₋₄ alkyl;
R^{a1} is selected from OH, NH₂, -NHC₁₋₄ alkyl, -N(C₁₋₄ alkyl)₂, -NHC₃₋₁₀ cycloalkyl, C₁₋₄ alkoxy, C₁₋₆ alkyl, C₃₋₁₀ cycloalkyl, 6- to 10-membered aryl, 5- to 10-membered heteroaryl containing 1 to 3 heteroatoms selected from N, O, or S;
X is selected from CR¹⁰ or N;
X₁ is selected from O or S;
each of R⁴ and R⁵ is independently selected from H, deuterium, C₃₋₁₀ cycloalkyl, C₂₋₆ alkenyl, C₂₋₆ alkynyl, halogen, amino,-NH-C₁₋₄ alkyl, -N(C₁₋₄ alkyl)₂, C₁₋₄ alkyl, haloC₁₋₄ alkyl, 4- to 10-membered heterocycloalkyl containing 1 to 3 heteroatoms selected from N, O, or S, 5- to 10-membered heteroaryl containing 1 to 3 heteroatoms selected from N, O, or S, or 6- to 10-membered aryl;
each of R⁶, R⁷, R⁸, R⁹, and R¹⁰ is independently selected from H, deuterium, R^{A2}, halogen, OH, CN, amino, C₁₋₄ alkyl, C₂₋₆ alkenyl, C₂₋₆ alkynyl, C₁₋₄ alkoxy, haloC₁₋₄ alkyl, -NH-C₁₋₄ alkyl, -N(C₁₋₄ alkyl)₂, -S(O)R^{c}, -S(O)²R^{c}, -C(O)R^{c}, - C(O)OR^{c}, -C(O)N(R^{c})₂, or -OC(O)R^{c}, and the alkyl, alkenyl, alkynyl, or alkoxy is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
R^{A2} is selected from -(CH₂)ᵣ-(C₃₋₁₀ cycloalkyl), -(CH₂)ᵣ-(4- to 10-membered heterocycloalkyl containing 1 to 3 heteroatoms selected from N, O, S, or Si), or - (CH₂)ᵣ-(5- to 10-membered heteroaryl containing 1 to 3 heteroatoms selected from N, O, or S), -P(O)(R^{c})₂, -Si(R^{c})₃, -SF₅, N₃, B(OH)₂, or -S(O)(=NH)R^{c}, and the cycloalkyl, heterocycloalkyl, or heteroaryl is optionally further substituted with 1 to 3 groups selected from halogen, =O, D, CN, OH, C₁₋₄ alkyl, haloC₁₋₄ alkyl, deuterated C₁₋₄ alkyl, C₁₋₄ alkoxy, haloC₁₋₄ alkoxy, C₃₋₆ cycloalkyl, 4- to 7-membered heterocycloalkyl containing 1 to 3 heteroatoms selected from N, O, or S, deuterated C₁₋₄ alkoxy and NH₂;
each R^{c} is independently selected from H, OH, C₁₋₄ alkyl, C₃₋₇ cycloalkyl, haloC₁₋₄ alkyl, deuterated C₁₋₄ alkyl, C₁₋₄ alkoxy, haloC₁₋₄ alkoxy, deuterated C₁₋₄ alkoxy, or NH₂;
each r is independently selected from 0, 1, 2 or 3;
L₁ is selected from a bond, O, NH, S, -CD₂-, -CHD-, -CR^{L1}R^{L2}-, C₁₋₄ alkyl, - C(O)-, S(O), or S(O)₂, and the alkyl is optionally further substituted with R^{L1};
L₂ is selected from a bond, O, NH, S, -CH₂-, -CD₂-, -CHD-, -CR^{L1}R^{L2}-, - CHR^{L2}- -CDR^{L2}-, C₂₋₄ alkyl, -C(O)-, S(O), or S(O)₂, and the alkyl is optionally further substituted with 1 to 3 R^{L1};
each of R^{L1} and R^{L2} is independently selected from halogen, OH, CN, amino, C₁₋₄ alkyl, C₁₋₄ alkoxy, C₃₋₆ cycloalkyl, or 4- to 6-membered heterocycloalkyl containing 1 to 3 heteroatoms selected from N, O, or S;
alternatively, R², R⁴ and L₁ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
alternatively, R⁴, R⁵ together with the atoms to which they are attached form a 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally substituted with 1 to 3 groups selected from R^{A3};
alternatively, R^{L2} and R⁹ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
each R^{A3} is independently selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂ group substitution;
provided that: one of the combinations of R⁶ and R¹⁰, R¹⁰ and R⁷, R⁷ and R⁸, and R⁸ and R⁹ together with the atoms to which they are attached form a 4- to 8-membered carbocyclic ring or 5- to 7-membered heterocycloalkyl containing 1 to 3 heteroatoms selected from N, O, S, B, or Si, and the carbocyclic ring or heterocycloalkyl is optionally further substituted with 1 to 3 groups selected from =O, 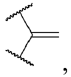 halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, 5- to 7-membered heterocycloalkyl containing 1 to 3 heteroatoms selected from N, O, S, B, or Si, 5-to 7-membered heteroaryl containing 1 to 3 heteroatoms selected from N, O, S, B, or Si and NH₂.

As an alternative to the thirty-first technical solution of the present invention, the compound of the present invention, a stereoisomer, deuterated substance, solvate, or pharmaceutically acceptable salt or eutectic crystal thereof, has the structure of formula (I): 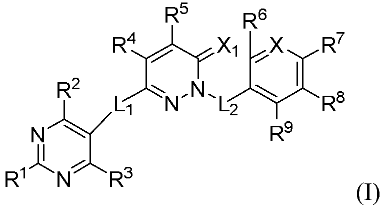 wherein
each of R¹, R² and R³ is independently selected from H, deuterium, R^{A1}, -O-haloC₁₋₄ alkyl, -NH-haloC₁₋₄ alkyl, -OC₁₋₄ alkyl, C₁₋₆ alkoxy, halogen, cyano, nitro, C₁₋₄ alkyl, -NH-C₁₋₄ alkyl, -N(C₁₋₄ alkyl)₂, -NH-C₃₋₁₀ cycloalkyl, -NHC(O)C₁₋₄ alkyl, or 4- to 10-membered heterocycloalkyl containing 1 to 3 heteroatoms selected from N, O, and S, and the alkyl, alkoxy, cycloalkyl, or heterocycloalkyl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, C₃₋₁₀ cycloalkyl, 4- to 6-membered heterocycloalkyl containing 1 to 3 heteroatoms selected from N, O, or S, phenyl, and NH₂, provided that R¹, R² and R³ are not simultaneously selected from H;
R^{A1} is selected from haloC₂₋₆ alkenyl, C₂₋₆ alkynyl, -O-(CH₂)ᵣ-R^{a}, C₃₋₁₀ cycloalkyl, 5- to 10-membered heteroaryl containing 1 to 3 heteroatoms selected from N, O, or S, -C₁₋₄ alkyl-R^{a}, -C₁₋₄ alkyl-OR^{a}, -C₁₋₄ alkyl-NR^{b}R^{a}, -NR^{b}-S(O)₂-R^{a}, -NR^{b}-S(O)₂-NR^{b}R^{a}, -O-NR^{b}R^{a}, -NH-OR^{b}, or -Se-(CH₂)ᵣ-R^{a}, and the alkynyl, heteroaryl, alkyl, or cycloalkyl is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{a} is selected from CN, haloC₁₋₆ alkyl, C₃₋₁₀ cycloalkyl, 4- to 10-membered heterocycloalkyl containing 1 to 3 heteroatoms selected from N, O, or S, 6- to 10-membered aryl, 5- to 10-membered heteroaryl containing 1 to 3 heteroatoms selected from N, O, or S, or -C(O)-R^{a1}, and the alkyl, cycloalkyl, heterocycloalkyl, aryl, or heteroaryl is optionally further substituted with 1 to 3 groups selected from halogen, =O, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{b} is selected from H, deuterium, C₁₋₄ alkyl, C₃₋₆ cycloalkyl, haloC₁₋₄ alkyl, or deuterated C₁₋₄ alkyl;
R^{a1} is selected from OH, NH₂, -NHC₁₋₄ alkyl, -N(C₁₋₄ alkyl)₂, -NHC₃₋₁₀ cycloalkyl, C₁₋₄ alkoxy, C₁₋₆ alkyl, C₃₋₁₀ cycloalkyl, 6- to 10-membered aryl, 5- to 10-membered heteroaryl containing 1 to 3 heteroatoms selected from N, O, or S;
X is selected from CR¹⁰ or N;
X₁ is selected from O or S;
each of R⁴ and R⁵ is independently selected from H, deuterium, C₃₋₁₀ cycloalkyl, C₂₋₆ alkenyl, C₂₋₆ alkynyl, halogen, amino,-NH-C₁₋₄ alkyl, -N(C₁₋₄ alkyl)₂, C₁₋₄ alkyl, haloC₁₋₄ alkyl, 4- to 10-membered heterocycloalkyl containing 1 to 3 heteroatoms selected from N, O, or S, 5- to 10-membered heteroaryl containing 1 to 3 heteroatoms selected from N, O, or S, or 6- to 10-membered aryl;
each of R⁶, R⁷, R⁸, R⁹, and R¹⁰ is independently selected from H, deuterium, R^{A2}, halogen, OH, CN, amino, C₁₋₄ alkyl, C₂₋₆ alkenyl, C₂₋₆ alkynyl, C₁₋₄ alkoxy, haloC₁₋₄ alkyl, -NH-C₁₋₄ alkyl, -N(C₁₋₄ alkyl)₂, -S(O)R^{c}, -S(O)²R^{c}, -C(O)R^{c}, - C(O)OR^{c}, -C(O)N(R^{c})₂, or -OC(O)R^{c}, and the alkyl, alkenyl, alkynyl, or alkoxy is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
R^{A2} is selected from -(CH₂)ᵣ-(4- to 10-membered heterocycloalkyl containing 1 to 3 heteroatoms selected from N, O, S, or Si), or -(CH₂)ᵣ-(5- to 10-membered heteroaryl containing 1 to 3 heteroatoms selected from N, O, or S), -P(O)(R^{c})₂, - Si(R^{c})₃, -SF₅, N₃, B(OH)₂, or -S(O)(=NH)R^{c}, and the heterocycloalkyl or heteroaryl is optionally further substituted with 1 to 3 groups selected from halogen, =O, D, CN, OH, C₁₋₄ alkyl, haloC₁₋₄ alkyl, deuterated C₁₋₄ alkyl, C₁₋₄ alkoxy, haloC₁₋₄ alkoxy, C₃₋₆ cycloalkyl, 4- to 7-membered heterocycloalkyl containing 1 to 3 heteroatoms selected from N, O, or S, deuterated C₁₋₄ alkoxy and NH₂;
each R^{c} is independently selected from H, OH, C₁₋₄ alkyl, C₃₋₇ cycloalkyl, haloC₁₋₄ alkyl, deuterated C₁₋₄ alkyl, C₁₋₄ alkoxy, haloC₁₋₄ alkoxy, deuterated C₁₋₄ alkoxy, or NH₂;
each r is independently selected from 0, 1, 2 or 3;
L₁ is selected from a bond, O, NH, S, -CD₂-, -CHD-, -CR^{L1}R^{L2}-, C₁₋₄ alkyl, - C(O)-, S(O), or S(O)₂, and the alkyl is optionally further substituted with R^{L1};
L₂ is selected from a bond, O, NH, S, -CH₂-, -CD₂-, -CHD-, -CR^{L1}R^{L2}-, - CHR^{L2}- -CDR^{L2}-, C₂₋₄ alkyl, -C(O)-, S(O), or S(O)₂, and the alkyl is optionally further substituted with 1 to 3 R^{L1};
each of R^{L1} and R^{L2} is independently selected from halogen, OH, CN, amino, C₁₋₄ alkyl, C₁₋₄ alkoxy, C₃₋₆ cycloalkyl, or 4- to 6-membered heterocycloalkyl containing 1 to 3 heteroatoms selected from N, O, or S;
alternatively, R², R⁴ and L₁ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
alternatively, R⁴, R⁵ together with the atoms to which they are attached form a 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally substituted with 1 to 3 groups selected from R^{A3};
alternatively, R^{L2} and R⁹ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
each R^{A3} is independently selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂ group substitution;
provided that: one of the combinations of R⁶ and R¹⁰, R¹⁰ and R⁷, R⁷ and R⁸, and R⁸ and R⁹ together with the atoms to which they are attached form a 4- to 8-membered carbocyclic ring or 5- to 7-membered heterocycloalkyl containing 1 to 3 heteroatoms selected from N, O, S, B, or Si, and the carbocyclic ring or heterocycloalkyl is optionally further substituted with 1 to 3 groups selected from =O, 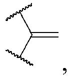 halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, 5- to 7-membered heterocycloalkyl containing 1 to 3 heteroatoms selected from N, O, S, B, or Si, 5-to 7-membered heteroaryl containing 1 to 3 heteroatoms selected from N, O, S, B, or Si and NH₂; in some embodiments, provided that: one of the combinations of R⁶ and R¹⁰, R¹⁰ and R⁷, R⁷ and R⁸, and R⁸ and R⁹ together with the atoms to which they are attached form 4- to 8-membered cycloalkyl or 5- to 7-membered heterocycloalkyl containing 1 to 3 heteroatoms selected from N, O, S, B, or Si, and the cycloalkyl or heterocycloalkyl is optionally further substituted with 1 to 3 groups selected from =O, , halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, 5- to 7-membered heterocycloalkyl containing 1 to 3 heteroatoms selected from N, O, S, B, or Si, 5- to 7-membered heteroaryl containing 1 to 3 heteroatoms selected from N, O, S, B, or Si and NH₂.

A thirty-second technical solution of the present invention is a compound of the present invention, a stereoisomer, a deuterated substance, a solvate, or a pharmaceutically acceptable salt or a eutectic crystal thereof, wherein
each of R⁶, R⁷, R⁸, R⁹, and R¹⁰ is independently selected from H, deuterium, - SF₅, N₃, halogen, OH, CN, amino, C₁₋₂ alkyl, C₂₋₄ alkenyl, C₂₋₄ alkynyl, C₁₋₂ alkoxy, haloC₁₋₂ alkyl, -NH-C₁₋₂ alkyl, or -N(C₁₋₂ alkyl)₂, and the alkyl, alkenyl, alkynyl, or alkoxy is optionally further substituted with 1 to 3 groups selected from halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
and the rest of the groups are as described in any of the above technical solutions.

A thirty-third technical solution of the present invention is a compound of the present invention, a stereoisomer, a deuterated substance, a solvate, or a pharmaceutically acceptable salt or a eutectic crystal thereof, having the structure of formula (II) or (II-a): 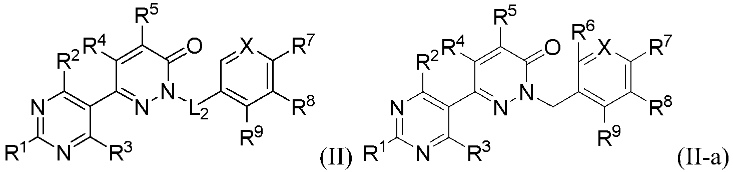
L₂ is selected from a bond, -CH₂-, -CR^{L1}R^{L2}-, -CHR^{L2}-, -CDR^{L2}-, or -C(O)-;
each of R^{L1} and R^{L2} is independently selected from halogen, OH, CN, amino, C₁₋₂ alkyl, C₁₋₂ alkoxy, C₃₋₄ cycloalkyl, or 4-membered heterocycloalkyl containing 1 to 3 heteroatoms selected from N, O, or S;
each of R² and R³ is independently selected from H, deuterium, C₁₋₂ alkoxy, halogen, cyano, nitro, or C₁₋₂ alkyl, and the alkyl or alkoxy is optionally further substituted with 1 to 3 groups selected from halogen, D, CN, OH and NH₂;
R¹ is selected from R^{A1}, -O-haloC₁₋₄ alkyl, -NH-haloC₁₋₄ alkyl, C₁₋₄ alkoxy, halogen, cyano, nitro, C₁₋₄ alkyl, -NH-C₁₋₄ alkyl, -N(C₁₋₄ alkyl)₂, -NH-C₃₋₁₀ cycloalkyl, -NHC(O)C₁₋₄ alkyl, or 4- to 10-membered heterocycloalkyl containing 1 to 3 heteroatoms selected from N, O, and S, and the alkyl, alkoxy, cycloalkyl, or heterocycloalkyl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, C₃₋₁₀ cycloalkyl, 4- to 6-membered heterocycloalkyl containing 1 to 3 heteroatoms selected from N, O, or S, phenyl, and NH₂;
X is selected from CR¹⁰ or N;
each of R⁴ and R⁵ is independently selected from H, deuterium, C₃₋₇ cycloalkyl, C₂₋₄ alkenyl, C₂₋₄ alkynyl, F, Cl, amino,-NH-C₁₋₂ alkyl, -N(C₁₋₂ alkyl)₂, C₁₋₂ alkyl, or 4- to 7-membered heterocycloalkyl containing 1 to 3 heteroatoms selected from N, O, or S;
each of R⁷ and R⁸ is independently selected from H, deuterium, F, Cl, OH, CN, amino, C₁₋₄ alkyl, C₂₋₄ alkenyl, C₂₋₄ alkynyl, C₁₋₄ alkoxy, haloC₁₋₄ alkyl, -NH-C₁₋₂ alkyl, -N(C₁₋₂ alkyl)₂, -S(O)R^{c}, -S(O)²R^{c}, -C(O)R^{c}, -C(O)OR^{c}, -C(O)N(R^{c})₂, or -OC(O)R^{c};
each of R⁶, R⁹, and R¹⁰ is independently selected from H, deuterium, F, Cl, OH, CN, amino, or C₁₋₂ alkyl;
alternatively, R², R⁴ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
each R^{A3} is independently selected from =O, halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
provided that: one of the combinations of R¹⁰ and R⁷, R⁷ and R⁸, and R⁸ and R⁹ together with the atoms to which they are attached form 4- to 8-membered cycloalkyl or 5- to 7-membered heterocycloalkyl containing 1 to 3 heteroatoms selected from N, O, S, B, or Si, and the cycloalkyl or heterocycloalkyl is optionally further substituted with 1 to 3 groups selected from =O, 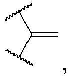 halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, 5- to 7-membered heterocycloalkyl containing 1 to 3 heteroatoms selected from N, O, S, B, or Si, 5- to 7-membered heteroaryl containing 1 to 3 heteroatoms selected from N, O, S, B, or Si and NH₂; in some embodiments, provided that: one of the combinations of R¹⁰ and R⁷, R⁷ and R⁸, and R⁸ and R⁹ together with the atoms to which they are attached form 4- to 8-membered cycloalkyl or 5- to 7-membered heterocycloalkyl containing 1 to 3 heteroatoms selected from N, O, S, B, or Si, and the cycloalkyl or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, 5- to 7-membered heterocycloalkyl containing 1 to 3 heteroatoms selected from N, O, S, B, or Si, 5- to 7-membered heteroaryl containing 1 to 3 heteroatoms selected from N, O, S, B, or Si and NH₂;
and the rest of the groups are as described in any of the above technical solutions.

A thirty-fourth technical solution of the present invention is a compound of the present invention, a stereoisomer, a deuterated substance, a solvate, or a pharmaceutically acceptable salt or a eutectic crystal thereof, having the structure of formula (III) or (III-a): 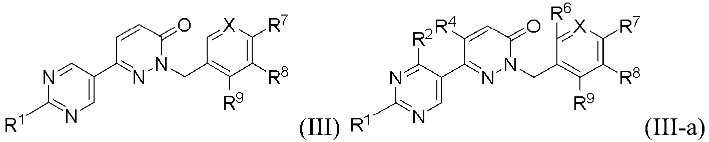
X is selected from CR¹⁰ or N;
each of R⁶, R⁷, R⁸, R⁹ and R¹⁰ is independently selected from H, deuterium, F, Cl, OH, CN, amino, or C₁₋₂ alkyl;
alternatively, R², R⁴ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
provided that: one of the combinations of R¹⁰ and R⁷, R⁷ and R⁸, and R⁸ and R⁹ together with the atoms to which they are attached form 4- to 8-membered cycloalkyl or 5- to 7-membered heterocycloalkyl containing 1 to 3 heteroatoms selected from N, O, S, B, or Si, and the cycloalkyl or heterocycloalkyl is optionally further substituted with 1 to 3 groups selected from =O, 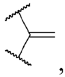 halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, 5- to 7-membered heterocycloalkyl containing 1 to 3 heteroatoms selected from N, O, S, B, or Si, 5- to 7-membered heteroaryl containing 1 to 3 heteroatoms selected from N, O, S, B, or Si and NH₂; and the rest of the groups are as described in any of the above technical solutions.

A thirty-fifth technical solution of the present invention is a compound of the present invention, a stereoisomer, a deuterated substance, a solvate, or a pharmaceutically acceptable salt or a eutectic crystal thereof, wherein
R¹ is selected from R^{A1}, -O-haloC₁₋₄ alkyl or C₁₋₄ alkyl, and the alkyl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each of R² and R³ is independently selected from H, deuterium, C₂₋₄ alkenyl, C₂₋₄ alkynyl, halogen, cyano, nitro, OH, C₁₋₄ alkyl, haloC₁₋₄ alkyl, or deuterated C₁₋₄ alkyl;
R^{A1} is selected from haloC₂₋₄ alkenyl, C₂₋₄ alkynyl, -O-(CH₂)ᵣ-R^{a}, C₃₋₆ monocyclic cycloalkyl, C₅₋₈ bicyclic bridged cycloalkyl, C₆₋₁₀ bicyclic spirocyclic cycloalkyl, 5- to 6-membered monocyclic heteroaryl containing 1 to 3 heteroatoms selected from N, O, or S, 8- to 10-membered bicyclic heteroaryl containing 1 to 3 heteroatoms selected from N, O, or S, -C₁₋₂ alkyl-R^{a}, -C₁₋₂ alkyl-OR^{a}, -C₁₋₂ alkyl-NR^{b}R^{a}, -NR^{b}-S(O)₂-R^{a}, -NR^{b}-S(O)₂-NR^{b}R^{a}, -O-NR^{b}R^{a}, -NH-OR^{b}, or -Se-(CH₂)ᵣ-R^{a}, and the alkynyl, heteroaryl, alkyl, or cycloalkyl is optionally further substituted with 1 to 3 groups selected from halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
each R^{a} is selected from CN, haloC₁₋₄ alkyl, C₃₋₆ monocyclic cycloalkyl, C₇₋₁₀ spirocyclic cycloalkyl, C₄₋₈ bridged cycloalkyl, C₄₋₉ fused cycloalkyl, 4- to 6-membered monocyclic heterocycloalkyl containing 1 to 3 heteroatoms selected from N, O, or S, 7- to 10-membered spirocyclic heterocycloalkyl containing 1 to 3 heteroatoms selected from N, O, or S, 4- to 8-membered bridged heterocycloalkyl containing 1 to 3 heteroatoms selected from N, O, or S, 4- to 6-membered fused heterocycloalkyl containing 1 to 3 heteroatoms selected from N, O, or S, phenyl, 8-to 10- membered aryl, 5- to 6-membered monocyclic heteroaryl containing 1 to 3 heteroatoms selected from N, O, or S, 8- to 10-membered bicyclic heteroaryl containing 1 to 3 heteroatoms selected from N, O, or S, or -C(O)-R^{a1}, and the alkyl, cycloalkyl, heterocycloalkyl, aryl, or heteroaryl is optionally further substituted with 1 to 3 groups selected from halogen, =O, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
each R^{b} is selected from H, deuterium, C₁₋₂ alkyl, or C₃₋₄ cycloalkyl; and the rest of the groups are as described in any of the above technical solutions.

A thirty-sixth technical solution of the present invention is a compound of the present invention, a stereoisomer, a deuterated substance, a solvate, or a pharmaceutically acceptable salt or a eutectic crystal thereof, wherein
R¹ is selected from R^{A1}, -O-haloC₁₋₄ alkyl or C₁₋₄ alkyl, and the alkyl is optionally further substituted with 1 to 3 groups selected from halogen, D, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
each of R² and R³ is independently selected from H, deuterium, F, Cl, cyano, OH, C₁₋₂ alkyl, haloC₁₋₂ alkyl, or deuterated C₁₋₂ alkyl;
R^{A1} is selected from -O-(CH₂)ᵣ-R^{a}, C₃₋₆ monocyclic cycloalkyl, C₅₋₈ bicyclic bridged cycloalkyl, C₆₋₁₀ bicyclic spirocyclic cycloalkyl, 5- to 6-membered monocyclic heteroaryl containing 1 to 3 heteroatoms selected from N, O, or S, 8-to 10-membered bicyclic heteroaryl containing 1 to 3 heteroatoms selected from N, O, or S, -C₁₋₂ alkyl-R^{a}, -C₁₋₂ alkyl-OR^{a}, or -Se-(CH₂)ᵣ-R^{a}, and the heteroaryl, alkyl, or cycloalkyl is optionally further substituted with 1 to 3 groups selected from halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
each R^{a} is selected from haloC₁₋₄ alkyl, C₃₋₆ monocyclic cycloalkyl, C₇₋₁₀ spirocyclic cycloalkyl, C₄₋₈ bridged cycloalkyl, C₄₋₉ fused cycloalkyl, 4- to 6-membered monocyclic heterocycloalkyl containing 1 to 3 heteroatoms selected from N, O, or S, 7- to 10-membered spirocyclic heterocycloalkyl containing 1 to 3 heteroatoms selected from N, O, or S, 4- to 8-membered bridged heterocycloalkyl containing 1 to 3 heteroatoms selected from N, O, or S, 4- to 6-membered fused heterocycloalkyl containing 1 to 3 heteroatoms selected from N, O, or S, phenyl, 8-to 10- membered aryl, 5- to 6-membered monocyclic heteroaryl containing 1 to 3 heteroatoms selected from N, O, or S, or 8- to 10-membered bicyclic heteroaryl containing 1 to 3 heteroatoms selected from N, O, or S, and the alkyl, cycloalkyl, heterocycloalkyl, aryl, or heteroaryl is optionally further substituted with 1 to 3 groups selected from halogen, =O, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂; and the rest of the groups are as described in any of the above technical solutions.

A thirty-seventh technical solution of the present invention is a compound of the present invention, a stereoisomer, a deuterated substance, a solvate, or a pharmaceutically acceptable salt or a eutectic crystal thereof, having the structure of formula (IV) or (V): 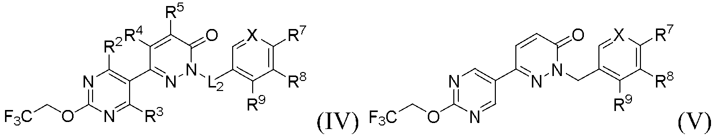
X is selected from CR¹⁰ or N;
each of R⁹ and R¹⁰ is independently selected from H, deuterium, F, Cl, OH, CN, amino, or C₁₋₂ alkyl;
L₂ is selected from a bond, -CH₂-, -CD₂-, -CHD-, -CR^{L1}R^{L2}-, -CHR^{L2}-, - CDR^{L2}-, C₂₋₄ alkyl, or -C(O)-, and the alkyl is optionally further substituted with 1 to 3 R^{L1};
provided that: one of the combinations of R¹⁰ and R⁷, R⁷ and R⁸, and R⁸ and R⁹ together with the atoms to which they are attached form 4- to 8-membered cycloalkyl, or 5- to 7-membered heterocycloalkyl containing 1 to 3 heteroatoms selected from N, O, S, B, or Si, and the cycloalkyl or heterocycloalkyl is optionally further substituted with 1 to 3 groups selected from =O, 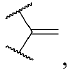 halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂; and the rest of the groups are as described in any of the above technical solutions.

A thirty-eighth technical solution of the present invention is a compound of the present invention, a stereoisomer, a deuterated substance, a solvate, or a pharmaceutically acceptable salt or a eutectic crystal thereof, wherein
one of the combinations of R⁶ and R¹⁰, R¹⁰ and R⁷, R⁷ and R⁸, and R⁸ and R⁹ together with the atoms to which they are attached form 4-, 5-, 6-, 7-, or 8-membered carbocyclic ring or 5-, 6-, or 7-membered heterocycloalkyl containing 1, 2, or 3 heteroatoms selected from N, O, S, B, or Si, and the carbocyclic ring or heterocycloalkyl is optionally further substituted with 1, 2, or 3 groups selected from =O, 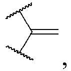 halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy, 5-, 6-, 7-membered heterocycloalkyl containing 1 to 3 heteroatoms selected from N, O, S, B, or Si, 5-, 6-, 7-membered heteroaryl containing 1 to 3 heteroatoms selected from N, O, S, B, or Si and NH₂;
in some embodiments, one of the combinations of R⁶ and R¹⁰, R¹⁰ and R⁷, R⁷ and R⁸, and R⁸ and R⁹ together with the atoms to which they are attached form 4-, 5-, 6-, 7-, or 8-membered cycloalkyl or 5-, 6-, or 7-membered heterocycloalkyl containing 1, 2, or 3 heteroatoms selected from N, O, S, B, or Si, and the cycloalkyl or heterocycloalkyl is optionally further substituted with 1, 2, or 3 groups selected from =O, 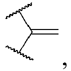 halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy, 5-, 6-, 7-membered heterocycloalkyl containing 1 to 3 heteroatoms selected from N, O, S, B, or Si, 5-, 6-, 7-membered heteroaryl containing 1 to 3 heteroatoms selected from N, O, S, B, or Si and NH₂;
in some embodiments, one of the combinations of R⁶ and R¹⁰, R¹⁰ and R⁷, R⁷ and R⁸, and R⁸ and R⁹ together with the atoms to which they are attached form 4-, 5-, 6-, 7-, or 8-membered cycloalkyl or 5-, 6-, or 7-membered heterocycloalkyl containing 1, 2, or 3 heteroatoms selected from N, O, S, B, or Si, and the cycloalkyl or heterocycloalkyl is optionally further substituted with 1, 2, or 3 groups selected from =O, halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy, 5-, 6-, 7-membered heterocycloalkyl containing 1 to 3 heteroatoms selected from N, O, S, B, or Si, 5-, 6-, 7-membered heteroaryl containing 1 to 3 heteroatoms selected from N, O, S, B, or Si and NH₂;
In some embodiments, one of the combinations of R⁷ and R⁸, and R⁸ and R⁹ together with the atoms to which they are attached form 4-, 5-, 6-, 7-, 8-membered cycloalkyl, 5- or 6-membered heterocycloalkyl containing 1, 2, or 3 heteroatoms selected from N, O, S, B, or Si, and the cycloalkyl or heterocycloalkyl is optionally further substituted with 1, 2, or 3 groups selected from =O, halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
in some embodiments, R⁷ and R⁸ together with the atoms to which they are attached form 4-, 5-, 6-, 7-, 8-membered cycloalkyl, 5- or 6-membered heterocycloalkyl containing 1, 2, or 3 heteroatoms selected from N, O, S, B, or Si, and the cycloalkyl or heterocycloalkyl is optionally further substituted with 1, 2, or 3 groups selected from =O, halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
in some embodiments, R⁷ and R⁸ together with the atoms to which they are attached form 4- or 5-membered cycloalkyl, 5-membered heterocycloalkyl containing 1, 2, or 3 heteroatoms selected from N, O, S, B, or Si, and the cycloalkyl or heterocycloalkyl is optionally further substituted with 1, 2, or 3 groups selected from =O, halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
and the rest of the groups are as described in any of the above technical solutions.

A thirty-ninth technical solution of the present invention is a compound of the present invention, a stereoisomer, a deuterated substance, a solvate, or a pharmaceutically acceptable salt or a eutectic crystal thereof, wherein
L₁ is selected from a bond;
L₂ is selected from a bond, -CH₂-, -CD₂-, -CHD-, -C(O)-, -CHF-, -CDF-, - CF₂-, CH(CH₃)-, CD(CH₃)-, or C(CH₃)₂-;
each of R⁶, R⁹, and R¹⁰ is independently selected from H, deuterium, F, Cl, OH, CN, or methyl;
each of R⁷ and R⁸ is independently selected from H, deuterium, F, Cl, OH, CN, or methyl;
each of R² and R³ is independently selected from H, deuterium, vinyl, or ethynyl;
each of R⁴ and R⁵ is independently selected from H, deuterium, cyclopropyl, vinyl, or ethynyl;
alternatively, R⁴, R⁵ together with the atoms to which they are attached form phenyl, cyclohexyl, cyclopentyl, thienyl, furyl, pyrrolyl, pyrazolyl, or isoxazolyl;
alternatively, R², R⁴, L₁ together with the atoms to which they are attached form cyclohexyl, cycloheptyl, phenyl or cyclopentyl;
alternatively, R^{L1} and R⁹ together with the atoms to which they are attached form cyclopentyl or cyclohexyl;
R¹ is selected from -CF₃, -CH₂CH₃, -CH₂CH₂CH₃, 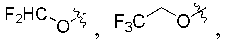 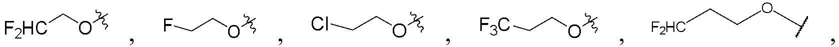 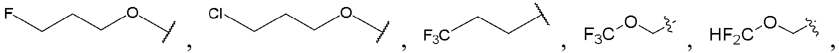 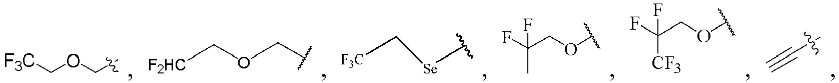 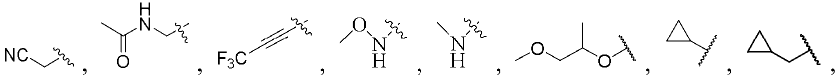 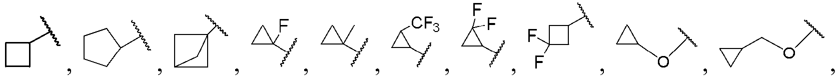 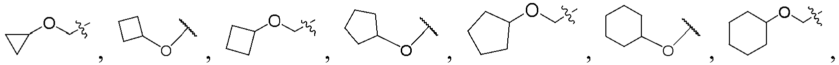 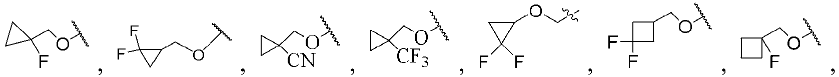 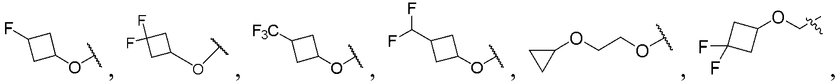 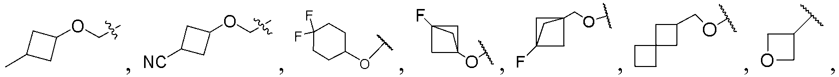 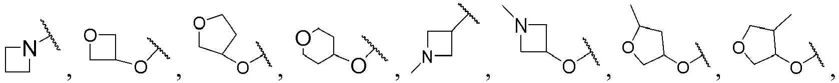 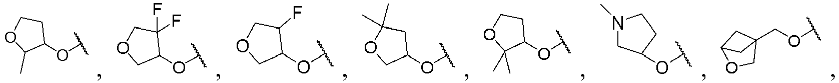 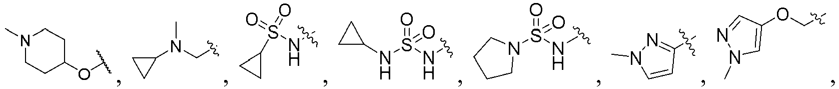 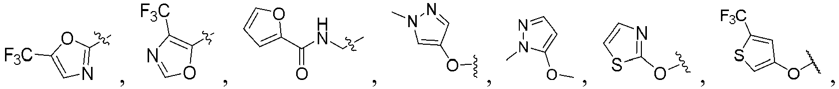 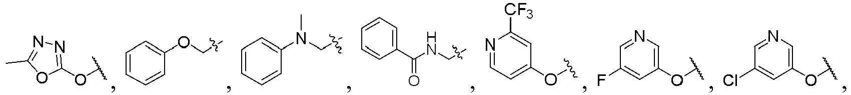 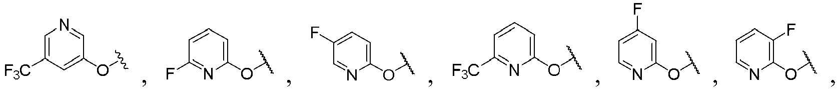 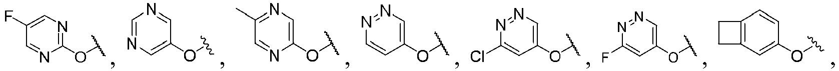 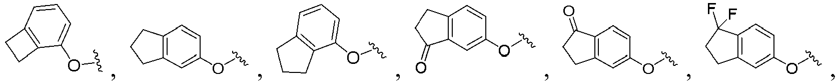 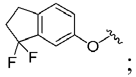
In some embodiments, R¹ is selected from 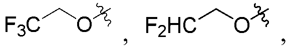 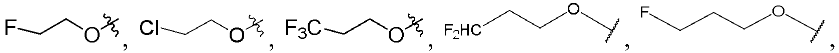 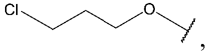 -CH₂CH₃, -CH₂CH₂CH₃, 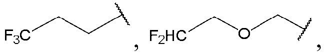 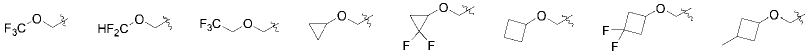 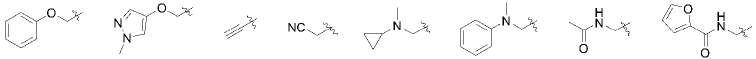 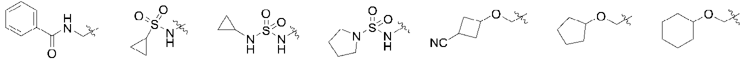 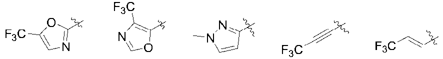 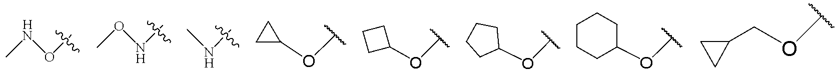 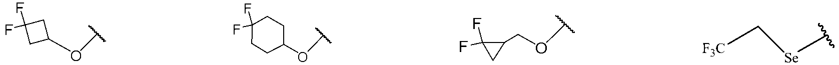 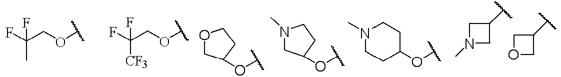 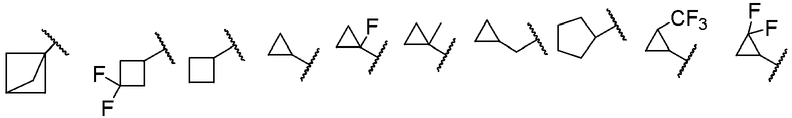 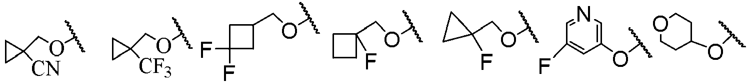 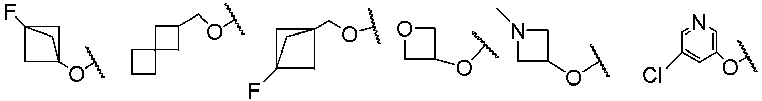 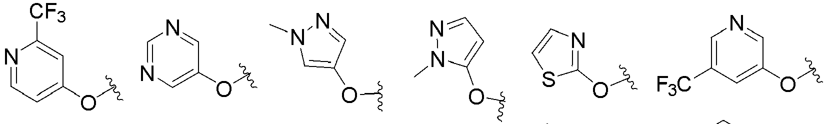 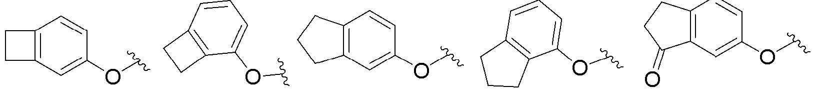 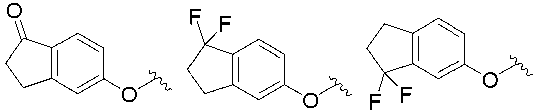 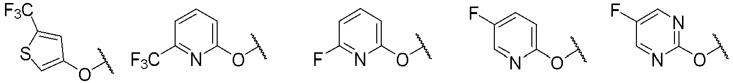 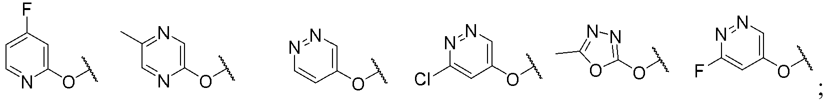
provided that: one of the combinations of R⁶ and R¹⁰, R¹⁰ and R⁷, R⁷ and R⁸, and R⁸ and R⁹ together with the atoms to which they are attached form 4- to 8-membered cycloalkyl or 5- to 7-membered heterocycloalkyl containing 1 Si atom, and the cycloalkyl or heterocycloalkyl is optionally further substituted with 1 to 3 groups selected from =O, F, Cl, deuterium, OH, C₁₋₂ alkyl, haloC₁₋₂ alkyl, 5- to 6-membered heteroaryl containing 1 to 3 heteroatoms selected from N, O, S, B, or Si;
in some embodiments, provided that: one of the combinations of R⁶ and R¹⁰, R¹⁰ and R⁷, R⁷ and R⁸, and R⁸ and R⁹ together with the atoms to which they are attached form 4- to 8-membered cycloalkyl or 5- to 7-membered heterocycloalkyl containing 1 Si atom, and the cycloalkyl or heterocycloalkyl is optionally further substituted with 1 to 3 groups selected from =O, 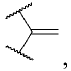 F, Cl, deuterium, OH, C₁₋₂ alkyl, haloC₁₋₂ alkyl, 5- to 6-membered heteroaryl containing 1 to 3 heteroatoms selected from N, O, S, B, or Si;
in some embodiments, provided that: one of the combinations of R⁶ and R¹⁰, R¹⁰ and R⁷, R⁷ and R⁸, and R⁸ and R⁹ together with the atoms to which they are attached form 4-, 5- or 6-membered cycloalkyl or 5- or 6-membered heterocycloalkyl containing 1 Si atom, and the cycloalkyl or heterocycloalkyl is optionally further substituted with 1 to 3 groups selected from =O, 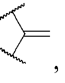 F, Cl, deuterium, OH, C₁₋₂ alkyl, haloC₁₋₂ alkyl, 5- to 6-membered heteroaryl containing 1 to 3 heteroatoms selected from N, O, S, B, or Si;
and the rest of the groups are as described in any of the above technical solutions.

Further, provided is a compound of the present invention, a stereoisomer, a deuterated substance, a solvate, or a pharmaceutically acceptable salt or a eutectic crystal thereof, having the structure of formula (VA): 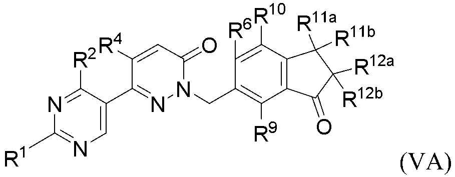
each of R^{11a}, R^{11b}, R^{12a}, and R^{12b} is independently selected from H, deuterium, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, or C₃₋₆ cycloalkyl; alternatively, any two of R^{11a}, R^{11b}, R^{12a}, and R^{12b} together with the atoms to which they are attached form C₃₋₆ cycloalkyl, which is optionally further substituted with 1 to 3 groups selected from =O, 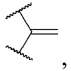 halogen, deuterium, CN, OH, C₁₋₃ alkyl, haloC₁₋₃ alkyl, deuterated C₁₋₃ alkyl, C₁₋₃ alkoxy, haloC₁₋₃ alkoxy, or deuterated C₁₋₃ alkoxy;
in certain embodiments, each of R^{11a}, R^{11b}, R^{12a}, and R^{12b} is independently selected from H, deuterium, OH, C₁₋₃ alkyl, or haloC₁₋₃ alkyl; alternatively, R^{11a} and R^{11b}, or R^{12a} and R^{12b} together with the atoms to which they are attached form C₃₋₆ cycloalkyl;
in certain embodiments, each of R^{11a}, R^{11b}, R^{12a}, and R^{12b} is independently selected from H, deuterium, OH, C₁₋₃ alkyl, or haloC₁₋₃ alkyl; alternatively, R^{11a} and R^{11b}, or R^{12a} and R^{12b} together with the atoms to which they are attached form cyclopropyl;
and the rest of the groups are as described in any of the above technical solutions.

A fortieth technical solution of the present invention is a compound of the present invention, a stereoisomer, a deuterated substance, a solvate, or a pharmaceutically acceptable salt or a eutectic crystal thereof, having the structure of formula (I): 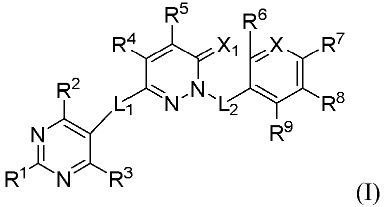 wherein
each of R¹, R² and R³ is independently selected from H, deuterium, R^{A1}, -O-haloC₁₋₄ alkyl, -NH-haloC₁₋₄ alkyl, -OC₁₋₄ alkyl, C₁₋₄ alkoxy, C₅₋₆ alkoxy, halogen, cyano, nitro, C₁₋₄ alkyl, C₂₋₆ alkenyl, -NH-C₁₋₄ alkyl, -N(C₁₋₄ alkyl)₂, -NH-C₃₋₁₀ cycloalkyl, -NHC(O)C₁₋₄ alkyl, or 4- to 10-membered heterocycloalkyl containing 1 to 3 heteroatoms selected from N, O, and S, and the alkyl, alkoxy, cycloalkyl, or heterocycloalkyl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, C₃₋₁₀ cycloalkyl, 4- to 6-membered heterocycloalkyl containing 1 to 3 heteroatoms selected from N, O, or S, phenyl, and NH₂, provided that R¹, R² and R³ are not simultaneously selected from H;
R^{A1} is selected from haloC₂₋₆ alkenyl, C₂₋₆ alkynyl, -O-(CH₂)ᵣ-R^{a}, C₃₋₁₀ cycloalkyl, 5- to 10-membered heteroaryl containing 1 to 3 heteroatoms selected from N, O, or S, -C₁₋₄ alkyl-R^{a}, -C₁₋₄ alkyl-OR^{a}, -C₁₋₄ alkyl-NR^{b}R^{a}, -NR^{b}-S(O)₂-R^{a}, -NR^{b}-S(O)₂-NR^{b}R^{a}, -O-NR^{b}R^{a}, -NH-OR^{b}, or -Se-(CH₂)ᵣ-R^{a}, and the alkynyl, heteroaryl, alkyl, or cycloalkyl is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{a} is selected from CN, haloC₁₋₆ alkyl, C₃₋₁₀ cycloalkyl, 4- to 10-membered heterocycloalkyl containing 1 to 3 heteroatoms selected from N, O, or S, 6- to 10-membered aryl, 5- to 10-membered heteroaryl containing 1 to 3 heteroatoms selected from N, O, or S, or -C(O)-R^{a1}, and the alkyl, cycloalkyl, heterocycloalkyl, aryl, or heteroaryl is optionally further substituted with 1 to 3 groups selected from halogen, =O, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{b} is selected from H, deuterium, C₁₋₄ alkyl, C₃₋₆ cycloalkyl, haloC₁₋₄ alkyl, or deuterated C₁₋₄ alkyl;
R^{a1} is selected from OH, NH₂, -NHC₁₋₄ alkyl, -N(C₁₋₄ alkyl)₂, -NHC₃₋₁₀ cycloalkyl, C₁₋₄ alkoxy, C₁₋₆ alkyl, C₃₋₁₀ cycloalkyl, 6- to 10-membered aryl, 5- to 10-membered heteroaryl containing 1 to 3 heteroatoms selected from N, O, or S;
X is selected from CR¹⁰ or N;
X₁ is selected from O or S;
each of R⁴ and R⁵ is independently selected from H, deuterium, C₃₋₁₀ cycloalkyl, C₂₋₆ alkenyl, C₂₋₆ alkynyl, halogen, amino,-NH-C₁₋₄ alkyl, -N(C₁₋₄ alkyl)₂, C₁₋₄ alkyl, haloC₁₋₄ alkyl, 4- to 10-membered heterocycloalkyl containing 1 to 3 heteroatoms selected from N, O, or S, 5- to 10-membered heteroaryl containing 1 to 3 heteroatoms selected from N, O, or S, or 6- to 10-membered aryl;
each of R⁶, R⁷, R⁸, R⁹, and R¹⁰ is independently selected from H, deuterium, R^{A2}, halogen, OH, CN, amino, C₁₋₄ alkyl, C₂₋₆ alkenyl, C₂₋₆ alkynyl, C₁₋₄ alkoxy, haloC₁₋₄ alkyl, -NH-C₁₋₄ alkyl, -N(C₁₋₄ alkyl)₂, -S(O)R^{c}, -S(O)²R^{c}, -C(O)R^{c}, - C(O)OR^{c}, -C(O)N(R^{c})₂, or -OC(O)R^{c}, and the alkyl, alkenyl, alkynyl, or alkoxy is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
R^{A2} is selected from -(CH₂)ᵣ-(4- to 10-membered heterocycloalkyl containing 1 to 3 heteroatoms selected from N, O, S, or Si), or -(CH₂)ᵣ-(5- to 10-membered heteroaryl containing 1 to 3 heteroatoms selected from N, O, or S), -P(O)(R^{c})₂, - Si(R^{c})₃, -SF₅, N₃, B(OH)₂, or -S(O)(=NH)R^{c}, and the heterocycloalkyl or heteroaryl is optionally further substituted with 1 to 3 groups selected from halogen, =O, D, CN, OH, C₁₋₄ alkyl, haloC₁₋₄ alkyl, deuterated C₁₋₄ alkyl, C₁₋₄ alkoxy, haloC₁₋₄ alkoxy, C₃₋₆ cycloalkyl, 4- to 7-membered heterocycloalkyl containing 1 to 3 heteroatoms selected from N, O, or S, deuterated C₁₋₄ alkoxy and NH₂;
each R^{c} is independently selected from H, OH, C₁₋₄ alkyl, C₃₋₇ cycloalkyl, haloC₁₋₄ alkyl, deuterated C₁₋₄ alkyl, C₁₋₄ alkoxy, haloC₁₋₄ alkoxy, deuterated C₁₋₄ alkoxy, or NH₂;
each r is independently selected from 0, 1, 2 or 3;
L₁ is selected from a bond, O, NH, S, -CD₂-, -CHD-, -CR^{L1}R^{L2}-, C₁₋₄ alkyl, - C(O)-, S(O), or S(O)₂, and the alkyl is optionally further substituted with R^{L1};
L₂ is selected from O, NH, S, -CH₂-, -CD₂-, -CHD-, -CR^{L1}R^{L2}-, -CHR^{L2}-, - CDR^{L2}-, C₂₋₄ alkyl, -C(O)-, S(O), S(O)₂, the alkyl is optionally further substituted with 1 to 3 R^{L1};
each of R^{L1} and R^{L2} is independently selected from halogen, OH, CN, amino, C₁₋₄ alkyl, C₁₋₄ alkoxy, C₃₋₆ cycloalkyl, or 4- to 6-membered heterocycloalkyl containing 1 to 3 heteroatoms selected from N, O, or S;
alternatively, R², R⁴ and L₁ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
alternatively, R⁴, R⁵ together with the atoms to which they are attached form a 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally substituted with 1 to 3 groups selected from R^{A3};
alternatively, R^{L2} and R⁹ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
each R^{A3} is independently selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂ group substitution;
provided that: one of the combinations of R⁶ and R¹⁰, R¹⁰ and R⁷, R⁷ and R⁸, and R⁸ and R⁹ together with the atoms to which they are attached form 4- to 8-membered carbocyclic ring, or 5- to 7-membered heterocycloalkyl containing 1 to 3 heteroatoms selected from N, O, S, B, or Si, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂.

A forty-first technical solution of the present invention is a compound of the present invention, a stereoisomer, a deuterated substance, a solvate, or a pharmaceutically acceptable salt or a eutectic crystal thereof, having the structure of formula (II): 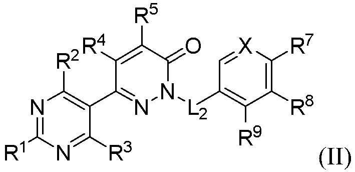
L₂ is selected from -CH₂-, -CR^{L1}R^{L2}-, -CHR^{L2}-, -CDR^{L2}-, or -C(O)-;
X is selected from CR¹⁰ or N;
each of R⁴ and R⁵ is independently selected from H, deuterium, C₃₋₇ cycloalkyl, C₂₋₄ alkenyl, C₂₋₄ alkynyl, F, Cl, amino,-NH-C₁₋₂ alkyl, -N(C₁₋₂ alkyl)₂, C₁₋₂ alkyl, or 4- to 7-membered heterocycloalkyl containing 1 to 3 heteroatoms selected from N, O, or S;
each of R⁷ and R⁸ is independently selected from H, deuterium, R^{A2}, F, Cl, OH, CN, amino, C₁₋₄ alkyl, C₂₋₄ alkenyl, C₂₋₄ alkynyl, C₁₋₄ alkoxy, haloC₁₋₄ alkyl, - NH-C₁₋₂ alkyl, -N(C₁₋₂ alkyl)₂, -S(O)R^{c}, -S(O)²R^{c}, -C(O)R^{c}, -C(O)OR^{c}, - C(O)N(R^{c})₂, or -OC(O)R^{c};
each of R⁹ and R¹⁰ is independently selected from H, deuterium, F, Cl, OH, CN, amino, or C₁₋₂ alkyl;
R^{A2} is selected from -(CH₂)ᵣ-(4- to 6-membered monocyclic heterocycloalkyl containing 1 to 3 heteroatoms selected from N, O, or S), -(CH₂)ᵣ-(7- to 10-membered spirocyclic heterocycloalkyl containing 1 to 3 heteroatoms selected from N, O, or S), -(CH₂)ᵣ-(5- to 6-membered monocyclic heteroaryl containing 1 to 3 heteroatoms selected from N, O, or S), -(CH₂)ᵣ-(8- to 10-membered bicyclic heteroaryl containing 1 to 3 heteroatoms selected from N, O, or S), -P(O)(R^{c})₂, - Si(R^{c})₃, -SF₅, or -S(O)(=NH)R^{c}, and the heterocycloalkyl or heteroaryl is optionally further substituted with 1 to 3 groups selected from halogen, D, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
provided that: one of the combinations of R¹⁰ and R⁷, R⁷ and R⁸, and R⁸ and R⁹ together with the atoms to which they are attached form 4- to 8-membered carbocyclic ring, or 5- to 7-membered heterocycloalkyl containing 1 to 3 heteroatoms selected from N, O, S, B, or Si, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂; and the rest of the groups are as described in any of the above technical solutions.

A forty-second technical solution of the present invention is a compound of the present invention, a stereoisomer, a deuterated substance, a solvate, or a pharmaceutically acceptable salt or a eutectic crystal thereof, wherein
R², R⁴, L₁ together with the atoms to which they are attached form 5- to 7-membered cycloalkyl, or phenyl, and the cycloalkyl is optionally further substituted with 1 to 3 groups selected from R^{A3}, and R¹ is not heterocycloalkyl; or
R⁴, R⁵ together with the atoms to which they are attached form phenyl, 5- to 6-membered cycloalkyl, or 5- to 6-membered heteroaryl containing 1 to 3 heteroatoms selected from N, O, or S, and the cycloalkyl is optionally substituted with 1-3 groups selected from R^{A3}, and R¹ is selected from R^{A1} or -O-haloC₁₋₄ alkyl; or
R^{L2} and R⁹ together with the atoms to which they are attached form 5- to 6-membered cycloalkyl, and the cycloalkyl is optionally further substituted with 1 to 3 groups selected from R^{A3};
each R^{A3} is independently selected from =O, halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
and the rest of the groups are as described in any of the above technical solutions.

A forty-third technical solution of the present invention is a compound of the present invention, and a stereoisomer, deuterated substance, solvate, or pharmaceutically acceptable salt thereof, wherein
R¹ is selected from R^{A1} or -O-haloC₁₋₄ alkyl, and the alkyl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each of R² and R³ is independently selected from H, deuterium, C₂₋₄ alkenyl, C₂₋₄ alkynyl, halogen, cyano, nitro, OH, C₁₋₄ alkyl, haloC₁₋₄ alkyl, or deuterated C₁₋₄ alkyl;
R^{A1} is selected from haloC₂₋₄ alkenyl, C₂₋₄ alkynyl, -O-(CH₂)ᵣ-R^{a}, C₃₋₆ monocyclic cycloalkyl, 5- to 6-membered monocyclic heteroaryl containing 1 to 3 heteroatoms selected from N, O, or S, 8- to 10-membered bicyclic heteroaryl containing 1 to 3 heteroatoms selected from N, O, or S, -C₁₋₂ alkyl-R^{a}, -C₁₋₂ alkyl-OR^{a}, -C₁₋₂ alkyl-NR^{b}R^{a}, -NR^{b}-S(O)₂-R^{a}, -NR^{b}-S(O)₂-NR^{b}R^{a}, -O-NR^{b}R^{a}, -NH-OR^{b}, or -Se-(CH₂)ᵣ-R^{a}, and the alkynyl, heteroaryl, alkyl or cycloalkyl is optionally further substituted with 1 to 3 groups selected from halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
r is selected from 0 or 1;
each R^{a} is selected from CN, haloC₁₋₄ alkyl, C₃₋₆ monocyclic cycloalkyl, C₇₋₁₀ spirocyclic cycloalkyl, C₄₋₈ bridged cycloalkyl, C₄₋₉ fused cycloalkyl, 4- to 6-membered monocyclic heterocycloalkyl containing 1 to 3 heteroatoms selected from N, O, or S, 7- to 10-membered spirocyclic heterocycloalkyl containing 1 to 3 heteroatoms selected from N, O, or S, 4- to 8-membered bridged heterocycloalkyl containing 1 to 3 heteroatoms selected from N, O, or S, 4- to 6-membered fused heterocycloalkyl containing 1 to 3 heteroatoms selected from N, O, or S, phenyl, 8-to 10- membered aryl, 5- to 6-membered monocyclic heteroaryl containing 1 to 3 heteroatoms selected from N, O, or S, 8- to 10-membered bicyclic heteroaryl containing 1 to 3 heteroatoms selected from N, O, or S, or -C(O)-R^{a1}, and the alkyl, cycloalkyl, heterocycloalkyl, aryl, or heteroaryl is optionally further substituted with 1 to 3 groups selected from halogen, =O, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
each R^{b} is selected from H, deuterium, C₁₋₂ alkyl, or C₃₋₄ cycloalkyl;
and the rest of the groups are as described in any of the above technical solutions.

A forty-fourth technical solution of the present invention is a compound of the present invention, a stereoisomer, a deuterated substance, a solvate, or a pharmaceutically acceptable salt or a eutectic crystal thereof, having the structure of formula (III): 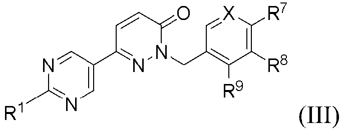
X is selected from CR¹⁰ or N;
each of R⁹ and R¹⁰ is independently selected from H, deuterium, F, Cl, OH, CN, amino, or C₁₋₂ alkyl;
provided that: one of the combinations of R¹⁰ and R⁷, R⁷ and R⁸, and R⁸ and R⁹ together with the atoms to which they are attached form 4- to 8-membered carbocyclic ring, or 5- to 7-membered heterocycloalkyl containing 1 to 3 heteroatoms selected from N, O, S, B, or Si, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
and the rest of the groups are as described in any of the above technical solutions.

A forty-fifth technical solution of the present invention is a compound of the present invention, a stereoisomer, a deuterated substance, a solvate, or a pharmaceutically acceptable salt or a eutectic crystal thereof, having the structure of formula (IV) or (V): 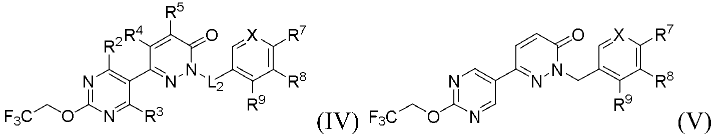
X is selected from CR¹⁰ or N;
each of R⁹ and R¹⁰ is independently selected from H, deuterium, F, Cl, OH, CN, amino, or C₁₋₂ alkyl;
L₂ is selected from -CH₂-, -CD₂-, -CHD-, -CR^{L1}R^{L2}-, -CHR^{L2}-, -CDR^{L2}-, C₂₋₄ alkyl, or -C(O)-, and the alkyl is optionally further substituted with 1 to 3 R^{L1};
provided that: one of the combinations of R¹⁰ and R⁷, R⁷ and R⁸, and R⁸ and R⁹ together with the atoms to which they are attached form 4- to 8-membered carbocyclic ring, or 5- to 7-membered heterocycloalkyl containing 1 to 3 heteroatoms selected from N, O, S, B, or Si, and the carbocyclic ring or heterocycloalkyl is optionally further substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂; and the rest of the groups are as described in any of the above technical solutions.

A forty-sixth technical solution of the present invention is a compound of the present invention, a stereoisomer, a deuterated substance, a solvate, or a pharmaceutically acceptable salt or a eutectic crystal thereof, R^{A2} is selected from - (CH₂)ᵣ-(4- to 6-membered monocyclic heterocycloalkyl containing 1 to 3 heteroatoms selected from N, O, or S), or -(CH₂)ᵣ-(5- to 6-membered monocyclic heteroaryl containing 1 to 3 heteroatoms selected from N, O, or S), and the heterocycloalkyl or heteroaryl is optionally further substituted with 1 to 3 groups selected from halogen, D, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
r is selected from 0, 1;
and the rest of the groups are as described in any of the above technical solutions.

A forty-seventh technical solution of the present invention is a compound of the present invention, a stereoisomer, a deuterated substance, a solvate, or a pharmaceutically acceptable salt or a eutectic crystal thereof, wherein
L₁ is selected from a bond, or O;
L₂ is selected from -CH₂-, -C(O)-, -CHF-, -CF₂-, or C(CH₃)₂-;
each of R^{L1} and R^{L2} is independently selected from H, deuterium, or F;
each of R⁶, R⁹, and R¹⁰ is independently selected from H, deuterium, F, Cl, OH, CN, or methyl;
each of R⁷ and R⁸ is independently selected from H, deuterium, F, Cl, OH, CN, methyl, or R^{A2}
R^{A2} is selected from 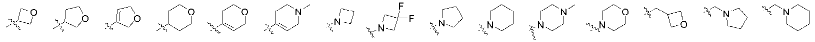 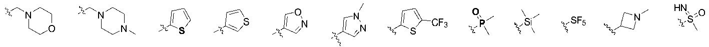 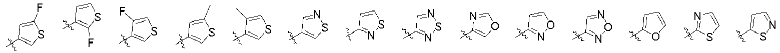 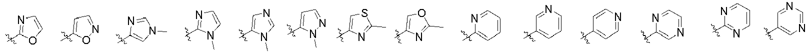 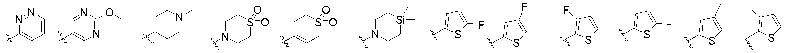 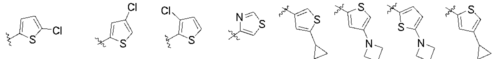 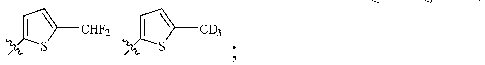
each of R² and R³ is independently selected from H, deuterium, vinyl, or ethynyl;
each of R⁴ and R⁵ is independently selected from H, deuterium, cyclopropyl, vinyl, or ethynyl;
alternatively, R⁴, R⁵ together with the atoms to which they are attached form phenyl, cyclohexyl, cyclopentyl, thienyl, furyl, pyrrolyl, pyrazolyl, or isoxazolyl;
alternatively, R², R⁴, L₁ together with the atoms to which they are attached form cyclohexyl, phenyl or cyclopentyl;
alternatively, R^{L1} and R⁹ together with the atoms to which they are attached form cyclopentyl or cyclohexyl;
R¹ is selected from 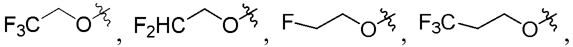 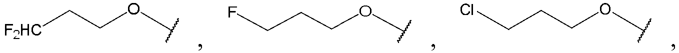 -CH₂CH₃, - CH₂CH₂CH₃, 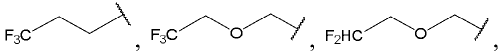 R^{A1};
R^{A1} is selected from 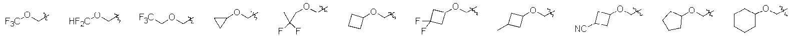 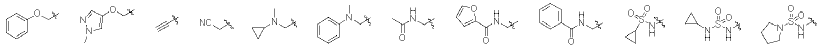 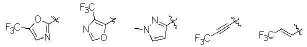 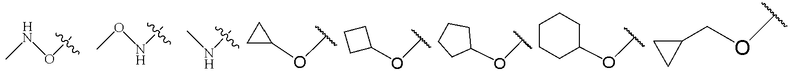 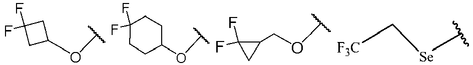 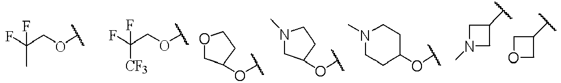 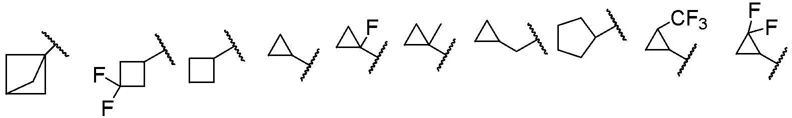 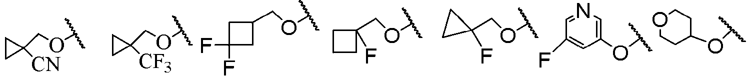 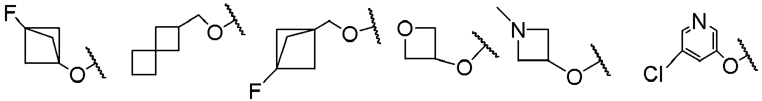 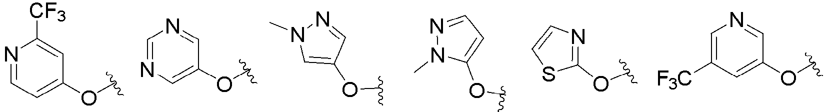 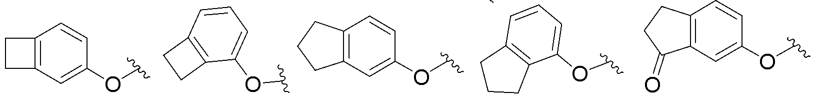 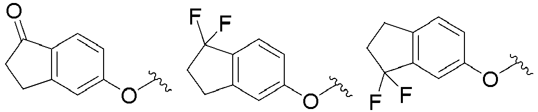 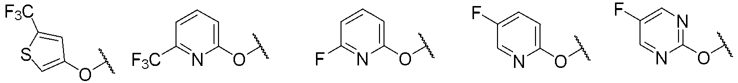 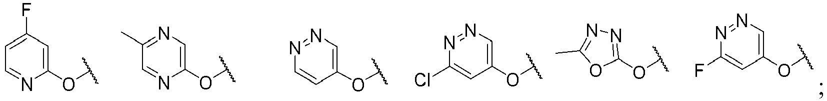
provided that: one of the combinations of R⁶ and R¹⁰, R¹⁰ and R⁷, R⁷ and R⁸, and R⁸ and R⁹ together with the atoms to which they are attached form 4- to 8-membered carbocyclic ring or 5- to 7-membered heterocycloalkyl containing 1 Si atom, and the carbocyclic ring or heterocycloalkyl is optionally further substituted with 1 to 3 groups selected from =O, F, Cl, deuterium, OH, C₁₋₂ alkyl, or haloC₁₋₂ alkyl; and the rest of the groups are as described in any of the above technical solutions.

Further, The compound of formula (I), formula (I-a), formula (I-b), formula (II), formula (II-a), formula (III), formula (III-a), formula (IV), formula (V), or formula (VA) of the present invention, and a stereoisomer, a deuterated substance, a solvate, or a pharmaceutically acceptable salt or a eutectic crystal thereof, wherein R⁷ and R⁸ together with the atoms to which they are attached form C₄₋₈ cycloalkyl, and the cycloalkyl is optionally further substituted with 1 to 3 groups selected from =O, 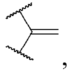 halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, 5- to 7-membered heterocycloalkyl containing 1 to 3 heteroatoms selected from N, O, S, B, or Si, 5- to 7-membered heteroaryl containing 1 to 3 heteroatoms selected from N, O, S, B, or Si and NH₂.

Further, The compound of formula (I), formula (I-a), formula (I-b), formula (II), formula (II-a), formula (III), formula (III-a), formula (IV), formula (V), or formula (VA) of the present invention, and a stereoisomer, a deuterated substance, a solvate, or a pharmaceutically acceptable salt or a eutectic crystal thereof, wherein R⁷ and R⁸ together with the atoms to which they are attached form C₄₋₆ monocyclic cycloalkyl, C₆₋₈ bicyclic bridged cycloalkyl, C₆₋₈ bicyclic fused cycloalkyl, or C₆₋₈ bicyclic spiro cycloalkyl, and the cycloalkyl is optionally further substituted with 1 to 3 groups selected from =O, 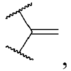 halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂.

Further, the compound of formula (I), formula (I-a), formula (I-b), formula (II), formula (II-a), formula (III), formula (III-a), formula (IV), formula (V), or formula (VA) of the present invention, and a stereoisomer, a deuterated substance, a solvate, or a pharmaceutically acceptable salt or a eutectic crystal thereof, wherein R⁷ and R⁸ together with the atoms to which they are attached form C₄₋₆ monocyclic cycloalkyl, and the cycloalkyl is optionally further substituted with 1 to 3 groups selected from =O, 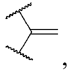 halogen, deuterium, CN, OH, C1-4 alkyl, haloC1-4 alkyl, deuterated C1-4 alkyl, C₁₋₄ alkoxy, haloC₁₋₄ alkoxy, deuterated C₁₋₄ alkoxy and NH₂.

Further, the compound of formula (I), formula (I-a), formula (I-b), formula (II), formula (II-a), formula (III), formula (III-a), formula (IV), formula (V), or formula (VA) of the present invention, and a stereoisomer, a deuterated substance, a solvate, or a pharmaceutically acceptable salt or a eutectic crystal thereof, wherein R⁷ and R⁸ together with the atoms to which they are attached form cyclobutyl, cyclopentyl, cyclohexyl, cyclobutenyl, cyclopentenyl, cyclohexenyl, 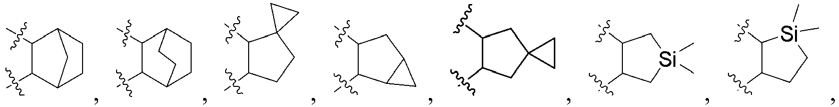 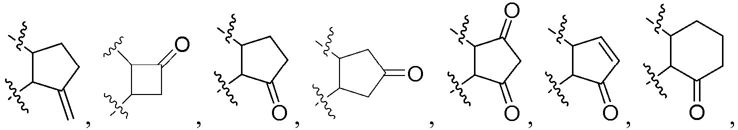 which is optionally further substituted with 1 to 3 groups selected from halogen, deuterium, CN, OH, C₁₋₃ alkyl, haloC₁₋₃ alkyl, deuterated C₁₋₃ alkyl, C₁₋₃ alkoxy, haloC₁₋₃ alkoxy, deuterated C₁₋₃ alkoxy and NH₂.

The compound of formula (I), formula (I-a), formula (I-b), formula (II), formula (II-a), formula (III), formula (III-a), formula (IV), formula (V), formula (VA), formula (VI), formula (VII), formula (VIII), or formula (IX) of the present invention, a stereoisomer, a deuterated substance, a solvate, or a pharmaceutically acceptable salt or a eutectic crystal thereof, wherein the compound is selected from but not limited to the structures in Table I and Table II below:

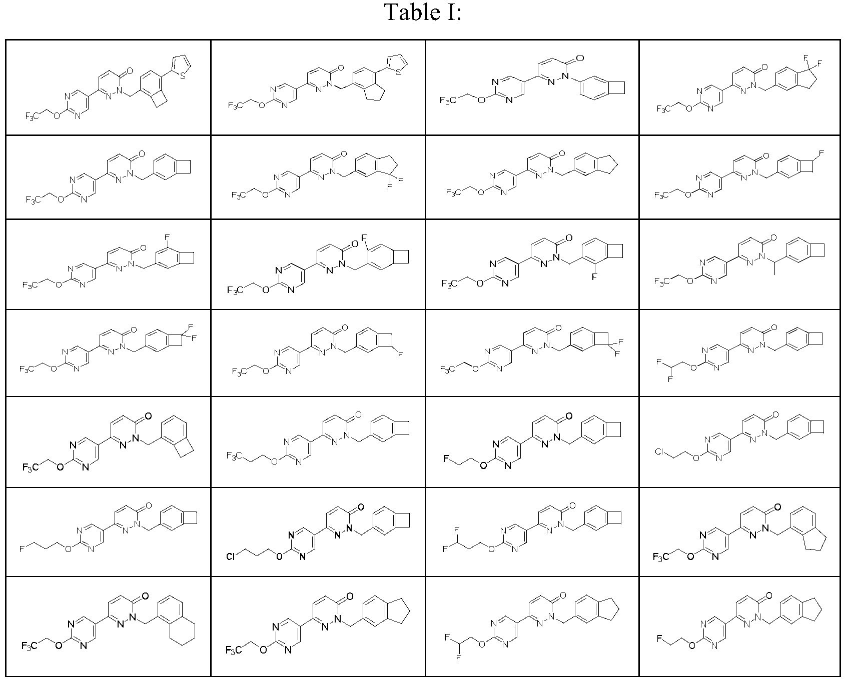

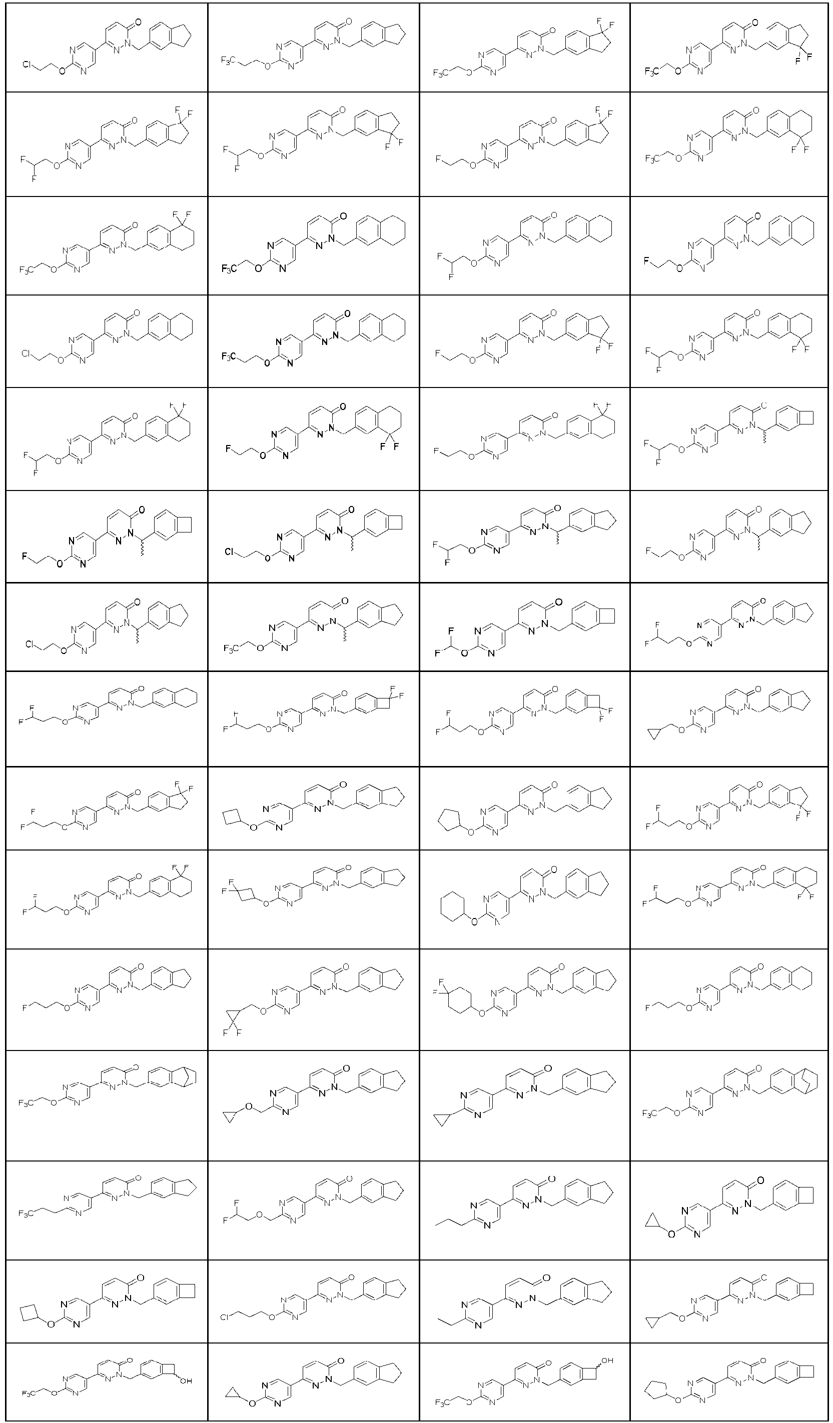

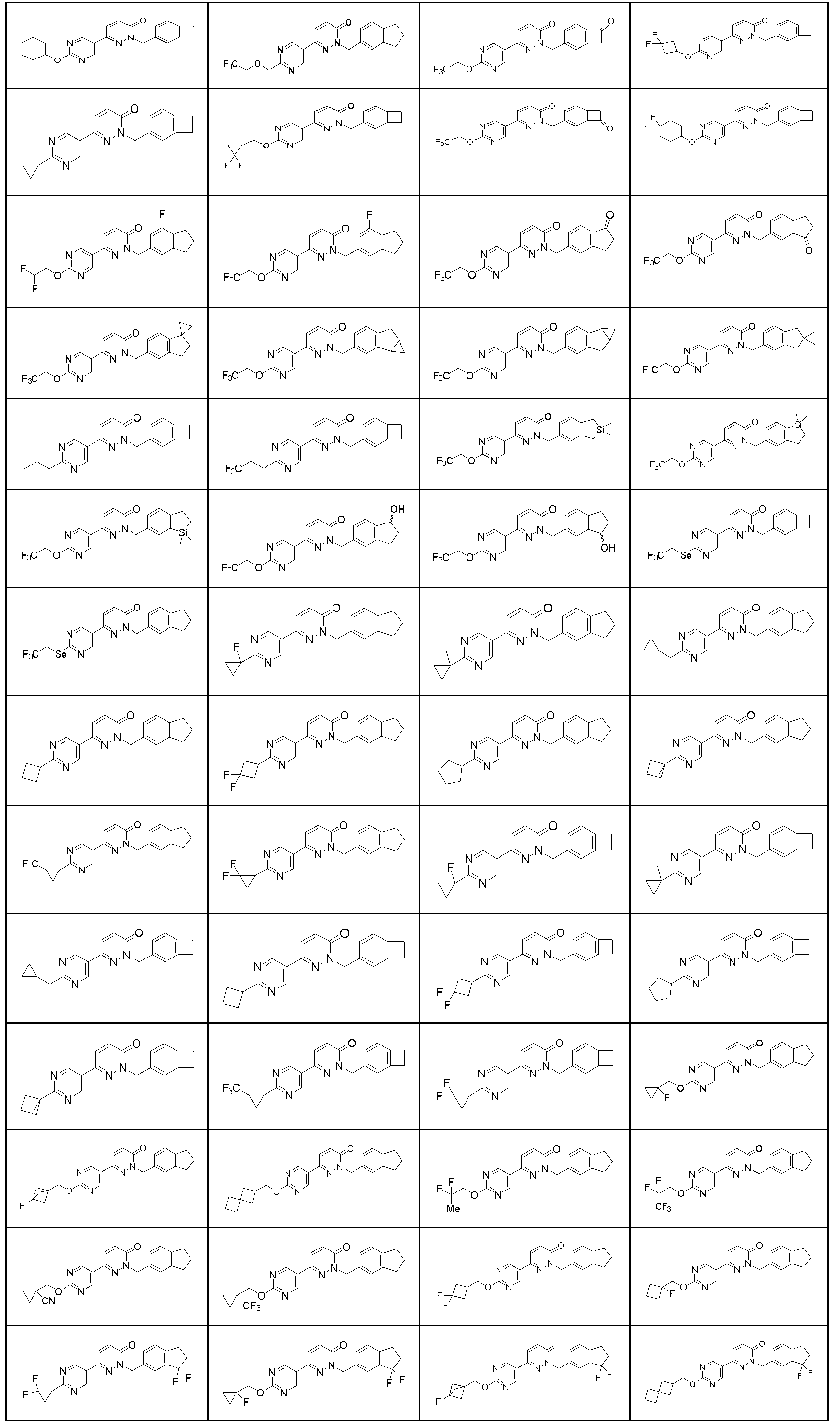

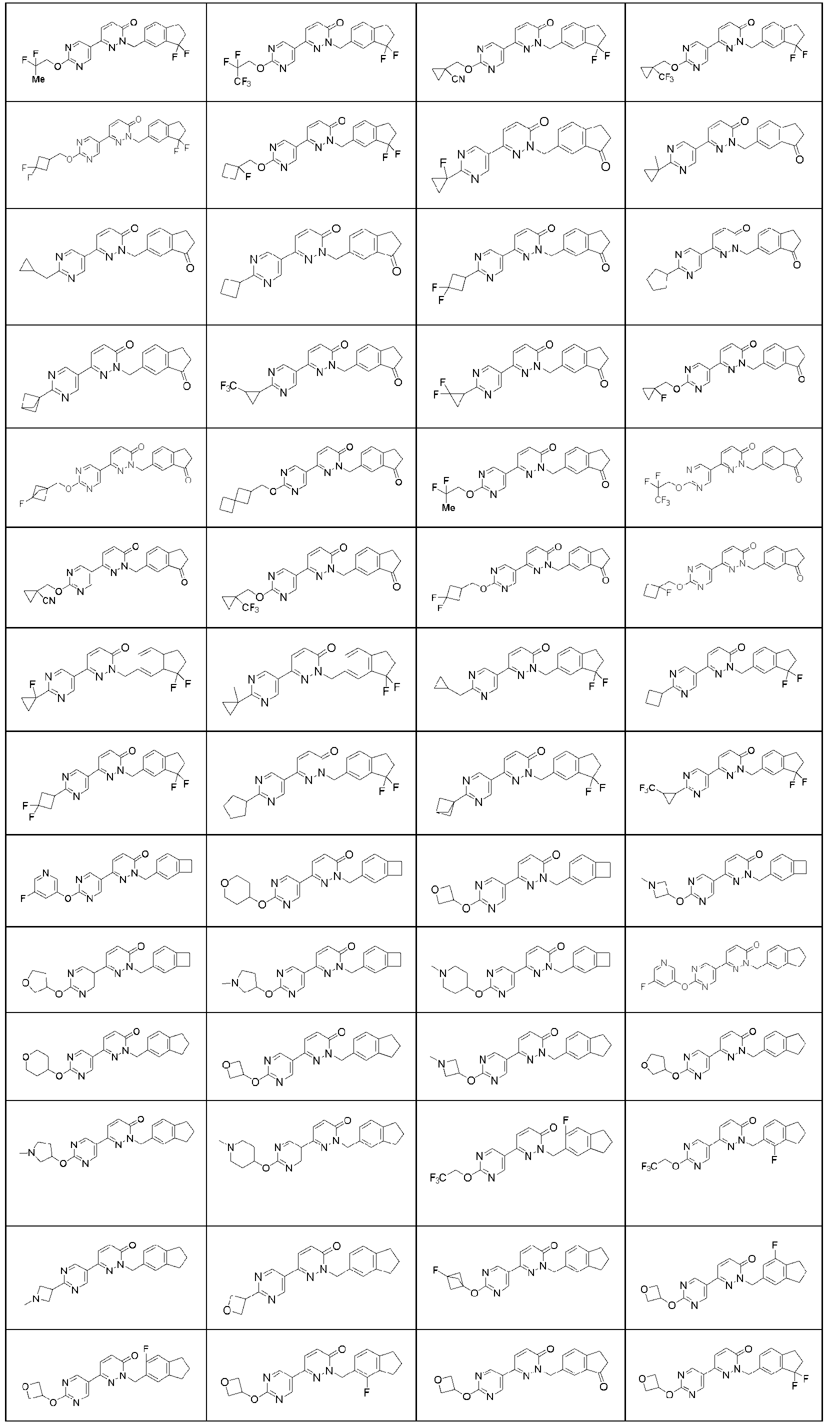

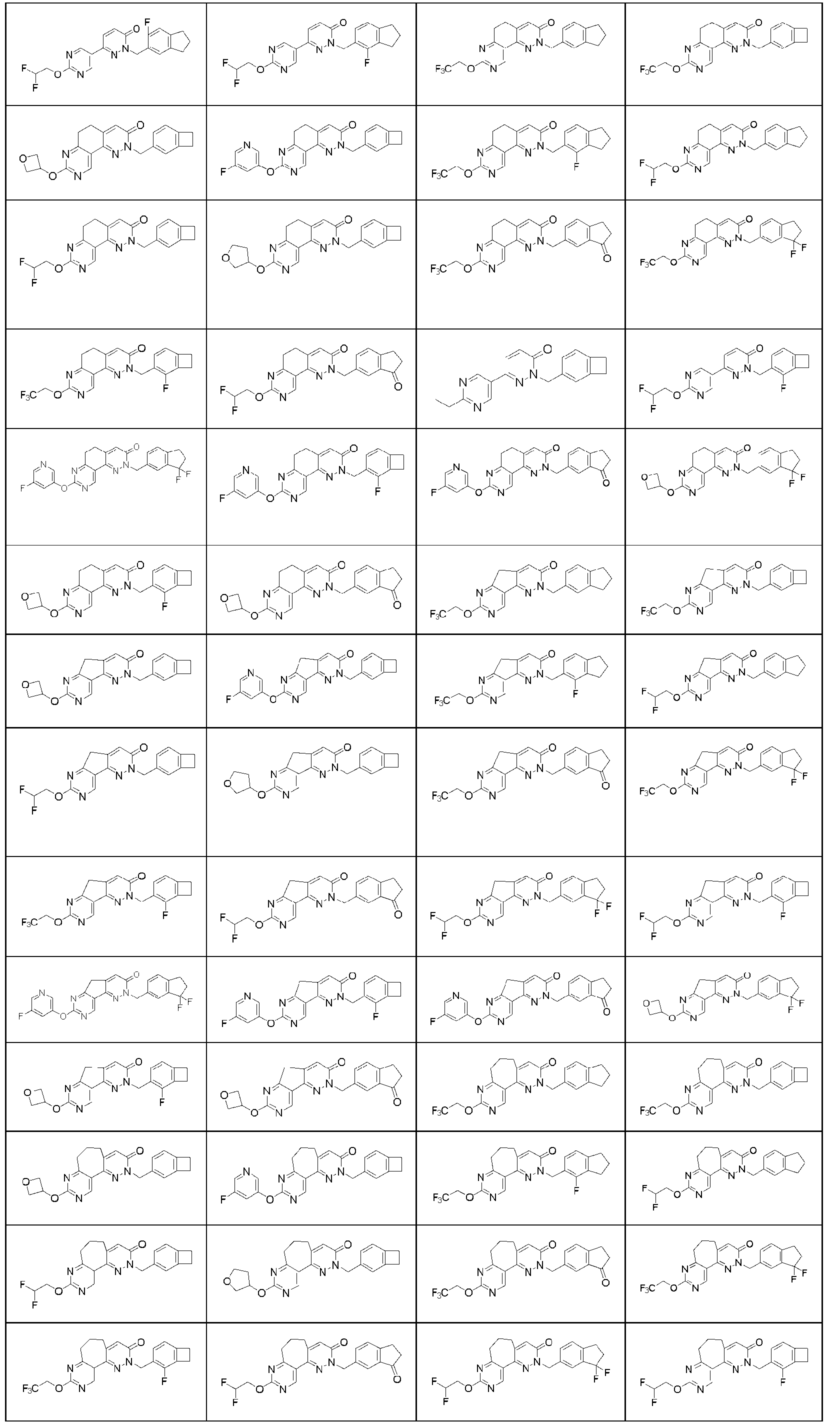

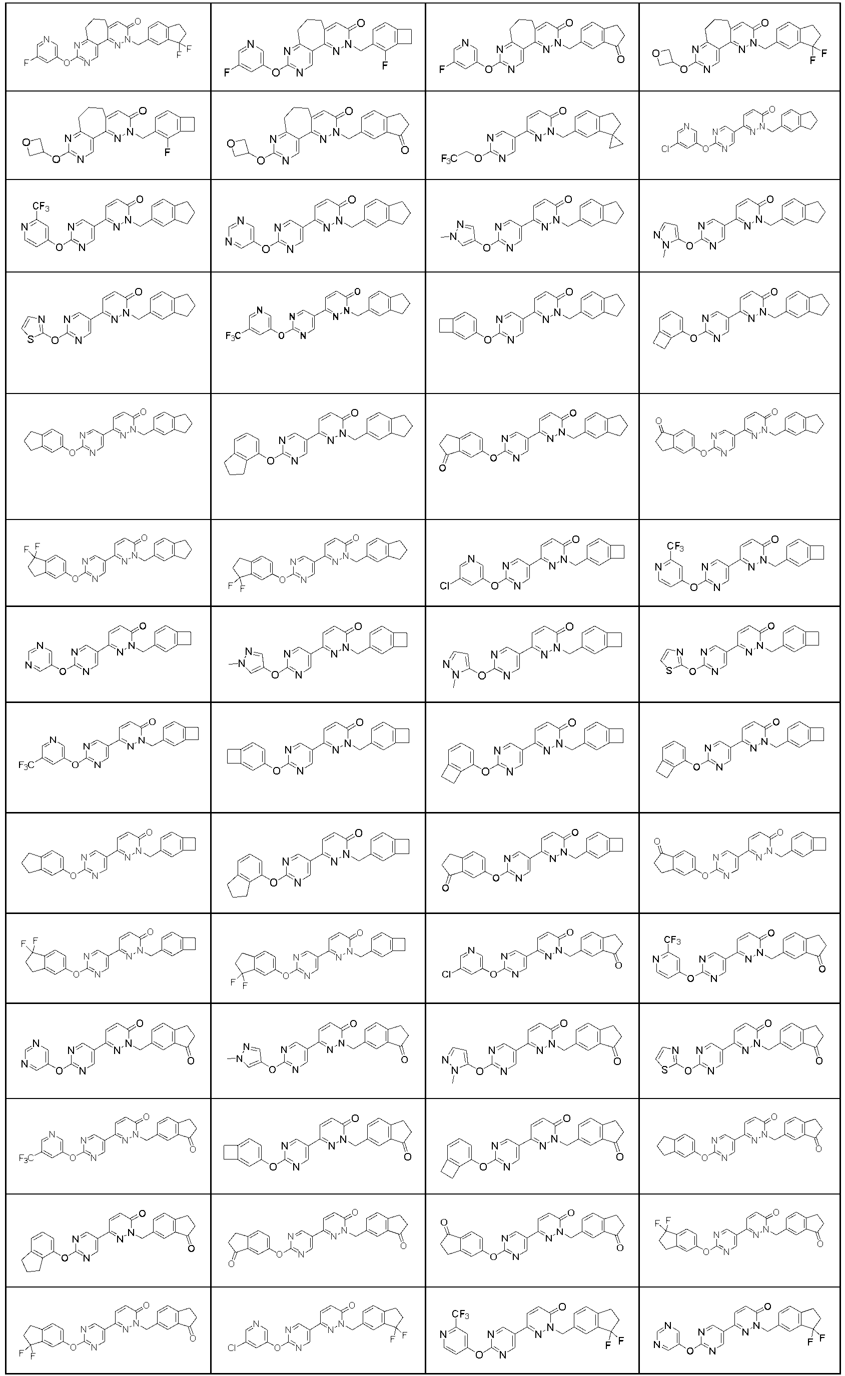

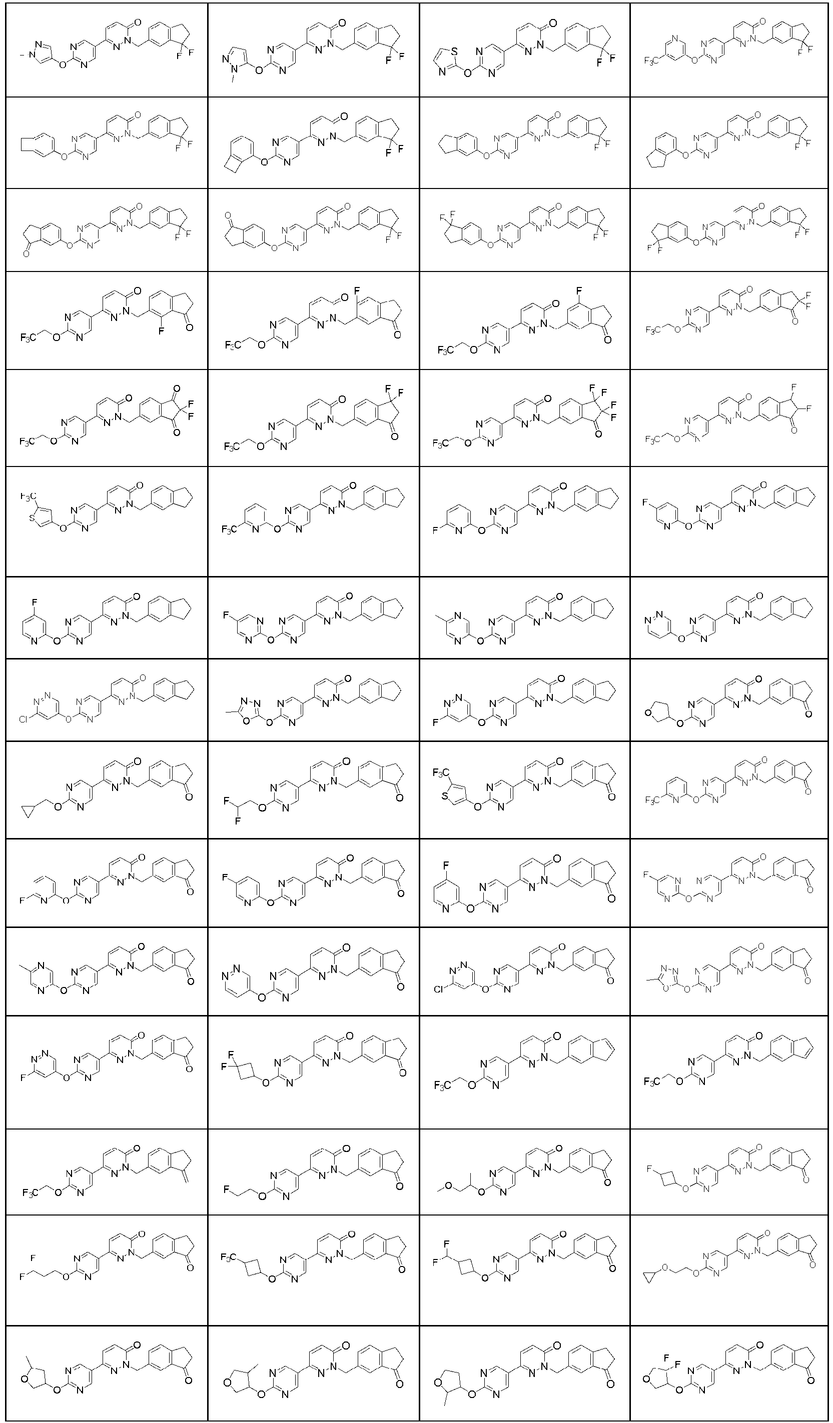

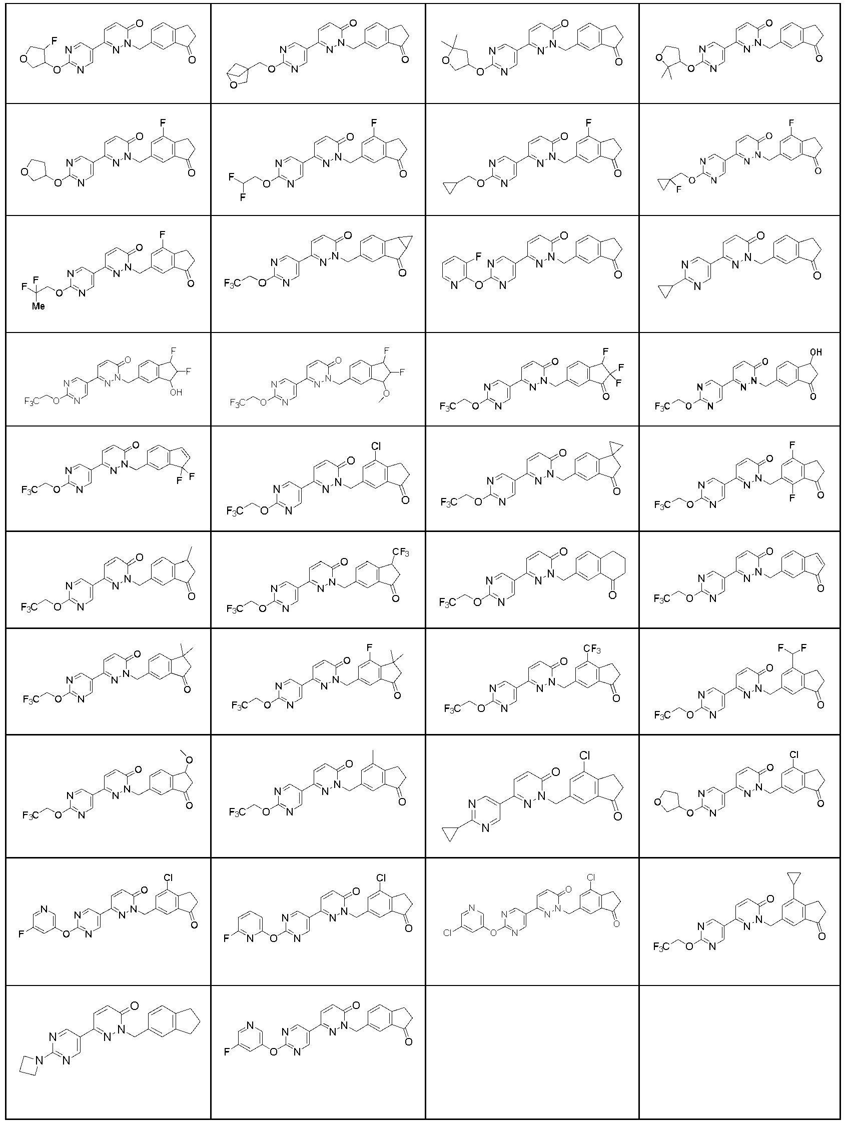

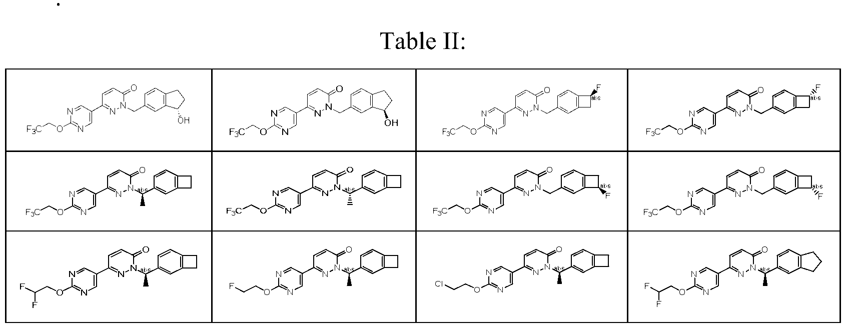

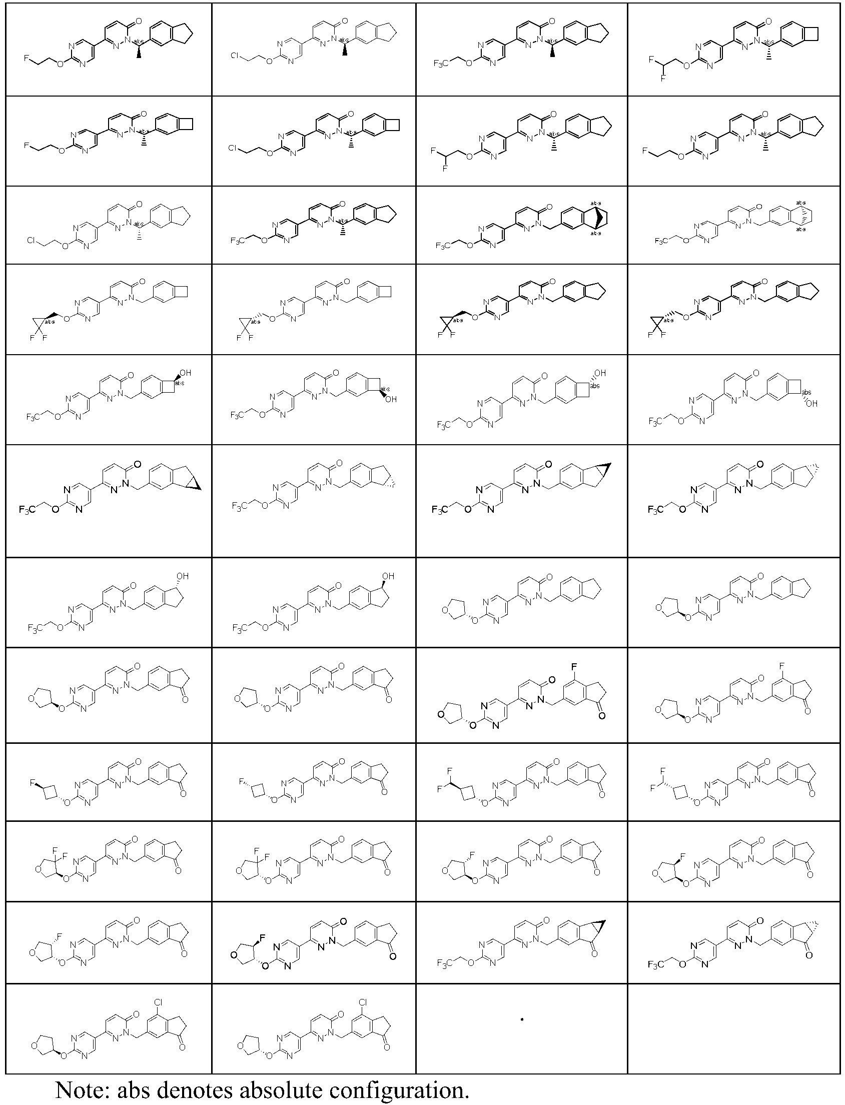

Secondly, the present invention also provides a pharmaceutical composition, comprising the compound, or the stereoisomer, solvate, deuterated substance, or pharmaceutically acceptable salt thereof according to any one of the preceding technical solutions, and a pharmaceutically acceptable carrier and/or excipient.

Further, the pharmaceutical composition or pharmaceutical preparation comprises 1-1500 mg of the compound, or the stereoisomer, deuterated substance, solvate, pharmaceutically acceptable salt or eutectic crystal thereof according to any one of the preceding technical solutions, and a pharmaceutically acceptable carrier and/or excipient.

Further, the present invention also provides the use of the compound, or the stereoisomer, solvate, deuterated substance, or pharmaceutically acceptable salt thereof, or the pharmaceutical composition according to any one of the preceding embodiments in the preparation of a drug for treating/preventing a Myosin II-mediated disease. Further, Myosin II-mediated diseases include, but are not limited to, muscular dystrophy.

The present invention further provides a method for treating a disease in a mammal, the method comprising administering to a subject a therapeutically effective amount of the compound, or the stereoisomer, deuterated substance, solvate, pharmaceutically acceptable salt or eutectic crystal thereof according to any one of the preceding technical solutions, and a pharmaceutically acceptable carrier and/or excipient, wherein the therapeutically effective amount is preferably 1-1500 mg; the disease is preferably muscular dystrophy.

The present invention further provides a method for treating a disease in a mammal, the method comprising administering to the mammal a therapeutically effective amount of the compound, or the stereoisomer, deuterated substance, solvate, pharmaceutically acceptable salt or eutectic crystal thereof or the pharmaceutical composition according to the present invention. In some embodiments, the mammal mentioned in the present invention includes human.

The "effective amount" or "therapeutically effective amount" as described in the present application refers to administration of a sufficient amount of the compound disclosed in the present application that will alleviate to some extent one or more symptoms of the diseases or conditions being treated. In some embodiments, the outcome is the reduction and/or remission of signs, symptoms or causes of the disease, or any other desired change in the biological system. For example, an "effective amount" in terms of the therapeutic use is an amount of the including compound disclosed in the present application that is required to provide clinically significant reduction of the symptoms of the disease. Examples of the therapeutically effective amount include, but are not limited to 1-1500 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1500 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1500 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, 10-20 mg; 20-1500 mg, 20-1000 mg, 20-900 mg, 20-800 mg, 20-700 mg, 20-600 mg, 20-500 mg, 20-400 mg, 20-350 mg, 20-300 mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, 20-30 mg; 50-1500 mg, 50-1000 mg, 50-900 mg, 50-800 mg, 50-700 mg, 50-600 mg, 50-500 mg, 50-400 mg, 50-300 mg, 50-250 mg, 50-200 mg, 50-150 mg, 50-125 mg, 50-100 mg; 100-1500 mg, 100-1000 mg, 100-900 mg, 100-800 mg, 100-700 mg, 100-600 mg, 100-500 mg, 100-400 mg, 100-300 mg, 100-250 mg, or 100-200 mg.

The present invention relates to a pharmaceutical composition or pharmaceutical preparation comprising a therapeutically effective amount of the compound, or the stereoisomer, deuterated substance, solvate, pharmaceutically acceptable salt or eutectic crystal thereof according to the present invention, and a carrier and/or excipient. The pharmaceutical composition can be in a unit preparation form (the amount of the active drug in the unit preparation is also referred to as the "preparation specification"). In some embodiments, the pharmaceutical composition comprises the compound, or the stereoisomer, deuterated substance, solvate, pharmaceutically acceptable salt, or eutectic crystal thereof according to the present invention in an amount including but not limited to 1-1500 mg, 5-1000 mg, 10-800 mg, 20-600 mg, 25-500 mg, 40-200 mg, 50-100 mg, 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg, and 1500 mg.

The present invention further provides a method for treating a disease in a mammal, the method comprising administering to a subject a therapeutically effective amount of the compound, or the stereoisomer, deuterated substance, solvate, pharmaceutically acceptable salt or eutectic crystal thereof according to the present invention, and a pharmaceutically acceptable carrier and/or excipient, wherein the therapeutically effective amount is preferably 1-1500 mg; the disease is preferably muscular dystrophy.

The present invention further provides a method for treating a disease in a mammal, the method comprising administering to a subject a drug, i.e., the compound, or the stereoisomer, deuterated substance, solvate, pharmaceutically acceptable salt or eutectic crystal thereof according to the present invention, and a pharmaceutically acceptable carrier and/or excipient in a daily dose of 1-1500 mg/day, wherein the daily dose can be a single dose or divided doses; in some embodiments, the daily dose includes, but is not limited to 10-1500 mg/day, 20-1500 mg/day, 25-1500 mg/day, 50-1500 mg/day, 75-1500 mg/day, 100-1500 mg/day, 200-1500 mg/day, 10-1000 mg/day, 20-1000 mg/day, 25-1000 mg/day, 50-1000 mg/day, 75-1000 mg/day, 100-1000 mg/day, 200-1000 mg/day, 25-800 mg/day, 50-800 mg/day, 100-800 mg/day, 200-800 mg/day, 25-400 mg/day, 50-400 mg/day, 100-400 mg/day, or 200-400 mg/day; in some embodiments, the daily dose includes, but is not limited to 1 mg/day, 5 mg/day, 10 mg/day, 20 mg/day, 25 mg/day, 50 mg/day, 75 mg/day, 100 mg/day, 125 mg/day, 150 mg/day, 200 mg/day, 400 mg/day, 600 mg/day, 800 mg/day, 1000 mg/day, 1200 mg/day, 1400 mg/day, or 1500 mg/day.

The present invention relates to a kit, wherein the kit can comprise a composition in the form of a single dose or multiple doses and comprises the compound, or the stereoisomer, deuterated substance, solvate, pharmaceutically acceptable salt or eutectic crystal thereof according to the present invention, and the amount of the compound, or the stereoisomer, deuterated substance, solvate, pharmaceutically acceptable salt or eutectic crystal thereof according to the present invention is identical to the amount of same in the above-mentioned pharmaceutical composition.

In the present invention, the amount of the compound, or the stereoisomer, deuterated substance, solvate, pharmaceutically acceptable salt or eutectic crystal thereof according to the present invention is calculated in the form of a free base in each case.

The term "preparation specification" refers to the weight of the active drug contained in each vial, tablet or other unit preparation.

### Synthetic route

Those skilled in the art would have been able to prepare the compounds of the present invention according to known organic synthesis techniques, and the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

Specific and similar reactants can be selectively identified by the indexes of known chemicals prepared by the Chemical Abstracts Service of the American Chemical Society, wherein the indexes are available in most public libraries and university libraries and online. Chemicals that are known but not commercially available in the catalog are optionally prepared by custom chemical synthesis plants, wherein many of standard chemical supply plants (such as those listed above) provide custom synthesis services.

### Term

Unless otherwise specified, the terms of the present invention have the following meanings.

The carbon, hydrogen, oxygen, sulfur, nitrogen and halogen involved in the groups and compounds of the present invention all include isotopes thereof, and are optionally further replaced by one or more of the corresponding isotopes thereof, wherein the isotopes of carbon include ¹²C, ¹³C and ¹⁴C; the isotopes of hydrogen include protium (H), deuterium (D, also known as heavy hydrogen) and tritium (T, also known as superheavy hydrogen); the isotopes of oxygen include ¹⁶O, ¹⁷O and ¹⁸O; the isotopes of sulfur include ³²S, ³³S, ³⁴S and ³⁶S; the isotopes of nitrogen include ¹⁴N and ¹⁵N; the isotope of fluorine includes ¹⁹F; the isotopes of chlorine include ³⁵Cl and ³⁷Cl; and the isotopes of bromine include ⁷⁹Br and ⁸¹Br.

The term "halogen" herein refers to F, Cl, Br, I, or isotopes thereof.

The term "halo" or "substituted with halogen" refers to being substituted with one or more groups selected from F, Cl, Br, I, or isotopes thereof, wherein the upper limit of the number of halogen substituents is equal to the sum of the number of hydrogens that can be substituted in the group to be substituted. Without particular limitation, the number of halogen substituents is any integer between 1 and the upper limit, and when the number of halogen substituents is greater than 1, the group to be substituted can be substituted with the same or different halogen. Generally, the circumstances of being substituted with 1-5 halogen, 1-3 halogen, 1-2 halogen, and 1 halogen are included.

The term "deuterium" refers to the isotope deuterium of hydrogen (H), which is synonymous with "D".

The term "deuterated" or "deuterated substance" refers to the case where a hydrogen atom on a group, such as alkyl, cycloalkyl, alkylene, aryl, heteroaryl, mercapto, heterocycloalkyl, alkenyl and alkynyl is substituted with at least one deuterium atom, wherein the upper limit of the number of deuterium substituents is equal to the sum of the number of hydrogens that can be substituted in the group to be substituted. Without particular limitation, the number of deuterium substituents is any integer between 1 and the upper limit, for example, 1-20 deuterium atoms, 1-10 deuterium atoms, 1-6 deuterium atoms, 1-3 deuterium atoms, 1-2 deuterium atoms or 1 deuterium atom.

Group "C_{x-y}" refers to a group comprising x to y carbon atoms, for example, "C₁₋₆ alkyl" refers to alkyl comprising 1-6 carbon atoms.

The term "alkyl" refers to a monovalent straight or branched saturated aliphatic hydrocarbon group, usually an alkyl group with 1 to 20 carbon atoms, or an alkyl group with 1 to 8 carbon atoms, or an alkyl group with 1 to 6 carbon atoms, or an alkyl group with 1 to 4 carbon atoms. Non-limiting examples of alkyl include methyl, ethyl, n-propyl, isopropyl, n-butyl, sec-butyl, neobutyl, tert-butyl, n-pentyl, isopentyl, neopentyl, n-hexyl, etc., and alkyl may be further substituted with a substituent.

The term "alkylene" refers to a divalent straight or branched saturated alkyl group. Examples of alkylene include, but are not limited to methylene, ethylidene, *etc.*

The term "haloalkyl" refers to an alkyl group in which one or more hydrogens are replaced by one or more halogen atoms (e.g., fluorine, chlorine, bromine, iodine, or isotopes thereof), wherein the upper limit of the number of halogen substituents is equal to the sum of the number of hydrogens that can be substituted in the alkyl group. Without particular limitation, the number of halogen substituents is any integer between 1 and the upper limit. Generally, the alkyl group is substituted with 1-5 halogen, 1-3 halogen, 1-2 halogen or 1 halogen; and when the number of halogen substituents is greater than 1, the group to be substituted can be substituted with the same or different halogen. Specific examples include, but are not limited to -CF₃, -CH₂Cl, -CH₂CF₃, -CCl₂, CF₃, etc.

The term "alkoxy" or "alkyloxy" refers to -O-alkyl, such as -O-C₁₋₈ alkyl, -O-C₁₋₆ alkyl, -O-C₁₋₄ alkyl or -O-C₁₋₂ alkyl. Non-limiting and specific examples of alkoxy or alkyloxy include methoxy, ethoxy, n-propoxy, isopropoxy, n-butoxy, sec-butoxy, tert-butoxy, n-pentoxy, n-hexyloxy, cyclopropoxy, cyclobutoxy, etc. The alkoxy may be optionally substituted with a substituent.

The term "haloalkoxy" refers to -O-haloalkyl, such as -O-halo C₁₋₈ alkyl, -O-halo C₁₋₆ alkyl, -O-halo C₁₋₄ alkyl or -O-halo C₁₋₂ alkyl; the upper limit of the number of halogen substituents is equal to the sum of the number of hydrogens that can be substituted in the group to be substituted. Without particular limitation, the number of halogen substituents is any integer between 1 and the upper limit, preferably 1-5 halogen, 1-3 halogen, 1-2 halogen, and 1 halogen; and when the number of halogen substituents is greater than 1, the group to be substituted can be substituted with the same or different halogen. Non-limiting examples of haloalkoxy include monofluoromethoxy, difluoromethoxy, trifluoromethoxy, difluoroethyloxy, *etc.*

The term "alkenyl" refers to a straight or branched hydrocarbon group comprising at least one carbon-carbon double bond (C=C) and generally comprises 2 to 18 carbon atoms, such as 2 to 8 carbon atoms, further such as 2 to 6 carbon atoms, and still further such as 2 to 4 carbon atoms. Examples of alkenyl include, but are not limited to ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc.; and the alkenyl may further be optionally substituted with a substituent.

The term "alkenylene" refers to a linear or branched divalent unsaturated hydrocarbon group comprising at least one carbon-carbon double bond (C=C) and generally comprises 2 to 18 carbon atoms, such as 2 to 8 carbon atoms, further such as 2 to 6 carbon atoms, and still further such as 2 to 4 carbon atoms. Non-limiting examples of alkenylene include ethynylene and the alkenylene may be optionally substituted with a substituent.

The term "alkynyl" refers to a straight or branched hydrocarbon group comprising at least one carbon-carbon triple bond (C≡C) and generally comprises 2 to 18 carbon atoms, further comprises 2 to 8 carbon atoms, further comprises 2 to 6 carbon atoms, and still further comprises 2 to 4 carbon atoms. Examples of alkynyl include, but are not limited to ethynyl, 1-propynyl, 2-propynyl, butynyl, 2-butynyl, 3-butynyl, 1-methyl-2-propynyl, 4-pentynyl, 3-pentynyl, 1-methyl-2-butynyl, 2-hexynyl, 3-hexynyl, 2-heptynyl, 3-heptynyl, 4-heptynyl, 3-octynyl, 3-nonynyl, 4-decynyl, etc.; and the alkynyl may be optionally substituted with a substituent.

The term "alkynylene" refers to a linear or branched divalent unsaturated hydrocarbon group containing a carbon-carbon triple bond (C≡C) and generally comprises 2 to 18 carbon atoms, further comprises 2 to 8 carbon atoms, further comprises 2 to 6 carbon atoms, and further comprises 2 to 4 carbon atoms. Non-limiting examples of alkynylene include ethynylene, propynylene and butynylene; and the alkynylene may be optionally substituted with a substituent.

The term "cycloalkyl" refers to a saturated or partially unsaturated, non-aromatic carbocyclic hydrocarbon group containing no ring heteroatoms. The cycloalkyl may be monocyclic, bicyclic or polycyclic, the bicyclic or polycyclic cycloalkyl may be in the form of a fused ring, a spiro ring, a bridged ring or a combination thereof, and may comprise one or more aromatic rings, but the ring system is non-aromatic as a whole, and the attachment site may be on an aromatic ring or a non-aromatic ring. Generally, the cycloalkyl contains 3 to 20 carbon atoms, further contains 3-8 carbon atoms, and still further contains 3-6 carbon atoms; when the cycloalkyl is monocyclic cycloalkyl, the cycloalkyl contains 3-15 carbon atoms, or 3-10 carbon atoms, or 3-8 carbon atoms, or 3-6 carbon atoms; when the cycloalkyl is bicyclic or polycyclic cycloalkyl, the cycloalkyl contains 5-12 carbon atoms, or 5-11 carbon atoms, or 6-10 carbon atoms. Non-limiting examples of cycloalkyl include cyclopropyl, cyclobutyl, cyclopentyl, cyclohexyl, cycloheptyl, butenyl, cyclopentenyl, cyclohexenyl, 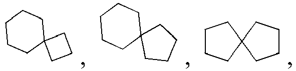 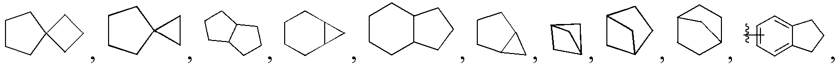 etc., and the cycloalkyl may be optionally substituted with a substituent.

The term "cycloalkylene" refers to a divalent group of cycloalkyl.

"Aryl" refers to a carbocyclic ring having aromaticity that does not contain heteroatoms and includes monocyclic aryl and fused aryl as well as aryl fused cycloalkyl, in the case of aryl fused cycloalkyl, the aryl is the linking site. Generally, the aryl contains 6 to 14 carbon atoms, and further contains 6 to 10 carbon atoms. Non-limiting examples of aryl include phenyl, naphthyl, anthryl, phenanthryl, 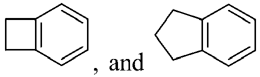 and the aryl may be optionally substituted with a substituent.

"Carbocycle" or "carbocyclyl" refers to a saturated, partially unsaturated, or aromatic carbocycle, and its meaning includes aryl and cycloalkyl. The carbocycle may be monocyclic, bicyclic or polycyclic, and the bicyclic or polycyclic carbocycle may be in the form of a bridged ring, a fused ring, a spiro ring and a combination thereof. Generally, the carbocycle contains 3-12 carbon atoms, or 3-10 carbon atoms, or 3-6 carbon atoms. Non-limiting examples of the monocyclic carbocycle include cyclopropyl, cyclobutyl, cyclopentyl, cyclohexyl, cycloheptyl, phenyl, *etc.* A bicyclic bridged ring includes 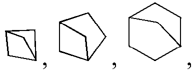 *etc.*, a bicyclic fused ring includes 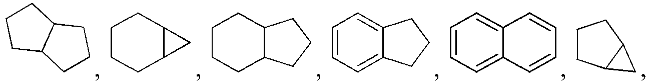 *etc.*, and a bicyclic spiro ring includes 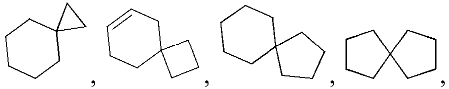 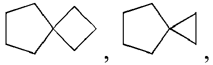 *etc.* The carbocycle may be optionally substituted with a substituent.

"Heterocycloalkyl" refers to a saturated or partially unsaturated non-aromatic carbocycle containing 1, 2, 3, or 4 heteroatoms selected from N, S or O. The heterocycloalkyl may be monocyclic, bicyclic or polycyclic, the bicyclic or polycyclic heterocycloalkyl may be in the form of a bridged ring, a fused ring, a spiro ring or a combination thereof, and may comprise one or more aromatic rings or heteroaromatic rings, but the ring system is non-aromatic as a whole, and the attachment site may be on an aromatic ring or a non-aromatic ring. Generally, the heterocycloalkyl is a 3- to 20-membered ring. When the heterocycloalkyl is monocyclic heterocycloalkyl, the heterocycloalkyl is usually a 3- to 15-membered ring, or a 3- to 10-membered ring, or a 3- to 8-membered ring, or a 3- to 6-membered ring; when the heterocycloalkyl is bicyclic or polycyclic heterocycloalkyl, the heterocycloalkyl is usually a 5- to 12-membered ring, or a 5-to 11-membered ring, or a 6- to 9-membered ring. The heteroatoms N and S include their oxidation states. Non-limiting examples of heterocycloalkyl include azetidinyl, morpholinyl, piperazinyl, piperidyl, tetrahydropyranyl, oxetanyl, pyranyl, azacyclopentenyl, azacyclohexenyl, oxacyclopentenyl, oxacyclohexenyl, etc., and the heterocycloalkyl may be optionally substituted with a substituent.

"Heteroaromatic ring" or "heteroaryl", unless otherwise specified, refers to an aromatic ring containing 1 to 4 heteroatoms selected from N, O or S and their oxidation states, which may be monocyclic, bicyclic or polycyclic, wherein the bicyclic or polycyclic heteroaromatic ring or heteroaryl may be in the form of a bridged ring, a fused ring, a spiro ring and a combination thereof. The bicyclic or polycyclic heteroaromatic ring or heteroaryl can be formed by fusion of heteroaryl to aryl, or of heteroaryl to heteroaryl, wherein the heteroaryl or aryl may be the attachment site. Non-limiting examples of heteroaromatic ring or heteroaryl include furyl, thienyl, pyrrolyl, oxazolyl, thiazolyl, imidazolyl, pyrazolyl, pyridyl, pyrimidyl, pyridazinyl, pyrazinyl, indolyl, purinyl, 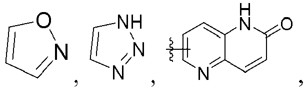 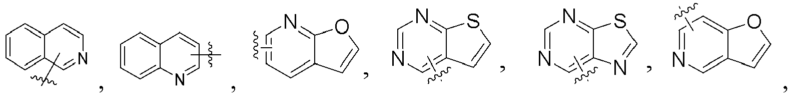 etc. The heteroaryl may be optionally substituted with a substituent.

The term "heterocycle" or "heterocyclyl" refers to a saturated or unsaturated, aromatic or non-aromatic ring containing 1 to 4 heteroatoms selected from N, O or S and their oxidation states, and its meaning includes heteroaryl and heterocycloalkyl. The heterocycle may be in the form of a monocyclic heterocycle, a bicyclic bridged heterocycle, a bicyclic fused heterocycle, a bicyclic spiro heterocycle or a combination thereof. The heterocycle is usually a 3- to 12-membered heterocycle, or a 5- to 12-membered heterocycle, or a 5- to 7-membered heterocycle. Heterocyclyl can be connected to a heteroatom or a carbon atom. Non-limiting examples of heterocyclyl include oxiranyl, azacyclopropyl, oxetanyl, azetidinyl, 1,3-dioxolanyl, 1,4-dioxolanyl, 1,3-dioxanyl, piperazinyl, azacycloheptyl, pyridyl, furyl, thienyl, pyranyl, N-alkylpyrrolyl, pyrimidyl, pyrazinyl, pyrazolyl, pyridazinyl, imidazolyl, piperidyl, piperadinyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, oxazolyl, dihydrooxazolyl, tetrahydrooxazolyl, tetrahydrothiazolyl, tetrahydropyranyl, benzoimidazolyl, benzopyridyl, pyrrolopyridyl, benzodihydrofuryl, azabicyclo[3.2.1]octanyl, azabicyclo[5.2.0]nonanyl, oxatricyclo[5.3.1.1]dodecyl, azaadamantyl and oxaspiro[3.3]heptanyl, 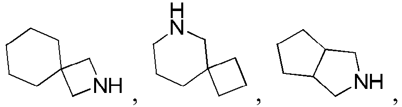 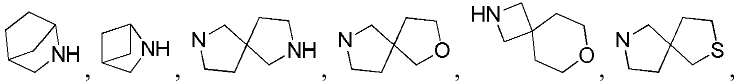 etc., and the heterocycle may be optionally substituted with a substituent.

The term "heterocyclene" refers to a substituted or unsubstituted, saturated or unsaturated, aromatic or non-aromatic, divalent heterocyclyl group. Non-limiting examples of heterocyclene include 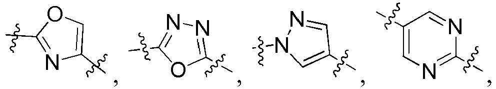 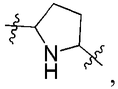 *etc.*

The term "spiro ring" refers to a polycyclic group sharing one carbon atom (referred to as a spiro atom) between rings, which may contain 0 or at least 1 double or triple bond, and may contain 0 to 5 heteroatoms selected from N, O, S, P, Si and their oxidation states. Generally, a spiro ring is a 6- to 14-membered ring, or a 6- to 12-membered ring, or a 6- to 10-membered ring. Generally, a spiro ring is a spiro ring formed by a three-membered ring and a three-membered ring, a three-membered ring and a four-membered ring, a three-membered ring and a five-membered ring, a three-membered ring and a six-membered ring, a four-membered ring and a four-membered ring, a four-membered ring and a five-membered ring, a four-membered ring and a six-membered ring, a five-membered ring and a five-membered ring or a five-membered ring and a six-membered ring. Non-limiting examples of the spiro ring include: 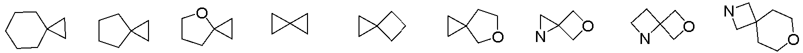 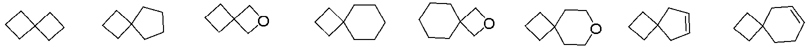 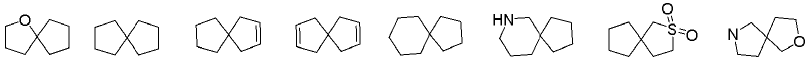 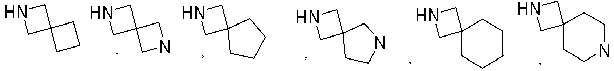 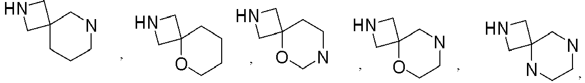 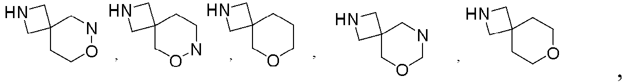 and the spiro ring may be optionally substituted with a substituent.

The term "fused ring ( 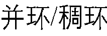 )" refers to a polycyclic group in which the rings share two adjacent ring atoms and one chemical bond. The fused ring may contain one or more double or triple bonds, and may contain 0 to 5 heteroatoms selected from N, S, O, P, Si and their oxidation states. Generally, a fused ring is a 5- to 20-membered ring, or a 5- to 14-membered ring, or a 5- to 12-membered ring or a 5- to 10-membered ring. Generally, a fused ring is in the form of a three-membered ring fused a four-membered ring (indicating a fused ring formed by a three-membered ring and a four-membered ring, and either the three-membered ring or the four-membered ring may be possibly used as the basic ring according to the IUPC nomenclature; similarly hereinafter), a three-membered ring fused a five-membered ring, a three-membered ring fused a six-membered ring, a four-membered ring fused a four-membered ring, a four-membered ring fused a five-membered ring, a four-membered ring fused a six-membered ring, a five-membered ring fused a five-membered ring, a five-membered ring fused a six-membered ring, and a six-membered ring fused a six-membered ring. Non-limiting examples of fused ring include purine, quinoline, isoquinoline, benzopyran, benzofuran, benzothiophene, and 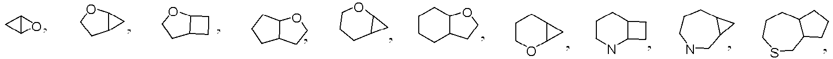 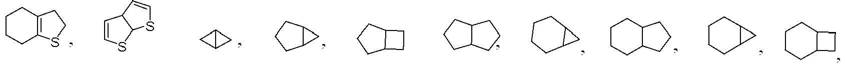 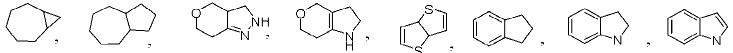 ; and the fused ring may be aromatic or non-aromatic and is optionally substituted with a substituent.

The term "bridged ring" refers to a ring system in which two non-adjacent ring atoms are shared between two rings, which may contain one or more double or triple bonds. The bridged ring may contain 0 to 5 heteroatoms selected from N, S, O, P, Si and their oxidation states. Generally, the bridged ring has 5 to 20, or 5 to 14, or 5 to 12, or 5 to 10 ring atoms. Non-limiting examples of the bridged ring include adamantane,

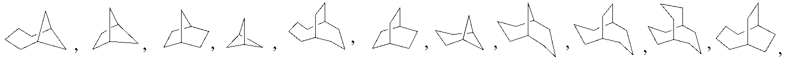

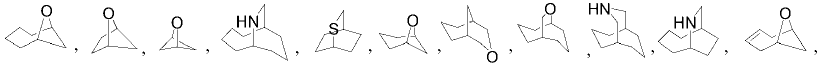

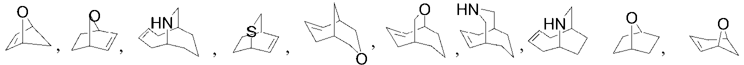

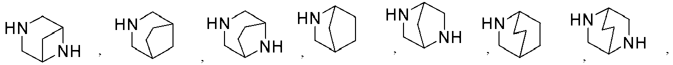

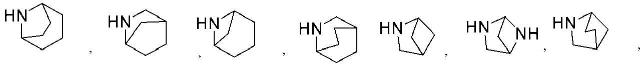

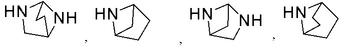

Unless otherwise specified, the term "substitution" or "substituent" refers to any substitution at a position allowed by chemical theory, and the number of substituents conforms to the rules of chemical bonding. Exemplary substituents include, but are not limited to: C₁₋₆ alkyl, C₂₋₆ alkenyl, C₂₋₆ alkynyl, C₃₋₈ heteroalkyl, C₅₋₁₂ aryl, 5- to 12-membered heteroaryl, hydroxyl, C₁₋₆ alkoxy, C₅₋₁₂ aryloxy, thiol, C₁₋₆ alkylthio, cyano, halogen, C₁₋₆ alkylthiocarbonyl, C₁₋₆ alkylcarbamoyl, N-carbamoyl, nitro, silyl, sulfinyl, sulfonyl, sulfoxide, halo C₁₋₆ alkyl, halo C₁₋₆ alkoxy, amino, phosphonic acid, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, -OC(=O)NH(C₁₋₆ alkyl), -NHC(=O)(C₁₋₆ alkyl), -N(C₁₋₆ alkyl)C(=O)(C₁₋₆ alkyl), -NHCO₂(C₁₋₆ alkyl), -NHC(=O)N(C₁₋₆ alkyl)₂, -HC(=O)NH(C₁₋₆ alkyl), -NHC(=O)NH₂, -NHSO₂(C₁₋₆ alkyl), -SO₂N(C₁₋₆ alkyl)₂, -SO₂NH(C₁₋₆ alkyl), -SO₂NH₂, -SO₂C₁₋₆ alkyl, etc.

The term "optional" or "optionally" refers to that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds described herein or the stereoisomers, deuterated substances, solvates, pharmaceutically acceptable salts or eutectic crystals or eutectic crystals thereof and other components comprising physiologically/pharmaceutically acceptable carriers and/or excipients.

The term "carrier" refers to: a system that does not cause significant irritation to the organism and does not eliminate the biological activity and characteristics of the administered compound and can change the way the drug enters the human body and the distribution of the drug in the body, control the release rate of the drug and delivery the drug to targeted organs. Non-limiting examples of the carrier include microcapsule, microsphere, nanoparticle, liposome, etc.

The term "excipient" refers to: a substance that is not a therapeutic agent per se, but used as a diluent, adjuvant, adhesive and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, an acceptable excipient can provide various functions and can be described as a wetting agent, a buffer, a suspending agent, a lubricant, an emulsifier, a disintegrating agent, an absorbent, a preservative, a surfactant, a colorant, a flavoring agent and a sweetening agent. Examples of acceptable excipients include, but are not limited to: (1) sugars, such as lactose, glucose and sucrose; (2) starch, such as corn starch and potato starch; (3) cellulose and its derivatives, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatin; (7) talc; (8) excipients, such as cocoa butter or suppository wax; (9) oils, such as peanut oil, cottonseed oil, safflower oil, sesame oil, olive oil, corn oil and soybean oil; (10) diols, such as propylene glycol; (11) polyols, such as glycerin, sorbitol, mannitol and polyethylene glycol; (12) esters, such as ethyl oleate and ethyl laurate; (13) agar; (14) buffers, such as magnesium hydroxide and aluminum hydroxide; (15) alginic acid; (16) pyrogen-free water; (17) isotonic saline; (18) Ringer's solution; (19) ethanol; (20) pH buffered solution; (21) polyester, polycarbonate and/or polyanhydride; and (22) other non-toxic compatible substances used in a pharmaceutical preparation.

The compounds of the present invention may exist in specific geometric or stereoisomeric forms. All such compounds of the present invention include cis and trans isomers, (-)- and (+)-enantiomers, (R)- and (S)-enantiomers, diastereomers, (D)-isomers, (L)-isomers, and racemic mixtures and other mixtures thereof, such as enantiomerically or diastereomerically enriched mixtures, all of which fall within the scope of the present invention . Additional asymmetric carbon atoms may be present in the substituents of the compounds of the present invention. All such isomers, as well as mixtures thereof, are included within the scope of the present invention. In certain embodiments, preferred compounds are those isomeric compounds that exhibit superior biological activity. Purified or partially purified isomers and stereoisomers, or racemic mixtures or diastereomeric mixtures of the compounds of the present invention are also included within the scope of the present invention. Purification and isolation of such materials can be achieved by standard techniques known in the art.

The compounds of the present invention also include tautomers thereof, for example, when the present invention describes the left side compound in which the pyrimidine ring is substituted with OH, the right side tautomer compound is also included.

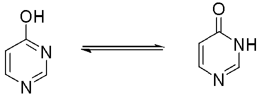

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "eutectic crystal" refers to a crystal formed by the combination of active pharmaceutical ingredient (API) and eutectic crystal former (CCF) under the action of hydrogen bonds or other non-covalent bonds. The pure state of API and CCF are both solid at room temperature, and there is a fixed stoichiometric ratio between various components. The eutectic crystal is a multi-component crystal, which includes both a binary eutectic crystal formed between two neutral solids and a multi-element eutectic crystal formed between a neutral solid and a salt or solvate.

### Detailed Description of Embodiments

The content of the present invention is described in detail with the following examples. If a specific condition is not indicated in the examples, a conventional condition is used in an experimental method. The listed examples are intended to better illustrate the content of the present invention but should not be construed as limiting the content of the present invention. According to the above-mentioned content of the invention, those skilled in the art can make unsubstantial modifications and adjustments to the embodiments, which still fall within the protection scope of the present invention.

### Test method

The structure of the compound is determined by nuclear magnetic resonance (NMR) or (and) mass spectrometry (MS). The NMR shift (δ) is given in the unit of 10⁻⁶ (ppm). NMR is measured with (Bruker Avance III 400 and Bruker Avance 300) NMR instrument, and the solvent for determination is deuterated dimethyl sulfoxide (DMSO-*d*₆), deuterated chloroform (CDCl₃), and deuterated methanol (CD₃OD), and the internal standard is tetramethylsilane (TMS);
MS is determined with Agilent 6120B (ESI) and Agilent 6120B (APCI); and
HPLC is determined with Agilent 1260DAD high pressure liquid chromatograph (Zorbax SB-C₁₈ 100 × 4.6 mm, 3.5 µM);
Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm;
and for the column chromatography, Yantai Huanghai silica gel of 200-300 mesh silica gel is generally used as a carrier.

### Preparation of intermediate 1

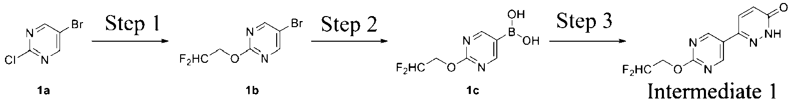

Step 1: 2,2-difluoroethanol-1-ol (16.5 g, 201.2 mmol) was dissolved in tetrahydrofuran (50 mL), and sodium hydride (7.44 g, 310 mmol) was added in portions at 0°C, the obtained system was returned to room temperature and stirred for 40 min, then 5-bromo-2-chloropyrimidine (compound 1a) (30 g, 155.1 mmol) was dissolved in tetrahydrofuran (50 mL), the obtained solution was injected into the above system, which was then continuously stirred for 3 h. The obtained system was diluted with water, extracted 3 times with ethyl acetate, and the organic phase was collected, dried and concentrated to give compound 1b (36 g of crude product), which was used directly for the next reaction.

LC-MS (ESI): m/z = 239.1 [M+H]⁺.

Step 2: Compound 1b (38 g, 158.98 mmol), potassium acetate (39.01 g, 397.45 mmol), bis(pinacolato)diboron (52.48 g, 206.67 mmol) and chloro(2-dicyclohexylphosphino-2',4',6'-triisoporphyrinyl-1,1'-biphenyl)[2-(2'-amino-1,1'-biphenyl)]palladium(II) (12.51 g, 15.90 mmol) was dissolved in 1,4-dioxane (500 mL), the obtained system was subjected to nitrogen replacement 3 times, then heated to 100°C under a nitrogen atmosphere and stirred for 3 h. The reaction liquid was filtered when it was still hot, the filter cake was rinsed with ethyl acetate and dichloromethane, respectively (4 times for each), the filtrate was collected and concentrated to obtain compound 1c (32 g of crude product), which was used directly for the next reaction.

LC-MS (ESI): m/z = 205.2 [M+H]⁺.

Step 3: Compound 1c (30 g, 147.10 mmol), 6-bromo-3-pyridazinone (30.71 g, 176.52 mmol), tripotassium phosphate (40.59 g, 191.23 mmol) and (1,3-bis(2,6-diisopropylphenyl)imidazolylidene)(3-chloropyridinyl)palladium(II)dichloride (10.02 g, 14.71 mmol) was dissolved in a mixed solvent of 1,4-dioxane (360 mL) and water (120 mL), the obtained system was subjected to nitrogen replacement 3 times, then heated to 90°C under a nitrogen atmosphere and stirred for 3 h. After cooling, the reaction liquid was filtered, the filter cake was rinsed with water, ethyl acetate, and dichloromethane, respectively, then collected and dried to obtain the intermediate 1 (27 g of crude product), which was used directly.

LC-MS (ESI): m/z = 255.1 [M+H]⁺.

### Example 56

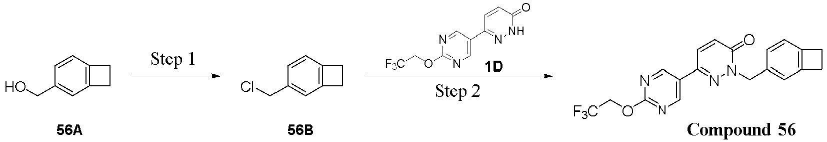

Step 1: Compound 56A (0.7 g, 5.22 mmol) was dissolved in dichloromethane (12 mL), thionyl chloride (1.24 g, 10.44 mmol) was added, and the obtained system was stirred at room temperature for 2 h. The obtained system was concentrated directly under reduced pressure to obtain compound 56B (0.8 g of crude product, 100%), which was used directly for the next reaction.

¹H NMR (400 MHz, DMSO-*d*₆) δ 7.24 (d, 1H), 7.15 (t, 1H), 7.07 (d, 1H), 4.72 (s, 2H), 3.12 (s, 4H).

Step 2: Compound 1D (0.6 g, 2.20 mmol, synthesis method reference: WO 2020097258A1), compound 56B (0.34 g, 2.20 mmol) and cesium carbonate (2.15 g, 6.60 mmol) were dissolved in acetonitrile (20 mL) and then the obtained system was stirred at 80°C for 2 h. After cooling, the obtained system was concentrated directly, water (10 mL) was added to the residue, and ethyl acetate was used for extraction for 3 times (15 mL × 3), the organic phase was collected, dried and concentrated, the resulting residue was purified by reverse phase column chromatography (acetonitrile : water (v : v)=45 : 55) to obtain compound 56 (0.6 g, 70.23%).
¹H NMR (400 MHz, DMSO-*d*₆) δ 9.15 (s, 2H), 8.10 (d, 1H), 7.24 (s, 1H), 7.16 - 7.10 (m, 2H), 7.04 (d, 1H), 5.27 (s, 2H), 5.11 (d, 2H), 3.09 (s, 4H);
LC-MS (ESI): m/z = 389.1 [M+H]⁺.

### Example 57

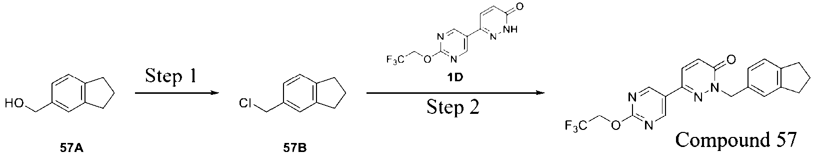

Step 1: (2,3-dihydro-1H-inden-5-yl)methanol (compound 57A) (0.3 g, 2.04 mmol) was dissolved in dichloromethane (6 mL), and then thionyl chloride (0.49 g, 4.12 mmol) was slowly added thereto in an ice-water bath, afterwards the obtained system was returned to room temperature and continuously reacted for 2 h. The reaction liquid was directly concentrated to obtain the compound 57B (0.5 g of crude product), which was used directly for the next reaction.

¹H NMR (400 MHz, DMSO-*d*₆) 7.19-7.11 (m, 3H), 4.60 (s, 2H), 2.85-2.77 (m, 4H), 2.04-1.88 (m, 2H).

Step 2: Compound 1D (0.25 g, 0.92 mmol), compound 57B (0.23 g, 1.38 mmol) and potassium carbonate (0.32 g, 2.32 mmol) were dissolved in N,N-dimethylformamide (8 mL), the obtained system was heated to 70°C and stirred for 2 h. The reaction liquid was diluted with water, and extracted with ethyl acetate 3 times, the organic phases were combined and concentrated, and the resulting residue was purified by preparative HPLC. Method: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.5% ammonium acetate); b. gradient elution, mobile phase A: 50%-80%; c. flow rate: 20 mL/min; d. elution time: 18 min. retention time: 17 min. Compound 57 (160 mg, 43%) was obtained.
¹H NMR (400 MHz, DMSO-*d*₆) δ 9.15 (s, 2H), 8.11 (d, 1H), 7.25 (s, 1H), 7.19 - 7.11 (m, 3H), 5.27 (s, 2H), 5.16-5.06 (m, 2H), 2.85-2.77 (m, 4H), 2.04-1.88 (m, 2H);
LC-MS (ESI): m/z = 403.2 [M+H]⁺.

### Example 60

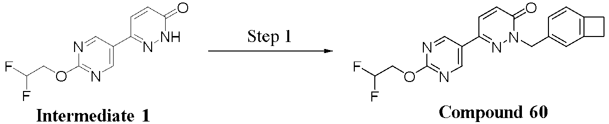

Step 1: Intermediate 1 (0.25 g, 0.98 mmol), compound 56B (0.19 g, 1.24 mmol) and potassium carbonate (0.34 g, 2.46 mmol) were dissolved in N,N-dimethylformamide (8 mL), the obtained system was heated to 70° and stirred for 3 h. The reaction liquid was diluted with water, and extracted with ethyl acetate 3 times, the organic phases were combined and concentrated, and the residue was purified by preparative HPLC. Method: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.5% ammonium acetate); b. gradient elution, mobile phase A: 30%-70%; c. flow rate: 20 mL/min; d. elution time: 20 min. retention time: 18 min. Compound 60 (120 mg, 33%) was obtained.
¹H NMR (400 MHz, DMSO-*d*₆) δ 9.11 (s, 2H), 8.08 (d, 1H), 7.25 (d, 1H), 7.15 - 7.10 (m, 2H), 7.04 (d, 1H), 6.63-6.26 (m, 1H), 5.27 (s, 2H), 4.78-4.62 (m, 2H), 3.08 (s, 4H);
LC-MS (ESI): m/z = 371.1 [M+H]⁺.

### Example 61

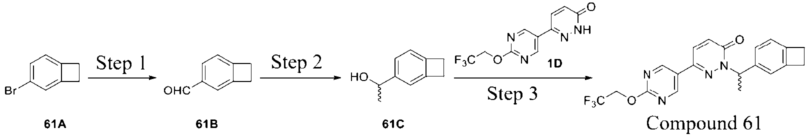

Step 1: Compound 61A (2.0 g, 10.91 mmol) was dissolved in tetrahydrofuran (25 mL), the obtained system was stirred under a nitrogen atmosphere, then n-butyllithium (8.7 mL, 13.09 mmol, 1.5M in n-hexane) was added dropwise at - 78°C, after the completion of addition, the obtained system was stirred at this temperature for 0.5 h, finally N,N-dimethylformamide (1.04 g, 14.18 mmol) was added dropwise thereto, and the obtained system was continuously stirred for 1 h. Water was slowly added to the system at -78°C to quench the reaction, the reaction liquid was then extracted with ethyl acetate (30 mL × 3), the organic phase was collected, dried and concentrated to obtain compound 61B (1.40 g of crude product, 97.1%), which was used directly for the next reaction.

Step 2: Compound 61B (1.4 g, 10.59 mmol) was dissolved in tetrahydrofuran (25 mL), the system was stirred at 0°C, methylmagnesium bromide (10.6 mL, 21.2 mmol, 2 M solution in ether) was added dropwise, and after the completion of addition, the obtained system was returned to room temperature and reacted for 2 h. Water was added in an ice bath to quench the reaction, the reaction liquid was extracted with ethyl acetate (30 mL×3), the organic phase was collected, dried and concentrated to obtain compound 61C (1.3 g of crude product, 82.83%), which was used directly for the next reaction.

Step 3: Compound 61C (0.60 g, 4.05 mmol) was dissolved in tetrahydrofuran (20 mL), then triphenylphosphine (1.59 g, 6.07 mmol) and Compound 1D (1.21 g, 4.46 mmol) were added, and diisopropyl azodicarboxylate (1.23 g, 6.07 mmol) was slowly added dropwise at 0°C, then the obtained system was slowly raised to room temperature and reacted overnight. The reaction liquid was concentrated directly, the resulting residue was purified by silica gel column chromatography (petroleum ether : ethyl acetate (v : v)=1 : 1) to obtain compound 61 (700 mg, 42.96%).
¹H NMR ((400 MHz, CDCl₃) δ 8.94 (s, 2H), 7.54 (d, 1H), 7.33 (d, 1H), 7.19 (s, 1H), 7.05-7.00 (m, 2H), 6.41-6.36 (m, 1H), 4.88 (q, 2H), 3.14 (s, 4H), 1.82 (d, 3H);
LC-MS (ESI): m/z = 403.1 [M+H]⁺.

### Example 62

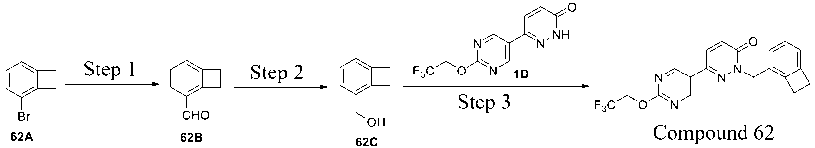

Step 1: Compound 62A (1.0 g, 5.46 mmol) and tetrahydrofuran (25 mL) were added into a three-necked flask, which was placed at -78°C under nitrogen protection and the obtained system was stirred, and then n-butyllithium (2.6 mL, 6.5 mmol, 2.5 M in n-hexane) was slowly added, after the completion of addition, the obtained system was stirred at this temperature for 0.5 h, finally N,N-dimethylformamide (0.52 g, 7.10 mmol) was slowly added thereto, and the obtained system was continuously stirred for 1 h. Water was slowly added dropwise to quench the reaction, the reaction liquid was then extracted with ethyl acetate (30 mL × 2), the organic phase was collected, dried and concentrated to obtain compound 62B (0.50 g of crude product, 69.3%), which was used directly for the next reaction.

Step 2: Compound 62B (0.22 g, 1.66 mmol) was dissolved in tetrahydrofuran (15 mL), the obtained system was stirred at 0°C, and then diisobutylaluminum hydride (2.5 mL, 2.5 mmol, 1M in n-hexane) was slowly added dropwise thereto, after the completion of addition, the obtained system was returned to room temperature and stirred for 2 h. The system was placed in an ice bath and stirred, then water (0.2 mL), 15% sodium hydroxide aqueous solution (0.2 mL) and water (0.5 mL) were added thereto in sequence, and stirred for 10 min, finally anhydrous sodium sulfate was added to the obtained system, which was dried and filtered, the filter cake was washed with ethyl acetate, the solution was collected and concentrated to obtain compound 62C (0.18 g, 80.8%), which was used directly for the next reaction.

Step 3: Compound 62C (0.18 g, 1.34 mmol) was dissolved in tetrahydrofuran (20 mL), then triphenylphosphine (0.53 g, 2.01 mmol) and Compound 1D (0.40 g, 1.47 mmol) were added, and diisopropyl azodicarboxylate (0.41 g, 2.01 mmol) was slowly added dropwise at 0°C, then the obtained system was slowly raised to room temperature and stirred overnight. The reaction liquid was concentrated directly, the resulting residue was purified by silica gel column chromatography (petroleum ether : ethyl acetate (v : v)=1 : 1) to obtain compound 62 (55 mg, 10.57%).
¹H NMR ((400 MHz, CDCl₃) δ 8.94 (s, 2H), 7.59 (d, 1H), 7.24 (d, 1H), 7.18 (t, 1H), 7.07 (d, 1H), 6.98 (d, 1H), 5.36 (s, 2H), 4.87 (q, 2H), 3.14-3.10 (m, 4H);
LC-MS (ESI): m/z = 389.1 [M+H]⁺.

### Example 63 and example 64

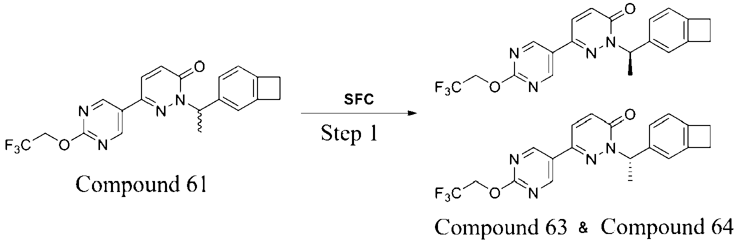

Compound 61 (700.0 mg) was subjected to chiral resolution to obtain P1 (retention time: 1.855 min, set as compound 63. SFC analytical method: instrument: SHIMADZU LC-30AD; chromatographic column: Chiralcel WHELK column; mobile phase: A for CO₂; B for 0.05% DEA in MeOH; gradient: 5-40% B gradient elution; flow rate: 3 mL/min; column temperature: 35°C; column pressure: 100 bar; wavelength: 220 nm.) and P2 (retention time: 2.536 min, set as compound 64). Preparation method: instruments: Waters 150 Prep-SFCA; chromatographic column: Chiralcel WHELK column; mobile phase: A for CO₂; B for 0.1% NH₃•H₂O in MeOH; gradient: 45% B gradient elution; flow rate: 100 mL/min; column temperature: 25°C; wavelength: 220 nm; cycle time: 5.0 min; sample preparation: sample concentration: 10 mg/mL, acetonitrile solution; sample injection: 10 mL/injection. After the separation, the solvent was dried and concentrated by a rotary evaporator at a bath temperature of 35°C, and then the solvent was dried by a lyophilizer at -80°C to obtain compound 63 (260 mg, 37.1%) and compound 64 (256 mg, 36.6%).

Compound 63: ¹H NMR ((400 MHz, CDCl₃) δ 8.94 (s, 2H), 7.54 (d, 1H), 7.32 (d, 1H), 7.19 (s, 1H), 7.04-7.00 (m, 2H), 6.41-6.36 (m, 1H), 4.88 (q, 2H), 3.14 (s, 4H), 1.82 (d, 3H); LC-MS (ESI): m/z = 403.1 [M+H]⁺.

Compound 64: ¹H NMR ((400 MHz, CDCl₃) δ 8.94 (s, 2H), 7.54 (d, 1H), 7.32 (d, 1H), 7.19 (s, 1H), 7.04-7.00 (m, 2H), 6.41-6.36 (m, 1H), 4.88 (q, 2H), 3.14 (s, 4H), 1.82 (d, 3H); LC-MS (ESI): m/z = 403.1 [M+H]⁺.

### Example 65

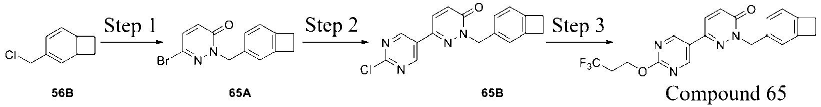

Step 1: Compound 56B (3.5 g, 20.00 mmol), 6-bromo-3-pyridazinol (3.94 g, 20.00 mmol) and cesium carbonate (19.55 g, 60.00 mmol) were dissolved in acetonitrile (100 mL), the obtained system was heated to 80°C and stirred for 2.5 h. The reaction liquid was concentrated directly, the resulting residue was purified by silica gel column chromatography (petroleum ether:ethyl acetate (v:v)=3:1) to obtain compound 65A (4 g, 68.70%).

LC-MS (ESI): m/z = 291.1 [M+H]⁺.

Step 2: Compound 65A (4 g, 13.74 mmol), 2-chloropyrimidine-5-boronic acid (4.35 g, 27.48 mmol), potassium carbonate (5.70 g, 41.22 mmol) and 1,3-bis(2,6-diisopropylphenyl)imidazol-2-ylidene](3-chloropyridinyl)palladium dichloride (0.94 g, 1.37 mmol) were dissolved in 1,4-dioxane (50 mL) and water (10 mL), then the obtained system was heated to 90°C and stirred for 2.5 h under nitrogen protection. The reaction liquid was concentrated directly, the resulting residue was purified by silica gel column chromatography (petroleum ether : ethyl acetate (v : v) = 1 : 1) to obtain compound 65B (3.5 g, 78.44%).

LC-MS (ESI): m/z = 325.5 [M+H]⁺.

Step 3: Trifluoropropanol (0.28 g, 2.48 mmol) was dissolved in tetrahydrofuran (5 mL) and sodium metal (0.043 g, 1.86 mmol) was added. After the sodium disappeared, compound 65B (0.20 g, 0.62 mmol) was added and the obtained system was stirred at room temperature for 1 h. Water and ethyl acetate were added for extraction, the organic phase was collected, dried and concentrated, the resulting residue was purified by reverse phase column chromatography (water : acetonitrile (v : v)=40 : 60) to obtain compound 65 (0.1 g, 40.09%).
¹H NMR (400 MHz, DMSO-*d*₆) δ 9.09 (s, 2H), 8.08 (d, 1H), 7.24 (d, 1H), 7.15 - 7.09 (m, 2H), 7.04 (d, 1H), 5.27 (s, 2H), 4.61-4.58(m, 2H), 3.09 (s, 4H), 2.94 - 2.79 (m, 2H);
LC-MS (ESI): m/z = 403.4 [M+H]⁺.

### Example 66

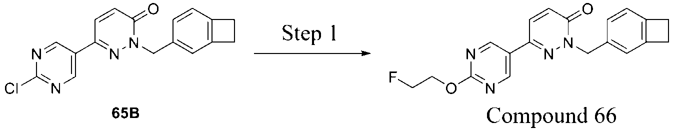

Step 1: 2-fluoroethanol (0.20 g, 3.1 mmol) was dissolved in tetrahydrofuran (5 mL), and sodium metal (0.057 g, 2.48 mmol) was added. After the sodium disappeared, compound 65B (0.20 g, 0.62 mmol) was added and the obtained system was stirred at room temperature for 1 h. Water and ethyl acetate were added for extraction, the organic phase was collected, dried and concentrated, and the obtained residue was purified by reverse phase column chromatography (water : acetonitrile (v : v) = 40 : 60) to give compound 66 (0.1 g, 45.77%).
¹H NMR (400 MHz, DMSO-*d*₆) δ 9.08 (s, 2H), 8.08 (d, 1H), 7.24 (d, 1H), 7.16 - 7.08 (m, 2H), 7.04 (d, 1H), 5.27 (s, 2H), 4.92 - 4.51 (m, 4H), 3.09 (s, 4H);
LC-MS (ESI): m/z = 353.4 [M+H]⁺.

### Example 67

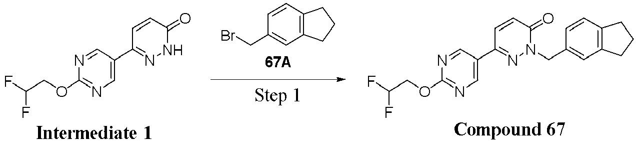

Step 1: Intermediate 1 (0.50 g, 1.97 mmol), compound 67A (0.50 g, 2.36 mmol), and cesium carbonate (1.30 g, 4.00 mmol) were added to acetonitrile (20 mL) in sequence, and the obtained system was heated to 80°C and stirred for 2 h. After cooling, water was added for dilution, then the obtained system was extracted with ethyl acetate 3 times, the combined organic phases were dried over anhydrous sodium sulfate and then concentrated, the resulting residue was purified by reverse phase column chromatography (acetonitrile : water (v : v)=10%-80%) to obtain compound 67 (420 mg, 55%).
¹H NMR (400 MHz, CDCl₃) δ 8.93 (s, 2H), 7.56 (d, 1H), 7.34 (s, 1H), 7.27-7.25 (m, 1H), 7.19-7.17 (m, 1H), 7.06 (d, 1H), 6.32-6.02(m, 1H), 5.36 (s, 2H), 4.69-4.61 (m, 2H), 2.90-2.85 (m, 4H), 2.90-2.00 (m, 2H);
LC-MS (ESI): m/z = 385.1 [M+H]⁺.

### Example 68

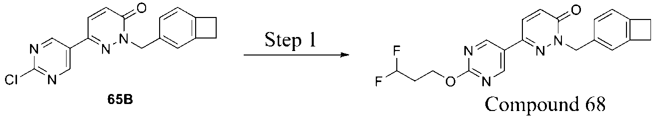

3,3-difluoropropan-1-ol (0.24 g, 2.48 mmol) was dissolved in tetrahydrofuran (5 mL) and sodium metal (0.043 g, 1.87 mmol) was added. After the sodium disappeared, compound 65B (0.20 g, 0.62 mmol) was added and the obtained system was reacted at room temperature for 1 h. Water and ethyl acetate were added to the system for extraction, the organic phase was collected and concentrated under reduced pressure, the resulting residue was purified by reverse phase column chromatography (acetonitrile : water (v : v)=10%-80%) to obtain compound 69 (0.1 g, 41.96%).
¹H NMR (400 MHz, DMSO-*d*₆) δ 9.08 (s, 2H), 8.07 (d, 1H), 7.24 (d, 1H), 7.15 - 7.08 (m, 2H), 7.04 (d, 1H), 6.42-6.11 (m, 1H), 5.27 (s, 2H), 4.52 (t, 2H), 3.09 (s, 4H), 2.46 - 2.29 (m, 2H);
LC-MS (ESI): m/z = 385.1 [M+H]⁺.

### Example 69

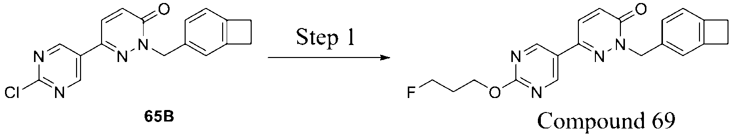

3-fluoropropan-1-ol (0.19 g, 2.48 mmol) was dissolved in tetrahydrofuran (5 mL) and sodium metal (0.043 g, 1.87 mmol) was added. After the sodium disappeared, compound 65B (0.20 g, 0.62 mmol) was further added and the obtained system was reacted at room temperature for 1 h. Water and ethyl acetate were added to the system for extraction, the organic phase was collected and concentrated under reduced pressure, and the obtained residue was purified by reverse phase column chromatography (acetonitrile : water (v : v) = 10% to 80%) to obtain compound 69 (0.08 g, 35.22%).
¹H NMR (400 MHz, DMSO-*d*₆) δ 9.07 (s, 2H), 8.07 (d, 1H), 7.24 (d, 1H), 7.14 - 7.07 (m, 2H), 7.04 (d, 1H), 5.27 (s, 2H), 4.67 (t, 1H), 4.55 (t, 1H), 4.47 (t, 2H), 3.09 (s, 4H), 2.23 - 2.06 (m, 2H);
LC-MS (ESI): m/z = 367.2 [M+H]⁺.

### Example 70

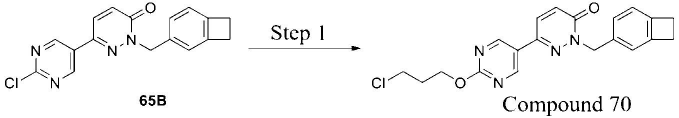

3-chloropropan-1-ol (0.23 g, 2.48 mmol) was dissolved in tetrahydrofuran (5 mL) and sodium metal (0.043 g, 1.87 mmol) was added. After the sodium disappeared, compound 65B (0.20 g, 0.62 mmol) was further added and the obtained system was reacted at room temperature for 1 h. Water and ethyl acetate were added to the system for extraction, the organic phase was collected and concentrated under reduced pressure, and the obtained residue was purified by reverse phase column chromatography (acetonitrile : water (v : v) = 10% to 80%) to obtain compound 70 (0.09 g, 37.92%).
¹H NMR (400 MHz, DMSO-*d*₆) δ 9.07 (s, 2H), 8.07 (d, 1H), 7.24 (d, 1H), 7.17 - 7.08 (m, 2H), 7.04 (d, 1H), 5.27 (s, 2H), 4.49 (t, 2H), 3.80 (t, 2H), 3.09 (s, 4H), 2.28 - 2.15 (m, 2H);
LC-MS (ESI): m/z = 383.1 [M+H]⁺.

### Example 71

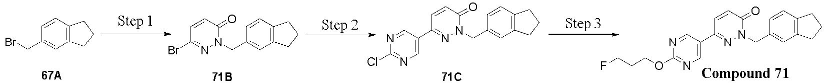

Step 1: Compound 67A (2 g, 9.47 mmol), 6-bromo-3-pyridazinol (1.82 g, 10.42 mmol) and potassium carbonate (3.27 g, 23.68 mmol) were dissolved in N,N-dimethylformamide (30 mL), and the obtained system was heated to 70°C and stirred for 3 h. After cooling, the system was diluted with water, then extracted with ethyl acetate 3 times, the combined organic phases were concentrated under reduced pressure, and the resulting residue was purified by silica gel column chromatography (ethyl acetate : petroleum ether (v : v)=15%) to obtain compound 71B (2.5 g, 86%).

LC-MS (ESI): m/z = 305.2 [M+H]⁺.

Step 2: Compound 71B (2.5 g, 8.19 mmol), (2-chloropyrimidin-5-yl)boronic acid (1.95 g, 12.29 mmol), potassium phosphate (2.61 g, 12.29 mmol) and 1,3-bis(2,6-diisopropylphenyl)imidazol-2-ylidene](3-chloropyridinyl)palladium(II) dichloride (0.56 g, 0.82 mmol) were dissolved in a mixed solvent of 1,4-dioxane (30 mL) and water (10 mL). The obtained system was then reacted under stirring at 90°C for 3 h under nitrogen protection. After cooling, water was added directly to precipitate the solid, which was filtered, the filter cake was ethyl acetate 3 times, then it was collected and dried to obtain compound 71C (1.8 g, 65%).

LC-MS (ESI): m/z = 339.3 [M+H]⁺.

Step 3: 3-fluoropropanol (0.46 g, 5.90 mmol) was dissolved in solvent tetrahydrofuran (10 mL), sodium metal (68 mg, 2.96 mmol) was added, and the obtained system was reacted under stirring at room temperature until the sodium metal disappeared completely. Compound 71C (200 mg, 0.59 mmol) was further added to the reaction liquid, and the obtained system was reacted under stirring for 2 h. Water and ethyl acetate were added for extraction and liquid separation, the organic phase was further washed with saturated brine, the organic phase was collected and concentrated under reduced pressure, and the resulting residue was purified by reverse phase column chromatography (acetonitrile : water (v : v)=10%-80%) to obtain compound 71 (22 mg, 9.8%).
¹H NMR (400 MHz, CDCl₃) δ 8.90 (s, 2H), 7.55 (d, 1H), 7.35 (s, 1H), 7.28-7.25 (m, 1H), 7.19-7.17 (m, 1H), 7.05 (d, 1H), 5.36 (s, 2H), 4.74-4.72 (m, 1H), 4.63-4.55 (m, 3H), 2.90-2.85 (m, 4H), 2.30-2.18 (m, 2H), 2.09-2.00 (m, 2H);
LC-MS (ESI): m/z = 381.3 [M+H]⁺.

### Example 72

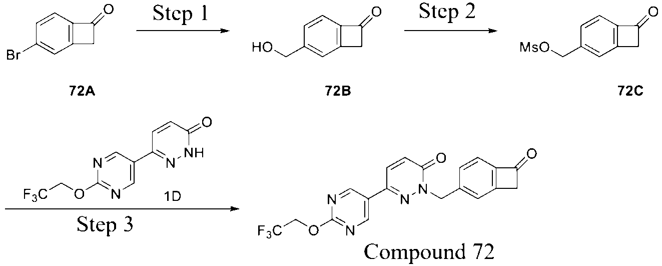

Step 1: Compound 72A (1.00 g, 5.08 mmol), (tributyltin)methanol (1.96 g, 6.10 mmol) and chloro(2-dicyclohexylphosphino-2',4',6'-triisopropyl-1,1'-biphenyl)[2-(2'-amino-1,1'-biphenyl)]palladium(II) (0.40 g, 0.51 mmol) were added to the solvent 1,4-dioxane (30 mL) in sequence, the obtained system was then subjected to nitrogen replacement 3 times, raised to 110°C and reacted under stirring for 2 h. After the reaction was completed, it was cooled to room temperature, then ethyl acetate (100 mL) and saturated brine (100 mL) were added for liquid separation, the organic phase was collected and washed with saturated brine 3 times, then dried over anhydrous sodium sulfate, and filtered, the filtrate was concentrated under reduced pressure, the resulting residue was purified by chromatography (ethyl acetate : petroleum ether (v : v)=0-100%) to obtain compound 72B (0.24 g, yield: 31.89%).

LC-MS (ESI): m/z = 149.1 [M+H]⁺.

Step 2: Compound 72B (0.24 g, 1.62 mmol) was added to the solvent dichloromethane (10 mL), then triethylamine (0.16 g, 1.62 mmol) was added, and methanesulfonyl chloride (0.19 g, 1.62 mmol) was slowly added dropwise in an ice bath, after the completion of dropwise addition, the obtained system was continuously reacted under stirring for 30 min. After the reaction was completed, ethyl acetate (100 mL) and aqueous sodium bicarbonate solution (100 mL) were added for liquid separation, the organic phase was collected and washed with saturated brine 3 times, then dried over anhydrous sodium sulfate, and filtered, the filtrate was concentrated under reduced pressure, the resulting residue was purified by column chromatography (ethyl acetate : petroleum ether (v : v)=0-50%) to obtain compound 72C (0.18 g, yield: 49.10%).

LC-MS (ESI): m/z = 227.20 [M+H]⁺.

Step 3: Compound 1D (90 mg, 0.33 mmol), compound 72C (75 mg, 0.33 mmol), and potassium carbonate (46 mg, 0.33 mmol) were added to acetonitrile (10 mL) in sequence, and the obtained system was heated to 70°C and reacted under stirring for 2 h. The system was cooled to room temperature, then ethyl acetate and saturated brine were added for liquid separation, the organic phase was collected and washed with saturated brine 3 times, and then concentrate under reduced pressure, the resulting residue was purified by reverse phase column chromatography (acetonitrile : water (v : v)=5% to 80%) to obtain compound 72 (51 mg, yield: 38.41%).
¹H NMR (400 MHz, CDCl₃) δ 8.94 (s, 2H), 7.70 - 7.59 (m, 2H), 7.53 - 7.48 (m, 1H), 7.34-7.32 (m, 1H), 7.11 (d, 1H), 5.45 (s, 2H), 4.91-4.85 (m, 2H), 3.95 (s, 2H);
LC-MS (ESI): m/z = 403.50 [M+H]⁺.

### Example 73

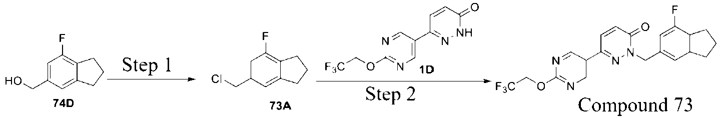

Step 1: Compound 74D (0.20 g, 1.20 mmol) and dichloromethane (15 mL) were added to a single-necked bottle, which was placed at 0°C, and the obtained system was stirred, then thionyl chloride (0.29 g, 2.40 mmol) was slowly added dropwise, and after the addition was completed, the obtained system was stirred at room temperature for 2 h. The obtained system was concentrated directly to obtain compound 73A (0.22 g, 99.29%), which was used directly for the next reaction.

Step 2: Compound 73A (0.22 g, 1.19 mmol) was dissolved in acetonitrile (15 mL), and compound 1D (0.36 g, 1.31 mmol) and cesium carbonate (1.18 g, 3.62 mmol) were added in sequence, and after the addition was completed, the obtained system was stirred at 80°C for 4 h. After cooling, the reaction liquid was filtered, and the filtrate was collected and concentrated, the resulting residue was purified by silica gel column chromatography (petroleum ether : ethyl acetate (v : v)=1 : 1) to obtain compound 73 (0.30 g, 59.97%).
¹H NMR ((400 MHz, CDCl₃) δ 8.94 (s, 2H), 7.58 (d, 1H), 7.11 (s, 1H), 7.08 (d, 1H), 6.94 (d, 1H), 6.34 (s, 2H), 4.88 (q, 2H), 2.93-2.88 (m, 4H), 2.13-2.06 (m, 2H);
LC-MS (ESI): m/z = 421.1 [M+H]⁺.

### Example 74

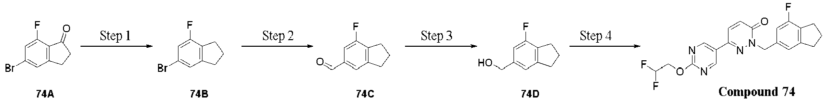

Step 1: Compound 74A (5 g, 21.83 mmol), triethylsilane (50 mL, 313.90 mmol) and trifluoroacetic acid (110 mL) were added in sequence to a 250 mL reaction flask, and after the addition was completed, the obtained system was stirred at 45°C for 16 h. The reaction liquid was concentrated under reduced pressure, and the resulting residue was extracted with ethyl acetate and water, the organic phase was collected and concentrated, and the resulting residue was purified by column chromatography (petroleum ether) to obtain compound 74B (3.7 g, yield: 78.81%).

Step 2: In a 100 mL reaction flask, compound 74B (3.7 g, 17.20 mmol) and tetrahydrofuran (40 mL) were added in sequence, and n-butyllithium (8.26 mL, 20.64 mmol, 2.5 M in hexane) was slowly added dropwise at -78°C under nitrogen protection, the obtained system was stirred for half an hour after the completion of addition, then N,N-dimethylformamide (3 mL) was further added dropwise, and the obtained system was stirred at -78°C for 1 h. After the reaction liquid was quenched by adding water, the obtained system was extracted with ethyl acetate, the organic phase was collected, washed and concentrated, the resulting residue was separated by column chromatography (petroleum ether : ethyl acetate (v : v)=1 : 0-0 : 1) to obtain compound 74C (2.3 g, yield: 81.45%).

Step 3: In a 100 mL reaction flask, compound 74C (2.3 g, 14.01 mmol) and methanol (30 mL) were added in sequence, followed by sodium borohydride (1.59 g, 42.03 mmol), and after the addition was completed, the obtained system was reacted under stirring at room temperature for 1 h. The reaction liquid was quenched with water and extracted with ethyl acetate. The organic phase was collected, washed and concentrated. The residue was separated by column chromatography (petroleum ether : ethyl acetate (v : v) = 1 : 0-0 : 1) to obtain compound 74D (2.2 g, yield: 94.49%).

Step 4: In a 100 mL reaction flask, compound 74D (100 mg, 0.60 mmol), intermediate 1 (0.18 g, 0.72 mmol), triphenylphosphine (0.24 g, 0.9 mmol) and tetrahydrofuran (4 mL) were added in sequence, and the obtained system was stirred at 0°C for 20 min after the completion of addition, then diisopropyl azodicarboxylate (0.18 g, 0.9 mmol) was slowly added dropwise under nitrogen protection, and the obtained system was reacted under stirring at room temperature for 3 h. The reaction liquid was concentrated, and the resulting residue was purified by preparative HPLC. Method: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.05% aqueous ammonia); b. gradient elution, mobile phase A: 25%-75%; c. flow rate: 15 mL/min; d. elution time: 20 min. retention time: 9.2 min. Compound 74 (95.8 mg, yield: 39.68%) was obtained.
¹H NMR (400 MHz, DMSO-*d*₆) δ 9.13 (s, 2H), 8.10 (d, 1H), 7.14 (d, 1H), 7.09 (s, 1H), 6.98 (s, 1H), 6.59-6.30 (m, 1H), 5.28 (s, 2H), 4.73-4.65 (m, 2H), 2.89-2.82 (m, 4H), 2.07-2.01 (m, 2H);
LCMS m/z = 403.4 [M+H]⁺.

### Example 75

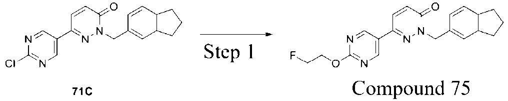

Step 1: 2-fluoroethanol (0.38 g, 5.90 mmol) was added to solvent tetrahydrofuran (10 mL), sodium (68 mg, 2.96 mmol) was added and the obtained system was stirred at room temperature until no visible sodium was found. Compound 71C (200 mg, 0.59 mmol) was added to the reaction liquid, and the obtained system was reacted under stirring at room temperature for 2 h. Water and ethyl acetate were added for liquid separation, the organic phase was collected, then washed with saturated brine, dried over anhydrous sodium sulfate and concentrated in sequence, the resulting residue was purified by reverse phase column chromatography (acetonitrile : water (v : v)=10%-80%) to obtain compound 75 (100 mg, yield: 46.2%).
¹H NMR (400 MHz, CDCl₃) δ 8.91 (s, 2H), 7.56 (d, 1H), 7.34 (s, 1H), 7.27 - 7.24 (m, 1H), 7.18 (d, 1H), 7.04 (d, 1H), 5.35 (s, 2H), 4.89 - 4.81 (m, 1H), 4.75-4.72 (m, 2H), 4.70 - 4.62 (m, 1H), 2.90-2.85 (m, 4H), 2.08-2.00 (m, 2H);
LC-MS (ESI): m/z = 367.40 [M+H]⁺.

### Example 76

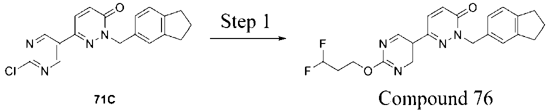

Step 1: 3,3-difluoropropan-1-ol (0.17 g, 1.77 mmol) was dissolved in tetrahydrofuran (10 mL), then sodium block (0.021 g, 0.90 mmol) was slowly added in an ice-water bath, the obtained system was heated to 60°C and stirred until the sodium block completely disappeared, afterwards it was cooled to room temperature and compound 71C (0.2 g, 0.59 mmol) was added, and then the obtained system was continuously reacted under stirring for 3 h. The reaction liquid was diluted with water, and extracted with ethyl acetate 3 times, the organic phases were combined and concentrated under reduced pressure, and the resulting residue was purified by preparative HPLC. Method: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.5% ammonium acetate); b. gradient elution, mobile phase A: 40%-70%; c. flow rate: 20 mL/min; d. elution time: 16 min. retention time: 15 min. Compound 76 (20 mg, yield: 8.5%) was obtained.
¹H NMR (400 MHz, DMSO-*d*₆) δ 9.08 (s, 2H), 8.08 (d, 1H), 7.24 (s, 1H), 7.19 - 7.11 (m, 3H), 6.45-6.06 (m, 1H), 5.27 (s, 2H), 4.55-4.48 (m, 2H), 2.83-2.77 (m, 4H), 2.44-2.30 (m, 2H), 2.01-1.95 (m, 2H);
LC-MS (ESI): m/z = 399.40 [M+H]⁺.

### Example 77

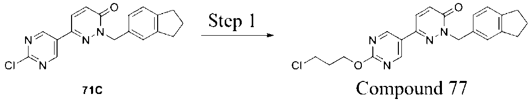

Step 1: 3-chloropropan-1-ol (0.17 g, 1.80 mmol) was dissolved in tetrahydrofuran (8 mL), sodium block (0.021 g, 0.90 mmol) was slowly added in an ice-water bath, and then the obtained system was heated to 60°C under stirring until the sodium block completely disappeared. Then, the obtained system was cooled to room temperature, compound 71C (0.2 g, 0.59 mmol) was added, and the reaction was continued under stirring for 3 h. The reaction liquid was diluted with water, and extracted with ethyl acetate 3 times, the organic phases were combined and concentrated under reduced pressure, and the resulting residue was purified by preparative HPLC. Method: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.5% ammonium acetate); b. gradient elution, mobile phase A: 40%-80%; c. flow rate: 20 mL/min; d. elution time: 16 min. retention time: 15 min. Compound 77 (25 mg, yield: 10.6%) was obtained after lyophilization.
¹H NMR (400 MHz, DMSO-*d*₆) δ 9.07 (s, 2H), 8.07 (d, 1H), 7.24 (s, 1H), 7.19 - 7.08 (m, 3H), 5.27 (s, 2H), 4.51-4.45 (m, 2H), 3.88-3.76 (m, 2H), 2.87-2.77 (m, 4H), 2.27-2.19 (m, 2H), 2.04-1.94 (m, 2H);
LC-MS (ESI): m/z = 397.40 [M+H]⁺.

### Example 78

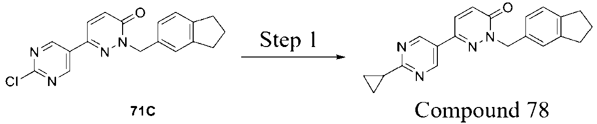

Step 1: Compound 71C (0.2 g, 0.59 mmol), and chloro(2-dicyclohexylphosphino-2',6'-diisopropoxy-1,1'-biphenyl)(2-amino-1,1'-biphenyl-2-yl)palladium (II) (0.046 g, 0.059 mmol) were dissolved in anhydrous dioxane (2 mL), the obtained system was subjected to nitrogen replacement, then a solution of cyclopropylmagnesium bromide in tetrahydrofuran (1.2 mL, 2.4 mmol) was added, and the obtained system was reacted under stirring at 100°C for 3 h under nitrogen protection. After cooling, water and ethyl acetate was added for extraction, the organic phase was collected and concentrated under reduced pressure, and the resulting residue was purified by preparative HPLC. Method: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: Pure water; b. gradient elution, mobile phase A: 10%-70%; c. flow rate: 15 mL/min. d. elution time: 20 min. retention time: 9.2 min. Compound 78 (3.3 mg, yield: 1.6%) was obtained.
¹H NMR (400 MHz, DMSO-*d*₆) δ 9.08 (s, 2H), 8.08 (d, 1H), 7.24 (s, 1H), 7.16 (s, 2H), 7.12 (d, 1H), 5.27 (s, 2H), 2.83 -2.78(m, 4H), 2.30 - 2.23 (m, 1H), 2.01 - 1.94 (m, 2H), 1.17 - 0.96 (m, 4H);
LC-MS (ESI): m/z = 345.1[M+H]⁺.

### Example 79

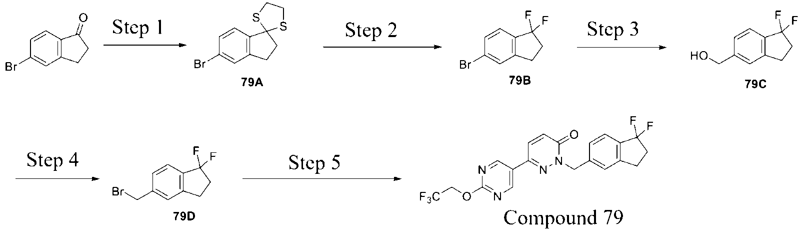

Step 1: 5-Bromo-2,3-dihydro-1H-inden-1-one (2.11 g, 10 mmol) was dissolved in toluene (100 mL), 1,2-ethanedithiol (1.88 g, 20 mmol) and p-toluenesulfonic acid (172 mg, 1 mmol) were added in sequence, and the obtained system was heated to 110°C and reacted overnight. The system was cooled to room temperature and concentrated, the resulting residue was separated by silica gel column chromatography (petroleum ether : ethyl acetate (v : v)=10 : 1) to obtain compound 79A (2.68 g, yield: 93.4%).

Step 2: N-iodosuccinimide (1.57 g, 7 mmol) was weighed into a 50 mL plastic centrifuge tube, dichloromethane (10 mL) was added, the obtained system was cooled to -78°C, and hydrogen fluoride-pyridine solution (4 mL) was added, then compound 79A (1 g, 3.5 mmol) dissolved in dichloromethane (10 mL) and the obtained mixture was added dropwise into the above system, which was continuously reacted at this temperature for 2 h. Saturated sodium bicarbonate solution was added to the system until no more bubbles were generated, petroleum ether (100 mL) was added and shaken thoroughly, then the organic phase was collected, dried over anhydrous sodium sulfate, and concentrated, the resulting residue was purified by silica gel column chromatography (100% petroleum ether) to obtain compound 79B (235 mg, yield: 28.9%).

¹H NMR (400 MHz, CDCl₃) δ 7.49 - 7.37 (m, 3H), 3.06 - 2.96 (m, 2H), 2.66 - 2.51 (m, 2H).

Step 3: Compound 79B (235 mg, 1 mmol) was dissolved in 1,4-dioxane (10 mL), then (tributyltin)methanol (353 mg, 1.1 mmol) and chloro(2-dicyclohexylphosphino-2',4',6'-triisopropyl-1,1'-biphenyl)[2-(2'-amino-1,1'-biphenyl)]palladium(II)(Xphos-pd-G2) (78 mg, 0.1 mmol) were added, the obtained system was subjected to nitrogen replacement and then reacted at 90°C for 4 h. After cooling, the reaction liquid was concentrated, and the resulting residue was purified by silica gel column chromatography (petroleum ether : ethyl acetate (v : v)=1 : 0-5 : 1) to obtain compound 79C (116 mg, yield: 63%).

LC-MS (ESI): m/z = 165.2 [M-HF+H]⁺, 167.1 [M-OH⁻]⁺.

Step 4: Compound 79C (116 mg, 0.6 mmol) was dissolved in dichloromethane (10 mL), then triphenylphosphine (471 mg, 1.8 mmol) and carbon tetrabromide (596 mg, 1.8 mmol) were added in sequence, and the obtained system was stirred at room temperature for 2 h. The reaction liquid was concentrated directly, and the resulting residue was purified by column chromatography (100% petroleum ether) to obtain compound 79D (128 mg, yield: 82.1%).

Step 5: Compound 79D (128 mg, 0.52 mmol) was dissolved in DMF (10 mL), compound 1D (163 mg, 0.6 mmol) and potassium carbonate (276 mg, 2 mmol) were added, and the obtained system was reacted at 70°C for 2 h. Water (50 mL) was added, and ethyl acetate was used for extraction (50 mL×2), the organic phase was collected, washed with saturated brine, and dried over anhydrous sodium sulfate, then filtered, the filtrate was collected, and concentrated, the resulting residue was purified by column chromatography (petroleum ether: ethyl acetate (v : v)=1 : 0-1 : 1) to obtain compound 79 (69 mg, yield: 30.4%).
¹H NMR (400 MHz, CDCl₃) δ 8.94 (s, 2H), 7.60 (d, 1H), 7.52 (d, 1H), 7.47 - 7.42 (m, 1H), 7.40 (s, 1H), 7.09 (d, 1H), 5.42 (s, 2H), 4.92 - 4.84 (m, 2H), 3.06 - 2.98 (m, 2H), 2.65 - 2.49 (m, 2H);
LC-MS (ESI): m/z = 439.2[M+H]⁺.

### Example 80

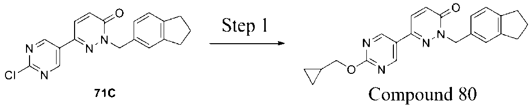

Step 1: Cyclopropylmethanol (0.12 g, 1.73 mmol) was dissolved in tetrahydrofuran (8 mL), and then sodium hydride (0.064 g, 2.67 mmol) was slowly added in portions in an ice-water bath, the obtained system was stirred for half an hour, then compound 71C (0.3 g, 0.89 mmol) was added, and then the obtained system was returned to room temperature and continuously reacted for 2 h. After the reaction was completed, the reaction liquid was diluted with water, and extracted with ethyl acetate 3 times, the organic phases were combined and concentrated under reduced pressure, and the resulting residue was purified by preparative HPLC. Method: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.5% ammonium acetate); b. gradient elution, mobile phase A: 40%-85%; c. flow rate: 20 mL/min; d. elution time: 18 min. retention time: 17 min. Compound 80 (100 mg, yield: 30%) was obtained.
¹H NMR (400 MHz, DMSO-*d*₆) δ 9.06 (s, 2H), 8.07 (d, 1H), 7.24 (s, 1H), 7.21 -7.15 (m, 2H), 7.16 -7.09 (m, 1H), 5.26 (s, 2H), 4.21 (d, 2H), 2.86-2.76 (m, 4H), 2.02-1.90 (m, 2H), 1.34-1.20 (m, 1H), 0.66 - 0.53 (m, 2H), 0.48 - 0.31 (m, 2H);
LC-MS (ESI): m/z = 375.10 [M+H]⁺.

### Example 81

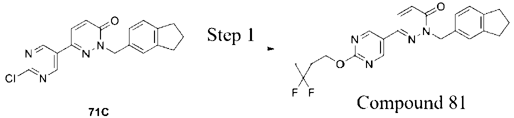

Step 1: (2,2-difluorocyclopropyl)methanol (0.19 g, 1.76 mmol) was dissolve in tetrahydrofuran (8 mL), then sodium hydride (0.064 g, 2.67 mmol) was slowly added in portions in an ice-water bath, the obtained system was continuously stirred for half an hour, then compound 71C (0.3 g, 0.89 mmol) was added, and the obtained system was returned to room temperature and continuously reacted for 2 h. After the reaction was completed, the reaction liquid was diluted with water, and extracted with ethyl acetate 3 times, the organic phases were combined and concentrated under reduced pressure, and the resulting residue was purified by preparative HPLC. Method: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.5% ammonium acetate); b. gradient elution, mobile phase A: 40%-80%; c. flow rate: 20 mL/min; d. elution time: 18 min. retention time: 17 min. Compound 81 (200 mg, yield: 54%) was obtained.
¹H NMR (400 MHz, DMSO-*d*₆) δ 9.09 (s, 2H), 8.09 (d, 1H), 7.24 (s, 1H), 7.20 - 7.16 (m, 2H), 7.13 (d, 1H), 5.27 (s, 2H), 4.62-4.24 (m, 2H), 2.87-2.75 (m, 4H), 2.36 - 2.23 (m, 1H), 2.04-1.90(m, 2H), 1.80-1.69 (m, 1H), 1.63-1.53 (m, 1H);
LC-MS (ESI): m/z = 411.10 [M+H]⁺.

### Example 82

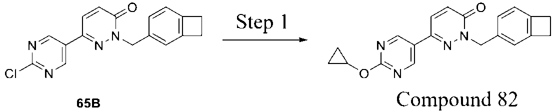

Step 1: Cyclopropanol (27 mg, 0.46 mmol) was added to solvent acetonitrile (10 mL), sodium hydride (9.2 mg, 0.23 mmol) was added in an ice bath, and the obtained system was reacted under stirring for 15 min. Compound 65B (50 mg, 0.15 mmol) was further added and the obtained system was reacted at room temperature for 1 h. Ethyl acetate and saturated brine were added for extraction and liquid separation. The organic phase was collected and washed 3 times with saturated brine, and then concentrated under reduced pressure, the resulting residue was purified by reverse phase column chromatography (acetonitrile : water (v : v)=5% to 80%) to obtain compound 82 (11 mg, yield: 21.17%).
¹H NMR (400 MHz, CDCl₃) δ 8.93-8.89 (m, 2H), 7.59-7.52 (m, 1H), 7.35-7.33 (m, 1H), 7.20 (s, 1H), 7.06-7.01 (m, 2H), 5.35 (s, 2H), 4.64 - 4.25 (m, 1H), 3.14 (s, 4H), 0.96-0.82 (m, 4H);
LC-MS (ESI): m/z = 347.1 [M+H]⁺.

### Example 83

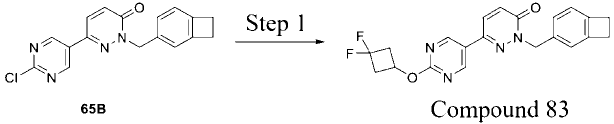

Step 1: 3,3-difluorocyclobutanol (49 mg, 0.45 mmol) was added to solvent acetonitrile (10 mL), sodium hydride (9.2 mg, 0.23 mmol) was further added in an ice bath, and the obtained system was reacted under stirring for 15 min. Compound 65B (50 mg, 0.15 mmol) was further added and the obtained system was reacted at room temperature for 1 h. Ethyl acetate and saturated brine were added for extraction and liquid separation. The organic phase was collected and washed 3 times with saturated brine, and then concentrated under reduced pressure, the resulting residue was purified by reverse phase column chromatography (acetonitrile : water (v : v)=5% to 80%) to obtain compound 83 (18 mg, yield: 30.27%).
¹H NMR (400 MHz, CDCl₃) δ 8.91 (s, 2H), 7.55 (d, 1H), 7.35-7.32 (m, 1H), 7.20 (s, 1H), 7.07-7.01 (m, 2H), 5.35 (s, 2H), 5.23-5.14 (m, 1H), 3.24 - 3.08 (m, 6H), 2.95-2.75 (m, 2H);
LC-MS (ESI): m/z = 397.1 [M+H]⁺.

### Example 84

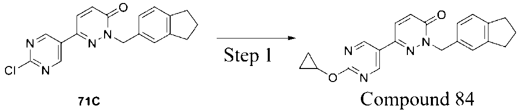

Step 1: Cyclopropanol (0.052 g, 0.89 mmol) was added to solvent tetrahydrofuran (5 mL), sodium hydride (0.024 g, 0.60 mmol) was added, and the obtained system was reacted under stirring for half an hour. Compound 71C (0.1 g, 0.30 mmol) was added to the reaction liquid, and the obtained system was reacted under stirring at room temperature for 2 h. Water and ethyl acetate were added for extraction and liquid separation, the organic phase was dried over anhydrous sodium sulfate and concentrated under reduced pressure, the resulting residue was purified by reverse phase column chromatography (acetonitrile : water (v : v)=3%-60%) to obtain compound 84 (0.05 g, yield: 46.24%).
¹H NMR (400 MHz, DMSO-*d*₆) δ 9.09 (s, 2H), 8.08 (d, 1H), 7.25 (s, 1H), 7.20-7.12 (m, 3H), 5.26 (s, 2H), 4.38-4.34 (m, 1H), 2.84-2.79 (m, 4H), 2.01-1.94 (m, 2H), 0.87 - 0.70 (m, 4H);
LC-MS (ESI): m/z = 361.1 [M+H]⁺.

### Example 85

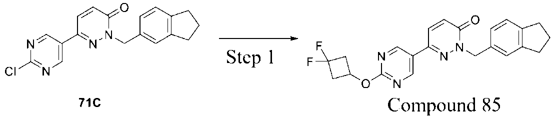

Step 1: 3,3-difluorocyclobutanol (0.097 g, 0.90 mmol) was added to solvent tetrahydrofuran (5 mL), sodium hydride (0.024 g, 0.60 mmol) was added, and the obtained system was reacted under stirring for half an hour. Compound 71C (0.1 g, 0.30 mmol) was added to the reaction liquid, and the obtained system was reacted under stirring at room temperature for 2 h. Water and ethyl acetate were added for extraction and liquid separation, the organic phase was dried over anhydrous sodium sulfate and concentrated under reduced pressure, the resulting residue was purified by reverse phase column chromatography (acetonitrile : water (v : v) = 3%-60%) to obtain compound 85 (0.047 g, yield: 38.17%).
¹H NMR (400 MHz, DMSO-*d*₆) δ 9.10 (s, 2H), 8.08 (d, 1H), 7.24 (s, 1H), 7.17-7.12 (m, 3H), 5.26 (s, 2H), 5.18-5.15 (m, 1H), 3.24-3.13 (m, 2H), 2.86-2.76 (m, 6H), 2.01-1.94 (m, 2H);
LC-MS (ESI): m/z = 411.1 [M+H]⁺.

### Example 86

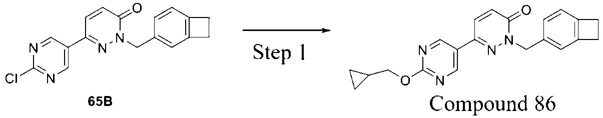

Step 1: Cyclopropylmethanol (0.054 g, 0.75 mmol) was added to solvent tetrahydrofuran (5 mL), sodium hydride (0.011 g, 0.45 mmol) was added, and the obtained system was reacted under stirring for half an hour. Compound 65B (0.05 g, 0.15 mmol) was added to the reaction liquid, and the obtained system was reacted under stirring at room temperature for 2 h. Water and ethyl acetate were added for extraction and liquid separation, the organic phase was dried over anhydrous sodium sulfate and concentrated under reduced pressure, the resulting residue was purified by reverse phase column chromatography (acetonitrile : water (v : v)=3%-60%) to obtain compound 86 (0.02 g, yield: 36.99%).
¹H NMR (400 MHz, DMSO-*d*₆) δ 9.05 (s, 2H), 8.06 (d, 1H), 7.24 (d, 1H), 7.14 - 7.08 (m, 2H), 7.04 (d, 1H), 5.26 (s, 2H), 4.21 (d, 2H), 3.09 (s, 4H), 1.32-1.25 (m, 1H), 0.65 - 0.50 (m, 2H), 0.44 - 0.31 (m, 2H);
LC-MS (ESI): m/z = 361.3 [M+H]⁺.

### Example 87

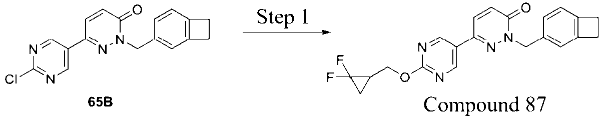

Step 1: 2,2-difluorocyclopropylmethanol (0.081 g, 0.75 mmol) was added to solvent tetrahydrofuran (5 mL), sodium hydride (0.011 g, 0.45 mmol) was added, and the obtained system was reacted under stirring for half an hour. Compound 65B (0.05 g, 0.15 mmol) was added to the reaction liquid, and the obtained system was reacted under stirring at room temperature for 2 h. Water and ethyl acetate were added for extraction and liquid separation, the organic phase was dried over anhydrous sodium sulfate and then concentrated under reduced pressure, the resulting residue was purified by reverse phase column chromatography (acetonitrile : water (v : v)=3%-60%) to obtain compound 86 (0.02 g, yield: 36.64%).
¹H NMR (400 MHz, DMSO-*d*₆) δ 9.09 (s, 2H), 8.08 (d, 1H), 7.24 (d, 1H), 7.16 -7.08 (m, 2H), 7.04 (d, 1H), 5.27 (s, 2H), 4.62 - 4.51 (m, 1H), 4.38 - 4.29 (m, 1H), 3.09 (s, 4H), 2.34-2.25(m, 1H), 1.83 - 1.67 (m, 1H), 1.63 - 1.48 (m, 1H);
LC-MS (ESI): m/z = 397.2 [M+H]⁺ .

### Example 88

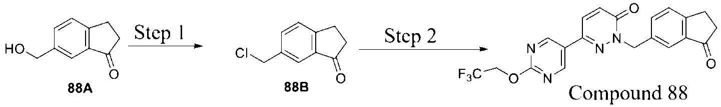

Step 1: Compound 88A (0.2 g, 1.23 mmol) was dissolved in dichloromethane (10 mL), thionyl chloride (0.16 g, 1.35 mmol) was added, and the obtained system was stirred at room temperature for 2 h. After the reaction was completed, the obtained system was concentrated under reduced pressure to obtain compound 88B (0.24 g of crude product), which was used directly for the next reaction.

Step 2: Compound 1D (0.3 g, 1.10 mmol), compound 88B (0.24 g, 1.32 mmol) and potassium carbonate (0.46 g, 3.30 mmol) were dissolved in N,N-dimethylformamide (10 mL) and then the obtained system was stirred at 70°C for 2 h. After cooling, the reaction liquid was concentrated directly, water (10 mL) was added to the resulting crude product and ethyl acetate (15 mL) was used for extraction for 3 times, the organic phases were combined, dried and concentrated, and then the resulting residue was purified by reverse phase column chromatography (acetonitrile : water (v : v)=5%-70%) to obtain compound 88 (0.22 g, yield: 48.04%).
¹H NMR (400 MHz, DMSO-*d*₆) δ 9.16 (s, 2H), 8.13 (d, 1H), 7.77 - 7.71 (m, 1H), 7.62 (s, 1H), 7.58 (d, 1H), 7.17 (d, 1H), 5.40 (s, 2H), 5.15-5.08 (m, 2H), 3.13 - 3.01 (m, 2H), 2.70 - 2.57 (m, 2H);
LC-MS (ESI): m/z = 417.1 [M+H]⁺.

### Example 89

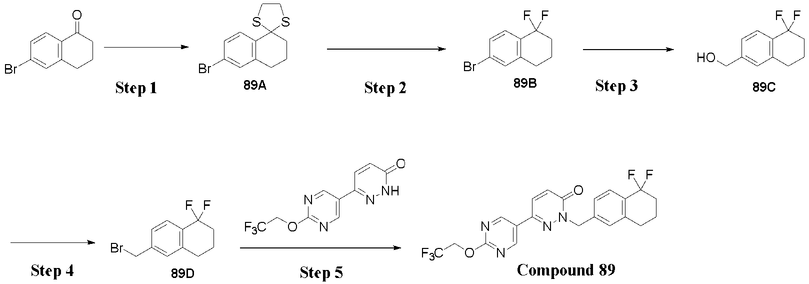

Step 1: 6-bromo-3,4-dihydronaphthalen-1(2H)-one (675 mg, 3 mmol) was dissolved in toluene (30 mL), 1,2-ethanedithiol (567 mg, 6 mmol) and p-toluenesulfonic acid (52 mg, 0.3 mmol) were added in sequence, and the obtained system was reacted overnight at 110°C. The system was cooled to room temperature and concentrated, the resulting residue was purified by silica gel column chromatography (petroleum ether : ethyl acetate (v : v)=10 : 1) to obtain compound 89A (685 mg, yield: 75.8%).

¹H NMR (400 MHz, CDCl₃) δ 8.04 (d, 1H), 7.23 - 7.18(m, 1H), 6.87 - 6.82 (m, 1H), 3.63 - 3.53 (m, 2H), 3.50 - 3.39 (m, 2H), 2.72 (t, 2H), 2.38 - 2.32 (m, 2H), 2.02 - 1.94 (m, 2H).

Step 2: N-iodosuccinimide (1.03 g, 4.6 mmol) was weighed into a 50 mL plastic centrifuge tube, dichloromethane (10 mL) was added, the obtained system was cooled to -78°C, and hydrogen fluoride-pyridine solution (4 mL) was added, then compound 89A (685 mg, 2.3 mmol) dissolved in dichloromethane (10 mL) and the obtained mixture was added dropwise into the above system, which was continuously reacted at this temperature for 2 h. Saturated sodium bicarbonate solution was added to the system until no more bubbles were generated, petroleum ether (100 mL) was added and shaken thoroughly, then the organic phase was collected, dried over anhydrous sodium sulfate, and concentrated, the resulting residue was purified by silica gel column chromatography (100% petroleum ether) to obtain compound 89B (463 mg, yield: 81.5%).

¹H NMR (400 MHz, CDCl₃) δ 7.72 (d, 1H), 7.39 - 7.32(m, 1H), 6.98 - 6.92 (m, 1H), 2.72 - 2.64 (m, 2H), 2.63 - 2.11 (m, 2H), 1.95 - 1.86 (m, 2H).

Step 3: Compound 89B (463 mg, 1.87 mmol) was dissolved in 1,4-dioxane (10 mL), then (tributyltin)methanol (642 mg, 2 mmol) and chloro(2-dicyclohexylphosphino-2',4',6'-triisopropyl-1,1'-biphenyl)[2-(2'-amino-1,1'-biphenyl)]palladium(II)(Xphos-Pd-G2) (78 mg, 0.1 mmol) were added, the obtained system was subjected to nitrogen replacement and then reacted at 90°C for 4 h. After cooling, the reaction liquid was concentrated, and the resulting residue was purified by silica gel column chromatography (petroleum ether : ethyl acetate (v : v)=1 : 0-5 : 1) to obtain compound 89C (259 mg, yield: 77%).

Step 4: Compound 89C (259 mg, 1.3 mmol) was dissolved in dichloromethane (10 mL), then triphenylphosphine (681 mg, 2.6 mmol) and carbon tetrabromide (860 mg, 2.6 mmol) were added in sequence, and the obtained system was stirred at room temperature for 2 h. The obtained system was concentrated directly, and the resulting residue was purified by column chromatography (100% petroleum ether) to obtain compound 89D (167 mg, yield: 49.2%).

Step 5: Compound 89D (167 mg, 0.64 mmol) was dissolved in N,N-dimethylformamide (10 mL), compound 1D (217 mg, 0.8 mmol) and potassium carbonate (276 mg, 2 mmol) were added, and the obtained system was reacted at 70°C for 2 h. water (50 mL) was added, and ethyl acetate (50 mL×2) was used for extraction, the organic phase was collected, washed with saturated brine, and dried over anhydrous sodium sulfate, then filtered, the filtrate was collected, and concentrated, the resulting residue was purified by column chromatography (petroleum ether: ethyl acetate (v : v)=1 : 0-1 : 1) to obtain compound 89 (75 mg, yield: 30.4%).
¹H NMR (400 MHz, CDCl₃) δ 8.92 (s, 2H), 7.77 (s, 1H), 7.57 (d, 1H), 7.47 (d, 1H), 7.15 (d, 1H), 7.07 (d, 1H), 5.40 (s, 2H), 4.93 - 4.81 (m, 2H), 2.83 - 2.76 (m, 2H), 2.33 - 2.19 (m, 2H), 2.03 - 1.94 (m, 2H);
LC-MS (ESI): m/z = 453.2 [M+H]⁺.

### Example 90 and example 91

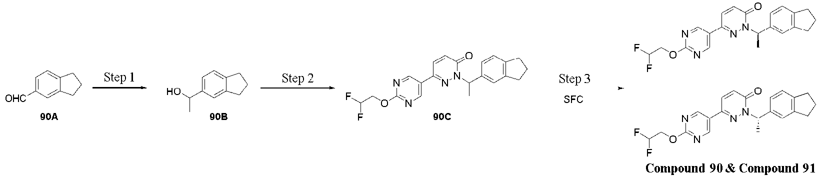

Step 1: Compound 90A (1.0 g, 6.82 mmol) was dissolved in tetrahydrofuran (25 mL), the obtained system was stirred at 0°C, and then methylmagnesium bromide (4.5 mL, 13.64 mmol, 3 M in diethyl ether) was slowly added dropwise, after the completion of addition, the obtained system was returned to room temperature and reacted. The reaction was monitored by TLC, when the reaction was completed, it was stirred in an ice bath, and quenched by adding water, then extracted with ethyl acetate (30 mL×3), the organic phase was collected and dried over anhydrous sodium sulfate, and filtered, the filtrate was concentrated to obtain compound 90B (1.0 g of crude product), which was used directly for the next reaction.

Step 2: Compound 90B (0.60 g, 3.70 mmol) was dissolved in tetrahydrofuran (20 mL), then triphenylphosphine (1.46 g, 5.55 mmol) and intermediate 1 (1.03 g, 4.07 mmol) were added, and diisopropyl azodicarboxylate (1.12 g, 5.55 mmol) was slowly added dropwise at 0°C, then the obtained system was slowly raised to room temperature and reacted overnight. The reaction liquid was concentrated directly, the resulting residue was purified by silica gel column chromatography (petroleum ether : ethyl acetate (v : v)=1 : 1) to obtain compound 90C (460.0 mg, yield: 31.21%).

Step 3: Compound 90C (460.0 mg) was subjected to chiral resolution to obtain P1 (retention time: 1.404 min, set as compound 90. SFC analytical method: instrument: SHIMADZU LC-30AD; chromatographic column: Chiralcel OJ column; mobile phase: A for CO₂; B for 0.05% DEA in MeOH; gradient: 40% B gradient elution; flow rate: 3 mL/min; column temperature: 35°C; column pressure: 100 bar; wavelength: 220 nm.) and P2 (retention time: 1.629 min, set as compound 91). Preparation method: instruments: Waters 150 Prep-SFC F; column: Chiralcel OJ column; mobile phase: A for CO₂; B for 0.1% NH₃•H₂O in MeOH; gradient: 30% B Gradient elution; flow rate: 100 mL/min; column temperature: 25°C; wavelength: 220 nm; cycle time: 3.6 min; sample preparation: sample concentration 10 mg/mL, acetonitrile solution; sample injection: 2.5 mL/injection. After the separation, the system was dried and concentrated by a rotary evaporator at a bath temperature of 35°C, and then the solvent was dried by a lyophilizer at -80°C to obtain compound 90 (180 mg) and compound 91 (163 mg).

Compound 90: ¹H NMR ((400 MHz, CDCl₃) δ 8.93 (s, 2H), 7.53 (d, 1H), 7.34 (s, 1H), 7.28-7.25 (m, 1H), 7.18 (d, 1H), 7.03 (d, 1H), 6.40 (q, 1H), 6.33-6.03 (m, 1H), 4.69-4.62 (m, 2H), 2.90-2.85 (m, 4H), 2.09-2.01 (m, 2H), 1.82 (d, 3H); LC-MS (ESI): m/z = 399.1 [M+H]⁺.

Compound 91: ¹H NMR ((400 MHz, CDCl₃) δ 8.93 (s, 2H), 7.53 (d, 1H), 7.34 (s, 1H), 7.28-7.25 (m, 1H), 7.18 (d, 1H), 7.03 (d, 1H), 6.40 (q, 1H), 6.33-6.03 (m, 1H), 4.69-4.62 (m, 2H), 2.90-2.85 (m, 4H), 2.09-2.01 (m, 2H), 1.82 (d, 3H); LC-MS (ESI): m/z = 399.1 [M+H]⁺.

### Example 92

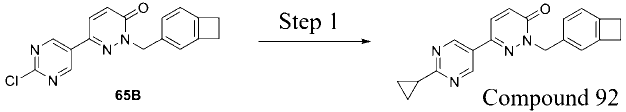

Step 1: Compound 65B (324 mg, 1 mmol), cyclopropylboronic acid (172 mg, 2 mmol), [1,1'-bis(diphenylphosphino)ferrocene]palladium(II) dichloride (73 mg, 0.1 mmol) and cesium carbonate (652 mg, 2 mmol) were dissolved in 1,4-dioxane (10 mL), the obtained system was subjected to nitrogen replacement, and then raised to 80°C and reacted for 5 h. After cooling to room temperature, the solid impurities in the system were removed by suction filtration, the filtrate was concentrated, and the resulting residue was purified by column chromatography (petroleum ether : ethyl acetate (v : v)=1 : 1) to obtain compound 92 (34 mg, yield :11 %).
¹H NMR (400 MHz, MeOD) δ 9.05 (s, 2H), 8.02 - 7.98 (m, 1H), 7.30 - 7.25 (m, 1H), 7.14 - 7.05 (m, 2H), 7.01 - 6.97 (m, 1H), 5.37 (s, 2H), 3.13 (s, 4H), 2.34 - 2.22 (m, 1H), 1.22 - 1.11 (m, 4H);
LCMS m/z =331.1 [M+H]⁺.

### Example 93

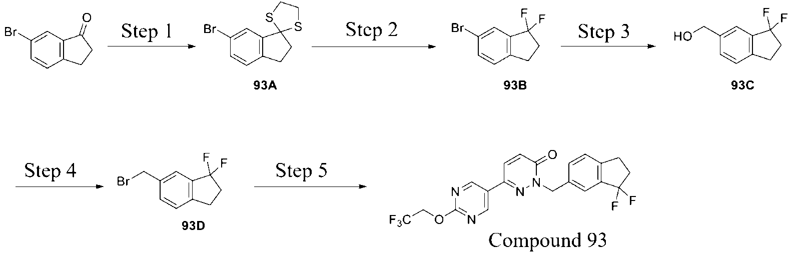

Step 1: 6-bromo-2,3-dihydro-1H-inden-1-one (4.2 g, 20 mmol) was dissolved in toluene (200 mL), 1,2-ethanedithiol (3.76 g, 40 mmol) and p-toluenesulfonic acid (344 mg, 2 mmol) were added in sequence, and the obtained system was heated to 110°C and reacted overnight. The system was cooled to room temperature and concentrated, the resulting residue was separated by silica gel column chromatography (petroleum ether : ethyl acetate (v : v)=10 : 1) to obtain compound 93A (5.4 g, yield: 94.1%).

Step 2: N-iodosuccinimide (1.57 g, 7 mmol) was weighed into a 50 mL plastic centrifuge tube, dichloromethane (10 mL) was added, the obtained system was cooled to -78°C, and hydrogen fluoride-pyridine solution (4 mL) was added, then compound 93A (1 g, 3.5 mmol) dissolved in dichloromethane (10 mL) and the obtained mixture was added dropwise into the above system, which was continuously reacted at this temperature for 2 h. Saturated sodium bicarbonate solution was added to the system until no more bubbles were generated, petroleum ether (100 mL) was added and shaken thoroughly, then the organic phase was collected, dried over anhydrous sodium sulfate, and concentrated, the resulting residue was purified by silica gel column chromatography (100% petroleum ether) to obtain compound 93B (228 mg, yield: 27.9%).

¹H NMR (400 MHz, CDCl₃) δ 7.67 (s, 1H), 7.56 - 7.52 (m, 1H), 7.19 - 7.15 (m, 1H), 3.02 - 2.95 (m, 2H), 2.65 - 2.53 (m, 2H).

Step 3: Compound 93B (228 mg, 0.98 mmol) was dissolved in 1,4-dioxane (10 mL), then (tributyltin)methanol (321 mg, 1 mmol) and chloro(2-dicyclohexylphosphino-2',4',6'-triisopropyl-1,1'-biphenyl)[2-(2'-amino-1,1'-biphenyl)]palladium(II)(Xphos-pd-G2) (78 mg, 0.1 mmol) were added, the obtained system was subjected to nitrogen replacement and then reacted at 90°C for 4 h. After cooling, the reaction liquid was concentrated, and the resulting residue was purified by silica gel column chromatography (petroleum ether : ethyl acetate (v : v)=1 : 0-5 : 1) to obtain compound 93C (137 mg, yield: 76.5%).

LC-MS (ESI): m/z = 165.2 [M-HF+H]⁺, 167.2 [M-OH⁻]⁺.

Step 4: Compound 93C (137 mg, 0.7 mmol) was dissolved in dichloromethane (10 mL), then triphenylphosphine (550 mg, 2.1 mmol) and carbon tetrabromide (695 mg, 2.1 mmol) were added in sequence, and the obtained system was stirred at room temperature for 1 h. The obtained system was concentrated directly, and the resulting residue was purified by column chromatography (100% petroleum ether) to obtain compound 93D (144 mg, yield: 83.2%).

Step 5: Compound 93D (144 mg, 0.58 mmol) was dissolved in DMF (10 mL), compound 1D (163 mg, 0.6 mmol) and potassium carbonate (276 mg, 2 mmol) were added, and the obtained system was reacted at 70°C for 2 h. Water (50 mL) was added, and ethyl acetate (50 mL×2) was used for extraction, the organic phase was collected, washed with saturated brine, and dried over anhydrous sodium sulfate, then filtered, the filtrate was collected, and concentrated, the resulting residue was purified by column chromatography (petroleum ether : ethyl acetate (v : v)=1 : 0-1 : 1) to obtain compound 93 (68 mg, yield: 26.7%).
¹H NMR (400 MHz, CDCl₃) δ 8.93 (s, 2H), 7.63 (s, 1H), 7.61 - 7.54 (m, 2H), 7.30 - 7.24 (m, 1H), 7.08 (d, 1H), 5.42 (s, 2H), 4.91 - 4.83 (m, 2H), 2.64 - 2.52 (m, 2H), 2.65 - 2.50 (m, 2H);
LC-MS (ESI): m/z = 439.2 [M+H]⁺.

### Example 94

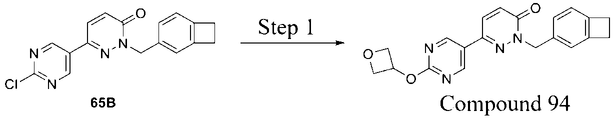

Step 1: Oxetan-3-ol (0.11 g, 1.48 mmol) was weighed into dry N,N-dimethylformamide (10 mL), then sodium hydride (0.053 g, 2.22 mmol) was added, the obtained system was stirred at room temperature for 30 min after the addition was completed, then compound 65B (0.24 g, 0.74 mmol) was weighed and added to the reaction liquid, which was then reacted at room temperature for 30 min. After the reaction was completed, water (30 mL) was added to the reaction liquid and ethyl acetate (100 mL×2) was used for extraction, the organic phases were combined, washed with saturated brine (150 mL×2), dried over anhydrous sodium sulfate, and filtered, the filtrate was collected and concentrated, and the resulting residue was purified by column chromatography (petroleum ether : ethyl acetate (v : v)=1 : 5) to obtain compound 94 (0.020 g, yield: 7.5%).
¹H NMR (400 MHz, DMSO-*d*₆) δ 9.07 (s, 2H), 8.07 - 8.05 (m, 1H), 7.25 - 7.23 (m, 1H), 7.16 - 7.08 (m, 2H), 7.05 - 7.03 (m, 1H), 5.64 - 5.61 m, 1H), 5.26 (s, 2H), 4.93 - 4.90 (m, 2H), 4.63 - 4.60 (m, 2H), 3.08 (s, 4H);
LC-MS (ESI): m/z = 363.1 [M+H]⁺.

### Example 95

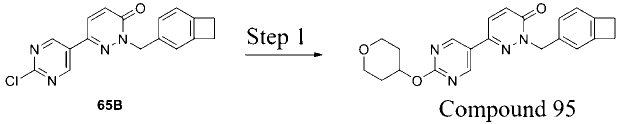

Step 1: Compound 65B (0.1 g, 0.31 mmol) and tetrahydro-2H-pyran-4-ol (47 mg, 0.46 mmol) were dissolved in anhydrous tetrahydrofuran (2 mL), then the obtained system was cooled to 0°C in an ice bath, and sodium hydride (15 mg, 0.37 mmol, 60%) was slowly added. The obtained system was naturally returned to room temperature and reacted under stirring overnight. After the reaction was completed, the reaction liquid was concentrated under reduced pressure, and the resulting residue was purified by preparative HPLC. Method: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: Pure water; b. gradient elution, mobile phase A: 10%-95%; c. flow rate: 15 mL/min; d. elution time: 20 min. Compound 95 (50 mg, yield: 41.3%) was obtained.
¹H NMR (400 MHz, DMSO-*d*₆) δ 9.06 (s, 2H), 8.06 (d, 1H), 7.27 - 7.20 (m, 1H), 7.15 - 7.07 (m, 2H), 7.04 (d, 1H), 5.26 (s, 2H), 5.24 - 5.18 (m, 1H), 3.90 - 3.85 (m, 2H), 3.57 - 3.46 (m, 2H), 3.09 (s, 4H), 2.09 - 2.00 (m, 2H), 1.74 - 1.65 (m, 2H);
LC-MS (ESI): m/z = 391.5 [M+H]⁺.

### Example 96

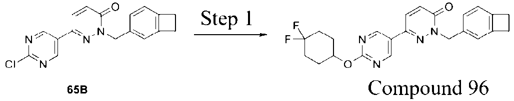

Step 1: 4,4-difluorocyclohexanol (0.38 g, 2.76 mmol) was added to solvent tetrahydrofuran (20 mL), sodium hydride (44 mg, 1.86 mmol) was further added in an ice bath, and the obtained system was reacted under stirring for 15 min. Compound 65B (0.3 g, 0.92 mmol) was further added and the obtained system was reacted at room temperature for 1 h. Ethyl acetate and saturated brine were added for liquid separation, the organic phase was collected and washed with saturated brine 3 times, and then concentrated, the resulting residue was purified by reverse phase column chromatography (acetonitrile : water (v : v)=5% to 80%) to obtain compound 96 (60 mg, yield: 15.37%).
¹H NMR (400 MHz, CDCl₃) δ 8.89 (s, 2H), 7.55 (d, 1H), 7.34-7.32 (m, 1H), 7.19 (d, 1H), 7.06-7.01 (m, 2H), 5.35 (s, 2H), 5.30-5.26 (m, 1H), 3.14 (s, 4H), 2.31 -1.89 (m, 8H);
LC-MS (ESI): m/z = 425.5 [M+H]⁺.

### Example 97

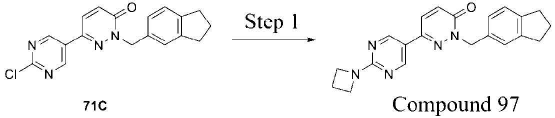

Step 1: Compound 71C (0.2 g, 0.59 mmol), azetidine (0.10 g, 1.75 mmol), potassium carbonate (0.16 g, 1.18 mmol) were dissolved in N,N-dimethylformamide (5 mL), and the obtained system was reacted under stirring at 60°C for 2 h. The system was cooled and concentrated under reduced pressure, and the resulting residue was purified by reverse phase column chromatography (acetonitrile : water (v : v)=3%-60%) to obtain compound 97 (0.02 g, yield: 9.43%)
¹H NMR (400 MHz, DMSO-*d*₆) δ 8.80 (s, 2H), 7.97 (d, 1H), 7.22 (s, 1H), 7.19-7.13 (m, 2H), 7.05 (d, 1H), 5.23 (s, 2H), 4.12-4.08 (m, 4H), 2.83-2.79 (m, 4H), 2.41 - 2.27 (m, 2H), 2.01-2.94 (m, 2H);
LC-MS (ESI): m/z = 360.1 [M+H]⁺.

### Example 98

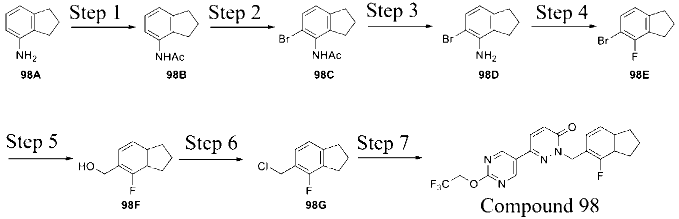

Step 1: Compound 98A (10.0 g, 75.08 mmol) was dissolved in dichloromethane (100 mL), and trifluoroacetic acid (7.3 mL, 97.60 mmol) was added at 0°C, and then acetic anhydride (7.8 mL, 82.60 mmol) was added dropwise, after the completion of dropwise addition, the obtained system was raised to room temperature and reacted for 3 h. Water (100 mL) was added for dilution, the organic phase was separated, and the aqueous phase was extracted once more with dichloromethane (100 mL), the organic phases was combined, dried over anhydrous sodium sulfate, and concentrated under reduced pressure, the resulting residue was purified by silica gel column chromatography (petroleum ether : ethyl acetate (v : v)=5 : 1) to obtain compound 98B (13.0 g, yield: 98.78%).
¹H NMR (400 MHz, DMSO-*d*₆) δ 9.26 (s, 1H), 7.40 (d, 1H), 7.08 - 7.04 (m, 1H), 6.99 - 6.97 (m, 1H), 2.88 - 2.84 (m, 2H), 2.81 - 2.77 (m, 2H), 2.04 (s, 3H), 2.00 - 1.95 (m, 2H);
LC-MS (ESI): m/z = 176.1[M+H]⁺.

Step 2: Compound 98B (13.0 g, 74.19 mmol), N-bromosuccinimide (14.5 g, 81.61 mmol), palladium acetate (1.6 g, 7.42 mmol) and p-toluenesulfonic acid (7.7 g, 44.51 mmol) were added to a reaction flask, the obtained system was mixed well with toluene (350 mL), and subjected to nitrogen replacement three times, then reacted at room temperature for 3 h. The reaction liquid was concentrated under reduced pressure, the resulting residue was purified by silica gel column chromatography (petroleum ether : ethyl acetate (v : v)=2 : 1) to obtain compound 98C (14.7 g, yield: 77.98%).
¹H NMR (400 MHz, DMSO-*d*₆) δ 9.54 (s, 1H), 7.38 (d, 1H), 7.06 (d, 1H), 2.88 - 2.84 (m, 2H), 2.74 - 2.70 (m, 2H), 2.03 (s, 3H), 2.00 - 1.94 (m, 2H);
LC-MS (ESI): m/z = 254.1 [M+H]⁺.

Step 3: Compound 98C (6.4 g, 25.28 mmol) was added to a reaction flask, dissolved in ethanol (120 mL), then concentrated hydrochloric acid (64 mL) was added and the obtained system was reacted at 80°C for 24 h. After cooling, the obtained system was concentrated under reduced pressure to give compound 98D (4.4 g of crude product), which was used directly for the next reaction.

LC-MS (ESI): m/z = 212.1 [M+H]⁺.

Step 4: Compound 98D (4.4 g, 17.70 mmol) was dissolved in pyridine hydrofluoride (40 mL), sodium nitrite (3.66 g, 53.10 mmol) was added at 0°C, and the obtained system was allowed to warm to room temperature and reacted for 1 h. The system was diluted by adding water (200 mL), and then extracted twice with ethyl acetate (150 mL×2), the organic phases were combined, dried, and concentrated, the resulting residue was purified by silica gel column chromatography (petroleum ether : ethyl acetate (v : v)=50 : 1) to obtain compound 98E (2.3 g, yield: 60.53%).

¹H NMR (400 MHz, CDCl₃) δ 7.30 - 7.25 (m, 1H), 6.87 (d, 1H), 2.98 - 2.95 (m, 2H), 2.91 - 2.88 (m, 2H), 2.16 - 2.09 (m, 2H);
Step 5: Compound 98E (2.2 g, 10.23 mmol), tri-n-butyltin methanol (3.63 g, 11.25 mmol) and chloro(2-dicyclohexylphosphino-2',4',6'-triisopropyl-1,1'-biphenyl)[2-(2'-amino-1,1'-biphenyl)]palladium(II)(Xphos Pd G2) (0.8 g, 1.02 mmol) were added to a reaction flask, and dissolved with 1,4-dioxane (100 mL) and the obtained system was heated to 90°C and reacted for 4 h. After cooling, the reaction liquid was concentrated under reduced pressure, and the resulting residue was purified by silica gel column chromatography (petroleum ether : ethyl acetate (v : v)=5 : 1) to obtain compound 98F (1.6 g, yield: 94.12%).

¹H NMR (400 MHz, DMSO-*d*₆) δ 7.22 - 7.18 (m, 1H), 7.02 (d, 1H), 5.11 - 5.0- (m, 1H), 4.49 (d, 2H), 2.89 - 2.84 (m, 4H), 2.10 - 2.01 (m, 2H);
Step 6: Compound 98F (0.5 g, 3.01 mmol) was added to a reaction flask, and dissolved with dichloromethane (10 mL), then thionyl chloride (0.5 mL) was added, and finally two drops of N,N-dimethylformamide was added, then the obtained system was reacted at room temperature for 1 h. The reaction liquid was concentrated under reduced pressure, the resulting residue was purified by silica gel column chromatography (petroleum ether : ethyl acetate (v : v)=50 : 1) to obtain compound 98G (0.37 g, yield: 66.67%).

¹H NMR (400 MHz, CDCl₃) δ 7.18 - 7.14 (m, 1H), 6.98 (d, 1H), 4.62 (s, 2H), 2.96 - 2.91 (m, 4H), 2.16 -2.08 (m, 2H).

Step 7: Compound 1D (0.2 g, 0.73 mmol), compound 98G (0.27 g, 1.46 mmol) and cesium carbonate (0.6 g, 1.82 mmol) were added to a reaction flask, and dissolved in acetonitrile (10 mL), and the obtained system was reacted at 80°C for 2 h. After cooling, the reaction liquid was concentrated under reduced pressure, and the resulting residue was purified by silica gel column chromatography (petroleum ether : ethyl acetate (v : v)=2 : 1) to obtain compound 98 (0.14 g, yield: 45.75%).
¹H NMR (400 MHz, DMSO-*d*₆) δ 9.09 (s, 2H), 8.12 - 8.10 (m, 1H), 7.15 (d, 1H), 7.13 (s, 1H), 7.02 (d, 1H), 5.35 (s, 2H), 5.14 - 5.07 (m, 2H), 2.90 - 2.85 (m, 4H), 2.09 - 2.02 (m, 2H);
LC-MS (ESI): m/z = 421.6 [M+H]⁺.

### Example 99

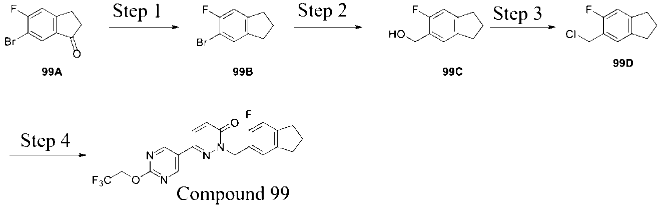

Step 1: Compound 99A (3.0 g, 13.10 mmol) was dissolved in dichloromethane (30 mL), trifluoromethanesulfonic acid (7.86 g, 52.40 mmol) and triethylsilane (6.0 g, 39.30 mmol) were added dropwise at 0°C in sequence, and after completion of the dropwise addition, the obtained system was allowed to warm to room temperature and reacted for 3 h. The reaction liquid was concentrated under reduced pressure, the resulting residue was purified by silica gel column chromatography (petroleum ether : ethyl acetate (v : v)=50 : 1) to obtain compound 99B (2.1 g, yield: 74.47%).

¹H NMR (400 MHz, CDCl₃) δ 7.26 (d, 1H), 6.87 (d, 1H), 2.79 - 2.75 (m, 4H), 2.09 - 1.98 (m, 2H).

Step 2: Compound 99B (2.1 g, 9.76 mmol), tri-n-butyltin methanol (3.45 g, 10.74 mmol) and chloro(2-dicyclohexylphosphino-2',4',6'-triisopropyl-1,1'-biphenyl)[2-(2'-amino-1,1'-biphenyl)]palladium(II)(Xphos Pd G2) (0.77 g, 0.98 mmol) were added to a reaction flask, and dissolved with 1,4-dioxane (100 mL) and then the obtained system was reacted at 90°C for 4 h. After cooling, the reaction liquid was concentrated under reduced pressure, and the resulting residue was purified by silica gel column chromatography (petroleum ether : ethyl acetate (v : v) = 5 : 1) to obtain compound 99C (1.2 g, yield: 74.07%).

¹H NMR (400 MHz, DMSO-*d*₆) δ 7.26 (d, 1H), 6.97 (d, 1H), 5.13 - 5.10 (m, 1H), 4.48 (d, 2H), 2.85 - 2.79 (m, 7.8 Hz, 4H), 2.06 - 1.98 (m, 2H).

Step 3: Compound 99C (0.5 g, 3.01 mmol) was added to a reaction flask, and dissolved with dichloromethane (10 mL), then thionyl chloride (0.5 mL) was added, and finally two drops of N,N-dimethylformamide was added, then the obtained system was reacted at room temperature for 1 h. The reaction liquid was concentrated under reduced pressure, the resulting residue was purified by silica gel column chromatography (petroleum ether : ethyl acetate (v : v)=50 : 1) to obtain compound 99D (0.4 g, yield: 71.94%).

¹H NMR (400 MHz, CDCl₃) δ 7.21 (d, 1H), 6.92 (d, 1H), 4.60 (s, 2H), 2.92 - 2.82 (m, 4H), 2.11 - 2.05 (m, 2H).

Step 4: Compound 1D (0.2 g, 0.73 mmol), compound 99D (0.27 g, 1.46 mmol) and cesium carbonate (0.6 g, 1.82 mmol) were added to a reaction flask, and dissolved in acetonitrile (10 mL), and the obtained system was reacted at 80°C for 2 h. After cooling, the reaction liquid was concentrated under reduced pressure, and the resulting residue was purified by silica gel column chromatography (petroleum ether : ethyl acetate (v : v)=2 : 1) to obtain compound 99 (0.21 g, yield: 68.40%).
¹H NMR (400 MHz, DMSO-*d*₆) δ 9.09 (s, 2H), 8.12 (d, 1H), 7.17 - 7.14 (m, 2H), 7.08 (d, 1H), 5.32 (s, 2H), 5.14 - 5.07 (m, 2H), 2.85 - 2.81 (m, 2H), 2.79 - 2.75 (m, 2H), 2.04 - 1.97 (m, 2H);
LC-MS (ESI): m/z = 421.1 [M+H]⁺.

### Example 100

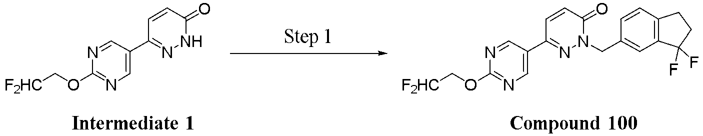

Step 1: Compound 93D (247 mg, 1 mmol) was dissolved in N,N-dimethylformamide (10 mL), intermediate 1 (254 mg, 1 mmol) and potassium carbonate (276 mg, 2 mmol) were added, and the obtained system was reacted at 70°C for 2 h. Water (50 mL) was added to the system, and ethyl acetate (50 mL×2) was used for extraction, the organic phase was collected, washed with saturated brine, and dried over anhydrous sodium sulfate, then filtered, the filtrate was collected, and concentrated, the resulting residue was purified by column chromatography (petroleum ether : ethyl acetate (v: v)=1 : 0-1 : 1) to obtain compound 100 (124 mg, yield: 29.5%).
¹H NMR (400 MHz, CDCl₃) δ 8.92 (s, 2H), 7.64 - 7.61 (m, 1H), 7.60 - 7.55 (m, 2H), 7.29 - 7.25 (m, 1H), 7.08 (d, 1H), 6.34 - 6.00 (m, 1H), 5.42 (s, 2H), 4.70 -4.60 (m, 2H), 3.05 - 2.97 (m, 2H), 2.64 - 2.52 (m, 2H);
LC-MS (ESI): m/z = 421.2 [M+H]⁺.

### Example 101

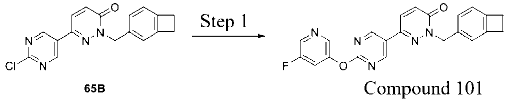

Step 1: Compound 65B (0.15 g, 0.46 mmol), 5-fluoropyridin-3-ol (0.062 g, 1.55 mmol) and potassium carbonate (0.16 g, 1.14 mmol) were dissolved in N,N-dimethylformamide (8 mL) and then the obtained system was stirred at 80°C for 2 h. After the reaction was completed, the reaction liquid was diluted with water, and extracted with ethyl acetate 3 times, the organic phases were combined and concentrated under reduced pressure, and the resulting residue was purified by preparative HPLC. Method: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.5% ammonium acetate); b. gradient elution, mobile phase A: 30%-80%; c. flow rate: 20 mL/min; d. elution time: 17 min. Compound 101 (80 mg, yield: 43%) was obtained after lyophilization.
¹H NMR (400 MHz, DMSO-*d*₆) δ 9.15 (s, 2H), 8.56 (d, 1H), 8.52-8.50 (m, 1H), 8.08 (d, 1H), 7.99-7.88 (m, 1H), 7.25 (d, 1H), 7.14 (d, 2H), 7.04 (d, 1H), 5.27 (s, 2H), 3.08 (s, 4H);
LC-MS (ESI): m/z = 402.30 [M+H]⁺.

### Example 102

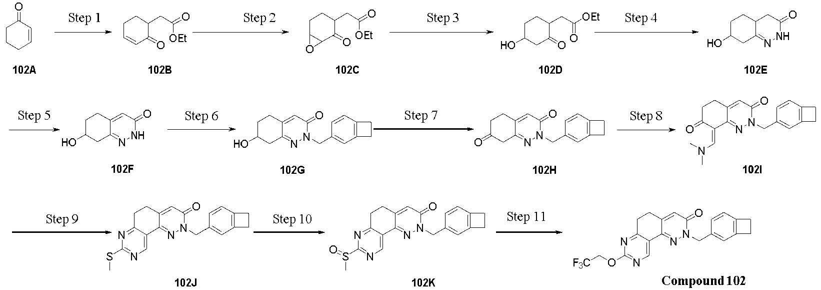

Step 1: Tetrahydrofuran (400 mL) was added to a flask, lithium diisopropylamide (312 mL, 624.21 mmol, 2M) was added at -78°C, and then a solution of compound 102A (50 g, 505.1 mmol) in tetrahydrofuran (100 mL) was added dropwise, after the completion of dropwise addition, the obtained system was continuously reacted for 1 h. Then, a solution of ethyl bromoacetate (104.3 g, 624.21 mmol) in tetrahydrofuran (100 mL) was further added, after the completion of dropwise addition, the obtained system was continuously reacted at -78°C for 1 h, then naturally returned to room temperature and reacted overnight. After the completion of reaction, saturated ammonium chloride solution (100 mL) was added dropwise at -78°C, water (500 mL) was added to the obtained system and then ethyl acetate (200 mL×3) was used for extraction, the organic phase was collected, washed, dried and concentrated, and the resulting residue was purified by column chromatography (petroleum ether : ethyl acetate (v : v)=5 : 1) to obtain compound 102B (23 g, yield: 24.26%).

LCMS (ESI): m/z = 183.2 [M+H]⁺.

Step 2: Compound 102B (17 g, 93.29 mmol) was added into a flask, followed by methanol (250 mL), then 30% hydrogen peroxide (64 mL, 626.64 mmol) was added in an ice bath, followed by sodium hydroxide solution (1.87 g, 46.65 mmol, dissolved in 13 mL H₂O), the obtained system was naturally warmed to room temperature and reacted for 16 h. Sodium thiosulfate solution was added in an ice bath, the obtained system was then extracted with ethyl acetate, the organic phase was collected, washed and concentrated, and the resulting residue was purified by silica gel column chromatography (petroleum ether : ethyl acetate (v : v)=10 : 1) to obtain compound 102C (8.2 g, yield: 44.34%).

LCMS (ESI): m/z = 199.1 [M+H]⁺.

Step 3: Diphenyl diselenide (23.63 g, 75.75 mmol) was added into a flask, then ethanol (290 mL) was added, sodium borohydride (5.76 g, 151.5 mmol) was added in portions at room temperature, the obtained system was reacted for 10 min until the yellow color disappeared, acetic acid (0.45 mL) was added, followed by a solution of compound 102C (10 g, 50.50 mmol) in ethanol (20 mL), and then the obtained system was reacted at room temperature for 3 h. Water was added to the system, which was extracted with dichloromethane, the organic phase was collected, washed, dried and concentrated, the resulting residue was purified by column chromatography (petroleum ether : ethyl acetate (v : v)=1 : 1) to obtain compound 102D (8.7 g, yield: 86.04%).

LCMS (ESI): m/z = 201.2 [M+H]⁺.

Step 4: Compound 102D (8.7 g, 43.45 mmol) was added to a flask, followed by ethanol (160 mL) and hydrazine hydrate (17 mL, 349.78 mmol), then the obtained system was reacted at 85°C for 1 h. After cooling, the reaction liquid was concentrated directly, and the resulting residue was purified by column chromatography (dichloromethane : methanol (v : v) = 1 : 10) to obtain compound 102E (6.4 g, yield: 87.58%).

LCMS (ESI): m/z = 169.1 [M+H]⁺.

Step 5: Compound 102E (6.4 g, 38.05 mmol) was added to a flask, acetonitrile (100 mL) and copper dichloride (10.23 g, 76.1 mmol) were added. Then the obtained system was reacted at 85°C for 1 h. After cooling, the reaction liquid was concentrated directly, and the resulting residue was purified by column chromatography (dichloromethane : methanol (v : v)=5 : 1) to obtain compound 102F (4.0 g, yield: 63.26%).

LCMS (ESI): m/z = 167.1 [M+H]⁺.

Step 6: Compound 102F (1.1 g, 6.62 mmol) was added to a flask, followed by acetonitrile (15 mL), cesium carbonate (6.47 g, 19.86 mmol) and compound 56B (1.52 g, 9.93 mmol), and then the obtained system was reacted at 85°C for 2 h. After cooling, the reaction liquid was directly filtered, and the filtrate was collected and concentrated, the resulting residue was purified by column chromatography (dichloromethane : methanol (v: v)=10 : 1) to obtain compound 102G (1.3 g, yield: 69.55%).

LCMS (ESI): m/z = 283.2 [M+H]⁺.

Step 7: Anhydrous dichloromethane (20 mL) was added to a flask and oxalyl chloride (0.43 mL, 5.1 mmol) was added. Anhydrous dimethyl sulfoxide (0.66 mL, 9.35 mmol) was added dropwise under nitrogen protection at -78°C. After the completion of dropwise addition, the reaction was continued for 30 min, and then a solution of compound 102G (1.2 g, 4.25 mmol) in dichloromethane (20 mL) was added dropwise. After the completion of dropwise addition, the obtained system was reacted at -78°C for 1 h, then triethylamine (4.71 mL, 34 mmol) was further added dropwise, and then reacted for another 1 h. Water was added to the system and dichloromethane was used for extraction, the organic phase was collected and washed to obtain a solution of compound 102H in dichloromethane, which solution was used directly for the next reaction.

LCMS (ESI): m/z = 281.1 [M+H]⁺.

Step 8: The solution of compound 102H in dichloromethane from the previous step was added into a flask, then N,N-dimethylformamide dimethylacetal (10 mL) was added. Then, dichloromethane was removed by concentration, and then the obtained system was reacted at 100°C for 1 h. After cooling, the obtained system was concentrated directly to give compound 1021 (2.1 g of crude product).

LCMS (ESI): m/z = 336.1 [M+H]⁺.

Step 9: Compound 102I (2.1 g, 6.26 mmol) was added into a flask, then dissolved by adding dioxane (60 mL), S-methylisothiourea sulfate (10.46 g, 37.56 mmol) and potassium carbonate (10.38 g, 75.10 mmol) were added in sequence, and then the obtained system was reacted at 110°C for 5 h. After cooling, the reaction liquid was filtered, and the filtrate was collected and concentrated, the resulting residue was purified by column chromatography (petroleum ether : ethyl acetate (v : v)=1 : 5) to obtain compound 102J (450 mg, yield for three steps: 29.25%).

LCMS (ESI): m/z = 363.1 [M+H]⁺.

Step 10: Compound 102J (150 mg, 0.41 mmol) was added to a flask, dichloromethane (10 mL) and m-chloroperoxybenzoic acid (100 mg, 0.49 mmol) were added, and then the obtained system was reacted at room temperature for 16 h. Sodium thiosulfate solution was added to the reaction liquid, which was extracted with dichloromethane, the organic phase was collected, washed and concentrated, and the resulting residue was purified by column chromatography (ethyl acetate : acetonitrile (v : v)=1 : 1) to obtain the compound 102K (70 mg, yield: 43.28%).

LCMS (ESI): m/z = 379.1 [M+H]⁺.

Step 11: Compound 102K (70 mg, 0.18 mmol) was added into a flask, and dissolved by adding acetonitrile (10 mL), then 2,2,2-trifluoroethanol (54 mg, 0.54 mmol) and potassium carbonate (75 mg, 0.54 mmol) were added in sequence, and the obtained system was reacted at 50°C for 1 h. After cooling, the reaction liquid was filtered directly, and the filtrate was concentrated and the residue was purified by preparative HPLC. Purification methods: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.05% aqueous ammonia); b. gradient elution, mobile phase A: 25%-75%; c. flow rate: 15 mL/min; d. elution time: 10 min. Compound 102 (33 mg, yield: 44.28%) was obtained.
¹H NMR (400 MHz, CDCl₃) δ 9.10 (s, 1H), 7.33 (d, 1H), 7.20 (s, 1H), 7.01 (d, 1H), 6.78 (s, 1H), 5.33 (s, 2H), 4.85 (q, 2H), 3.14 (s, 4H), 3.01 (t, 2H), 2.93 (t, 2H);
LCMS (ESI): m/z = 415.2 [M+H]⁺.

### Example 103

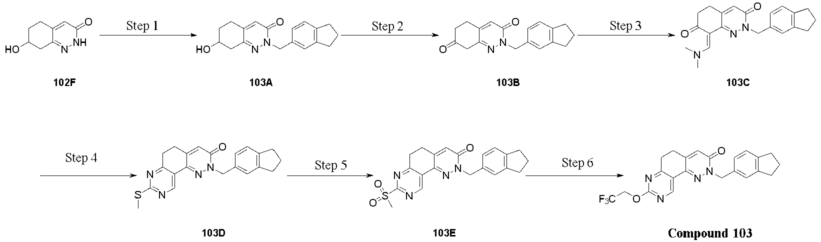

Step 1: Compound 102F (600 mg, 3.61 mmol) was added to a flask, followed by acetonitrile (10 mL), cesium carbonate (3.53 g, 10.83 mmol) and compound 57B (902 mg, 5.42 mmol), and then the obtained system was reacted at 85°C for 2 h. After cooling, the reaction liquid was filtered, and the filtrate was concentrated, the resulting residue was purified by column chromatography (dichloromethane : methanol (v : v)=10 : 1) to obtain compound 103A (940 mg, yield: 87.86%).

LCMS (ESI): m/z = 297.1 [M+H]⁺.

Step 2: Anhydrous dichloromethane (10 mL) was added to a flask and oxalyl chloride (0.32 mL, 3.80 mmol) was added. Anhydrous dimethyl sulfoxide (0.50 mL, 6.97 mmol) was added dropwise under nitrogen protection at -78°C. After the completion of dropwise addition, the reaction was continued for 30 min, and then a solution of compound 103A (940 mg, 3.17 mmol) in dichloromethane (20 mL) was added dropwise. After the completion of dropwise addition, the obtained system was reacted at -78°C for 1 h, then triethylamine (3.52 mL, 25.39 mmol) was further added dropwise, and then reacted for another 1 h. Water was added to the system and dichloromethane was used for extraction, the organic phase was collected and washed to obtain a solution of compound 103B in dichloromethane, which solution was used directly for the next reaction.

LCMS (ESI): m/z = 295.1 [M+H]⁺.

Step 3: The solution of compound 103B in dichloromethane from the previous step was added into a flask, then N,N-dimethylformamide dimethylacetal (10 mL) was added. Then, the dichloromethane was removed by concentration, and the remaining solution in the flask was continuously reacted at 100°C for 1 h. After cooling, the obtained system was concentrated to give compound 103C (1.5 g of crude product).

LCMS (ESI): m/z = 350.2 [M+H]⁺.

Step 4: Compound 103C (1.5 g, 4.29 mmol) was added into a flask, then dissolved by adding dioxane (45 mL), S-methylisothiourea sulfate (7.17 g, 25.74 mmol) and potassium carbonate (7.12 g, 51.52 mmol) were added in sequence, and then the obtained system was reacted at 110°C for 5 h. After cooling, the reaction liquid was filtered, and the filtrate was concentrated, the resulting residue was purified by column chromatography (petroleum ether : ethyl acetate (v : v)=1 : 5) to obtain compound 103D (700 mg, yield for three steps: 58.65%).

LCMS (ESI): m/z = 377.1 [M+H]⁺;

Step 5: Compound 103D (150 mg, 0.40 mmol) was added to a flask, dichloromethane (8 mL) and m-chloroperoxybenzoic acid (179 mg, 0.88 mmol) were added, and then the obtained system was reacted at room temperature for 16 h. Sodium thiosulfate solution was added to the reaction liquid, which was extracted with dichloromethane, the organic phase was collected, washed and concentrated, and the resulting residue was purified by column chromatography (ethyl acetate) to obtain compound 103E (60 mg, yield: 36.72%).

LCMS (ESI): m/z = 409.1 [M+H]⁺.

Step 6: Compound 103E (60 mg, 0.15 mmol) was added into a flask, and dissolved by adding acetonitrile (10 mL), then 2,2,2-trifluoroethanol (45 mg, 0.45 mmol) and potassium carbonate (62 mg, 0.45 mmol) were added in sequence, and the obtained system was reacted at 50°C for 1 h. After cooling, the reaction liquid was filtered, and the filtrate was concentrated and the residue was purified by preparative HPLC. Purification methods: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.05% aqueous ammonia); b. gradient elution, mobile phase A: 25%-75%; c. flow rate: 15 mL/min; d. elution time: 10 min. Compound 103 (32 mg, yield: 49.84%) was obtained.
¹H NMR (400 MHz, CDCl₃) δ 9.10 (s, 1H), 7.34 (s, 1H), 7.27 (s, 1H), 7.18 (d, 1H), 6.29 (s, 1H), 5.34 (s, 2H), 4.85 (q, 2H), 3.02-3.00 (m, 2H), 2.95-2.85 (m, 6H), 2.08-2.01 (m, 2H);
LCMS (ESI): m/z = 429.2 [M+H]⁺.

### Example 104

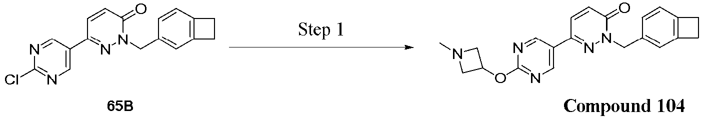

Step 1: 1-methylazetidin-3-ol (174 mg, 2 mmol) was dissolved in tetrahydrofuran (20 mL), sodium hydride (160 mg, 4 mmol) was added in an ice bath, stirring was continued for 30 min and then compound 65B (324 mg, 1 mmol) was added. After 10 min, the reaction was quenched with saturated ammonium chloride solution, extracted with ethyl acetate (20 mL×2), the organic phases were combined, washed with saturated brine, dried over anhydrous sodium sulfate, and filtered, the filtrate was collected, and concentrated, the resulting residue was purified by column chromatography (dichloromethane : ethyl acetate (v : v)=1 : 1) to obtain compound 104 (44 mg, yield: 11.7%).
¹H NMR (400 MHz, CDCl₃) δ 8.88 (s, 2H), 7.54 (d, 1H), 7.34 - 7.31 (m, 1H), 7.19 (s, 1H), 7.06 -7.03 (m, 1H), 7.03 -7.00 (m, 1H), 5.40 - 5.28 (m, 3H), 4.19 - 4.09 (m, 2H), 3.37 - 3.30 (m, 2H), 3.16 - 3.12 (m, 4H), 2.53 (s, 3H);
LC-MS (ESI): m/z = 376.2 [M+H]⁺.

### Example 105

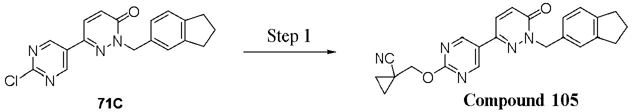

Step 1: Compound 1-(hydroxymethyl)cyclopropanecarbonitrile (58 mg, 0.60 mmol) was dissolved in tetrahydrofuran (3 mL), sodium hydride (30 mg, 0.75 mmol) was added at 0°C, the obtained system was stirred for 20 min, and then compound 71C (100 mg, 0.30 mmol) was added, and the obtained system was continuously reacted at 0°C for 1 h. 0.1 mL of saturated ammonium chloride solution was added to quench the reaction, and the resulting product was purified by column chromatography (dichloromethane : ethyl acetate (v : v)=1 : 1) to obtain compound 105 (46 mg, yield: 38.39%).
¹H NMR (400 MHz, DMSO-*d*₆) δ 9.09 (s, 2H), 8.09 (d, 1H), 7.24 (s, 1H), 7.18 - 7.14 (m, 2H), 7.12 (d, 1H), 5.27 (s, 2H), 4.42 (s, 2H), 2.84 - 2.79 (m, 4H), 2.02 - 1.94 (m, 2H), 1.41 - 1.38 (m, 2H), 1.27 - 1.24 (m, 2H);
LC-MS (ESI): m/z = 400.2 [M+H]⁺.

### Example 106

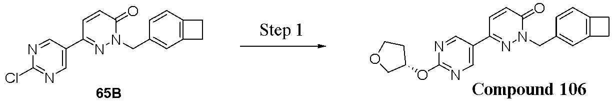

Step 1: Compound (S)-(+)-3-hydroxytetrahydrofuran (81 mg, 0.92 mmol) was dissolved in tetrahydrofuran (3 mL), sodium hydride (46 mg, 1.15 mmol) was added at 0°C, the obtained system was stirred for 20 min, and then compound 65B (150 mg, 0.46 mmol) was added, and the obtained system was continuously reacted at 0°C for 1 h. 0.1 mL of saturated ammonium chloride solution was added to quench the reaction, and the resulting product was purified via column chromatography (dichloromethane : ethyl acetate (v : v)=1 : 1) to obtain compound 106 (70 mg, yield: 40.43%).
¹H NMR (400 MHz, DMSO-*d*₆) δ 9.07 (s, 2H), 8.07 (d, 1H), 7.24 (d, 1H), 7.13 -7.09 (m, 2H), 7.04 (d, 1H), 5.56 - 5.53 (m, 1H), 5.26 (s, 2H), 3.97 - 3.78 (m, 4H), 3.09 (s, 4H), 2.33 - 2.02 (m, 2H);
LC-MS (ESI): m/z = 377.1 [M+H]⁺.

### Example 107

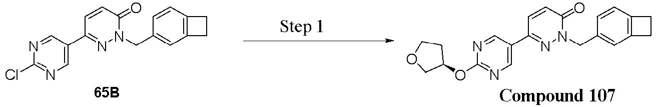

Step 1: Compound (R)-(+)-3-hydroxytetrahydrofuran (81 mg, 0.92 mmol) was dissolved in tetrahydrofuran (3 mL), sodium hydride (46 mg, 1.15 mmol) was added at 0°C, the obtained system was stirred for 20 min, and then compound 65B (150 mg, 0.46 mmol) was added, and the obtained system was continuously reacted at 0°C for 1 h. 0.1 mL of saturated ammonium chloride solution was added to quench the reaction, and the resulting product was purified via column chromatography (dichloromethane : ethyl acetate (v : v)=1 : 1) to obtain compound 107 (35 mg, yield: 20.23%).
¹H NMR (400 MHz, DMSO-*d*₆) δ 9.07 (s, 2H), 8.08 - 8.05 (m, 1H), 7.25 - 7.23 (m, 1H), 7.13 - 7.10 (m, 2H), 7.05 - 7.03 (m, 1H), 5.56 - 5.53 (m, 1H), 5.26 (s, 2H), 3.97 - 3.78 (m, 4H), 3.09 (s, 4H), 2.32 - 2.22 (m, 1H), 2.10 - 2.04 (m, 1H);
LC-MS (ESI): m/z = 377.2 [M+H]⁺.

### Example 108

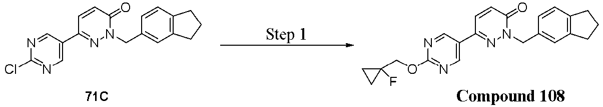

Step 1: Compound (1-fluorocyclopropyl)methanol (54 mg, 0.60 mmol) was dissolved in tetrahydrofuran (3 mL), sodium hydride (30 mg, 0.75 mmol) was added at 0°C, the obtained system was stirred for 20 min, and then compound 71C (100 mg, 0.30 mmol) was added, and the obtained system was continuously reacted at 0°C for 1 h. 0.1 mL of saturated ammonium chloride solution was added to quench the reaction, and the resulting product was purified via column chromatography (dichloromethane : ethyl acetate (v : v)=1 : 1) to obtain compound 108 (20 mg, yield: 17.0%).
¹H NMR (400 MHz, DMSO-*d*₆) δ 9.08 (s, 2H), 8.10 - 8.07 (m, 1H), 7.27 - 7.11 (m, 4H), 5.27 (s, 2H), 4.72 - 4.66 (m, 2H), 2.84 - 2.79 (m, 4H), 2.01 - 1.96 (m, 2H), 1.17 - 1.12 (m, 2H), 0.93 -0.92 (m, 2H);
LC-MS (ESI): m/z = 393.2 [M+H]⁺.

### Example 109

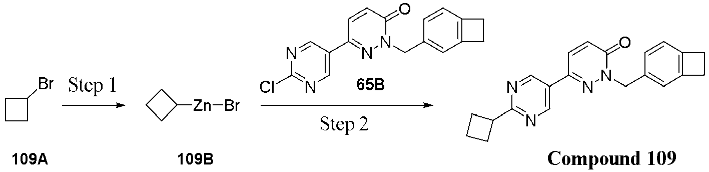

Step 1: Zinc powder (2.38 g, 36.35 mmol) was added to anhydrous N,N-dimethylacetamide (16 mL), the obtained system was subjected to nitrogen protection, and raised to 70°C, then 1,2-dibromoethane (0.84 g, 4.47 mmol) was added, the obtained system was stirred for 5 min, then cooled to room temperature. After trimethylchlorosilane (0.70 g, 6.43 mmol) was added, a solution of compound 109A (3.78 g, 28 mmol) in anhydrous N,N-dimethylacetamide (10 mL) was slowly added. After the addition was completed, the obtained system was raised to 70°C and stirred for 1 h. The reaction liquid was cooled to room temperature and stood still to obtain the supernatant of compound 109B, which supernatant was used directly for the next reaction.

Step 2: Compound 65B (0.32 g, 1 mmol) and RuPhos Pd G2 (0.078 g, 0.1 mmol) were dissolved in anhydrous N,N-dimethylacetamide (1 mL), the obtained system was subjected to nitrogen protection, the above solution of compound 109B (3 mL) was added, the obtained system was heated to 100°C and stirred for 3 h. After the reaction was completed, it was filtered, the filtrate was concentrated, and the resulting residue was purified by preparative HPLC. Purification methods: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.05% aqueous ammonia); b. gradient elution, mobile phase A: 40%-100%; c. flow rate: 15 mL/min; d. elution time: 25 min. Compound 109 (40 mg, yield: 11.6%) was obtained.
¹H NMR (400 MHz, DMSO-*d*₆) δ 9.19 (s, 2H), 8.11 (d, 1H), 7.28 - 7.21 (m, 1H), 7.13 (d, 2H), 7.04 (d, 1H), 5.28 (s, 2H), 3.88 - 3.74 (m, 1H), 3.09 (s, 4H), 2.45 -2.27 (m, 4H), 2.13 - 1.96 (m, 1H), 1.96 - 1.82 (m, 1H);
LC-MS (ESI): m/z = 345.7 [M+H]⁺.

### Example 110

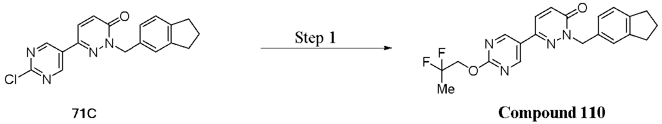

Step 1: Compound 2,2-difluoropropanol (96 mg, 1.0 mmol) was dissolved in tetrahydrofuran (10 mL), sodium hydride (60 mg, 1.5 mmol) was added at 0°C, the obtained system was stirred for 20 min, and then compound 71C (169 mg, 0.50 mmol) was added, and the obtained system was continuously reacted at 0°C for 1 h. 0.1 mL of saturated ammonium chloride solution was added to quench the reaction, and the resulting product was purified via column chromatography (dichloromethane : ethyl acetate (v : v)=1 : 1) to obtain compound 110 (107 mg, yield: 53.77%).
¹H NMR (400 MHz, DMSO-*d*₆) δ 9.11 (s, 2H), 8.11 - 8.08 (m, 1H), 7.24 - 7.12 (m, 4H), 5.27 (s, 2H), 4.72 - 4.67 (m, 2H), 2.84 - 2.79 (m, 4H), 2.01 - 1.94 (m, 2H), 1.81 - 1.71 (m, 3H);
LC-MS (ESI): m/z = 399.2 [M+H]⁺.

### Example 111

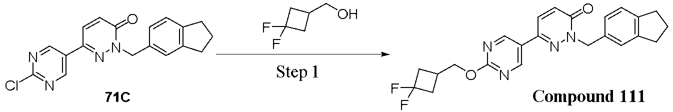

Step 1: (3,3-difluorocyclobutyl)methanol (122 mg, 1 mmol) was dissolved in tetrahydrofuran (10 mL), the obtained system was cooled in an ice-water bath, then sodium hydride (80 mg, 2 mmol) was added, the obtained system was continuously stirred for 30 min, and then compound 71C (339 mg, 1 mmol) was added. After 10 min, the reaction was quenched with saturated ammonium chloride solution, and extracted with ethyl acetate (20 mL×2), the organic phases were collected and combined, washed with saturated brine, dried over anhydrous sodium sulfate, and filtered, the filtrate was collected and concentrated, the resulting residue was purified by column chromatography (petroleum ether : ethyl acetate (v : v) = 1 : 1) to obtain compound 111 (124 mg, yield: 29.2%).
¹H NMR (400 MHz, CDCl₃) δ 8.90 (s, 2H), 7.55 (d, 1H), 7.34 (s, 1H), 7.29 - 7.23 (m, 1H), 7.20 - 7.16 (m, 1H), 7.05 (d, 1H), 5.35 (s, 2H), 4.50 - 4.45 (m, 2H), 2.91 - 2.84 (m, 4H), 2.82 - 2.65 (m, 3H), 2.56 - 2.42 (m, 2H), 2.09 - 2.00 (m, 2H);
LC-MS (ESI): m/z = 425.2 [M+H]⁺.

### Example 112

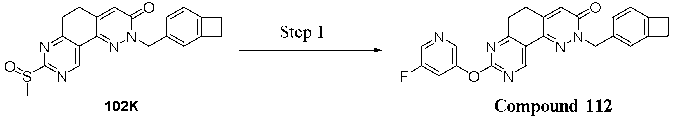

Step 1: Compound 102K (30 mg, 0.08 mmol) was added to a flask, acetonitrile (10 mL), 3-fluoro-5-hydroxypyridine (26 mg, 0.23 mmol) and potassium carbonate (32 mg, 0.23 mmol) were added, and then the obtained system was reacted at 50°C for 1 h. After cooling, the reaction liquid was filtered, and the filtrate was concentrated and the residue was purified by preparative HPLC. Purification method: 1. Instrument: waters 2767 (preparative liquid phase chromatographic instrument); chromatographic column: SunFire @ PrepC18 (19 mm x 250 mm). 2. The sample was filtered with a 0.45 um filter to prepare a sample liquid. 3. Preparative chromatographic conditions: a. composition of mobile phases A and B: mobile phase A: acetonitrile; mobile phase B: water (containing 0.05% aqueous ammonia); b. gradient elution, mobile phase A: 25%-75%; c. flow rate: 15 mL/min; d. elution time: 20 min. Compound 112 (8 mg, yield: 25%) was obtained.
¹H NMR (400 MHz, DMSO-*d*₆) δ 9.06 (s, 1H), 8.55 (d, 1H), 8.49 (s, 1H), 7.93-7.89 (m, 1H), 7.24 (d, 1H), 7.12 (s, 1H), 7.03 (d, 1H), 6.93 (s, 1H), 5.23 (s, 2H), 3.08 (s, 4H), 2.97 (s, 4H);
LCMS (ESI): m/z = 428.1 [M+H]⁺.

### Example 113

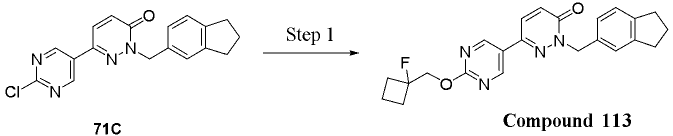

Step 1: 1-fluorocyclobutyl-1-methanol (0.28 g, 2.69 mmol) was dissolved in tetrahydrofuran (20 mL), sodium hydride (53 mg, 1.33 mmol) was added in an ice bath, and the obtained system was reacted under stirring for 15 min. Compound 71C (300 mg, 0.89 mmol) was further added to the reaction liquid, and the obtained system was stirred at room temperature for 2 h. After the reaction was completed, water and ethyl acetate were added for liquid separation, the organic phase was collected and washed with saturated brine 3 times, dried over anhydrous sodium sulfate and concentrated under reduced pressure, the residue was purified by reverse phase column chromatography (acetonitrile : water ( v : v) = 10%-80%) to obtain compound 113 (109 mg, yield: 30.13%).
¹H NMR (400 MHz, CDCl₃) δ 8.91 (s, 2H), 7.55 (d, 1H), 7.34 (s, 1H), 7.27-7.25 (m, 1H), 7.18 (d, 1H), 7.04 (d, 1H), 5.35 (s, 2H), 4.61 (d, 2H), 2.90-2.85 (m, 4H), 2.59 - 2.22 (m, 4H), 2.08-2.00 (m, 2H), 1.98 - 1.85 (m, 1H), 1.66 - 1.55 (m, 1H);
LC-MS (ESI): m/z = 407.1 [M+H]⁺.

### Example 114

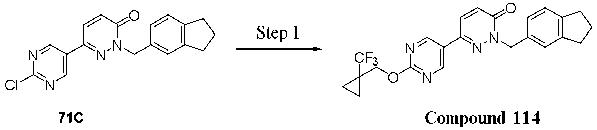

Step 1: (1-(trifluoromethyl)cyclopropyl)methanol (0.12 g, 0.88 mmol) was dissolved in tetrahydrofuran (8 mL), the obtained system was then cooled to 0°C, and sodium hydride (0.032 g, 1.32 mmol) was slowly added, and then the obtained system was stirred for 0.5 h, then compound 71C (0.15 g, 0.44 mmol) was added slowly and the obtained system was stirred for another 3 h. The system was diluted with water, and extracted with ethyl acetate 3 times, the organic phases were combined and concentrated, and the resulting residue was purified by preparative HPLC. Purification methods: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.5% ammonium acetate); b. gradient elution, mobile phase A: 30%-70%; c. flow rate: 15 mL/min; d. elution time: 20 min. Compound 114 (100 mg, yield: 51%) was obtained.
¹H NMR (400 MHz, DMSO-*d*₆) δ 9.07 (s, 2H), 8.08 (d, 1H), 7.24 (s, 1H), 7.20 - 7.09 (m, 3H), 5.27 (s, 2H), 4.55 (s, 2H), 2.88-2.76 (m, 4H), 2.03-1.91 (m, 2H), 1.15 - 1.07 (m, 4H);
LC-MS (ESI): m/z = 443.2 [M+H]⁺.

### Example 115

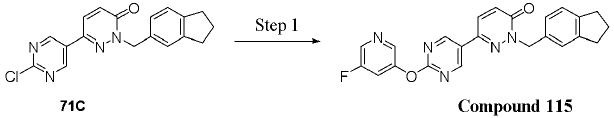

Step 1: Compound 71C (0.15 g, 0.44 mmol), 5-fluoropyridin-3-ol (0.065 g, 0.58 mmol) and potassium carbonate (0.15 g, 1.11 mmol) were dissolved in N,N-dimethylformamide (6 mL) and then the obtained system was stirred at 80°C for 6 h. After the reaction was completed, the reaction liquid was diluted with water, and extracted with ethyl acetate 3 times, the organic phases were combined and concentrated, and the resulting residue was purified by preparative HPLC. Purification methods: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.5% ammonium acetate); b. gradient elution, mobile phase A: 30%-70%; c. flow rate: 15 mL/min; d. elution time: 20 min. Compound 115 (80 mg, yield: 43%) was obtained.
¹H NMR (400 MHz, DMSO-*d*₆) δ 9.15 (s, 2H), 8.56 (d, 1H), 8.52-8.50 (m, 1H), 8.08 (d, 1H), 7.98-7.89 (m, 1H), 7.25 (s, 1H), 7.20 - 7.11 (m, 3H), 5.27 (s, 2H), 2.86-2.72 (m, 4H), 2.04-1.91 (m, 2H);
LC-MS (ESI): m/z = 416.2 [M+H]⁺.

### Example 116

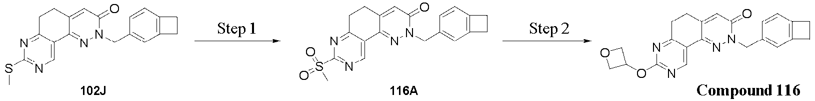

Step 1: Compound 102J (300.0 mg, 0.83 mmol) was added to a flask, dichloromethane (10 mL) and m-chloroperoxybenzoic acid (315.0 mg, 1.83 mmol) were added, and then the obtained system was reacted at room temperature for 16 h. The reaction liquid was quenched by adding sodium thiosulfate solution, then extracted with dichloromethane, the organic phase was collected, washed and concentrated, and the resulting residue was purified by column chromatography (petroleum ether : ethyl acetate (v : v)=1 : 50) to obtain compound 116A (200 mg, yield: 61.35%).

LC-MS (ESI): m/z = 395.1 [M+H]⁺.

Step 2: Compound 116A (30 mg, 0.076 mmol) was added to a flask, acetonitrile (6 mL), oxetan-3-ol (17.0 mg, 0.228 mmol), and potassium carbonate (31.5 mg, 0.228 mmol) were added, and then the obtained system was reacted at 80°C overnight. After cooling, the reaction liquid was concentrated directly, the resulting residue was purified by silica gel column chromatography (petroleum ether : ethyl acetate (v : v)=1 : 2) to obtain compound 116 (7 mg, yield: 23.33%).
¹H NMR ((400 MHz, CDCl₃) δ 9.06 (s, 1H), 7.33 (d, 1H), 7.19 (s, 1H), 7.01 (d, 1H), 6.77 (s, 1H), 5.70-5.64 (m, 1H), 5.32 (s, 2H), 4.99 (t, 2H), 4.81 (t, 2H), 3.13 (s, 4H), 3.00-2.90 (m, 4H);
LC-MS (ESI): m/z = 389.1 [M+H]⁺.

### Example 117

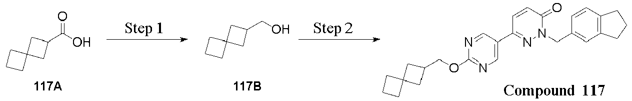

Step 1: Compound 117A (420 mg, 3 mmol) was dissolved in tetrahydrofuran (30 mL), and cooled to 0°C, and lithium aluminum tetrahydride (420 mg, 11 mmol) was added, and the obtained system was reacted at room temperature for 3 h. Water (0.42 mL), 10% aqueous sodium hydroxide solution (0.84 mL) and water (1.26 mL) were added in sequence to quench the reaction, the solid was removed by suction filtration, and the collected filtrate was concentrated to obtain compound 117B (320 mg of crude product), which was used directly for the next reaction.

Step 2: Compound 117B (320 mg of crude product) was dissolved in tetrahydrofuran (10 mL), sodium hydride (120 mg, 3 mmol) was added, and the obtained system was reacted at room temperature for 30 min. Compound 71C (200 mg, 0.59 mmol) was added and the obtained system was reacted under stirring for 1 h. Water and ethyl acetate were added for extraction and liquid separation, the organic phase was further washed with saturated brine, the organic phase was collected and concentrated under reduced pressure, and the resulting residue was purified by column chromatography (petroleum ether : ethyl acetate (v : v)=2 : 1) to obtain compound 117 (25 mg, yield: 9.9%).
¹H NMR (400 MHz, DMSO-*d*₆) δ 9.04 (s, 2H), 8.09 - 8.03 (m, 1H), 7.27 - 7.07 (m, 4H), 5.26 (s, 2H), 4.35 - 4.26 (m, 2H), 2.88 - 2.74 (m, 4H), 2.17 - 1.71 (m, 13H);
LCMS (ESI): m/z = 429.1 [M+H]⁺.

### Example 118

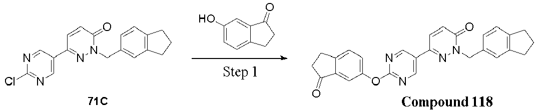

Step 1: 6-hydroxy-1-indenone (148 mg, 1 mmol) was dissolved in tetrahydrofuran (10 mL), the obtained system was cooled in an ice-water bath, sodium hydride (80 mg, 2 mmol) was added, the obtained system was continuously stirred for 30 min, and compound 71C (339 mg, 1 mmol) was added. After 10 min, the reaction was quenched with saturated ammonium chloride solution, and extracted with ethyl acetate (20 mL×2), the organic phases were combined, washed with saturated brine, dried over anhydrous sodium sulfate, and filtered, the filtrate was concentrated, and the resulting residue was purified by column chromatography (petroleum ether : ethyl acetate (v : v)=1 : 3) to obtain compound 118 (146 mg, yield: 32.4%).
¹H NMR (400 MHz, CDCl₃) δ 8.93 (s, 2H), 7.61 - 7.58 (m, 1H), 7.58 - 7.52 (m, 2H), 7.47 - 7.43 (m, 1H), 7.33 (s, 1H), 7.26 - 7.23 (m, 1H), 7.19 - 7.16 (m, 1H), 7.07 - 7.03 (m, 1H), 5.35 (s, 2H), 3.21 - 3.15 (m, 2H), 2.91 - 2.84 (m, 4H), 2.80 - 2.73 (m, 2H), 2.09 - 1.99 (m, 2H);
LC-MS (ESI): m/z = 451.6 [M+H]⁺.

### Example 119

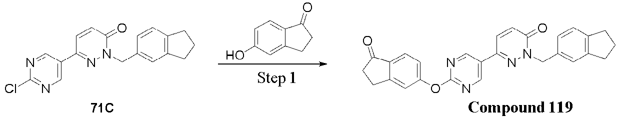

Step 1: 5-hydroxy-1-indenone (148 mg, 1 mmol) was dissolved in tetrahydrofuran (10 mL), the obtained system was cooled in an ice-water bath, sodium hydride (80 mg, 2 mmol) was added, the obtained system was continuously stirred for 30 min, and compound 71C (339 mg, 1 mmol) was added. After 10 min, the reaction was quenched with saturated ammonium chloride solution, and extracted with ethyl acetate (20 mL×2), the organic phases were combined, washed with saturated brine, dried over anhydrous sodium sulfate, and filtered, the filtrate was concentrated, and the resulting residue was purified by column chromatography (petroleum ether : ethyl acetate (v : v)=1 : 3) to obtain compound 119 (189 mg, yield: 42.0%).
¹H NMR (400 MHz, CDCl₃) δ 8.96 (s, 2H), 7.87 - 7.83 (m, 1H), 7.57 - 7.53 (m, 1H), 7.34 - 7.31 (m, 2H), 7.26 - 7.23 (m, 1H), 7.23 - 7.15 (m, 2H), 7.08 - 7.04 (m, 1H), 5.35 (s, 2H), 3.21 - 3.15 (m, 2H), 2.91 - 2.83 (m, 4H), 2.78 - 2.71 (m, 2H), 2.10 - 2.00 (m, 2H);
LC-MS (ESI): m/z = 451.6 [M+H]⁺.

### Example 120

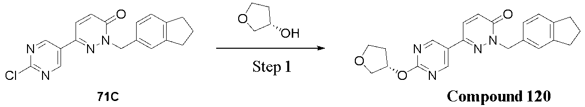

Step 1: (S)-tetrahydrofuran-3-ol (88 mg, 1 mmol) was dissolved in tetrahydrofuran (10 mL), the obtained system was cooled in an ice-water bath, sodium hydride (80 mg, 2 mmol) was added, the obtained system was continuously stirred for 30 min, and compound 71C (339 mg, 1 mmol) was added. After 10 min, the reaction was quenched with saturated ammonium chloride solution, and extracted with ethyl acetate (20 mL×2), the organic phases were combined, washed with saturated brine, dried over anhydrous sodium sulfate, and filtered, the filtrate was concentrated, and the resulting residue was purified by column chromatography (petroleum ether : ethyl acetate (v : v)=1 : 3) to obtain compound 120 (77 mg, yield: 19.7%).
¹H NMR (400 MHz, CDCl₃) δ 8.89 (s, 2H), 7.55 (d, 1H), 7.34 (s, 1H), 7.27 - 7.24 (m, 1H), 7.20 - 7.16 (m, 1H), 7.05 (d, 1H), 5.61 - 5.56 (m, 1H), 5.35 (s, 2H), 4.17 - 4.10 (m, 1H), 4.07 - 3.90 (m, 3H), 2.91 - 2.84 (m, 4H), 2.34 - 2.20 (m, 2H), 2.09 - 2.00 (m, 2H);
LC-MS (ESI): m/z =391.6 [M+H]⁺.

### Example 121

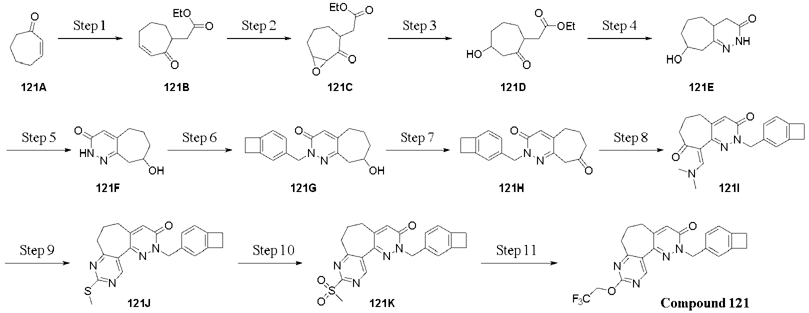

Step 1: Tetrahydrofuran (20 mL) was added to a flask, lithium diisopropylamide (10.9 mL, 21.8 mmol, 2M) was added at -78°C, and then compound 121A (2 g, 18.16 mmol) dissolved in tetrahydrofuran (5 mL) was added dropwise, after the completion of dropwise addition, the obtained system was continuously reacted for 1 h. Then, a solution of ethyl bromoacetate (3.64 g, 21.79 mmol) in tetrahydrofuran (5 mL) was further added dropwise, after the completion of dropwise addition, the obtained system was continuously reacted at -78°C for 2 h. After the completion of reaction, saturated ammonium chloride solution (10 mL) was added dropwise at -78°C, water (50 mL) was added and then ethyl acetate (20 mL*3) was used for extraction, the organic phase was collected, washed, dried and then concentrated, and the resulting residue was purified by column chromatography (petroleum ether : ethyl acetate (v : v)=5 : 1) to obtain compound 121B (2.5 g, yield: 70.15%).

LCMS (ESI): m/z = 197.2 [M+H]⁺.

Step 2: Compound 121B (2.4 g, 12.23 mmol) was added into a flask, followed by methanol (30 mL), then 30% hydrogen peroxide (5.83 mL, 73.38 mmol) was added in an ice bath, followed by sodium hydroxide solution (0.24 g, 6 mmol, dissolved in 1.84 mL of water), then the obtained system was naturally warmed to room temperature and reacted for 16 h. Sodium thiosulfate solution was added in an ice bath, the obtained system was then extracted with ethyl acetate, the organic phase was collected, washed and concentrated, and the resulting residue was purified by column chromatography (petroleum ether : ethyl acetate (v : v)=10 : 1) to obtain compound 121C (0.95 g, yield: 36.60%).

LCMS (ESI): m/z = 213.3 [M+H]⁺.

Step 3: Diphenyl diselenide (2.1 g, 6.72 mmol) was added into a flask, then ethanol (20 mL) was added, sodium borohydride (0.51 g, 13.48 mmol) was added in portions at room temperature, the obtained system was reacted for 10 min, then acetic acid (0.04 mL) and a solution of compound 121C (0.9 g, 4.48 mmol) in ethanol (50 mL) were added. Then the obtained system was reacted at room temperature for 3 h. Water was added, which was extracted with dichloromethane, the organic phase was collected, washed, dried and concentrated, the resulting residue was purified by column chromatography (petroleum ether : ethyl acetate (v : v)=1 : 1) to obtain compound 121D (0.8 g, yield: 83.34%).

LCMS (ESI): m/z = 215.3 [M+H]⁺.

Step 4: Compound 121D (0.8 g, 3.73 mmol) was added to a flask, followed by ethanol (10 mL) and hydrazine hydrate (1.6 mL, 32.92 mmol). The obtained system was then reacted at 85°C for 1 h. After cooling, the reaction liquid was concentrated, and the resulting residue was purified by column chromatography (dichloromethane : methanol (v : v) = 1 : 10) to obtain compound 121E (0.65 g, yield: 95.63%).

LCMS (ESI): m/z = 183.2 [M+H]⁺.

Step 5: Compound 121E (650 mg, 3.57 mmol) was added to a flask, acetonitrile (10 mL) and copper dichloride (0.96 g, 7.14 mmol) were added. The obtained system was then reacted at 85°C for 1 h. After cooling, the obtained system was concentrated directly, and the resulting residue was purified by column chromatography (dichloromethane : methanol (v : v)=1 : 5) to obtain compound 121F (390 mg, yield: 60.62%).

LCMS (ESI): m/z = 181.1 [M+H]⁺.

Step 6: Compound 121F (390 mg, 2.16 mmol) was added to a flask, followed by acetonitrile (10 mL), cesium carbonate (2.11 g, 6.48 mmol) and compound 56B (490 mg, 3.22 mmol), and then the obtained system was reacted at 85°C for 2 h. After cooling, the reaction liquid was directly filtered, and the filtrate was concentrated, the resulting residue was purified by column chromatography (dichloromethane : methanol (v : v)=10 : 1) to obtain compound 121G (550 mg, yield: 85.92%).

LCMS (ESI): m/z = 297.6 [M+H]⁺.

Step 7: Anhydrous dichloromethane (20 mL) was added to a flask, and oxalyl chloride (0.19 mL, 2.21 mmol) was added. Anhydrous dimethyl sulfoxide (0.29 mL, 4.09 mmol) was added dropwise at -78°C under nitrogen protection. After the completion of dropwise addition, the reaction was continued for 30 min, and then a solution of compound 121G (550 mg, 1.86 mmol) in dichloromethane (30 mL) was added dropwise. After the completion of dropwise addition, the obtained system was reacted at -78°C for 1 h, then triethylamine (2.06 mL, 14.86 mmol) was further added dropwise, and then reacted for another 1 h. Water was added and dichloromethane was used for extraction, the organic phase was collected and washed to obtain a solution of compound 121H in dichloromethane, which solution was used directly for the next reaction.

LCMS (ESI): m/z = 295.2 [M+H]⁺.

Step 8: The solution of compound 121H in dichloromethane was added into a flask, then N,N-dimethylformamide dimethylacetal (10 mL) was added, the obtained system was concentrated to remove dichloromethane, and the remaining solution in the flask was continuously stirred at 100°C for 1 h. After cooling, the obtained system was concentrated directly to give compound 121I (550 mg of crude product), which was used directly for the next reaction.

LCMS (ESI): m/z = 350.2 [M+H]⁺.

Step 9: Compound 121I (550 mg, 1.57 mmol) was added to a flask, and dioxane (20 mL), S-methylisothiourea sulfate (2.62 g, 9.40 mmol) and potassium carbonate (2.60 g, 18.81 mmol) were added, then the obtained system was reacted at 110°C for 5 h. After cooling, the reaction liquid was directly filtered, and the filtrate was concentrated, the resulting residue was purified by column chromatography (petroleum ether : ethyl acetate (v : v) = 1 : 5) to obtain compound 121J (200 mg, yield: 33.84%).

LCMS (ESI): m/z = 377.1 [M+H]⁺.

Step 10: Compound 121J (100 mg, 0.27 mmol) was added to a flask, dichloromethane (5 mL) and m-chloroperoxybenzoic acid (100 mg, 0.59 mmol) were added, and then the obtained system was reacted at room temperature for 16 h. Sodium thiosulfate solution was added to the reaction liquid, then dichloromethane was used for extraction, the organic phase was collected, washed and concentrated, and the resulting residue was purified by column chromatography (petroleum ether : ethyl acetate (v : v)=1 : 0-0 : 1) to obtain compound 121K (39 mg, yield: 35.36%).

LCMS (ESI): m/z = 409.6 [M+H]⁺.

Step 11: Compound 121K (39 mg, 0.09 mmol) was added to a flask, acetonitrile (5 mL), 2,2,2-trifluoroethanol (29 mg, 0.29 mmol) and potassium carbonate (39 mg, 0.29 mmol) were added, and the obtained system was reacted at 50°C for 1 h. After cooling, the reaction liquid was filtered directly, and the filtrate was concentrated and the residue was purified by preparative HPLC. Purification methods: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.05% aqueous ammonia); b. gradient elution, mobile phase A: 25%-75%; c. flow rate: 15 mL/min; d. elution time: 10 min. Compound 121 (8.76 mg, yield: 21.52%) was obtained.
¹H NMR (400 MHz, DMSO-*d*₆) δ 8.62 (s, 1H), 7.22 (d, 1H), 7.10 (s, 1H), 7.04 (d, 1H), 6.96 (s, 1H), 5.25 (s, 2H), 5.09 (q, 2H), 3.09 (s, 4H), 2.75 (t, 2H), 2.47 (t, 2H), 2.18-2.11 (m, 2H);
LCMS (ESI): m/z = 429.6 [M+H]⁺.

### Example 122

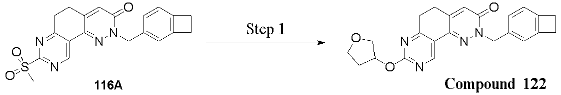

Step 1: Compound 116A (40 mg, 0.10 mmol) was added to a flask, and then acetonitrile (6 mL), 3-hydroxytetrahydrofuran (88 mg, 1.00 mmol) and cesium carbonate (98 mg, 0.30 mmol) were added, and then the obtained system was reacted at 50°C for 1 h. After cooling, the reaction liquid was concentrated directly, the resulting residue was purified by silica gel column chromatography (petroleum ether : ethyl acetate (v : v)=1 : 2) to obtain compound 122 (2 mg, yield: 5%).
¹H NMR ((400 MHz, CDCl₃) δ 9.07 (s, 1H), 7.33 (d, 1H), 7.20 (s, 1H), 7.01 (d, 1H), 6.77 (s, 1H), 5.60-5.58 (m, 1H), 5.32 (s, 2H), 4.15-3.92 (m, 4H), 3.13 (s, 4H), 3.00-2.90 (m, 4H), 2.31-2.20 (m, 2H);
LC-MS (ESI): m/z = 403.2 [M+H]⁺.

### Example 123

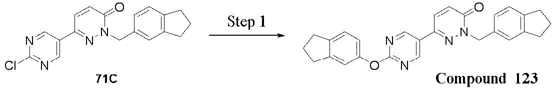

Step 1: Compound 71C (0.18 g, 0.53 mmol), 2,3-dihydro-1H-inden-5-ol (0.10 g, 0.74 mmol) and potassium carbonate (0.18 g, 1.33 mmol) were dissolved in N,N-dimethylformamide (6 mL), and the obtained system was reacted under stirring at 80°C for 3 h. After the reaction was completed, the reaction liquid was cooled, diluted with water, and extracted with ethyl acetate 3 times, the organic phases were combined and concentrated under reduced pressure, and the resulting residue was purified by preparative HPLC. Purification methods: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.5% ammonium acetate); b. gradient elution, mobile phase A: 30%-80%; c. flow rate: 20 mL/min; d. elution time: 17 min. Compound 123 (190 mg, yield: 82%) was obtained.
¹H NMR (400 MHz, DMSO-*d*₆) δ 9.07 (s, 2H), 8.05 (d, 1H), 7.31 - 7.22 (m, 2H), 7.19 - 7.10 (m, 3H), 7.06 (d, 1H), 6.97-6.90 (m, 1H), 5.26 (s, 2H), 2.90-2.84 (m, 4H), 2.84-2.77 (m, 4H), 2.12-2.02 (m, 2H), 2.01-1.91 (m, 2H);
LC-MS (ESI): m/z = 437.5 [M+H]⁺.

### Example 124

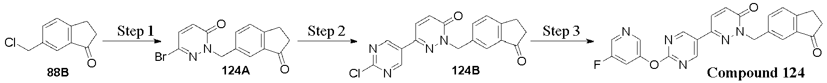

Step 1: 6-bromo-3-pyridazinol (1 g, 5.71 mmol), compound 88B (1.13 g, 6.28 mmol) and potassium carbonate (2.37 g, 17.13 mmol) were dissolved in N,N-dimethylformamide (10 mL) and the obtained system was reacted under stirring at 70°C for 2 h. The system was cooled to room temperature and concentrated, the resulting residue was purified by column chromatography (petroleum ether : ethyl acetate (v : v)= 5%-70%) to obtain compound 124A (1.4 g, yield: 76.82%).

LC-MS (ESI): m/z = 319.1 [M+H]⁺ .

Step 2: Compound 124A (1 g, 3.13 mmol), 2-chloropyrimidine-5-boronic acid (0.50 g, 3.16 mmol), potassium carbonate (1.30 g, 9.39 mmol) and [1,3-bis(2,6-diisopropylphenyl)imidazol-2-ylidene](3-chloropyridinyl)palladium dichloride (0.21 g, 0.31 mmol) were dissolved in a mixed solvent of 1,4-dioxane (10 mL) and water (2 mL), then the obtained system was reacted under stirring at 90°C for 2.5 h under nitrogen protection. The system was cooled to room temperature and concentrated, the resulting residue was purified by column chromatography (petroleum ether : ethyl acetate (v : v)= 5%-90%) to obtain compound 124B (0.87 g, yield: 78.79%).

LC-MS (ESI): m/z = 353.1 [M+H]⁺.

Step 3: 3-fluoro-5-hydroxypyridine (0.095 g, 0.84 mmol) was dissolved in dry tetrahydrofuran (5 mL), then sodium hydride (0.023 g, 0.57 mmol) was added, the obtained system was stirred for half an hour, and then compound 124B (0.1 g, 0.28 mmol) was further added, and the obtained system was stirred at 50°C overnight. The system was cooled and concentrated directly, and the resulting residue was purified by reverse phase column chromatography (acetonitrile : water (v : v)=3%-60%) to obtain compound 124 (20 mg, yield: 16.63%).
¹H NMR (400 MHz, DMSO-*d*₆) δ 9.16 (s, 2H), 8.56 (d, 1H), 8.51 (s, 1H), 8.11 (d, 1H), 7.96-7.93 (m, 1H), 7.78 - 7.70 (m, 1H), 7.61 (s, 1H), 7.57 (d, 1H), 7.17 (d, 1H), 5.40 (s, 2H), 3.09 - 3.05 (m, 2H), 2.64 - 2.61 (m, 2H);
LC-MS (ESI): m/z = 430.1 [M+H]⁺ .

### Example 125

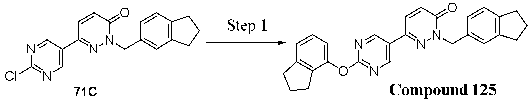

4-indanol (0.24 g, 1.77 mmol) was dissolved in dry tetrahydrofuran (10 mL), sodium hydride (0.047 g, 1.18 mmol) was added and stirred for half an hour, then compound 71C (0.2 g, 0.59 mmol) was added and the obtained system was reacted under stirring for half an hour. The reaction liquid was concentrated directly, and the resulting residue was purified by reverse phase column chromatography (acetonitrile : water (v : v)=3%-60%) to obtain compound 125 (150 mg, yield: 58.24%).
¹H NMR (400 MHz, DMSO-*d*₆) δ 9.08 (s, 2H), 8.06 (d, 1H), 7.26 - 7.19 (m, 2H), 7.19 - 7.15 (m, 3H), 7.13 (d, 1H), 6.97 (d, 1H), 5.26 (s, 2H), 2.96-2.93 (m, 2H), 2.83-2.78 (m, 4H), 2.64-2.60 (m, 2H), 2.01-1.96 (m, 4H);
LC-MS (ESI): m/z = 437.3 [M+H]⁺.

### Example 126

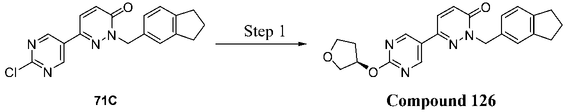

(R)-(-)-3-hydroxytetrahydrofuran (264 mg, 3 mmol) was dissolved in tetrahydrofuran (20 mL), sodium hydride (100 mg, 2.5 mmol) was added, the obtained system was reacted at room temperature for 30 min, compound 71C (338 mg, 1 mmol) was added, and the obtained system was reacted under stirring for 10 min. Water and ethyl acetate were added for extraction and liquid separation, the organic phase was further washed with saturated brine, the organic phase was collected and concentrated under reduced pressure, and the resulting residue was purified by column chromatography (petroleum ether : ethyl acetate (v : v)=1 : 2) to obtain compound 126 (140 mg, yield: 36%).
¹H NMR (400 MHz, DMSO-*d*₆) δ 9.07 (s, 2H), 8.09 - 8.04 (m, 1H), 7.26 - 7.07 (m, 4H), 5.57 - 5.52 (m, 1H), 5.26 (s, 2H), 3.98 - 3.75 (m, 4H), 2.86 - 2.76 (m, 4H), 2.34 - 2.21 (m, 1H), 2.11 - 1.90 (m, 3H);
LCMS m/z =391.1 [M+H]⁺.

### Example 127

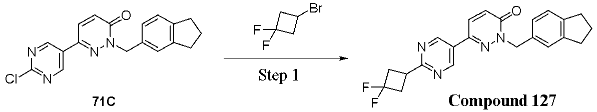

3-bromo-1,1-difluorocyclobutane (0.30 g, 1.75 mmol) was added to a mixed solvent of tetrahydrofuran (10 mL) and N,N-dimethylacetamide (20 mL), the obtained system was subjected to nitrogen replacement, then zinc powder (0.14 g, 2.18 mmol) was added, and the obtained system was stirred at room temperature for 1 h, then compound 71C (0.30 g, 0.89 mmol) and bis(triphenylphosphine)palladium dichloride (62 mg, 0.089 mmol) were added, and the obtained system was subjected to nitrogen replacement 3 times, then raised to 80°C and reacted overnight. After the reaction was completed, the reaction liquid was filtered, ethyl acetate (100 mL) and water (100 mL) were added to the filtrate for extraction and liquid separation, the organic phase was washed with saturated brine 3 times and concentrated, and the resulting residue was purified by a reverse phase preparative column (acetonitrile : water (v : v)=10%-80%) to obtain compound 127 (22 mg, yield: 6.27%).
¹H NMR (400 MHz, CDCl₃) δ 9.08 (s, 2H), 7.60 (d, 1H), 7.35 (s, 1H), 7.26 (d, 1H), 7.18 (d, 1H), 7.07 (d, 1H), 5.37 (s, 2H), 3.73-3.64 (m, 1H), 3.17 - 2.96 (m, 4H), 2.90-2.85 (m, 4H), 2.09-2.01 (m, 2H);
LC-MS (ESI): m/z = 395.20 [M+H]⁺.

### Example 128

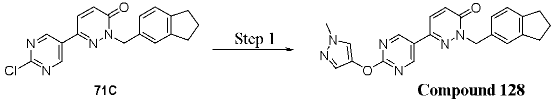

Compound 71C (0.17 g, 0.5 mmol), 1-methyl-1H-pyrazol-4-ol (0.074 g, 0.75 mmol) and potassium carbonate (0.14 g, 1 mmol) were dissolved in 2-methyltetrahydrofuran (2 mL), and the obtained system was reacted under stirring at 80°C for 3 h. After cooling, the reaction liquid was concentrated directly, and the resulting residue was purified by preparative HPLC. Purification methods: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.05% aqueous ammonia); b. gradient elution, mobile phase A: 40%-100%; c. flow rate: 15 mL/min; d. elution time: 25 min. Compound 128 (137 mg, yield: 68.4%) was obtained.
¹H NMR (400 MHz, DMSO-*d*₆) δ 9.12 (s, 2H), 8.08 (d, 1H), 7.91 (d, 1H), 7.50 (d, 1H), 7.25 (s, 1H), 7.20 - 7.15 (m, 2H), 7.13 (d, 1H), 5.27 (s, 2H), 3.84 (s, 3H), 2.84 - 2.79 (m, 4H), 2.01 - 1.94 (m, 2H).
LC-MS (ESI): m/z = 401.2 [M+H]⁺.

### Example 129

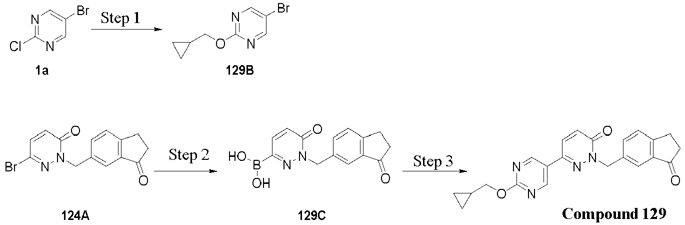

Step 1: Cyclopropylmethanol (0.45 g, 6.18 mmol) was dissolved in dry tetrahydrofuran (10 mL), then sodium hydride (0.31 g, 7.79 mmol) was added and stirred for half an hour, compound 1a (1 g, 5.19 mmol) was added and stirred at room temperature for 1 h. The reaction liquid was concentrated directly, a large amount of solid precipitated when water was added, filtration was performed, the filter cake was rinsed with water, collected and dried to obtain compound 129B (1.1 g, yield: 92.52%).

LC-MS (ESI): m/z = 229.1 [M+H]⁺.

Step 2: Compound 124A (1 g, 3.13 mmol), bis(pinacolato)diboron (3.97 g, 15.65 mmol), potassium acetate (0.92 g, 9.39 mmol) and chloro(2-dicyclohexylphosphino-2',4',6'-triisopropyl-1,1'-biphenyl)[2-(2'-amino-1,1'-biphenyl)]palladium(II) (0.25 g, 0.31 mmol) were dissolved in 1,4-dioxane (10 mL), the obtained system was reacted under stirring at 90°C for 3 h under nitrogen protection. The reaction liquid was cooled to room temperature, the funnel was filled with diatomaceous earth for filtration, the filter cake was washed once with methanol (20 mL), the filtrate was collected and concentrated under reduced pressure, a mixed solvent of petroleum ether and ethyl acetate (v : v=30 : 1, 30 mL) was added to the resulting residue, the obtained system was stirred for 12 h, then filtered, the filter cake was collected and dried to obtain compound 129C (0.8 g, yield: 89.97%).

LC-MS (ESI): m/z = 285.1 [M+H]⁺.

Step 3: Compound 129C (0.4 g, 1.09 mmol), compound 129B (0.25 g, 1.09 mmol), potassium carbonate (0.45 g, 3.26 mmol) and [1,3-bis(2,6-diisopropylphenyl)imidazol-2-ylidene](3-chloropyridinyl)palladium dichloride (0.074 g, 0.11 mmol) were dissolved in a mixed solvent of 1,4-dioxane (5 mL) and water (1 mL), then the obtained system was stirred at 90°C for 3 h under nitrogen protection. The reaction liquid was cooled to room temperature and concentrated, then ethyl acetate was added to the resulting residue and mixed evenly, the obtained system was filtered, the filtrate was collected and concentrated, the resulting residue was purified by reverse phase column chromatography (acetonitrile : water (v : v)=5%-70%) to obtain compound 129 (0.21 g, yield: 49.60%).
¹H NMR (400 MHz, DMSO-*d*₆) δ 9.06 (s, 2H), 8.08 (d, 1H), 7.74-7.71 (m, 1H), 7.61 (s, 1H), 7.57 (d, 1H), 7.13 (d, 1H), 5.39 (s, 2H), 4.21 (d, 2H), 3.13 - 3.02 (m, 2H), 2.65 - 2.60 (m, 2H), 1.36 - 1.21 (m, 1H), 0.66 - 0.53 (m, 2H), 0.42 - 0.32 (m, 2H);
LC-MS (ESI): m/z = 389.2 [M+H]⁺.

### Example 130

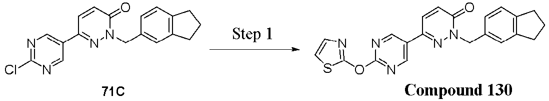

2-hydroxythiazole (0.18 g, 1.77 mmol) was dissolved in dry tetrahydrofuran (10 mL), sodium hydride (0.047 g, 1.18 mmol) was added and stirred for half an hour, then compound 71C (0.2 g, 0.59 mmol) was added and the obtained system was reacted under stirring for half an hour. The reaction liquid was concentrated directly, and the resulting residue was purified by reverse phase column chromatography (acetonitrile : water (v : v)=3%-60%) to obtain compound 130 (0.11 g, yield: 46.21 %).
¹H NMR (400 MHz, DMSO-*d*₆) δ 9.33 (s, 2H), 8.17 (d, 1H), 7.59 (d, 1H), 7.27 (s, 1H), 7.19-7.16 (m, 3H), 6.71 (d, 1H), 5.30 (s, 2H), 2.84-2.79 (m, 4H), 2.04 - 1.92 (m, 2H);
LC-MS (ESI): m/z = 404.1 [M+H]⁺.

### Example 131

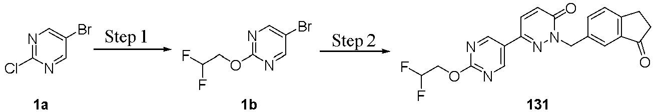

Step 1: 2,2-difluoroethanol (0.51 g, 6.23 mmol) was dissolved in dry tetrahydrofuran (5 mL), then sodium hydride (0.31 g, 7.79 mmol) was further added and stirred for half an hour, compound la (1 g, 5.19 mmol) was further added and stirred at room temperature for 1 h. The reaction liquid was concentrated directly, a large amount of solid precipitated when water was added, filtration was performed, the filter cake was rinsed with water, collected and dried to obtain compound 1b (1.1 g, yield: 88.67%).

LC-MS (ESI): m/z = 239.1 [M+H]⁺.

Step 2: Compound 129C (0.4 g, 1.09 mmol), compound 1b (0.26 g, 1.09 mmol), potassium carbonate (0.45 g, 3.26 mmol) and [1,3-bis(2,6-diisopropylphenyl)imidazol-2-ylidene](3-chloropyridinyl)palladium dichloride (0.074 g, 0.11 mmol) were dissolved in 1,4-dioxane (5 mL) and water (1 mL), then the obtained system was stirred at 90°C for 3 h under nitrogen protection. After the reaction liquid was cooled to room temperature, it was concentrated, the resulting residue was dissolved with ethyl acetate, and filtered, the filtrate was collected and concentrated, and the resulting residue was purified by reverse phase column chromatography (acetonitrile : water (v : v)=5%-70%) to obtain compound 131 (0.18 g, yield: 41.45%).
¹H NMR (400 MHz, DMSO-*d*₆) δ 9.12 (s, 2H), 8.11 (d, 1H), 7.74-7.72 (m, 1H), 7.62 (s, 1H), 7.57 (d, 1H), 7.15 (d, 1H), 6.62 - 6.24 (m, 1H), 5.39 (d, 2H), 4.73-4.69 (m, 2H), 3.11 - 3.03 (m, 2H), 2.66 - 2.59 (m, 2H);
LC-MS (ESI): m/z = 399.1 [M+H]⁺.

### Example 132

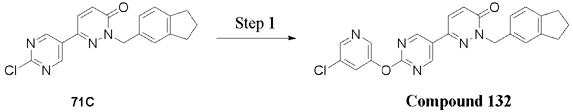

Step 1: 71C (0.15 g, 0.44 mmol), 5-chloropyridin-3-ol (0.068 g, 0.52 mmol) and potassium carbonate (0.15 g, 1.1 mmol) were dissolved in N,N-dimethylformamide (6 mL) and then the obtained system was stirred at 80°C for 3 h. After the reaction was completed, the reaction liquid was cooled to room temperature, diluted with water, and extracted with ethyl acetate 3 times, the organic phases were combined and concentrated, and the resulting residue was purified by preparative HPLC. Separation method: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.5% ammonium acetate); b. gradient elution, mobile phase A: 30%-70%; c. flow rate: 15 mL/min; d. elution time: 20 min. Compound 132 (110 mg, yield: 57.8%) was obtained.
¹H NMR (400 MHz, DMSO-*d*₆) δ 9.15 (s, 2H), 8.67 - 8.47 (m, 2H), 8.20 - 7.99 (m, 2H), 7.25 (s, 1H), 7.19 - 7.11 (m, 3H), 5.27 (s, 2H), 2.86-2.76 (m, 4H), 2.04 - 1.90 (m, 2H);
LC-MS (ESI): m/z = 432.6 [M+H]⁺.

### Example 133

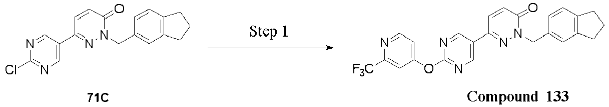

Step 1: Compound 71C (0.20 g, 0.59 mmol), 2-trifluoromethyl-4-hydroxypyridine (0.19 g, 1.16 mmol), and potassium carbonate (0.20 g, 1.47 mmol) were added to solvent acetonitrile (10 mL) in sequence, and the obtained system was heated to 80°C and reacted overnight. After the reaction was completed, it was cooled to room temperature and filtered, ethyl acetate (100 mL) and water (100 mL) were added to the filtrate for liquid separation, the organic phase was washed with saturated brine 3 times, and then concentrated to dryness, the resulting residue was purified by reverse phase column chromatography (acetonitrile : water (v : v)=10%-80%) to obtain the final product 133 (130 mg, yield: 47.34%).
¹H NMR (400 MHz, CDCl₃) δ 9.00 (s, 2H), 8.78 (d, 1H), 7.64 (d, 1H), 7.58 (d, 1H), 7.43-7.41 (m, 1H), 7.34 (s, 1H), 7.25 (d, 1H), 7.19 (d, 1H), 7.08 (d, 1H), 5.37 (s, 2H), 2.90-2.85 (m, 4H), 2.09-2.20 (m, 2H);
LC-MS (ESI): m/z = 466.20 [M+H]⁺.

### Example 134

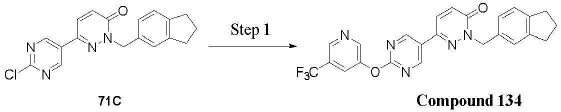

Step 1: Compound 71C (0.2 g, 0.59 mmol), 5-(trifluoromethyl) pyridin-3-ol (0.19 g, 1.18 mmol) and potassium carbonate (0.20 g, 1.47 mmol) were dissolved in N,N-dimethylformamide (8 mL), and the obtained system was heated to 80°C and stirred for 3 h. The system was cooled to room temperature and filtered, the filtrate was concentrated, and the resulting residue was purified by reverse phase column chromatography (acetonitrile : water (v : v)=3%-60%) to obtain compound 134 (0.15 g, yield: 54.62%).
¹H NMR (400 MHz, DMSO-*d*₆) δ 9.16 (s, 2H), 8.93-8.92 (m, 2H), 8.37 (s, 1H), 8.09 (d, 1H), 7.25 (s, 1H), 7.19 - 7.12 (m, 3H), 5.27 (s, 2H), 2.83-2.78 (m, 4H), 2.03 - 1.88 (m, 2H);
LC-MS (ESI): m/z = 466.2 [M+H]⁺.

### Example 135

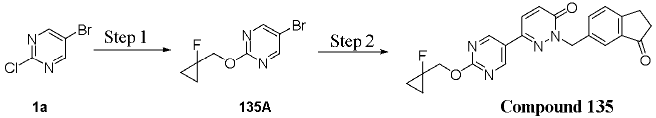

Step 1: 1-fluoropropylmethanol (0.17 g, 1.85 mmol) was dissolved in dry tetrahydrofuran (5 mL), then sodium hydride (0.092 g, 2.31 mmol) was added and stirred for half an hour, compound 1a (0.3 g, 1.54 mmol) was added and stirred at room temperature for one hour. The reaction liquid was concentrated, a large amount of solid precipitated when water was added, filtration was performed, the filter cake was rinsed with water, collected and dried to obtain compound 135A (0.3 g, yield: 78.85%).

LC-MS (ESI): m/z = 247.0 [M+H]⁺.

Step 2: Compound 129C (0.4 g, 1.09 mmol), compound 135A (0.27 g, 1.09 mmol), potassium carbonate (0.45 g, 3.26 mmol) and [1,3-bis(2,6-diisopropylphenyl)imidazol-2-ylidene](3-chloropyridinyl)palladium dichloride (0.074 g, 0.11 mmol) were dissolved in a mixed solvent of 1,4-dioxane (5 mL) and water (1 mL), then the obtained system was heated to 90°C and stirred for 3 h under nitrogen protection. After the reaction liquid was cooled to room temperature, it was concentrated, then dissolved with ethyl acetate, and filtered, the filtrate was collected and concentrated, and the resulting residue was purified by reverse phase column chromatography (acetonitrile : water (v : v)=5%-70%) to obtain compound 135 (0.22 g, yield: 49.66%).
¹H NMR (400 MHz, DMSO-*d*₆) δ 9.09 (s, 2H), 8.11 (d, 1H), 7.74-7.72 (m, 1H), 7.62 (s, 1H), 7.57 (d, 1H), 7.15 (d, 1H), 5.40 (s, 2H), 4.69 (d, 2H), 3.11 - 3.02 (m, 2H), 2.66 - 2.58 (m, 2H), 1.24-1.13 (m, 2H), 0.97 - 0.88 (m, 2H);
LC-MS (ESI): m/z = 407.2 [M+H]⁺.

### Example 136

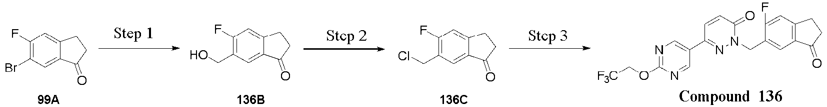

Step 1: Compound 99A (2.0 g, 8.73 mmol), tri-n-butyltin methanol (3.08 g, 9.60 mmol), and Xphos Pd G2 (0.69 g, 0.87 mmol) were dissolved in 1,4-dioxane (50 mL), the obtained system was subjected to nitrogen replacement three times, and reacted at 90°C for 16 h. The reaction liquid was cooled to room temperature and concentrated under reduced pressure, the resulting residue was purified by silica gel column chromatography (petroleum ether : ethyl acetate (v : v)=3 : 1) to obtain compound 136B (1.5 g, yield: 95.36%).
¹H NMR (400 MHz, DMSO-*d*₆) δ 7.73 (d, 1H), 7.38 (d, 1H), 5.40 - 5.37 (m, 1H), 4.57 (d, 2H), 3.12 - 3.05 (m, 2H), 2.68 - 2.61 (m, 2H);
LC-MS (ESI): m/z = 181.1 [M+H]⁺.

Step 2: Compound 136B (0.3 g, 1.67 mmol) was dissolved in dichloromethane (5 mL), then thionyl chloride (0.3 mL) was added, a drop of N,N-dimethylformamide was further added, then the obtained system was reacted at room temperature for 3 h. The obtained system was concentrated under reduced pressure, the residue was washed twice with petroleum ether (30 mL × 2), and the residue was dried to give compound 136C (0.31 g, yield: 93.46%).

LC-MS (ESI): m/z = 199.1 [M+H]⁺.

Step 3: Compound 1D (0.2 g, 0.73 mmol), compound 136C (0.3 g, 1.51 mmol) and cesium carbonate (0.6 g, 1.82 mmol) were dissolved in acetonitrile (10 mL) and then the obtained system was reacted at 80°C for 16 h. The reaction liquid was cooled to room temperature and concentrated under reduced pressure, and the resulting residue was purified by silica gel column chromatography (dichloromethane : ethyl acetate (v : v)=1 : 1) to obtain compound 136 (42 mg, yield: 13.25%).
¹H NMR (400 MHz, CDCl₃) δ 8.83 (s, 2H), 7.59 - 7.55 (m, 2H), 7.13 (d, 1H), 7.04 (d, 1H), 5.42 (s, 2H), 4.83 - 4.77 (m, 2H), 3.09 - 3.02 (m, 2H), 2.65 - 2.59 (m, 2H);
LC-MS (ESI): m/z = 435.1 [M+H]⁺.

### Example 137

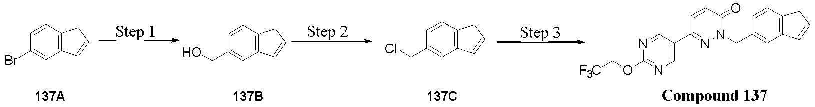

Step 1: Compound 137A (1 g, 5.14 mmol), (tributyltin)methanol (1.98 g, 6.17 mmol) and chloro(2-dicyclohexylphosphino-2',4',6'-triisopropyl-1,1'-biphenyl)[2-(2'-amino-1,1'-biphenyl)]palladium(II) (0.081 g, 0.10 mmol) were added to the solvent 1,4-dioxane (20 mL) in sequence, the obtained system was then subjected to nitrogen replacement 3 times, raised to 90°C and reacted under stirring for 3 h. After the reaction was completed, the reaction liquid was cooled to room temperature and concentrated, the resulting residue was purified by column chromatography (ethyl acetate : petroleum ether (v : v)=5%-50%) to obtain compound 137B (0.6 g, yield: 79.85%).

Step 2: Compound 137B (0.5 g, 3.42 mmol) was dissolved in dichloromethane (10 mL), thionyl chloride (0.53 g, 4.45 mmol) was added, and the obtained system was stirred at room temperature for 2 h. After the reaction was completed, the obtained system was concentrated under reduced pressure to give 137C (0.56 g of crude product).

Step 3: 1D (0.2 g, 0.73 mmol), compound 137C (0.12 g, 0.73 mmol) and potassium carbonate (0.30 g, 2.19 mmol) were dissolved in N,N-dimethylformamide (10 mL) and then the obtained system was stirred at 80°C for 2 h. After cooling, the reaction liquid was concentrated, diluted with water (10 mL), and then extracted with ethyl acetate 3 times (15 mL×3), the organic phase was collected and concentrated, and the resulting residue was purified by reverse phase column chromatography (acetonitrile : water (v : v)=3%-70%) to obtain compound 137 (0.2 g, yield: 68.43%).
¹H NMR (400 MHz, DMSO-*d*₆) δ 9.15 (d, 2H), 8.11 (d, 1H), 7.54 - 7.36 (m, 2H), 7.35 - 7.22 (m, 1H), 7.15 (d, 1H), 6.92-6.89 (m, 1H), 6.62-6.61 (m, 1H), 5.36 (s, 2H), 5.14-5.08 (m, 2H), 3.38 (d, 2H);
LC-MS (ESI): m/z = 401.1 [M+H]⁺.

### Example 138

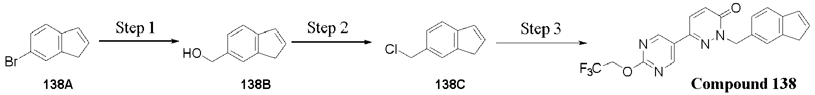

Step 1: Compound 138A (1 g, 5.14 mmol), (tributyltin)methanol (1.98 g, 6.17 mmol) and chloro(2-dicyclohexylphosphino-2',4',6'-triisopropyl-1,1'-biphenyl)[2-(2'-amino-1,1'-biphenyl)]palladium(II) (0.081 g, 0.10 mmol) were added to the solvent 1,4-dioxane (20 mL) in sequence, the obtained system was then subjected to nitrogen replacement 3 times, raised to 90°C and reacted under stirring for 3 h. After the reaction was completed, the reaction liquid was cooled to room temperature and concentrated, the resulting residue was purified by column chromatography (ethyl acetate : petroleum ether (v : v)=5%-50%) to obtain compound 138B (0.6 g, yield: 81.18%).

Step 2: Compound 138B (0.5 g, 3.42 mmol) was dissolved in dichloromethane (10 mL), thionyl chloride (0.53 g, 4.45 mmol) was added, and the obtained system was stirred at room temperature for 2 h. After the reaction was completed, the obtained system was concentrated under reduced pressure to give compound 138C (0.56 g of crude product).

Step 3: Compound 1D (0.2 g, 0.73 mmol), compound 138C (0.12 g, 0.73 mmol) and potassium carbonate (0.30 g, 2.19 mmol) were dissolved in N,N-dimethylformamide (10 mL) and then the obtained system was stirred at 80°C for 2 h. After cooling, the reaction liquid was concentrated directly, diluted with water (10 mL), and then extracted with ethyl acetate 3 times (15 mL×3), the organic phase was collected and concentrated, and the resulting residue was purified by reverse phase column chromatography (acetonitrile : water (v : v)=3%-70%) to obtain compound 138 (0.18 g, yield: 61.59%).
¹H NMR (400 MHz, DMSO-*d*₆) δ 9.15 (d, 2H), 8.11 (d, 1H), 7.55 - 7.36 (m, 2H), 7.34-7.23 (m, 1H), 7.15 (d, 1H), 6.92-6.89 (m, 1H), 6.65 - 6.58 (m, 1H), 5.36 (s, 2H), 5.14-5.08 (m, 2H), 3.38 (d, 2H);
LC-MS (ESI): m/z = 401.1 [M+H]⁺.

### Example 139

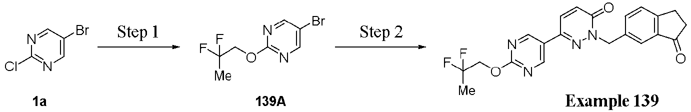

Step 1: 2,2-difluoropropanol (1.50 g, 15.59 mmol) was added to tetrahydrofuran (50 mL), the obtained system was cooled to 0-10°C under nitrogen protection, then sodium hydride (0.50 g) was added. After the obtained system was reacted under stirring for 30 min, compound 1a (2.00 g, 10.39 mmol) was added, and the obtained system was reacted at room temperature for 2 h. After the reaction was completed, ethyl acetate and water were added for extraction and liquid separation, the organic phase was collected and concentrated under reduced pressure, the resulting residue was purified by column chromatography (ethyl acetate : petroleum ether (v : v)=0-50%) to obtain compound 139A (2.10 g, yield: 79.88%).

LC-MS (ESI): m/z = 253.0 [M+H]⁺.

Step 2: Compound 139A (0.20 g, 0.79 mmol), compound 129C (0.25 g, 0.88 mmol) and potassium phosphate (0.17 g, 0.79 mmol) were added to solvent 1,4-dioxane (20 mL) in sequence, the obtained system was subjected to nitrogen replacement, then [1,3-bis(2,6-diisopropylphenyl)imidazol-2-ylidene](3-chloropyridinyl)palladium(II) dichloride (54 mg, 0.079 mmol) and water (0.5 mL) were added, the obtained system was subjected to nitrogen replacement 3 times, then heated to 100°C and reacted overnight. After the reaction was completed, the reaction liquid was filtered, the filter cake was washed with ethyl acetate, the filtrate was collected, then ethyl acetate and water were added for extraction and liquid separation, the organic phase was collected and concentrated under reduced pressure, and the resulting residue was purified by reverse phase column chromatography (acetonitrile : water (v : v)=10%-80%) to obtain compound 139 (45 mg, yield: 13.81%).
¹H NMR (400 MHz, CDCl₃) δ 8.91 (s, 2H), 7.80 - 7.69 (m, 2H), 7.60 (d, 1H), 7.50 - 7.44 (m, 1H), 7.08 (d, 1H), 5.45 (s, 2H), 4.65-4.59 (m, 2H), 3.19 - 3.06 (m, 2H), 2.76 - 2.65 (m, 2H), 1.85- 175 (m, 3H);
LC-MS (ESI): m/z = 413.6 [M+H]⁺.

### Example 140

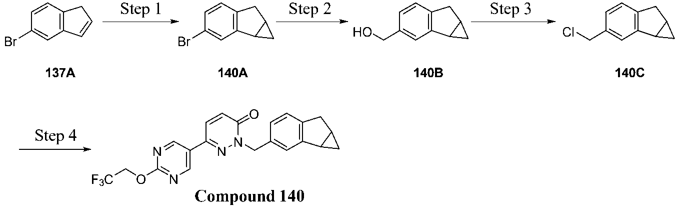

Step 1: Diethylzinc (10.28 mL, 10.28 mmol, 1 M in n-hexane) was added to dichloromethane (10 mL), the obtained system was subjected to nitrogen protection and cooled to 0°C, and then trifluoroacetic acid (1.17 g, 10.28 mmol) in dichloromethane (5 mL) was added, the obtained system was reacted for half an hour, then diiodomethane (2.75 g, 10.28 mmol) in dichloromethane (5 mL) was further added and reacted for half an hour, compound 137A (1 g, 5.14 mmol) in dichloromethane (5 mL) was further added, the obtained system was then slowly warmed to room temperature and reacted for 2 h. The reaction liquid was concentrated directly, and the resulting residue was purified by column chromatography (ethyl acetate : petroleum ether (v : v)=5%-50%) to obtain compound 140A (0.7 g, yield: 65.14%).

¹H NMR (400 MHz, DMSO-*d*₆) δ 7.51-7.50 (m, 1H), 7.25-7.24 (m, 1H), 7.13-7.11 (m, 1H), 3.12-3.06 (m, 1H), 2.89-2.85 (m, 1H), 2.46 - 2.37 (m, 1H), 1.99 - 1.86 (m, 1H), 1.15-1.10 (m, 1H), -0.01 - -0.02 (m, 1H).

Step 2: Compound 140A (1 g, 4.78 mmol), (tributyltin)methanol (3.07 g, 9.56 mmol) and chloro(2-dicyclohexylphosphino-2',4',6'-triisopropyl-1,1'-biphenyl)[2-(2'-amino-1,1'-biphenyl)]palladium(II) (0.075 g, 0.096 mmol) were added to the solvent 1,4-dioxane (20 mL) in sequence, the obtained system was then subjected to nitrogen replacement 3 times, raised to 90°C and reacted under stirring for 3 h. After the reaction was completed, the reaction liquid was cooled to room temperature and concentrated, the resulting residue was purified by column chromatography (ethyl acetate : petroleum ether (v : v)=5%-50%) to obtain compound 140B (0.6 g, yield: 78.35%).

¹H NMR (400 MHz, DMSO-*d*₆) δ 7.23 (s, 1H), 7.07 (d, 1H), 6.99 (d, 1H), 5.03 - 5.00 (m, 1H), 4.42 (d, 2H), 3.11 - 3.08 (m, 1H), 2.84 (d, 1H), 2.38 - 2.29 (m, 1H), 1.86 - 1.83 (m, 1H), 1.08 - 1.03 (m, 1H), -0.09 - -0.12(m, 1H).

Step 3: Compound 140B (0.1 g, 0.62 mmol) was dissolved in dichloromethane (10 mL), thionyl chloride (0.096 g, 0.81 mmol) was added, and the obtained system was stirred at room temperature for 2 h. After the reaction was completed, the obtained system was concentrated under reduced pressure to give 140C (0.11 g of crude product).

Step 4: Compound 1D (0.2 g, 0.73 mmol), compound 140C (0.13 g, 0.73 mmol) and potassium carbonate (0.30 g, 2.19 mmol) were dissolved in N,N-dimethylformamide (10 mL) and then the obtained system was stirred at 80°C for 2 h. After cooling, the reaction liquid was concentrated, water (10 mL) was added to the resulting residue, and ethyl acetate was used for extraction for 3 times (15 mL×3) , the organic phase was collected and concentrated, and the resulting residue was purified by reverse phase column chromatography (acetonitrile : water (v : v)=3%-70%) to obtain compound 140 (0.2 g, yield: 66.12%).
¹H NMR (400 MHz, DMSO-*d*₆) δ 9.15 (s, 2H), 8.11 (d, 1H), 7.31 (s, 1H), 7.14 (d, 1H), 7.10 (d, 2H), 5.27 (s, 2H), 5.14-5.08 (m, 2H), 3.09-3.03 (m, 1H), 2.84 (d, 1H), 2.35-2.33 (m, 1H), 1.87-1.84 (m, 1H), 1.07-1.02 (m, 1H), -0.09 - 0.11 (m, 1H);
LC-MS (ESI): m/z = 415.1 [M+H]⁺.

### Example 141

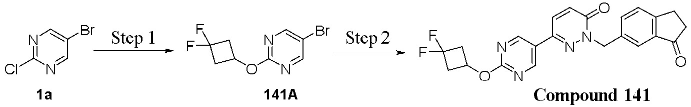

Step 1: 3,3-difluorocyclobutanol (0.56 g, 5.19 mmol) was dissolved in dry tetrahydrofuran (5 mL), then sodium hydride (0.31 g, 7.79 mmol) was further added and stirred for half an hour, compound 1a (1 g, 5.19 mmol) was further added and stirred at room temperature for 1 h. The reaction liquid was concentrated directly, a large amount of solid immediately precipitated when water was added to the residue, filtration was performed, the filter cake was rinsed with water, collected and dried to obtain compound 141A (1.1 g, yield: 79.96%).

LC-MS (ESI): m/z = 265.1 [M+H]⁺.

Step 2: Compound 129C (0.16 g, 0.55 mmol), compound 141A (0.15 g, 0.55 mmol), potassium carbonate (0.23 g, 1.65 mmol) and [1,3-bis(2,6-diisopropylphenyl)imidazol-2-ylidene](3-chloropyridinyl)palladium dichloride (0.037 g, 0.055 mmol) were dissolved in a mixed solvent of 1,4-dioxane (5 mL) and water (1 mL), then the obtained system was stirred at 90°C for 3 h under nitrogen protection. After the reaction liquid was cooled to room temperature, it was concentrated, the resulting product was dissolved with ethyl acetate, then filtered to remove insolubles, the filtrate was collected and concentrated, and the resulting residue was purified by reverse phase column chromatography (acetonitrile : water (v : v) = 5%-70%) to obtain compound 141 (0.16 g, yield: 68.55%).
¹H NMR (400 MHz, DMSO-*d*₆) δ 9.09 (s, 2H), 8.09 (d, 1H), 7.74-7.72 (m, 1H), 7.61 (s, 1H), 7.57 (d, J = 8.0 Hz, 1H), 7.14 (d, J = 9.7 Hz, 1H), 5.39 (s, 2H), 5.19-5.15 (m, 1H), 3.23-3.16 (m, 2H), 3.10 - 3.04 (m, 2H), 2.89 - 2.73 (m, 2H), 2.65 - 2.59 (m, 2H);
LC-MS (ESI): m/z = 425.2 [M+H]⁺.

### Example 142

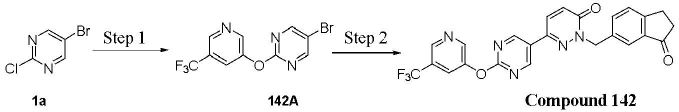

Step 1: Compound 1a (0.5 g, 2.60 mmol), 5-(trifluoromethyl) pyridine-3-hydroxy (0.42 g, 2.6 mmol) and potassium carbonate (0.36 g, 2.6 mmol) were dissolved in N,N-dimethylformamide (10 mL) and the obtained system was stirred at 80°C for 3 h. The reaction liquid was cooled to room temperature and concentrated, a large amount of solid immediately precipitated when water was added to the resulting product, filtration was performed, the filter cake was rinsed with water, collected and dried to obtain compound 142A (0.6 g, yield: 72.10%).

LC-MS (ESI): m/z = 320.2 [M+H]⁺.

Step 2: Compound 129C (0.16 g, 0.55 mmol), compound 142A (0.18 g, 0.55 mmol), potassium carbonate (0.23 g, 1.65 mmol) and [1,3-bis(2,6-diisopropylphenyl)imidazol-2-ylidene](3-chloropyridinyl)palladium dichloride (0.037 g, 0.055 mmol) were dissolved in 1,4-dioxane (5 mL) and water (1 mL), then the obtained system was stirred at 90°C for 3 h under nitrogen protection. The reaction liquid was cooled to room temperature and concentrated, the resulting product was dissolved with ethyl acetate, then filtered to remove insolubles, the filtrate was collected and concentrated, and the resulting residue was purified by reverse phase column chromatography (acetonitrile : water (v : v)=5%-70%) to obtain compound 142 (0.22 g, yield: 81.95%).
¹H NMR (400 MHz, DMSO-*d*₆) δ 9.17 (s, 2H), 8.92 (s, 2H), 8.36 (s, 1H), 8.11 (d, 1H), 7.75-7.73 (m, 1H), 7.61 (s, 1H), 7.57 (d, 1H), 7.17 (d, 1H), 5.40 (s, 2H), 3.14 - 3.01 (m, 2H), 2.65 - 2.60 (m, 2H);
LC-MS (ESI): m/z = 480.1 [M+H]⁺.

### Example 143

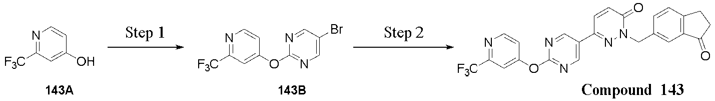

Step 1: Compound 143A (500 mg, 3.07 mmol), compound 1a (0.59 g, 3.07 mmol), and potassium carbonate (0.85 g, 6.14 mmol) were added to acetonitrile (20 mL) in sequence, and the obtained system was heated to 70°C and reacted for 2 h. After the reaction was completed, the reaction liquid was filtered and the filtrate was concentrated, the resulting residue was purified by silica gel column chromatography (ethyl acetate : petroleum ether (v : v)=0-20%) to obtain compound 143B (0.70 g, yield: 71.24%).

LC-MS (ESI): m/z = 320.00 [M+H]⁺.

Step 2: Compound 143B (0.30 g, 0.94 mmol), compound 129C (0.33 g, 0.94 mmol) and potassium phosphate (0.40 g, 1.88 mmol) were added to solvent 1,4-dioxane (20 mL) in sequence, the obtained system was subjected to nitrogen replacement, then [1,3-bis(2,6-diisopropylphenyl)imidazol-2-ylidene](3-chloropyridinyl)palladium dichloride (64 mg, 0.094 mmol) and water (1 mL) were added, the obtained system was subjected to nitrogen replacement 3 times, then heated to 100°C and reacted overnight. After the reaction was completed, the reaction liquid was filtered, the filter cake was washed with ethyl acetate, ethyl acetate and water were added to the filtrate for extraction and liquid separation, the organic phase was collected and concentrated under reduced pressure, and the resulting residue was purified by reverse phase column chromatography (acetonitrile : water (v : v)=10%-80%) to obtain compound 143 (52 mg, yield: 11.54%).
¹H NMR (400 MHz, CDCl₃) δ 8.99 (s, 2H), 8.78 (d, 1H), 7.80 - 7.70 (m, 2H), 7.67 - 7.58 (m, 2H), 7.49-7.42 (m, 2H), 7.10 (d, 1H), 5.46 (s, 2H), 3.23 - 3.05 (m, 2H), 2.77 - 2.63 (m, 2H);
LC-MS (ESI): m/z = 480.1 [M+H]⁺.

### Example 144

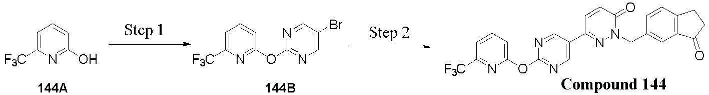

Step 1: Compound 144A (450 mg, 2.75 mmol), compound 1a (590 mg, 3.03 mmol) and potassium carbonate (0.76 g, 5.50 mmol) were added to a reaction flask, and dissolved in acetonitrile (30 mL), and the obtained system was reacted at 80°C for 16 h. The reaction was monitored by TLC, when the reaction was completed, the reaction liquid was cooled to room temperature and concentrated under reduced pressure, the resulting residue was purified by silica gel column chromatography (petroleum ether : ethyl acetate (v : v)=8 : 1) to obtain compound 144B (800 mg, yield: 90.89%).

LC-MS (ESI): m/z = 322.0 [M+H]⁺.

Step 2: Compound 144B (200 mg, 0.62 mmol), compound 129C (180 mg, 0.62 mmol), 1,3-bis(2,6-diisopropylphenyl)imidazol-2-ylidene](3-chloropyridinyl)palladium dichloride (49 mg, 0.06 mmol) and potassium phosphate (263 mg, 1.24 mmol) were dissolved in a mixed solvent of 1,4-dioxane (8 mL) and water (2 mL), and then the obtained system was subjected to nitrogen replacement three times and reacted at 90°C for 3 h. The reaction was monitored by TLC, when the reaction was completed, the reaction liquid was cooled to room temperature and concentrated under reduced pressure, and the resulting residue was purified by silica gel column chromatography (dichloromethane : ethyl acetate (v : v)=2 : 1) to obtain compound 144 (97 mg, yield: 32.63%).
¹H NMR (400 MHz, CDCl₃) δ 8.88 (s, 2H), 7.97 - 7.93 (m, 1H), 7.69 (s, 1H), 7.65 - 7.63 (m, 1H), 7.56 (s, 1H), 7.54 - 7.53 (m, 1H), 7.41 - 7.39 (m, 1H), 7.28 (d, 1H), 7.01 (d, 1H), 5.37 (s, 2H), 3.10 - 2.99 (m, 2H), 2.66 - 2.56 (m, 2H);
LC-MS (ESI): m/z = 480.5 [M+H]⁺.

### Example 145

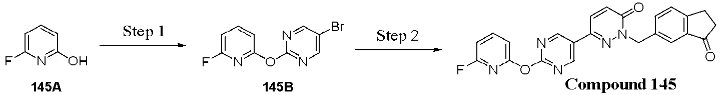

Step 1: Compound 145A (450 mg, 4.01 mmol), compound 1a (780 mg, 4.01 mmol) and potassium carbonate (1.1 g, 8.02 mmol) were dissolved in acetonitrile (30 mL) and the obtained system was reacted at 80°C for 16 h. The reaction was monitored by TLC, when the reaction was completed, the reaction liquid was cooled to room temperature and concentrated under reduced pressure, the resulting residue was purified by silica gel column chromatography (petroleum ether : ethyl acetate (v : v)=8 : 1) to obtain compound 145B (1.0 g, yield: 92.34%).

Step 2: Compound 145B (200 mg, 0.74 mmol), compound 129C (210 mg, 0.74 mmol), 1,3-bis(2,6-diisopropylphenyl)imidazol-2-ylidene](3-chloropyridinyl)palladium dichloride (56 mg, 0.07 mmol) and potassium phosphate (314 mg, 1.48 mmol) were dissolved in a mixed solvent of 1,4-dioxane (8 mL) and water (2 mL), and then the obtained system was subjected to nitrogen replacement three times and reacted at 90°C for 3 h. The reaction was monitored by TLC, when the reaction was completed, the reaction liquid was cooled to room temperature and concentrated under reduced pressure, and the resulting residue was purified by silica gel column chromatography (dichloromethane : ethyl acetate (v : v)=2 : 1) to obtain compound 145 (120 mg, yield: 37.76%).
¹H NMR (400 MHz, CDCl₃) δ 8.89 (s, 2H), 7.89 - 7.83 (m, 1H), 7.70 (s, 1H), 7.65 - 7.63 (m, 1H), 7.53 (d, 1H), 7.40 (d, 1H), 7.02 (d, 1H), 6.99 - 6.97 (m, 1H), 6.81 - 6.79 (m, 1H), 5.37 (s, 2H), 3.08 - 3.02 (m, 2H), 2.64 - 2.59 (m, 2H);
LC-MS (ESI): m/z = 430.1 [M+H]⁺.

### Example 146

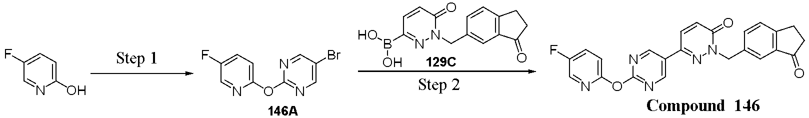

Step 1: 5-fluoropyridin-2-ol (1.6 g, 14 mmol) was dissolved in anhydrous acetonitrile (20 mL), 5-bromo-2-chloropyrimidine (2.7 g, 14 mmol) and potassium carbonate (3.9 g, 28 mmol) were added, and the obtained system was reacted at 90°C for 4 h. The system was cooled to room temperature, filtered, the filter cake was washed with ethyl acetate, the filtrate was collected and concentrated, and the resulting residue was purified by silica gel column chromatography (petroleum ether : ethyl acetate (v : v)=10 : 1) to obtain compound 146A (1.07 g, yield: 28.4%).

LC-MS (ESI): m/z = 270.2, 272.2 [M+H]⁺.

Step 2: 50 mL of eggplant-shaped flask was taken and compound 146A (500 mg, 1.85 mmol), compound 129C (525 mg, 1.85 mmol), potassium phosphate (980 mg, 4.6 mmol) and 1,3-bis(2,6-diisopropylphenyl)imidazol-2-ylidene](3-chloropyridinyl)palladium dichloride (125 mg, 0.18 mmol) were added in sequence, then 1,4-dioxane (20 mL) and water (4 mL) were added, the obtained system was subjected to nitrogen replacement and reacted at 90°C for 4 h. The reaction was monitored by LCMS, when the reaction was completed, the system was concentrated directly, the resulting residue was purified by silica gel column chromatography (petroleum ether : ethyl acetate (v : v)=1 : 2) to obtain compound 146 (96 mg, yield: 12.1%).
¹H NMR (400 MHz, CDCl₃) δ 8.94 (s, 2H), 8.24 - 8.22 (m, 1H), 7.77 - 7.75 (m, 1H), 7.73 - 7.69 (m, 1H), 7.61 - 7.55 (m, 2H), 7.48 - 7.44 (m, 1H), 7.18 - 7.13 (m, 1H), 7.08 (d, 1H), 5.44 (s, 2H), 3.14 - 3.08 (m, 2H), 2.72 - 2.64 (m, 2H);
LC-MS (ESI): m/z = 430.2 [M+H]⁺.

### Example 147

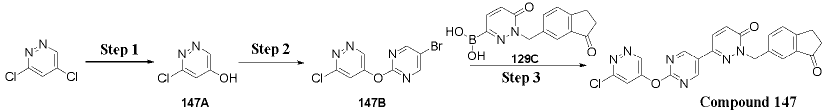

Step 1: 3,5-dichloropyridazine (1.49 g, 10 mmol) was dissolved in 1,4-dioxane (50 mL), 1 M sodium hydroxide solution (10 mL) was added, and the obtained system was heated to 100°C and reacted overnight. After cooling to room temperature and concentration, compound 147A (1.63 g of crude product) was obtained, which was used directly for the next reaction.

Step 2: Compound 147A (1.63 g of crude product) was dissolved in anhydrous acetonitrile (20 mL), 5-bromo-2-chloropyrimidine (1.93 g, 10 mmol) and potassium carbonate (2.76 g, 20 mmol) were added, and the obtained system was reacted at 90°C for 4 h. The system was cooled to room temperature, filtered, the filter cake was washed with ethyl acetate, the filtrate was collected and concentrated, and the resulting residue was purified by silica gel column chromatography (petroleum ether : ethyl acetate (v : v)=10 : 1) to obtain compound 147B (346 mg, yield: 12.1%).

LC-MS (ESI): m/z = 287.2 [M+H]⁺.

Step 3: 50 mL of eggplant-shaped flask was taken and compound 147B (287 mg, 1 mmol), compound 129C (284 mg, 1 mmol), potassium phosphate (530 mg, 2.5 mmol) and 1,3-bis(2,6-diisopropylphenyl)imidazol-2-ylidene](3-chloropyridinyl)palladium dichloride (68 mg, 0.1 mmol) were added in sequence, then 1,4-dioxane (20 mL) and water (4 mL) were added, the obtained system was subjected to nitrogen replacement and reacted at 90°C for 4 h. The reaction was monitored by LCMS, when the reaction was completed, the system was concentrated directly, the resulting residue was purified by silica gel column chromatography (petroleum ether : ethyl acetate (v : v)=1 : 5) to obtain compound 147 (32 mg, yield: 7.16%).
¹H NMR (400 MHz, DMSO-*d*₆) δ 9.47 (s, 2H), 8.36 - 8.12 (m, 2H), 7.77 - 7.73 (m, 1H), 7.67 - 7.63 (m, 1H), 7.60 - 7.55 (m, 1H), 7.52 - 7.49 (m, 1H), 7.21 (d, 1H), 5.45 (s, 2H), 3.12 - 3.04 (m, 2H), 2.67 - 2.59 (m, 2H);
LC-MS (ESI): m/z = 447.2 [M+H]⁺.

### Example 148

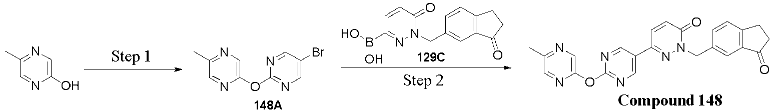

Step 1: 5-methylpyrazin-2-ol (550 mg, 5 mmol) was dissolved in anhydrous acetonitrile (20 mL), 5-bromo-2-chloropyrimidine (965 mg, 5 mmol) and potassium carbonate (1.8 g, 10 mmol) were added, and the obtained system was reacted at 90°C for 4 h. The system was cooled to room temperature, filtered, the filter cake was washed with ethyl acetate, the filtrate was collected and concentrated, and the resulting residue was purified by silica gel column chromatography (petroleum ether : ethyl acetate (v : v)=10 : 1) to obtain compound 148A (665 mg, yield: 49.8%).

LC-MS (ESI): m/z = 267.2, 269.2 [M+H]⁺.

Step 2: 50 mL of eggplant-shaped flask was taken and compound 148A (267 mg, 1 mmol), compound 129C (284 mg, 1 mmol), potassium phosphate (530 mg, 2.5 mmol) and 1,3-bis(2,6-diisopropylphenyl)imidazol-2-ylidene](3-chloropyridinyl)palladium dichloride (68 mg, 0.1 mmol) were added in sequence, then 1,4-dioxane (10 mL) and water (2 mL) were added, the obtained system was subjected to nitrogen replacement and reacted at 90°C for 4 h. The reaction was monitored by LCMS, when the reaction was completed, the system was concentrated directly, the resulting residue was purified by silica gel column chromatography (petroleum ether : ethyl acetate (v : v)=1 : 2) to obtain compound 148 (130 mg, yield: 30.5%).
¹H NMR (400 MHz, DMSO-*d*₆) δ 9.15 (s, 2H), 8.61 - 8.60 (m, 1H), 8.36 (s, 1H), 8.11 (d, 1H), 7.75 - 7.71 (m, 1H), 7.62 (s, 1H), 7.58 - 7.55 (m, 1H), 7.17 (d, 1H), 5.40 (s, 2H), 3.16 - 3.02 (m, 2H), 2.71 - 2.59 (m, 2H), 2.55 (s, 3H);
LC-MS (ESI): m/z = 427.2 [M+H]⁺.

### Example 149

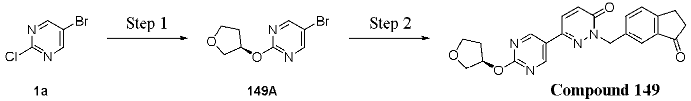

Step 1: (3R)-oxolan-3-ol (0.14 g, 1.59 mmol) was dissolved in tetrahydrofuran (6 mL), the obtained system was then cooled to 0°C, and sodium hydride (0.064 g, 2.67 mmol) was slowly added in portions, the obtained system was reacted for half an hour, then 5-bromo-2-chloropyrimidine (0.2 g, 1.06 mmol) was added, and the obtained system was continuously reacted for 2 h. After the reaction was completed, the reaction liquid was diluted with water, and extracted with ethyl acetate 3 times, the organic phases were combined and then concentrated to obtain 149A (0.22 g of crude product).

LC-MS (ESI): m/z = 245.1 [M+H]⁺.

Step 2: Compound 149A (0.22 g, 0.90 mmol), compound 129C (0.26 g, 0.90 mmol), potassium carbonate (0.31 g, 2.25 mmol) and 1,3-bis(2,6-diisopropylphenyl)imidazol-2-ylidene](3-chloropyridinyl)palladium dichloride (0.061 g, 0.090 mmol) were dissolved in a mixed solvent of 1,4-dioxane (8 mL) and water (2 mL), then the obtained system was subjected to nitrogen replacement 3 times and reacted at 90°C for 3 h. After the reaction was completed, the reaction liquid was diluted with water, and extracted with ethyl acetate 3 times, the organic phases were combined and concentrated, and the resulting residue was purified by preparative HPLC. Purification methods: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.5% ammonium acetate); b. gradient elution, mobile phase A: 30%-70%; c. flow rate: 15 mL/min; d. elution time: 20 min. Compound 149 (100 mg, yield: 27%) was obtained.
¹H NMR (400 MHz, DMSO-*d*₆) δ 9.08 (s, 2H), 8.09 (d, 1H), 7.75-7.70 (m, 1H), 7.64 - 7.53 (m, 2H), 7.14 (d, 1H), 5.57-5.51 (m, 1H), 5.39 (s, 2H), 4.02 - 3.74 (m, 4H), 3.11 - 3.03 (m, 2H), 2.66 - 2.59 (m, 2H), 2.31 - 2.22 (m, 1H), 2.13 - 2.02 (m, 1H);
LC-MS (ESI): m/z = 405.7 [M+H]⁺.

### Example 150

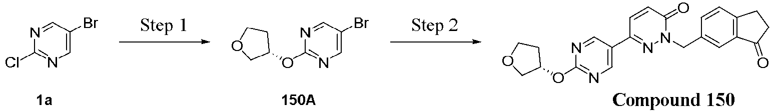

Step 1: (3S)-oxolan-3-ol (0.32 g, 3.64 mmol) was dissolved in tetrahydrofuran (10 mL), the obtained system was then cooled to 0°C, and then sodium hydride (0.16 g, 6.66 mmol) was slowly added in portions, the obtained system was continuously reacted for half an hour, then compound 1a (0.5 g, 2.60 mmol) was added, and the obtained system was continuously reacted for 2 h. After the reaction was completed, the reaction liquid was diluted with water, and extracted with ethyl acetate 3 times, the organic phases were combined and then concentrated to obtain compound 150A (0.63 g of crude product).

LC-MS (ESI): m/z = 245.1 [M+H]⁺.

Step 2: Compound 150A (0.3 g, 1.22 mmol), compound 129C (0.35 g, 1.22 mmol), 1,3-bis(2,6-diisopropylphenyl)imidazol-2-ylidene](3-chloropyridinyl)palladium dichloride (0.083 g, 0.12 mmol) and tripotassium phosphate (0.52 g, 2.44 mmol) were dissolved in a mixed solvent of 1,4-dioxane (8 mL) and water (2 mL), the obtained system was subjected to nitrogen replacement 3 times, then reacted at 90°C for 3 h. After the reaction was completed, the reaction liquid was diluted with water, and extracted with ethyl acetate 3 times, the organic phases were combined and concentrated, and the resulting residue was purified by preparative HPLC. Purification methods: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.5% ammonium acetate); b. gradient elution, mobile phase A: 30%-70%; c. flow rate: 15 mL/min; d. elution time: 20 min. Compound 150 (210 mg, yield: 42.5%) was obtained.
¹H NMR (400 MHz, DMSO-*d*₆) δ 9.08 (s, 2H), 8.09 (d, 1H), 7.76-7.70 (m, 1H), 7.64 - 7.54 (m, 2H), 7.14 (d, 1H), 5.57-5.51(m, 1H), 5.39 (s, 2H), 4.04 - 3.74 (m, 4H), 3.10 - 3.04 (m, 2H), 2.70 - 2.58 (m, 2H), 2.30-2.22 (m, 1H), 2.11 - 2.02 (m, 1H);
LC-MS (ESI): m/z = 405.7 [M+H]⁺.

### Example 151

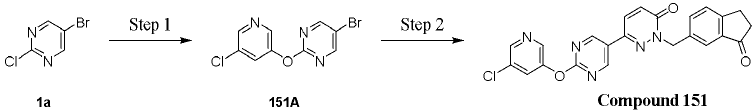

Step 1: 5-chloropyridin-3-ol (0.5 g, 3.86 mmol), compound 1a (0.90 g, 4.63 mmol) and potassium carbonate (1.33 g, 9.62 mmol) were dissolved in N,N-dimethylformamide (10 mL) and then the obtained system was reacted at 80°C for 4 h. After the reaction was completed, the reaction liquid was diluted with water, and extracted with ethyl acetate 3 times, the organic phases were combined and concentrated, the resulting residue was slurried with petroleum ether, the solid was collected by filtration and dried to obtain compound 151A (0.63 g, yield: 57%).

LC-MS (ESI): m/z = 286.0 [M+H]⁺.

Step 2: Compound 151A (0.2 g, 0.70 mmol), compound 129C (0.20 g, 1.70 mmol), tripotassium phosphate (0.3 g, 1.41 mmol) and 1,3-bis(2,6-diisopropylphenyl)imidazol-2-ylidene](3-chloropyridinyl)palladium dichloride (0.048 g, 0.070 mmol) were dissolved in a mixed solvent of 1,4-dioxane (8 mL) and water (2 mL), the obtained system was subjected to nitrogen replacement 3 times and then reacted at 90°C for 3 h. After the reaction was completed, the reaction liquid was diluted with water, and extracted with ethyl acetate 3 times, the organic phases were combined and concentrated, and the resulting residue was purified by preparative HPLC. Purification methods: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.5% ammonium acetate); b. gradient elution, mobile phase A: 30%-70%; c. flow rate: 15 mL/min; d. elution time: 20 min. Compound 151 (60 mg, yield: 19.2%) was obtained.
¹H NMR (400 MHz, DMSO-*d*₆) δ 9.16 (s, 2H), 8.59 (d, 2H), 8.14 - 8.05 (m, 2H), 7.77-7.70 (m, 1H), 7.64 - 7.54 (m, 2H), 7.17 (d, 1H), 5.40 (s, 2H), 3.11 - 3.03 (m, 2H), 2.65 - 2.58 (m, 2H);
LC-MS (ESI): m/z = 446.1 [M+H]⁺.

### Example 152

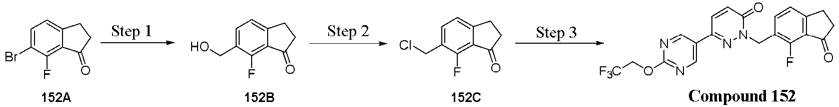

Step 1: Compound 152A (1 g, 4.4 mmol), tri-n-butyltin methanol (1.5 g, 4.6 mmol) and X-Phos Pd G2 (345 mg, 0.44 mmol) were dissolved in 1,4-dioxane (30 mL), the reaction system was subjected to nitrogen replacement, and heated to 90°C, and reacted for 16 h. The reaction system was cooled to room temperature and concentrated, the resulting residue was purified by column chromatography (petroleum ether : ethyl acetate (v : v)=2 : 1) to obtain compound 152B (510 mg, yield: 64%).

LCMS (ESI): m/z = 181.1 [M+H]⁺.

Step 2: Compound 152B (300 mg, 1.7 mmol) was dissolved in a mixed solvent of dichloromethane/thionyl chloride (v : v = 2 : 1, 5 mL) and the obtained system was reacted overnight at room temperature. The reaction system was concentrated to give compound 152C (340 mg of crude product), which was used directly for the next reaction.

LCMS (ESI): m/z = 199.1 [M+H]⁺.

Step 3: Compound 152C (340 mg of crude product), compound 1D (300 mg, 1.1 mmol) and cesium carbonate (652 mg, 2 mmol) were dissolved in acetonitrile (20 mL) and the system was heated to 90°C and reacted overnight. The reaction system was cooled to room temperature and concentrated, the residue was separated by column chromatography (petroleum ether : ethyl acetate (v : v)=1 : 2) to obtain compound 152 (37 mg, yield: 8%).
¹H NMR (400 MHz, DMSO-*d*₆) δ 9.10 (s, 2H), 8.14 - 8.10 (m, 1H), 7.74 - 7.68 (m, 1H), 7.38 -7.33 (m, 1H), 7.18 - 7.12 (m, 1H), 5.41 (s, 2H), 5.15 - 5.06 (m, 2H), 3.12 - 3.04 (m, 2H), 2.68 - 2.62 (m, 2H);
LCMS (ESI): m/z = 435.1 [M+H]⁺.

### Example 153

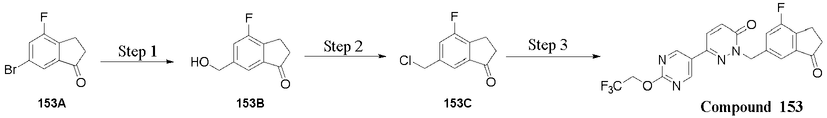

Step 1: Compound 153A (1 g, 4.4 mmol), tri-n-butyltin methanol (1.5 g, 4.6 mmol) and X-Phos Pd G2 (345 mg, 0.44 mmol) were dissolved in 1,4-dioxane (30 mL), the reaction system was subjected to nitrogen replacement, and heated to 90°C, and reacted for 16 h. The reaction system was cooled to room temperature and concentrated, the residue was separated by column chromatography (petroleum ether : ethyl acetate (v : v)=2 : 1) to obtain compound 153B (610 mg, yield: 76%).

LCMS (ESI): m/z = 181.1 [M+H]⁺.

Step 2: Compound 153B (300 mg, 1.7 mmol) was dissolved in a mixed solvent of dichloromethane/thionyl chloride (v : v = 2 : 1, 5 mL) and the obtained system was reacted overnight at room temperature. The reaction system was concentrated to give compound 153C (330 mg of crude product), which was used directly for the next reaction.

LCMS (ESI): m/z = 199.1 [M+H]⁺.

Step 3: Compound 153C (330 mg of crude product), compound 1D (300 mg, 1.1 mmol) and cesium carbonate (652 mg, 2 mmol) were dissolved in acetonitrile (20 mL) and the system was heated to 90°C and reacted overnight. The reaction system was cooled to room temperature and concentrated, the residue was separated by column chromatography (petroleum ether : ethyl acetate (v : v)=1 : 2) to obtain compound 153 (42 mg, yield: 9%).
¹H NMR (400 MHz, DMSO-*d*₆) δ 9.16 (s, 2H), 8.17 - 8.11 (m, 1H), 7.61 - 7.55 (m, 1H), 7.48 (s, 1H), 7.20 - 7.15 (m, 1H), 5.41 (s, 2H), 5.16 - 5.06 (m, 2H), 3.11 - 3.03 (m, 2H), 2.72 - 2.64 (m, 2H);
LCMS (ESI): m/z = 435.1 [M+H]⁺.

### Example 154

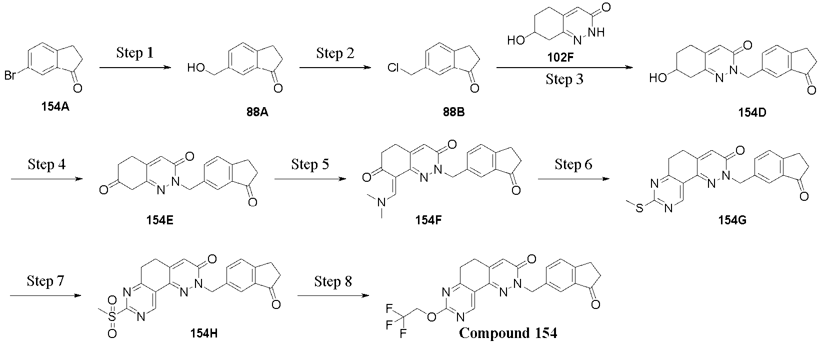

Step 1: Compound 154A (8 g, 37.90 mmol), tri-n-butyltin methanol (13.39 g, 41.69 mmol) and chloro(2-dicyclohexylphosphino-2',4',6'-triisopropyl-1,1'-biphenyl)[2-(2'-amino-1,1'-biphenyl)]palladium(II)(Xphos Pd G2) (2.98 g, 3.79 mmol) were added to a reaction flask, and dissolved with 1,4-dioxane (100 mL) and then the obtained system was reacted at 90°C for 16 h. After cooling, the reaction liquid was concentrated under reduced pressure, and the resulting residue was purified by silica gel column chromatography (petroleum ether : ethyl acetate (v : v)=5 : 1) to obtain compound 88A (4 g, yield: 65%).

LCMS (ESI): m/z = 163.2 [M+H]⁺.

Step 2: Compound 88A (1 g, 6.17 mmol) was dissolved in dichloromethane (10 mL), then thionyl chloride (0.54 mL, 7.40 mmol) was added dropwise at 0°C under nitrogen protection, and the obtained system was reacted at room temperature for 5 h. After the reaction was completed, it was quenched with saturated sodium bicarbonate solution, the obtained system was extracted with dichloromethane, the organic phase was collected, washed and concentrated, and the resulting residue was purified by column chromatography (petroleum ether : ethyl acetate (v/v)=5 : 1) to obtain compound 88B (490 mg, yield: 43%).

LCMS (ESI): m/z = 181.1 [M+H]⁺.

Step 3: Compound 88B (390 mg, 2.16 mmol) was used as raw material, and compound 154D (0.4 g, yield: 62%) was obtained with reference to the operation in step 6 of Example 102.

LCMS (ESI): m/z = 311.2 [M+H]⁺.

Step 4: Compound 154D (390 mg, 2.16 mmol) was used as raw material, and a solution of compound 154E in dichloromethane was obtained with reference to the operation in step 7 of Example 102.

LCMS (ESI): m/z = 309.2 [M+H]⁺.

Step 5: A solution of compound 154E in dichloromethane was used as raw material, and compound 154F (0.4 g of crude product) was obtained with reference to the operation in step 8 of Example 102, and used directly for the next reaction.

LCMS (ESI): m/z = 364.4 [M+H]⁺.

Step 6: Compound 154F (0.4 g) was used as raw material, and compound 154G (50 mg, yield: 11%) was obtained with reference to the operation in step 9 of Example 102.

LCMS (ESI): m/z = 391.3 [M+H]⁺.

Step 7: Compound 154G (50 mg, 0.13 mmol) was used as raw material, and compound 154H (26 mg, yield: 47%) was obtained with reference to the operation in step 10 of Example 102.

LCMS (ESI): m/z = 423.1 [M+H]⁺.

Step 8: Compound 154H (26 mg, 0.06 mmol) was used as raw material, and compound 154 (12.34 mg, yield: 44%) was obtained with reference to the operation in step 11 of Example 102.
¹H NMR (400 MHz, CDCl₃) δ 9.07 (s, 1H), 7.75 (s, 1H), 7.4-7.71 (m, 1H), 7.47 (d, 1H), 6.80 (s, 1H), 5.42 (s, 2H), 4.86 (q, 2H), 312 (t, 2H), 3.06-3.02 (m, 2H), 2.98-2.94 (m, 2H), 2.70-2.67 (m, 2H);
LCMS (ESI): m/z = 443.0 [M+H]⁺.

### Example 155

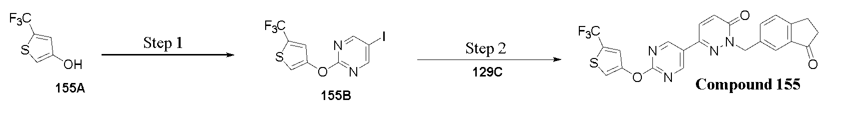

Step 1: Compound 155A (0.25 g, 1.47 mmol), 2-chloro-5-iodopyrimidine (0.35 g, 1.47 mmol) and potassium carbonate (0.61 g, 4.41 mmol) were dissolved in acetonitrite (20 mL), and after the addition was completed, the obtained system was heated under reflux and reacted for 3 h. The reaction was monitored by TLC, after thereaction was completed, the reaction liquid was cooled to room temperature and concentrated under reduced pressure, the resulting residue was purified by column chromatography (ethyl acetate: petroleum ether (v:v) = 1:40) to obtain compound 155 B (0.40 g, yield: 73.13%).

LC-MS (ESI): m/z = 373.0 [M+H]⁺.

Step 2: Compound 155B (200 mg, 0.54 mmol), compound 129C (150 mg, 0.54 mmol), potassium phosphate (230 mg, 1.08 mmol) and [1,3-bis(2,6-diisopropylphenyl)imidazol-2-ylidene](3-chloropyridinyl)palladium(II) dichloride (37 mg, 0.054 mmol) were added into a 50 mL three-necked flask in sequence, then 1,4-dioxane (10 mL) and water (2 mL) were added, the obtained system was subjected to nitrogen replacement and reacted at 90°C for 4 h. The reaction was monitored by LCMS, when the reaction was completed, the system was concentrated directly, the resulting residue was purified by silica gel column chromatography (petroleum ether : ethyl acetate (v : v)=1 : 2) to obtain compound 155 (13 mg, yield: 4.97%).
¹H NMR (400 MHz, CDCl₃) δ 8.96 (s, 2H), 7.83 - 7.68 (m, 2H), 7.61 (d, 1H), 7.51 - 7.34 (m, 3H), 7.09 (d, 1H), 5.45 (s, 2H), 3.20 - 3.02 (m, 2H), 2.74 - 2.60 (m, 2H);
LC-MS (ESI): m/z = 485.0 [M+H]⁺.

### Example 156

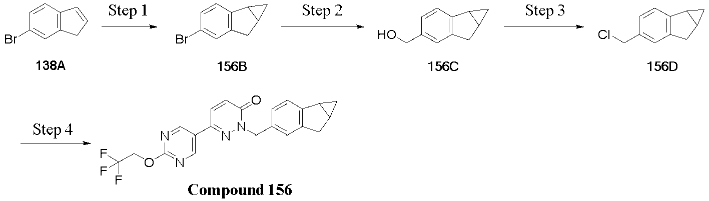

Step 1: Diethylzinc (10.28 mL, 10.28 mmol, 1 M) solution was added to dichloromethane (10 mL), the obtained system was subjected to nitrogen protection and cooled to 0°C, and then a solution of trifluoroacetic acid (1.17 g, 10.28 mmol) in dichloromethane (5 mL) was added, the obtained system was reacted for half an hour, then a solution of diiodomethane (2.75 g, 10.28 mmol) in dichloromethane (5 mL) was further added and reacted for half an hour, a solution of compound 138A (1 g, 5.14 mmol) in dichloromethane (5 mL) was further added, the obtained system was then slowly warmed to room temperature and reacted for 2 h. The reaction liquid was concentrated directly, and the resulting residue was purified by column chromatography (ethyl acetate : petroleum ether (v : v)=0%-5%) to obtain compound 156B (0.7 g, yield: 63.96%).

¹H NMR (400 MHz, DMSO-*d*₆) δ 7.32 (s, 1H), 7.23 (d, 2H), 3.16 - 3.08 (m, 1H), 2.94 - 2.84 (m, 1H), 2.40 - 2.30 (m, 1H), 1.93 - 1.82 (m, 1H), 1.11 - 1.05 (m, 1H), -0.02 - -0.09 (m, 1H).

Step 2: Compound 156B (0.2 g, 1.01 mmol), (tributyltin)methanol (0.39 g, 1.21 mmol) and chloro(2-dicyclohexylphosphino-2',4',6'-triisopropyl-1,1'-biphenyl)[2-(2'-amino-1,1'-biphenyl)]palladium(II) (0.016 g, 0.020 mmol) were added to the solvent 1,4-dioxane (3 mL) in sequence, the obtained system was then subjected to nitrogen replacement 3 times, raised to 90°C and reacted under stirring for 3 h. After the reaction was completed, the reaction liquid was cooled to room temperature and concentrated, the resulting residue was purified by column chromatography (ethyl acetate : petroleum ether (v : v)=0%-10%) to obtain compound 156C (0.1 g, yield: 64.34%).

¹H NMR (400 MHz, DMSO-*d*₆) δ 7.21 (d, 1H), 7.08 (s, 1H), 7.03 - 6.96 (m, 1H), 4.99 (s, 1H), 4.40 (s, 2H), 3.15 - 3.05 (m, 1H), 2.85 (d, 1H), 2.36 - 2.30 (m, 1H), 1.91 - 1.79 (m, 1H), 1.09 - 1.01 (m, 1H), 0.92 - 0.85 (m, 1H), -0.11 - -0.19 (m, 1H).

Step 3: 156C (0.1 g, 0.62 mmol) was dissolved in dichloromethane (10 mL), thionyl chloride (0.096 g, 0.81 mmol) was added, and the obtained system was stirred at room temperature for 2 h. After the reaction was completed, the obtained system was concentrated under reduced pressure to obtain 156D (0.11 g of crude product), which was used directly for the next reaction.

Step 4: Compound 1D (0.17 g, 0.62 mmol), 156D (0.11 g, 0.62 mmol) and potassium carbonate (0.26 g, 1.86 mmol) were dissolved in N,N-dimethylformamide (10 mL) and then the obtained system was stirred at 80°C for 2 h. After cooling, the reaction liquid was concentrated, water (10 mL) was added to the resulting product, and ethyl acetate was used for extraction (15 mL×3), the organic phase was collected and concentrated, and the resulting residue was purified by column chromatography (petroleum ether : ethyl acetate (v : v) =10%-30%) to obtain compound 156 (0.09 g, yield: 35.03%).

¹H NMR (400 MHz, DMSO-*d*₆) δ 9.14 (s, 2H), 8.11 (d, 1H), 7.31 (s, 1H), 7.14 (d, 1H), 7.10 (d, 2H), 5.24 (s, 2H), 5.15 - 5.05 (m, 2H), 3.10 - 3.01 (m, 1H), 2.84 (d, 1H), 2.38 - 2.31 (m, 1H), 1.90 - 1.78 (m, 1H), 1.11 - 1.01 (m, 1H), -0.08 - -0.12 (m, 1H).

### Example 157

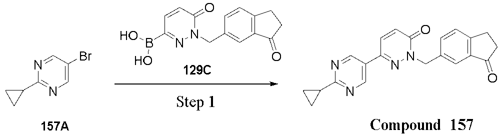

Step 1: Compound 157A (0.2 g, 1.00 mmol), compound 129C (0.31 g, 1.1 mmol), chloro(2-dicyclohexylphosphino-2',4',6'-triisopropyl-1,1'-biphenyl)[2-(2'-amino-1,1'-biphenyl)]palladium(II) (XPhos Pd G2) (0.079 g, 0.10 mmol), cuprous chloride (0.11 g, 1.1 mmol) and potassium trimethylsilanolate (0.19 g, 1.5 mmol) were dissolved in anhydrous N,N-dimethylformamide (3 mL), and the obtained system was reacted at 90°C for 3 h under nitrogen protection. Ethyl acetate and water were added for extraction, the organic phase was collected, dried and concentrated, and the resulting residue was purified by preparative HPLC. Method: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.05% aqueous ammonia); b. gradient elution, mobile phase A: 40%-100%; c. flow rate: 15 mL/min; d. elution time: 22 min. Compound 157 (20 mg, yield: 5.58%) was obtained.
¹H NMR (400 MHz, DMSO-*d*₆) δ 9.08 (s, 2H), 8.10 (d, 1H), 7.73 - 7.71(m, 1H), 7.61 (d, 1H), 7.57 (d, 1H), 7.14 (d, 1H), 5.40 (s, 2H), 3.12 - 3.01 (m, 2H), 2.66 - 2.58 (m, 2H), 2.30 - 2.23 (m, 1H), 1.13 - 1.09 (m, 2H), 1.07 - 1.03 (m, 2H);
LC-MS (ESI): m/z = 359.4 [M+H]⁺.

### Example 158

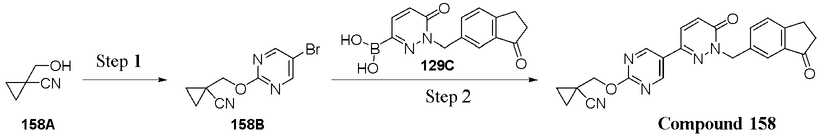

Step 1: Compound 158A (0.23 g, 2.37 mmol) and 5-bromo-2-chloropyrimidine (0.4 g, 2.12 mmol) were dissolved in anhydrous tetrahydrofuran (3 mL), the system was stirred in an ice bath, and cooled to 0°C, and sodium hydride (0.10 g, 2.54 mmol) was added slowly, and then the obtained system was warmed to room temperature and stirred. After the reaction was completed, the reaction liquid was concentrated to remove the solvent, water was added to the residue, the obtained system was stirred and then filtered, the filter cake was collected and dried to obtain compound 158B (0.5 g, yield: 92.8%).

LC-MS (ESI): m/z = 254.0, 256.0 [M+H]⁺.

Step 2: Compound 158B (0.254 g, 1.0 mmol), compound 129C (0.31 g, 1.09 mmol), [1,3-bis(2,6-diisopropylphenyl)imidazol-2-ylidene](3-chloropyridinyl)palladium dichloride (0.068 g, 0.10 mmol) and potassium carbonate (0.27 g, 1.93 mmol) were dissolved in 1,4-dioxane (3 mL) and water (1 mL), and the obtained system was reacted at 90°C for 3 h under nitrogen protection. After cooling, the reaction liquid was filtered, and the filtrate was collected and concentrated, and the resulting residue was purified by preparative HPLC. Method: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.05% aqueous ammonia); b. gradient elution, mobile phase A: 40%-100%; c. flow rate: 15 mL/min; d. elution time: 21 min. Compound 158 (11 mg, yield: 2.66%) was obtained.
¹H NMR (400 MHz, DMSO-*d*₆) δ 9.10 (s, 2H), 8.11 (d, 1H), 7.74 - 7.72(m, 1H), 7.67 - 7.54 (m, 2H), 7.15 (d, 1H), 5.40 (s, 2H), 4.42 (s, 2H), 3.11 - 3.03 (m, 2H), 2.66 - 2.58 (m, 2H), 1.43 - 1.35 (m, 2H), 1.29 - 1.21 (m, 2H);
LC-MS (ESI): m/z = 414.5 [M+H]⁺.

### Example 159 and example 160

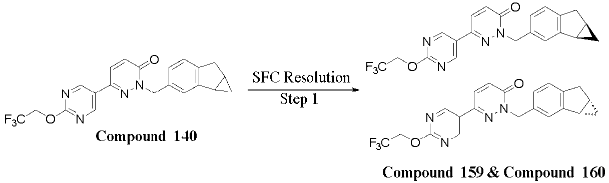

Compound 140 (300.0 mg) was subjected to chiral resolution to obtain P1 (appearance time: 13.61-14.72 min, set as compound 159) and P2 (appearance time: 15.11-16.33 min, set as compound 160). Preparation method: instrument: SFC Prep 150 AP; column: Daicel AD-H (19 mm * 250 mm); mobile phase: A for CO2; B for ethanol; gradient: 25% B gradient elution; flow rate: 40 mL/min; column temperature: 25°C; wavelength: 220 nm; sample preparation: acetonitrile solution. After the separation, the system was dried and concentrated by a rotary evaporator at a bath temperature of 35°C, and then the solvent was dried by a lyophilizer at - 80°C to obtain compound 159 (125 mg, yield: 41.67%) and compound 160 (130 mg, yield: 43.33%).

Compound 159: ¹H NMR (400 MHz, DMSO-*d*₆) δ 9.15 (s, 2H), 8.11 (d, 1H), 7.31 (s, 1H), 7.14 (d, 1H), 7.10 (s, 2H), 5.26 (s, 2H), 5.14-5.08 (m, 2H), 3.09-3.03 (m, 1H), 2.86-2.81 (m, 1H), 2.35-2.33 (m, 1H), 1.90 - 1.80 (m, 1H), 1.07-1.02 (m, 1H), -0.09 - -0.11 (m, 1H);
LC-MS (ESI): m/z = 415.5 [M+H]⁺.

Compound 160: ¹H NMR (400 MHz, DMSO-*d*₆) δ 9.15 (s, 2H), 8.11 (d, 1H), 7.31 (s, 1H), 7.14 (d, 1H), 7.10 (s, 2H), 5.26 (s, 2H), 5.14-5.08 (m, 2H), 3.09-3.03 (m, 1H), 2.86-2.81 (m, 1H), 2.35-2.33 (m, 1H), 1.89-1.80 (m, 1H), 1.09 - 0.96 (m, 1H), -0.09 - -0.11 (m, 1H);
LC-MS (ESI): m/z = 415.5 [M+H]⁺.

### Example 161 and example 162

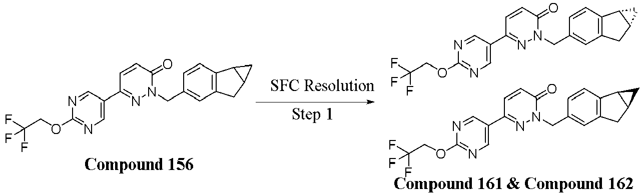

Compound 156 (220.0 mg) was subjected to chiral resolution to obtain P1 (appearance time: 12.56-13.72 min, set as compound 161) and P2 (appearance time: 14.21-15.36 min, set as compound 162). Resolution method: instrument: SFC Prep 150 AP; chromatographic column: Daicel IG-H (19 mm×250 mm); mobile phase: A for CO₂; B for methanol; gradient: isocratic elution, mobile phase B: 40%; flow rate: 40 mL/min; column temperature: 25°C; wavelength: 220 nm; sample preparation: methanol solution. After the separation, the system was dried and concentrated by a rotary evaporator at a bath temperature of 35°C, and then the solvent was dried by a lyophilizer at -80°C to obtain compound 161 (120 mg, yield: 41.67%) and compound 160 (90 mg, yield: 43.33%).

Compound 161: ¹H NMR (400 MHz, DMSO-*d*₆) δ 9.14 (s, 2H), 8.10 (d, 1H), 7.24 (d, 1H), 7.20 - 7.09 (m, 3H), 5.24 (s, 2H), 5.14 - 5.07 (m, 2H), 3.11 - 3.05(m, 1H), 2.84 (d, 1H), 2.37 - 2.28 (m, 1H), 1.89 - 1.79 (m, 1H), 1.05 - 1.01 (m, 1H), - 0.11- -0.14 (m, 1H);
LC-MS (ESI): m/z = 415.0 [M+H]⁺.

Compound 162: ¹H NMR (400 MHz, DMSO-*d*₆) δ 9.14 (s, 2H), 8.10 (d, 1H), 7.24 (d, 1H), 7.19 - 7.09 (m, 3H), 5.24 (s, 2H), 5.14-5.07 (m, 2H), 3.11-3.04 (m, 1H), 2.84 (d, 1H), 2.36 - 2.29 (m, 1H), 1.88 - 1.80 (m, 1H), 1.07 -1.01(m, 1H), - 0.08 - -0.12(m, 1H);
LC-MS (ESI): m/z = 415.1 [M+H]⁺.

### Example 163

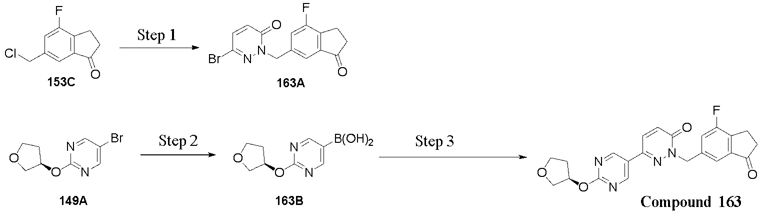

Step 1: Compound 153C (4 g, 20 mmol) was dissolved in anhydrous acetonitrile (40 mL), 6-bromopyridazin-3(2H)-one (4 g, 22.8 mmol) and potassium carbonate (5.5 g, 40 mmol) were added, and the obtained system was reacted at 70°C for 4 h. The system was cooled to room temperature, filtered, the filter cake was washed with ethyl acetate, the filtrate was collected, concentrated, and the resulting residue was purified by silica gel column chromatography (petroleum ether : ethyl acetate (v : v)=1 : 0-3 : 1) to obtain compound 163A (5.62 g, yield: 83.4%).

LC-MS (ESI): m/z = 337.2, 339.2 [M+H]⁺.

Step 2: Compound 149A (0.35 g, 1.43 mmol), potassium acetate (0.28 g, 2.86 mmol), bis(pinacolato)diboron (0.51 g, 2.00 mmol) and chloro(2-dicyclohexylphosphino-2',4',6'-triisoporphyrinyl-1,1'-biphenyl)[2-(2'-amino-1,1'-biphenyl)]palladium(II) (0.11 g, 0.14 mmol) were dissolved in 1,4-dioxane (8 mL), the obtained system was subjected to nitrogen replacement 3 times, then reacted at 95°C for 3 h. After the reaction was completed, the reaction liquid was filtered, and the filtrate was concentrated to obtain compound 163B (0.28 g of crude product).

LC-MS (ESI): m/z = 211.2 [M+H]⁺.

Step 3: Compound 163B (0.25 g, 1.19 mmol), 163A (0.44 g, 1.31 mmol), potassium carbonate (0.33 g, 2.38 mmol) and [1,3-bis(2,6-diisopropylphenyl)imidazol-2-ylidene](3-chloropyridinyl)palladium(II) dichloride (0.081 g, 0.12 mmol) were dissolved in 1,4-dioxane (8 mL) and water (2 mL), the obtained system was subjected to nitrogen replacement 3 times and then reacted at 90°C for 2 h. After the reaction was completed, the reaction liquid was concentrated directly, the resulting residue was coarsely purified by silica gel column chromatography (dichloromethane : ethyl acetate (v : v)=4 : 1) to obtain crude compound 163, which was then slurried (petroleum ether : ethyl acetate=1 : 1) to obtain compound 163 (0.16 g, yield: 31.8%).
¹H NMR (400 MHz, DMSO-*d*₆) δ 9.09 (s, 2H), 8.11 (d, 1H), 7.58 (d, 1H), 7.47 (s, 1H), 7.15 (d, 1H), 5.58-5.51 (m, 1H), 5.40 (s, 2H), 4.02 - 3.74 (m, 4H), 3.11 - 3.02 (m, 2H), 2.74 - 2.63 (m, 2H), 2.31-2.21 (m, 1H), 2.13 - 2.02 (m, 1H);
LC-MS (ESI): m/z = 423.2 [M+H]⁺.

### Example 164

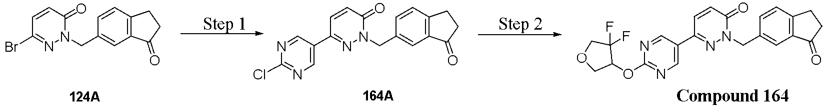

### Step 1:

Compound 124A (3.00 g, 7.52 mmol), 2-chloropyrimidine-5-boronic acid pinacolate (1.99 g, 8.75 mmol) and potassium phosphate (3.19 g, 15.04 mmol) were added to a mixed solution of 1,4-dioxane (50 mL) and water (5 mL) in sequence, the obtained system was subjected to nitrogen replacement 3 times, then [1,3-bis(2,6-diisopropylphenyl)imidazol-2-ylidene](3-chloropyridyl)palladium dichloride (0.51 g, 0.75 mmoL) was further added, and the obtained system was further subjected to nitrogen replacement 3 times, then was raised to 100°C and reacted overnight. After the reaction was completed, the reaction liquid was filtered, dichloromethane and water were added to the filtrate for extraction and liquid separation, the organic phase was collected and concentrated to dryness, and the resulting residue was purified by silica gel column chromatography (methanol : dichloromethane (v : v)=1 : 10) to obtain compound 164A (2.20 g, yield: 82.93%).

LC-MS (ESI): m/z = 353.1 [M+H]⁺.

### Step 2:

Compound 164A (0.1 g, 0.28 mmol), 4,4-difluorotetrahydrofuran-3-ol (0.035 g, 0.28 mmol) and potassium carbonate (0.097 g, 0.70 mmol) were dissolved in acetonitrile (5 mL) and then the obtained system was stirred at 70°C for 8 h. After the reaction was completed, the reaction liquid was cooled to room temperature, filtered, the filtrate was concentrated under reduced pressure, and the resulting residue was purified by reverse phase column chromatography (acetonitrile : water (v : v)=3%-70%) to obtain compound 164 (0.1 g, yield: 81.10%).
¹H NMR (400 MHz, DMSO-*d*₆) δ 9.14 (s, 2H), 8.12 (d, 1H), 7.75-7.72 (m, 1H), 7.62 (s, 1H), 7.57 (d, 1H), 7.15 (d, 1H), 5.76 - 5.56 (m, 1H), 5.40 (s, 2H), 4.43-4.39 (m, 1H), 4.19 - 3.96 (m, 3H), 3.15 - 3.01 (m, 2H), 2.65 - 2.58 (m, 2H);
LC-MS (ESI): m/z = 441.4 [M+H]⁺.

### Example 165

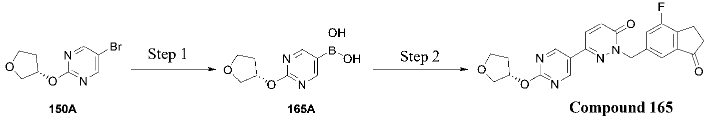

Step 1: 100 mL of eggplant-shaped flask was taken and compound 150A (764 mg, 3.1 mmol), bis(pinacolato)diboron (2.37 g, 9.3 mmol), chloro(2-dicyclohexylphosphino-2',4',6'-triisopropyl-1,1'-biphenyl)[2-(2'-amino-1,1'-biphenyl)]palladium(II) (235 mg, 0.3 mmol) and potassium acetate (1.52 g, 15.5 mmol) were weighed, and dissolved with 1,4-dioxane (30 mL), the obtained system was subjected to nitrogen replacement, and reacted at 90°C for 3 h. After the raw materials were completely converted, the funnel was filled with diatomaceous earth for filtration, and the filtrate was collected and concentrated to obtain compound 165A (1.74 g of crude product), which was used directly for the next reaction.

LC-MS (ESI): m/z = 211.2 [M+H]⁺.

Step 2: 50 mL of eggplant-shaped flask was taken and compound 165A (1.74 g of crude product), compound 163A (674 mg, 2 mmol), potassium phosphate (1.27 g, 6 mmol) and [1,3-bis(2,6-diisopropylphenyl)imidazol-2-ylidene](3-chloropyridinyl)palladium(II) dichloride (139 mg, 0.2 mmol) were added in sequence, then 1,4-dioxane (30 mL) and water (6 mL) were added, the obtained system was subjected to nitrogen replacement and then reacted at 90°C for 4 h. The system was concentrated directly, the resulting residue was purified by silica gel column chromatography (petroleum ether : ethyl acetate (v : v)=1 : 4) to obtain compound 165 (244 mg, total yield for two steps: 28.9%).
¹H NMR (400 MHz, CDCl₃) δ 8.89 (s, 2H), 7.62 - 7.57 (m, 2H), 7.44 - 7.39 (m, 1H), 7.10 - 7.06 (m, 1H), 5.62 - 5.57 (m, 1H), 5.42 (s, 2H), 4.15 - 3.89 (m, 4H), 3.17 - 3.07 (m, 2H), 2.75 - 2.66 (m, 2H), 2.37 - 2.15 (m, 2H);
LC-MS (ESI): m/z = 423.2 [M+H]⁺.

### Example 166

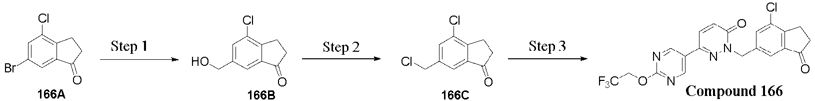

Step 1: Compound 166A (0.6 g, 2.44 mmol), Xphos Pd G2 (0.2 g, 0.25 mmol) and tributyltin methanol (0.78 g, 2.44 mmol) were added into a reaction flask, and dissolved with 1,4-dioxane (20 mL), the obtained system was subjected to nitrogen replacement three times, and reacted at 90°C for 16 h. The reaction was monitored by TLC, when the reaction was completed, the reaction liquid was cooled to room temperature and concentrated under reduced pressure, the resulting residue was purified by column chromatography (petroleum ether : ethyl acetate (v : v)=2 : 1) to obtain compound 166B (0.3 g, yield: 62.53%).

LC-MS (ESI): m/z = 197.1 [M+H]⁺.

Step 2: Compound 166B (0.3 g, 1.53 mmol) was added to a reaction flask, dissolved in dichloromethane (10 mL), then thionyl chloride (0.3 mL) was added, and two drops of DMF were further added, and the obtained system was reacted at room temperature for 2 h. The reaction was monitored by TLC, when the reaction was completed, the reaction liquid was concentrated under reduced pressure, the resulting residue was purified by column chromatography (petroleum ether : ethyl acetate (v : v)=10 : 1) to obtain compound 166C (0.19 g, yield: 57.74%).
¹H NMR (400 MHz, CDCl₃) δ 7.67 - 7.64 (m, 2H), 4.60 (s, 2H), 3.15 - 3.10 (m, 2H), 2.78 - 2.74 (m, 2H);
LC-MS(ESI): m/z = 215.1 [M+H]⁺.

Step 3: Compound 1D (0.19 g, 0.70 mmol), compound 166C (0.19 g, 0.88 mmol) and cesium carbonate (0.45 g, 1.40 mmol) were added to a reaction flask, and dissolved in acetonitrile (10 mL), and then the obtained system was reacted at 80°C for 2 h. The reaction was monitored by TLC, when the reaction was completed, the reaction liquid was cooled to room temperature and concentrated under reduced pressure, and the resulting residue was purified by column chromatography (dichloromethane : ethyl acetate (v : v)=1 : 1) to obtain compound 166 (13 mg, yield: 4.12%).
¹H NMR (400 MHz, DMSO-*d*₆) δ 9.16 (s, 2H), 8.13 (d, 1H), 7.84 (s, 1H), 7.59 (s, 1H), 7.17 (d, 1H), 5.42 (s, 2H), 5.14 - 5.08 (m, 2H), 3.06 - 3.01 (m, 2H), 2.72 - 2.67 (m, 2H);
LC-MS(ESI): m/z = 451.0 [M+H]⁺.

### Example 167

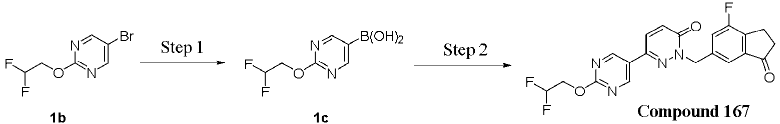

Step 1: Compound 1b (0.3 g, 1.26 mmol), bis(pinacolato)diboron (0.64 g, 2.52 mmol), potassium acetate (0.25 g, 2.52 mmol) and chloro(2-dicyclohexylphosphino-2',4',6'-triisopropyl-1,1'-biphenyl)[2-(2'-amino-1,1'-biphenyl)]palladium(II) (0.099 g, 0.13 mmol) were dissolved in 1,4-dioxane (8 mL), the obtained system was subjected to nitrogen replacement 3 times and then reacted at 95°C for 3 h. After the reaction was completed, the reaction liquid was filtered, and the filtrate was collected and concentrated to obtain compound 1c (0.25 g of crude product).

LC-MS (ESI): m/z = 205.1 [M+H]⁺.

Step 2: Compound 1c (0.25 g, 1.23 mmol), compound 163A (0.46 g, 1.35 mmol), potassium carbonate (0.34 g, 2.46 mmol) and [1,3-bis(2,6-diisopropylphenyl)imidazol-2-ylidene](3-chloropyridinyl)palladium(II) dichloride (0.084 g, 0.12 mmol) were dissolved in 1,4-dioxane (8 mL) and water (2 mL), then the obtained system was subjected to nitrogen replacement 3 times and reacted at 90°C for 2 h. After the reaction was completed, the reaction liquid was concentrated directly and purified by column chromatography (dichloromethane : ethyl acetate=4 : 1) to obtain crude compound 167, which was then purified by HPLC. Purification methods: instrument: waters 2767 preparative liquid chromatographic instrument; chromatographic column: SunFire @ Prep C18 (19 mm × 150 mm); composition of mobile phase: mobile phase A: acetonitrile; mobile phase B: water (containing 0.1% trifluoroacetic acid); gradient: 20%-60% acetonitrile gradient; elution; cycle time: 5 min. Compound 167 (200 mg, yield: 39%) was obtained.
¹H NMR (400 MHz, DMSO-*d*₆) δ 9.13 (s, 2H), 8.12 (d, 1H), 7.58 (d, 1H), 7.48 (d, 1H), 7.16 (d, 1H), 6.62-6.21 (m, 1H), 5.41 (s, 2H), 4.78-4.61 (m, 2H), 3.13 - 3.01 (m, 2H), 2.73 - 2.61 (m, 2H);
LC-MS (ESI): m/z = 417.6 [M+H]⁺.

### Example 168

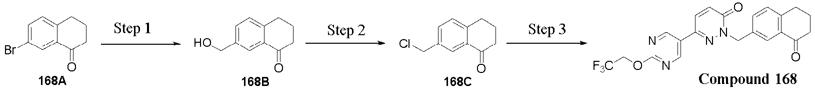

Step 1: Compound 168A (1.0 g, 4.43 mmol), Xphos Pd G2 (0.35 g, 0.44 mmol) and tributyltin methanol (2.1 g, 6.65 mmol) were added into a reaction flask, and dissolved with 1,4-dioxane (10 mL), the obtained system was subjected to nitrogen replacement three times, and reacted at 90°C for 16 h. The reaction was monitored by TLC, when the reaction was completed, the reaction liquid was cooled to room temperature and concentrated under reduced pressure, the resulting residue was purified by column chromatography (petroleum ether : ethyl acetate (v : v)=2 : 1) to obtain compound 168B (0.75 g, yield: 96.15%).
¹H NMR (400 MHz, DMSO-*d*₆) δ 7.83 (s, 1H), 7.49 - 7.46 (m, 1H), 7.31 - 7.29 (m, 1H), 5.26 - 5.23 (m, 1H), 4.51 - 4.50 (m, 2H), 2.93 - 2.90 (m, 2H), 2.62 - 2.56 (m, 2H), 2.07 - 1.99 (m, 2H);
LC-MS (ESI): m/z = 177.3 [M+H]⁺.

Step 2: Compound 168B (0.75 g, 4.26 mmol) was added to a reaction flask, dissolved in dichloromethane (10 mL), then thionyl chloride (0.8 mL) was added, and two drops of DMF were further added, and the obtained system was reacted at room temperature for 2 h. The reaction was monitored by TLC, when the reaction was completed, the reaction liquid was concentrated under reduced pressure, the resulting residue was purified by column chromatography (petroleum ether : ethyl acetate (v : v)=10 : 1) to obtain compound 168C (0.5 g, yield: 63.39%).

¹H NMR (400 MHz, DMSO-*d*₆) δ 7.94 - 7.91 (m, 1H), 7.62 - 7.58 (m, 1H), 7.39 - 7.35 (m, 1H), 4.81 (s, 2H), 2.98 - 2.91 (m, 2H), 2.63 - 2.58 (m, 2H), 2.08 - 2.00 (m, 2H).

Step 3: Compound 1D (0.4 g, 1.46 mmol), compound 168C (0.43 g, 2.19 mmol) and cesium carbonate (0.96 g, 2.92 mmol) were added to a reaction flask, and dissolved in acetonitrile (20 mL), and then the obtained system was reacted at 80°C for 2 h. The reaction was monitored by TLC, when the reaction was completed, the reaction liquid was cooled to room temperature and concentrated under reduced pressure, and the resulting residue was purified by column chromatography (dichloromethane : ethyl acetate (v : v)=1 : 1) to obtain compound 168 (0.29 g, yield: 45.84%).
¹H NMR (400 MHz, DMSO-*d*₆) δ 9.14 (s, 2H), 8.11 (d, 1H), 7.89 (s, 1H), 7.63 - 7.57 (m, 1H), 7.37 - 7.31 (m, 1H), 7.18 - 7.12 (m, 1H), 5.35 (s, 2H), 5.16 - 5.06 (m, 2H), 2.95 - 2.87 (m, 2H), 2.61 - 2.54 (m, 2H), 2.06 - 1.97 (m, 2H);
LC-MS (ESI): m/z = 431.1 [M+H]⁺.

### Example 169

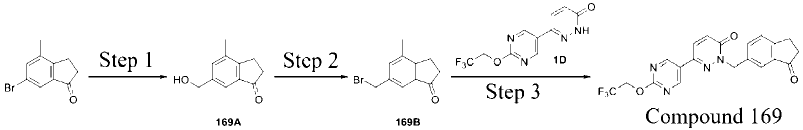

Step 1: 6-bromo-4-methyl-2,3-dihydro-1H-inden-1-one (450 mg, 2 mmol) was dissolved in 1,4-dioxane (20 mL), then (tributyltin)methanol (706 mg, 2.2 mmol) and chloro(2-dicyclohexylphosphino-2',4',6'-triisopropyl-1,1'-biphenyl)[2-(2'-amino-1,1'-biphenyl)]palladium(II) (xphos Pd G2) (156 mg, 0.2 mmol) were added, the obtained system was subjected to nitrogen replacement and then reacted at 90°C for 4 h. After the raw materials disappeared completely, the reaction liquid was concentrated directly, and the resulting residue was purified by silica gel column chromatography (petroleum ether : ethyl acetate (v : v)=1 : 0-3 : 1) to obtain compound 169A (321 mg, yield: 91.2%).

Step 2: Compound 169A (321 mg, 1.8 mmol) was dissolved in dichloromethane (20 mL), then triphenylphosphine (707 mg, 2.7 mmol) and carbon tetrabromide (893 mg, 2.7 mmol) were added in sequence, and the obtained system was stirred at room temperature for 0.5 h. After the reaction was completed, the reaction liquid was concentrated directly, and purified by column chromatography (petroleum ether : ethyl acetate (v : v)=1 : 0-10 : 1) to obtain compound 169B (317 mg, yield: 73.7%).

LC-MS (ESI): m/z = 239.1, 241.1 [M+H]⁺,

Step 3: Compound 169B (317 mg, 1.3 mmol) was dissolved in anhydrous acetonitrile (20 mL), compound 1D (353 mg, 1.3 mmol) and potassium carbonate (276 mg, 2 mmol) were added, and the obtained system was reacted at 60°C for 2 h. After the reaction was completed, the reaction liquid was concentrated directly, and the resulting residue was purified by column chromatography (dichloromethane : ethyl acetate (v : v)=1 : 0-3 : 1) to obtain compound 169 (332 mg, yield: 59.3%).
¹H NMR (400 MHz, CDCl₃) δ 8.93 (s, 2H), 7.67 - 7.50 (m, 3H), 7.09 (s, 1H), 5.42 (s, 2H), 4.93 - 4.83 (m, 2H), 3.05 - 2.93 (m, 2H), 2.72 - 2.67 (m, 2H), 2.36 (s, 3H);
LC-MS (ESI): m/z = 431.0 [M+H]⁺.

### Example 170

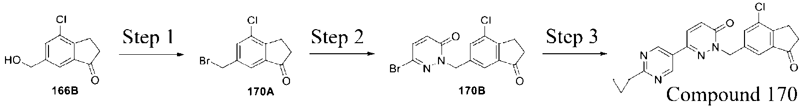

Step 1: Compound 166B (2.98 g, 15.16 mmol) was added to a reaction flask, and dissolved in dichloromethane (50 mL), then carbon tetrabromide (7.54 g, 22.74 mmol) was added, and finally triphenylphosphine (5.96 g, 22.74 mmol) was added, the obtained system was reacted at room temperature for 20 min. The reaction liquid was concentrated under reduced pressure, the resulting residue was purified by silica gel column chromatography (petroleum ether : ethyl acetate (v : v)=10 : 1) to obtain compound 170A (3.10 g, yield: 78.88%).

¹H NMR (400 MHz, DMSO-*d*₆) δ 7.89 - 7.87 (m, 1H), 7.71 - 7.69 (m, 1H), 4.80 (s, 2H), 3.08 - 3.03 (m, 2H), 2.74 - 2.70 (m, 2H).

Step 2: Compound 170A (3.10 g, 11.94 mmol), 6-bromo-3-pyridazinone (2.30 g, 13.13 mmol) and potassium carbonate (3.30 g, 23.88 mmol) were added to a reaction flask, then dissolved in acetonitrile (50 mL) and the obtained system was reacted at 70°C for 3 h. The reaction was monitored by TLC, when the reaction was completed, the reaction liquid was cooled to room temperature and concentrated directly under reduced pressure, and the resulting residue was purified by silica gel column chromatography (dichloromethane : ethyl acetate (v : v)=1 : 1) to obtain compound 170B (4.10 g, yield: 97.16%).
¹H NMR (400 MHz, DMSO-*d*₆) δ 7.74 - 7.72 (m, 1H), 7.62 (d, 1H), 7.49 (s, 1H), 7.00 (d, 1H), 5.29 (s, 2H), 3.07 - 3.00 (m, 2H), 2.73 - 2.67 (m, 2H);
LC-MS (ESI): m/z = 352.9, 354.9 [M+H]⁺.

Step 3: Compound 170B (200 mg, 0.57 mmol), 2-(cyclopropyl)pyrimidine-5-boronic acid pinacolate (210 mg, 0.85 mmol), potassium phosphate (360 mg, 1.71 mmol) and [1,1'-bis(diphenylphosphino)ferrocene]palladium dichloride (44 mg, 0.06 mmol) were dissolved in 1,4-dioxane (10 mL) and water (2 mL), the obtained system was subjected to nitrogen replacement three times, and reacted at 100°C for 3 h. After cooling to the room temperature, the reaction liquid was concentrated directly under reduced pressure, and the resulting residue was purified by silica gel column chromatography (dichloromethane : ethyl acetate (v : v)=1 : 1) to obtain compound 170 (130 mg, yield: 58.56%).
¹H NMR (400 MHz, DMSO-*d*₆) δ 9.09 (s, 2H), 8.12 (d, 1H), 7.84 - 7.82 (m, 1H), 7.58 (s, 1H), 7.16 (d, 1H), 5.41 (s, 2H), 3.05 - 3.01 (m, 2H), 2.71 - 2.67 (m, 2H), 2.30 - 2.23 (m, 1H), 1.13 - 1.08 (m, 2H), 1.08 - 1.04 (m, 2H);
LC-MS (ESI): m/z = 393.4 [M+H]⁺.

### Example 171

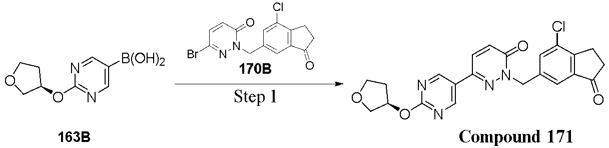

Step 1: Compound 163B (0.25 g, 1.19 mmol), compound 170B (0.46 g, 1.30 mmol), tripotassium phosphate (0.51 g, 2.40 mmol) and [1,3-bis(2,6-diisopropylphenyl)imidazol-2-ylidene](3-chloropyridinyl)palladium(II) dichloride (PEPPSI-IPR catalyst) (0.081 g, 0.12 mmol) were dissolved in 1,4-dioxane (6 mL) and water (2 mL), then the obtained system was subjected to nitrogen replacement 3 times, and reacted at 90°C for 3 h. After the reaction was completed, the reaction liquid was concentrated directly, and the resulting residue was purified by column chromatography (dichloromethane : ethyl acetate (v : v) = 1 : 1) to obtain the crude product, which was then slurried and purified (petroleum ether : ethyl acetate (v : v)=1 : 1) to obtain compound 171 (90 mg, yield: 17.2%).
¹H NMR (400 MHz, DMSO-*d*₆) δ 9.09 (s, 2H), 8.13-8.05 (m, 1H), 7.85-7.80 (m, 1H), 7.61-7.55 (m, 1H), 7.18-7.10 (m, 1H), 5.59-5.50( m, 1H), 5.40 (s, 2H), 4.01 - 3.72 (m, 4H), 3.08 - 2.99 (m, 2H), 2.75 - 2.64 (m, 2H), 2.33-2.21 (m, 1H), 2.15 - 2.01 (m, 1H);
LC-MS (ESI): m/z = 439.4 [M+H]⁺.

### Example 172

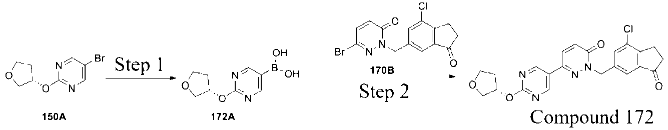

Step 1: Compound 150A (0.24 g, 1 mmol), bis(pinacolato)diboron (0.3 g, 1.2 mmol), potassium acetate (0.29 g, 3 mmol) and 1,1-bis(diphenylphosphino)ferrocene palladium dichloride (0.036 g, 0.05 mmol) were dissolved in 1,4-dioxane (8 mL), the obtained system was subjected to nitrogen replacement 3 times, and then reacted at 90°C overnight. After cooling, the reaction liquid was filtered directly, the filtrate was collected and concentrated to obtain compound 172A (0.3 g of crude product), which was used directly for the next reaction.

LC-MS(ESI): m/z = 211.1 [M+H]⁺.

Step 2: Compound 172A (0.3 g, 1 mmol), compound 170B (0.3 g, 0.85 mmol), potassium phosphate (0.41 g, 2 mmol) and [1,3-bis(2,6-diisopropylphenyl)imidazol-2-ylidene](3-chloropyridinyl)palladium(II) dichloride (PEPPSI-IPR catalyst) (0.068 g, 0.1 mmol) were dissolved in 1,4-dioxane (8 mL) and water (2 mL), then the obtained system was subjected to nitrogen replacement 3 times, and reacted at 90°C for 2 h. After the reaction was completed, the reaction liquid was concentrated directly, and the resulting residue was purified by column chromatography (dichloromethane : ethyl acetate (v : v) = 2 : 1) to obtain the crude product, which was then slurried with ethyl acetate and purified to obtain compound 172 (160 mg, yield: 43%).
¹H NMR (400 MHz, DMSO-*d*₆) δ 9.09 (s, 2H), 8.12-8.09 (m, 1H), 7.84-7.83 (m, 1H), 7.58 (s, 1H), 7.16-7.14 (m, 1H), 5.56-5.53 (m, 1H), 5.40 (s, 2H), 3.96-3.77 (m, 4H), 3.04-3.01 (m, 2H), 2.70-2.67 (m, 2H), 2.29-2.24(m, 1H), 2.08-1.98 (m, 1H);
LC-MS (ESI): m/z = 439.5 [M+H]⁺.

### Example 173

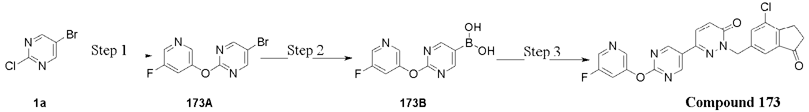

Step 1: 3-fluoro-5-hydroxypyridine (1 g, 8.87 mmol), compound 1a (1.72 g, 8.87 mmol) and potassium carbonate (3.68 g, 26.63 mmol) were dissolved in N,N-dimethylformamide (10 mL) and the obtained system was reacted at 80°C for 3 h. After the reaction was completed, the reaction liquid was cooled to room temperature, and concentrated under reduced pressure, a large amount of solid precipitated when 30 mL of water was added, filtration was performed, the filter cake was collected and washed twice with water (10 mL), then dried to obtain compound 173A (1.8 g of crude product), which was used directly for the next reaction.

LC-MS (ESI): m/z = 269.9 [M+H]⁺.

Step 2: Compound 173A (0.5 g, 1.85 mmol), bis(pinacolato)diboron (0.56 g, 2.22 mmol), potassium acetate (0.54 g, 5.55 mmol) and chloro(2-dicyclohexylphosphino-2',4',6'-triisopropyl-1,1'-biphenyl)[2-(2'-amino-1,1'-biphenyl)]palladium(II) (0.15 g, 0.19 mmol) were dissolved in 1,4-dioxane (10 mL), the obtained system was reacted under stirring at 90°C for 3 h under nitrogen protection. The reaction liquid was cooled to room temperature, the funnel was filled with diatomaceous earth for filtration, the filter cake was washed once with methanol (20 mL), the filtrate was collected and concentrated under reduced pressure to obtain compound 173B (1 g of crude product), which was used directly for the next reaction.

LC-MS (ESI): m/z = 236.1 [M+H]⁺.

Step 3: Compound 170B (0.2 g, 0.57 mmol), compound 173B (0.14 g, 0.63 mmol), 1,3-bis(2,6-diisopropylphenyl)imidazol-2-ylidene](3-chloropyridinyl)palladium dichloride (0.039 g, 0.057 mmol) and potassium carbonate (0.24 g, 1.71 mmol) were dissolved in a mixed solvent of 1,4-dioxane (5 mL) and water (1 mL), and then the obtained system was subjected to nitrogen replacement three times and reacted at 90°C for 3 h. After the reaction was completed, the reaction liquid was cooled to room temperature and concentrated under reduced pressure, the resulting residue was purified by reverse phase column chromatography (water : acetonitrile (v : v)=3%-60%) to obtain compound 173 (0.020 g, yield: 7.56%).
¹H NMR (400 MHz, DMSO-*d*₆) δ 9.17 (s, 2H), 8.56 (d, 1H), 8.51 (s, 1H), 8.12 (d, 1H), 7.96-7.93 (m, 1H), 7.84 (s, 1H), 7.59 (s, 1H), 7.18 (d, 1H), 5.41 (s, 2H), 3.08 - 3.00 (m, 2H), 2.72 - 2.67 (m, 2H);
LC-MS (ESI): m/z = 464.1 [M+H]⁺.

### Example 174

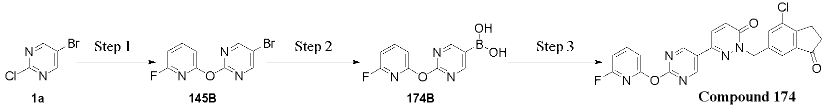

Step 1: 2-fluoro-6-hydroxypyridine (1 g, 8.87 mmol), compound 1a (1.72 g, 8.87 mmol) and potassium carbonate (3.68 g, 26.63 mmol) were dissolved in N,N-dimethylformamide (10 mL) and the obtained system was reacted at 80°C for 3 h. After the reaction was completed, the reaction liquid was cooled to room temperature, and concentrated under reduced pressure, a large amount of solid precipitated when 30 mL of water was added, filtration was performed, the filter cake was collected and washed twice with water (10 mL), then dried to obtain compound 145B (1.7 g of crude product), which was used directly for the next reaction.

LC-MS (ESI): m/z = 269.9 [M+H]⁺.

Step 2: Compound 145B (0.5 g, 1.85 mmol), bis(pinacolato)diboron (0.56 g, 2.22 mmol), potassium acetate (0.54 g, 5.55 mmol) and chloro(2-dicyclohexylphosphino-2',4',6'-triisopropyl-1,1'-biphenyl)[2-(2'-amino-1,1'-biphenyl)]palladium(II) (0.15 g, 0.19 mmol) were dissolved in 1,4-dioxane (10 mL), the obtained system was reacted under stirring at 90°C for 3 h under nitrogen protection. The reaction liquid was cooled to room temperature, the funnel was filled with diatomaceous earth for filtration, the filter cake was washed once with methanol (20 mL), the filtrate was collected and concentrated under reduced pressure to obtain compound 174B (1 g of crude product), which was used directly for the next reaction.

LC-MS (ESI): m/z = 236.1 [M+H]⁺.

Step 3: Compound 170B (0.2 g, 0.57 mmol), compound 174B (0.14 g, 0.63 mmol), 1,3-bis(2,6-diisopropylphenyl)imidazol-2-ylidene](3-chloropyridinyl)palladium dichloride (0.039 g, 0.057 mmol) and potassium carbonate (0.24 g, 1.71 mmol) were dissolved in a mixed solvent of 1,4-dioxane (5 mL) and water (1 mL), and then the obtained system was subjected to nitrogen replacement three times and reacted at 90°C for 3 h. After the reaction was completed, the reaction liquid was cooled to room temperature and concentrated under reduced pressure, the resulting residue was purified by reverse phase column chromatography (water : acetonitrile (v : v)=3%-60%) to obtain compound 174 (0.05 g, yield: 18.91%).
¹H NMR (400 MHz, DMSO-*d*₆) δ 9.19 (s, 2H), 8.23 - 8.11 (m, 2H), 7.85 (s, 1H), 7.60 (s, 1H), 7.29-7.27 (m, 1H), 7.20 - 7.12 (m, 2H), 5.41 (s, 2H), 3.10 - 2.96 (m, 2H), 2.72 - 2.67 (m, 2H);
LC-MS (ESI): m/z = 464.1 [M+H]⁺.

### Example 175

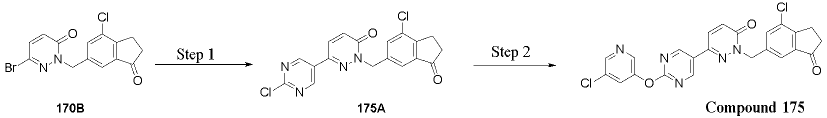

Step 1: Compound 170B (0.5 g, 1.41 mmol), 2-chloropyrimidine-5-boronic acid (0.25 g, 1.55 mmol), 1,3-bis(2,6-diisopropylphenyl)imidazol-2-ylidene](3-chloropyridinyl)palladium dichloride (0.096 g, 0.14 mmol) and potassium carbonate (0.58 g, 4.23 mmol) were dissolved in a mixed solvent of 1,4-dioxane (5 mL) and water (1 mL), then the obtained system was subjected to nitrogen replacement three times, and reacted at 90°C for 3 h. After the reaction was completed, the reaction liquid was cooled to room temperature and concentrated under reduced pressure, and the resulting residue was purified by column chromatography (ethyl acetate : petroleum ether v : v=1%-50%) to obtain compound 175A (0.2 g, yield: 36.63%).

LC-MS (ESI): m/z = 387.4 [M+H]⁺.

Step 2: Compound 175A (0.1 g, 0.26 mmol), 5-chloro-3-hydroxypyridine (0.034 g, 0.26 mmol) and potassium carbonate (0.072 g, 0.52 mmol) were dissolved in N,N-dimethylformamide (5 mL) and the obtained system was reacted at 80°C for 3 h. The reaction liquid was concentrated, ethyl acetate was added, and filtration was performed, the filtrate was collected and concentrated, and the resulting residue was purified by reverse phase column chromatography (acetonitrile : water (v : v)=3%-60%) to obtain compound 175 (0.06 g, yield: 48.24%).
¹H NMR (400 MHz, DMSO-*d*₆) δ 9.17 (s, 2H), 8.59 (d, 2H), 8.13-8.10 (m, 2H), 7.84 (s, 1H), 7.59 (s, 1H), 7.18 (d, 1H), 5.41 (s, 2H), 3.07-3.02 (m, 2H), 2.74 - 2.63 (m, 2H);
LC-MS (ESI): m/z = 480.0 [M+H]⁺.

### Example 176

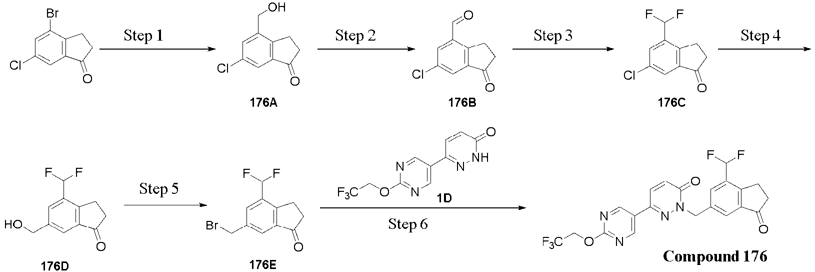

Step 1: 4-bromo-6-chloro-2,3-dihydro-1H-inden-1-one (1.5 g, 6 mmol) was dissolved in 1,4-dioxane (60 mL), then (tributyltin)methanol (2.1 g, 6.6 mmol) and chloro(2-dicyclohexylphosphino-2',4',6'-triisopropyl-1,1'-biphenyl)[2-(2'-amino-1,1'-biphenyl)]palladium(II) (xphos Pd G2) (468 mg, 0.6 mmol) were added, the obtained system was subjected to nitrogen replacement and then reacted at 90°C for 4 h. After cooling, the reaction liquid was concentrated directly, and the resulting residue was purified by column chromatography (petroleum ether : ethyl acetate (v : v)=1 : 0-3 : 1) to obtain compound 176A (756 mg, yield: 64.1%).

LC-MS (ESI): m/z = 197.1 [M+H]⁺.

Step 2: Compound 176A (756 mg, 3.8 mmol) was dissolved in dichloromethane (40 mL), then excess manganese dioxide powder was added, and the obtained system was stirred at room temperature overnight. The reaction liquid was filtered directly, the filter cake was washed with dichloromethane, and the filtrate was concentrated to obtain compound 176B (720 mg of crude product), which was used directly for the next reaction.

LC-MS (ESI): m/z = 195.1 [M+H]⁺.

Step 3: Compound 176B (720 mg, 3.7 mmol) was dissolved in dichloromethane (40 mL), diethylaminosulfur trifluoride (6.0 g, 37 mmol) was added and the obtained system was reacted at room temperature for 3 h. The system was slowly poured into a saturated sodium bicarbonate solution, the obtained system was then subjected to liquid separation and extraction, the organic phase was collected, dried over anhydrous sodium sulfate, and concentrated, the resulting residue was separated by silica gel column chromatography (petroleum ether : ethyl acetate (v : v)= 1 : 0-10 : 1) to obtain compound 176C (544 mg, yield: 67.8%).

LC-MS (ESI): m/z = 217.1 [M+H]⁺.

Step 4: Compound 176C (544 mg, 2.5 mmol) was dissolved in 1,4-dioxane (20 mL), then (tributyltin)methanol (963 mg, 3 mmol) and chloro(2-dicyclohexylphosphino-2',4',6'-triisopropyl-1,1'-biphenyl)[2-(2'-amino-1,1'-biphenyl)]palladium(II) (xphos Pd G2) (156 mg, 0.2 mmol) were added, the obtained system was subjected to nitrogen replacement and then reacted at 90°C for 4 h. After cooling, the reaction liquid was concentrated directly, and the resulting residue was purified by silica gel column chromatography (petroleum ether : ethyl acetate (v : v)=1 : 0-3 : 1) to obtain compound 176D (414 mg, yield: 78.1%).

Step 5: Compound 176D (380 mg, 1.8 mmol) was dissolved in dichloromethane (20 mL), then triphenylphosphine (707 mg, 2.7 mmol) and carbon tetrabromide (893 mg, 2.7 mmol) were added in sequence, and the obtained system was stirred at room temperature for 0.5 h. After the reaction was completed, the reaction liquid was concentrated directly, and the resulting residue was purified by column chromatography (petroleum ether : ethyl acetate (v : v)=1 : 0-10 : 1) to obtain compound 176E (309 mg, yield: 62.4%).

Step 6: Compound 176E (309 mg, 1.1 mmol) was dissolved in anhydrous acetonitrile (20 mL), and compound 1D (353 mg, 1.3 mmol) and potassium carbonate (276 mg, 2 mmol) were added, and the obtained system was reacted at 60°C for 2 h. After the reaction was completed, the reaction liquid was concentrated directly, and the resulting residue was purified by column chromatography (dichloromethane : ethyl acetate (v : v)=1 : 0-3 : 1) to obtain compound 176 (228 mg, yield: 44.5%).
¹H NMR (400 MHz, CDCl₃) δ 8.93 (s, 2H), 7.87 (s, 1H), 7.86 - 7.83 (m, 1H), 7.63 (d, 1H), 7.10 (d, 1H), 6.92 - 6.61 (m, 1H), 5.48 (s, 2H), 4.92 - 4.84 (m, 2H), 3.30 - 3.23(m, 2H), 2.77 - 2.70 (m, 2H);
LC-MS (ESI): m/z = 467.5 [M+H]⁺.

### Example 177

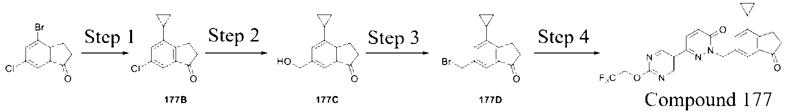

Step 1: 4-bromo-6-chloro-2,3-dihydro-1H-inden-1-one (1.2 g, 4.89 mmol), cyclopropylboronic acid (0.63 g, 7.33 mmol), potassium carbonate (1.35 g, 9.78 mmol) and 1,1-bis(diphenylphosphino)ferrocene palladium(II) dichloride (0.36 g, 0.49 mmol) were dissolved in 1,4-dioxane (16 mL) and water (4 mL), then the obtained system was subjected to nitrogen replacement 3 times, and reacted at 95°C for 5 h. After cooling, the reaction liquid was concentrated directly, the resulting residue was purified by column chromatography (petroleum ether : ethyl acetate (v : v)=12 : 1) to obtain compound 177B (0.88 g, yield: 87%).

LC-MS (ESI): m/z = 207.1 [M+H]⁺.

Step 2: Compound 177B (0.5 g, 2.42 mmol), (tributylstannyl)methanol (1.09 g, 3.39 mmol) and chloro(2-dicyclohexylphosphino-2',4',6'-triisopropyl -1,1'-biphenyl)[2-(2'-amino-1,1'-biphenyl)]palladium(II) (xphos Pd G2) (0.19 g, 0.24 mmol) were dissolved in 1,4-dioxane (10 mL), then the obtained system was subjected to nitrogen replacement 3 times, and reacted at 90°C for 4 h. After cooling, the reaction liquid was concentrated directly, and the resulting residue was purified by column chromatography (petroleum ether : ethyl acetate (v : v)=2 : 1) to obtain compound 177C (0.46 g, yield: 94%).

LC-MS (ESI): m/z = 203.1 [M+H]⁺.

Step 3: Compound 177C (0.1 g, 0.49 mmol) was dissolved in dichloromethane (5 mL), then carbon tetrabromide (0.23 g, 0.69 mmol) and triphenylphosphine (0.18 g, 0.69 mmol) were added and the obtained system was reacted at room temperature for half an hour. After the reaction was completed, the reaction liquid was concentrated directly by spinning to obtain compound 177D (0.12 g of crude product), which was used directly for the next reaction.

LC-MS (ESI): m/z = 265.0 [M+H]⁺.

Step 4: Compound 1D (0.1 g, 0.37 mmol), compound 177D (0.12 g, 0.44 mmol) and potassium carbonate (0.10 g, 0.74 mmol) were dissolved in N,N-dimethylformamide (6 mL) and the obtained system was heated to 80°C and reacted for 3 h. After cooling, water was added to the system, and ethyl acetate was used for extraction 3 times, the organic phases were combined and concentrated, and the resulting residue was purified by preparative HPLC. Method: 1. Instruments: waters 2767 (preparative liquid phase chromatographic instrument); chromatographic column: SunFire @ PrepC18 (19 mm × 250 mm). 2. The sample was filtered with a 0.45 µm filter to prepare a sample solution. 3. Preparative chromatography conditions: a. composition of mobile phases A and B: mobile phase A: acetonitrile; mobile phase B: water (containing 0.05% aqueous ammonia); b. gradient elution, mobile phase A: 40%-100%; c. flow rate: 15 mL/min. Compound 177 (50 mg, yield: 29.5%) was obtained.
¹H NMR (400 MHz, DMSO-*d*₆) δ 9.14 (s, 2H), 8.12 (d, 1H), 7.36 (d, 1H), 7.29 (d, 1H), 7.15 (d, 1H), 5.35 (s, 2H), 5.17-5.05 (m, 2H), 3.15 - 3.06 (m, 2H), 2.69 - 2.60 (m, 2H), 2.05-1.95 (m, 1H), 1.09 - 0.97 (m, 2H), 0.81 - 0.63 (m, 2H);
LC-MS (ESI): m/z = 457.8 [M+H]⁺.

### Biological test

The following Comparative Example 1 of the present invention is as shown below, and is prepared with reference to the method of Example 96 in Patent WO 2021231615,

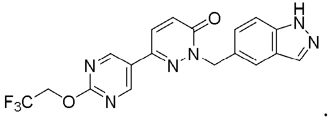

### I. Method for analysis of myosin activity in rabbit skeletal muscle

The purpose of this test was to evaluate the ability of compounds to inhibit the hydrolysis of ATP by rabbit skeletal muscle myosin. The determination method was as follows:
1. Solution formulation:
- Preformed F-actin filaments (# AKF99-B, Cytoskeleton) were diluted to 1 mg/mL with Reaction buffer (25 mM Tris-HCL, pH 7.5 + 35 mM KCl + 0.1 mM EGTA + 1 mM MgCl2);
- Rabbit myosin (# MY02-A, Cytoskeleton) was diluted to 0.1 mg/mL with Resuspension buffer (15 mM Tris-HCL, PH7.5 + 0.2 M KCl + 1 mM MgCl2);
- ATP (# BSA04-001, Cytoskeleton) was diluted to 3 mM working solution with 15 mM Tris-HCL (PH = 7.5);

2. Experimental steps:
- adding 13 µL reaction buffer + 12 µL F-actin to actin control wells;
- adding 10 µL reaction buffer + 12 µL F-actin + 3 µL myosin II to positive control wells;
- adding 10 µL of compound working solution + 12 µL of F-actin + 3 µL of myosin II to the compound wells;
- adding 5 µL of ATP working solution to all test wells to start the reaction, and simultaneously adding a sample to the Pi standard wells according to the instruction (#BK054, Cytoskeleton);
- gently mixing and accurately incubating at 37°C for 60 min;
- adding 70 µL of CytoPhos Reagent (# BK054, Cytoskeleton) to each well and incubating for 10 min to stop the reaction.

3. Detection:
the OD value of each well was detected at 650 nm using the endpoint method. The standard curve was fitted according to Pi concentration and OD value, and the Pi concentration of each test well was obtained. Inhibition (%) = (Pi positive control well-Pi test well)/Pi positive control well * 100%. The Inhibition%-Enzyme concentration curve was fitted with Graphpad Prism 8 software, and IC₅₀ was calculated.
4. Detection results: the compound has an IC₅₀ of less than 1000 µM for inhibitory activity on hydrolysis of ATP by rabbit skeletal muscle myosin, and the specific results of some examples are as shown in Table 1.

**Table 1 Inhibitory activity of compounds on hydrolysis of ATP by rabbit skeletal muscle myosin**

| Compound No. | Rabbit skeletal muscle myosin (IC₅₀, µM) |
|---|---|
| 56 | AA |
| 57 | AA |
| 60 | AA |
| 61 | A |
| 64 | AA |
| 65 | AA |
| 66 | AA |
| 67 | AA |
| 68 | AA |
| 69 | AA |
| 70 | AA |
| 71 | AA |
| 72 | AA |
| 73 | AA |
| 74 | AA |
| 75 | AA |
| 76 | AA |
| 77 | AA |
| 78 | AA |
| 79 | A |
| 80 | AA |
| 81 | AA |
| 82 | AA |
| 83 | AA |
| 84 | AA |
| 85 | A |
| 86 | AA |
| 87 | AA |
| 88 | AA |
| 89 | A |
| 90 | A |
| 91 | A |
| 92 | AA |
| 93 | AA |
| 94 | AA |
| 95 | A |
| 96 | A |
| 98 | AA |
| 99 | AA |
| 100 | AA |
| 101 | AA |
| 102 | AA |
| 104 | AA |
| 105 | AA |
| 106 | AA |
| 107 | AA |
| 108 | AA |
| 109 | AA |
| 110 | AA |
| 112 | AA |
| 113 | AA |
| 115 | AA |
| 120 | AA |
| 122 | A |
| 124 | AA |
| 126 | AA |
| 127 | AA |
| 128 | A |
| 129 | AA |
| 130 | A |
| 131 | AA |
| 132 | AA |
| 133 | AA |
| 134 | AA |
| 135 | AA |
| 136 | AA |
| 137 | AA |
| 138 | AA |
| 139 | AA |
| 140 | AA |
| 141 | AA |
| 142 | A |
| 143 | A |
| 144 | A |
| 145 | AA |
| 146 | A |
| 149 | AA |
| 150 | AA |
| 151 | AA |
| 152 | AA |
| 153 | AA |
| 154 | A |
| 155 | AA |
| 156 | AA |
| 157 | AA |
| 158 | AA |
| 159 | AA |
| 160 | AA |
| 161 | AA |
| 162 | AA |
| 163 | AA |
| 164 | AA |
| 165 | A |
| 166 | AA |
| 167 | AA |
| 168 | A |
| 169 | AA |
| 170 | AA |
| 171 | AA |
| 172 | AA |
| 173 | AA |
| 174 | AA |
| 175 | AA |
| 176 | AA |
| 177 | A |

| | |
|---|---|
| AA denotes 0 µM < IC₅₀ ≤ 2.5 µM, A denotes 2.5 µM < IC₅₀ ≤ 20 µM, B denotes 20 µM < IC₅₀ ≤ 50 µM, C denotes 50 µM < IC₅₀ ≤ 100 µM, and D denotes 100 µM < IC₅₀ ≤ 200 µM. | |

Conclusion: The compounds of the present invention, such as the compounds of the examples, had strong ability to inhibit the hydrolysis of ATP by myosin in rabbit skeletal muscle, and the IC₅₀ of some compounds was less than 1 µM, the IC₅₀ of compound 56 was 0.34 µM, the IC₅₀ of compound 57 was 0.33 µM, the IC₅₀ of compound 60, compound 68 and compound 69 were all 0.1 µM, the IC₅₀ of compound 72 was 0.56 µM, the IC₅₀ of compound 73 was 0.55 µM, the IC₅₀ of compound 74 was 0.29 µM, the IC₅₀ of compound 75 and compound 80 were all 0.5 µM, the IC₅₀ of compound 82 and compound 86 were all 0.2 µM, the IC₅₀ of compound 87 was 0.3 µM, the IC₅₀ of compound 88 was 0.8 µM, the IC₅₀ of compound 92 was 0.2 µM, the IC₅₀ of compound 93 was 0.7 µM, the IC₅₀ of compound 94 was 0.2 µM, the IC₅₀ of compound 98 was 0.2 µM, the IC₅₀ of compound 99 was 0.8 µM, the IC₅₀ of compound 100 was 0.1 µM, the IC₅₀ of compound 101 was 0.1 µM, the IC₅₀ of compound 102 was 0.4 µM, the IC₅₀ of compound 104 was 0.8 µM, the IC₅₀ of compound 105 was 0.9 µM, the IC₅₀ of compound 106 was 0.2 µM, the IC₅₀ of compound 107 was 0.2 µM, the IC₅₀ of compound 108 was 0.2 µM, the IC₅₀ of compound 109 was 0.1 µM, the IC₅₀ of compound 110 was 0.8 µM, the IC₅₀ of compound 120 was 0.7 µM, the IC₅₀ of compound 126 was 0.5 µM, the IC₅₀ of compound 129 was 0.2 µM, the IC₅₀ of compound 131 was 0.4 µM, the IC₅₀ of compound 132 was 0.4 µM, the IC₅₀ of compound 135 was 0.3 µM, the IC₅₀ of compound 137 was 0.3 µM, the IC₅₀ of compound 138 was 0.2 µM, the IC₅₀ of compound 139 was 0.6 µM, the IC₅₀ of compound 140 was 0.8 µM, the IC₅₀ of compound 141 was 0.8 µM, the IC₅₀ of compound 145 was 0.8 µM, the IC₅₀ of compound 151 was 0.7 µM, the IC₅₀ of compound 153 was 0.5 µM, the IC₅₀ of compound 156 was 0.9 µM, the IC₅₀ of compound 159 was 0.4 µM, the IC₅₀ of compound 161 was 0.5 µM, the IC₅₀ of compound 162 was 0.8 µM, the IC₅₀ of compound 167 was 0.27 µM, the IC₅₀ of compound 169 was 0.8 µM, the IC₅₀ of compound 170 was 0.9 µM, the IC₅₀ of compound 171 was 0.9 µM, the IC₅₀ of compound 172 was 1.0 µM, the IC₅₀ of compound 173 was 0.53 µM, the IC₅₀ of compound 175 was 0.53 µM.

### II. Method for analysis of cardiac myosin SII activity

The purpose of this test was to evaluate the ability of compounds to inhibit the hydrolysis of ATP by cardiac myosin SII. The determination method was as follows:
1. Solution formulation:
   - Preformed F-actin filaments (# AKF99-B, Cytoskeleton) were diluted to 1 mg/mL with Reaction buffer (15mM Tris-HCL, PH7.5 + 10mM KCl + 0.1mM EGTA + 2mM MgCl2);
   - Cardiac Muscle myosin (# MY03-A, Cytoskeleton) was diluted to 1 mg/mL with Resuspension buffer (15mM Tris-HCL, PH7.5 + 0.2M KCl + 1mM MgCl2);
   - ATP (# BSA04-001, Cytoskeleton) was diluted to 3 mM working solution with 15 mM Tris-HCL (PH = 7.5);
2. Formulation of working solution of compounds: the final compound concentration was 100 µM
3. Experimental steps:
   - adding 10 µL of compound working solution + 12 µL of Preformed F-actin filaments + 3 µL of Cardiac Muscle myosin to the compound wells;
   - adding 10 µL of reaction buffer containing DMSO + 12 µL Preformed F-actin filaments + 3 µL Cardiac Muscle myosin to positive control wells;
   - adding 10 µL of reaction buffer containing DMSO + 12 µL Preformed F-actin filaments + 3 µL Resuspension buffer to negative control wells;
   - adding 5 µL of 3 mM ATP working solution to all test wells to start the reaction, gently mixing and incubating at 37°C for 2 h;
   - after the reaction was completed, taking 6 µL of reaction liquid, adding 24 µL of reaction buffer for 5-fold dilution, then detecting the content of the product Pi with a phosphate kit (#BK054, Cytoskeleton);
   - adding a sample to Pi standard wells according to the instructions;
   - adding 70 µL of CytoPhos Reagent (# BK054, Cytoskeleton) to each well and incubating at room temperature for 10 min before detection.
4. Detection:
   the OD value of each well was detected at 650 nm. The standard curve was fitted according to Pi standard concentration and OD value, and the Pi concentration of each test well was obtained. Inhibition (%) = (positive control well-test well)/positive control well * 100%.

**Table 2 Inhibition rate of compounds on cardiac myosin SII**

| Compound No. | Cardiac myosin SII (Inhibition @ 100 µM) |
|---|---|
| 57 | 1.5% |
| 67 | 2% |
| 69 | 19% |
| 70 | 18% |
| 71 | 21% |
| 73 | 10.2% |
| 74 | 15.8% |
| 80 | -17.8% |
| 81 | -5.6% |
| 84 | -3.8% |
| 89 | 13.7% |
| 90 | 14.3% |
| 92 | 17% |
| 93 | -16% |
| 95 | 2% |
| 96 | -9% |
| 102 | -5% |
| 104 | 8.9% |
| 105 | 16% |
| 108 | 4.5% |
| 109 | 9.7% |
| 110 | 1% |
| 112 | 2% |
| 113 | 0% |
| 115 | 3% |
| 120 | 14% |
| 124 | 4% |
| 126 | 7% |
| 127 | 8% |
| 128 | -10% |
| 129 | 22% |
| 130 | 3% |
| 132 | 1% |
| 133 | -4% |
| 134 | 1% |
| 136 | -7% |
| 139 | 6% |
| 140 | -6% |
| 141 | 14% |
| 142 | -8% |
| 145 | -8% |
| 149 | 10% |
| 150 | 15% |
| 151 | -2% |
| 155 | 1% |
| 156 | 7% |
| 157 | 10% |
| 158 | 10% |
| 159 | 7% |
| 160 | 8% |
| 161 | 5% |
| 162 | 13% |
| 164 | 4% |
| 166 | 17% |
| 167 | 4% |

Conclusion: The compounds of the present invention, such as the compounds in the examples, have no or very weak inhibitory activity on cardiac myosin SII.

### III. Analysis method for activity of smooth muscle myosin S1 fragments

The purpose of this test was to evaluate the ability of compounds to inhibit the hydrolysis of ATP by smooth muscle myosin S1 fragments. The determination method was as follows:
1. Solution formulation
   - F-actin polymer: Cardiac Actin powder (# CS-AD99, Cytoskeleton) was dissolved in a buffer (5 mM Pipes-KOH solution, PH = 7.0, containing 100 µM ATP and 500 µM DTT) to form a 2 mg/mL solution and the solution was left at room temperature for 30 min; then 2 mM MgCl₂ and 2 mM EGTA were added and the obtained system was left at room temperature for 40 min;
   - Smooth muscle S1 fragment (#CS-MYS05, Cytoskeleton) was diluted with a pre-cooled PM12 buffer containing 1 mM DTT (12 mM Pipes-KOH solution, PH = 7.0, containing 2 mM MgCl₂) to form a 0.7 mg/mL solution;
   - ATP (# BSA04-001, Cytoskeleton) was diluted to 5 mM working solution with 15 mM Tris-HCL (PH = 7.5);
2. Working solution of compounds: the final compound concentration was 100 µM
3. Experimental steps:
   - adding 10 µL of compound working solution + 6 µL of F-actin polymer + 4 µL of Smooth muscle S1 fragment solution to the compound wells;
   - adding 10 µL of PM12 buffer containing DMSO + 6 µL of F-actin polymer + 4 µL of Smooth muscle S1 fragment solution to positive control wells;
   - adding 10 µL of PM12 buffer containing DMSO + 6 µL of F-actin polymer + 4 µL of PM12 buffer to negative control wells;
   - adding 10µL of 5mM ATP working solution to all test wells to start the reaction, gently mixing and incubating at 37°C for 20 min;
   - after the reaction was completed, taking 6 µL of reaction liquid, adding 24 µL of PM12 buffer for 5-fold dilution, then detecting the content of the product Pi with a phosphate kit (#BK054, Cytoskeleton);
   - adding a standard according to the instructions, adding 70 µL of CytoPhos Reagent (# BK054, Cytoskeleton) to all wells and incubating at room temperature for 10 min before detection.
4. Detection:
   the OD value of each well was detected at 650 nm. The standard curve was fitted according to Pi standard concentration and OD value, and the Pi concentration of each test well was obtained. Inhibition (%) = (positive control well-test well)/positive control well * 100%.

**Table 3 Inhibition rate of compounds on smooth muscle myosin S1**

| Compound No. | Smooth muscle myosin S1 (Inhibition @ 100 µM) |
|---|---|
| 57 | 3.3% |
| 67 | 3% |
| 72 | 23.6% |
| 73 | -3% |
| 74 | 5.3% |
| 80 | 9.3% @ 50µM |
| 81 | 10.9% @ 50µM |
| 84 | 20.2% @ 50µM |
| 89 | 17.6% |
| 90 | 12.2% |
| 93 | -14% |
| 95 | -2% |
| 102 | -2% |
| 104 | 20.7% |
| 105 | 16% |
| 108 | -0.9% |
| 109 | -3.5% |
| 110 | -6% |
| 112 | -9% |
| 113 | -13% |
| 115 | 3% |
| 120 | -3% |
| 124 | -12% |
| 126 | 7% |
| 127 | -6% |
| 128 | -2% |
| 129 | -2% |
| 130 | 2% |
| 131 | -9% |
| 133 | 8% |
| 134 | 8% |
| 135 | 8% |
| 136 | -4% |
| 137 | 11% |
| 139 | 6% |
| 140 | 2% |
| 141 | 3% |
| 142 | 2% |
| 145 | 8% |
| 149 | 2% |
| 150 | 4% |
| 151 | 2% |
| 152 | -5% |
| 153 | -12% |
| 155 | 5% |
| 156 | -11% |
| 157 | -3% |
| 158 | 13% |
| 159 | 5% |
| 160 | 3% |
| 162 | 16% |
| 163 | 14% |
| 164 | 17% |
| 165 | 12% |
| 166 | 6% |
| 167 | 17% |

Conclusion: The compounds of the present invention, such as the compounds in the examples, have no or very weak inhibitory activity on smooth muscle myosin S1.

### IV. Pharmacokinetic experiment in mice

1. Experimental animals: Male C57 mice, 22-25 g, 6 mice/compound. Purchased from Chengdu Ddossy Experimental Animals Co., Ltd.
2. Experimental design: On the day of the experiment, 12 C57 mice were randomly grouped according to their body weight. The animals were fasted with water available for 12 to 14 h one day before the administration and were fed 4 h after the administration.

**Table 4.1 Administration information**

| Group | Number | Administration information | | | | | |
|---|---|---|---|---|---|---|---|
| | Male | Test compound | Administra tion dosage (mg/kg) | Administra tion concentrati on (mg/mL) | Administrati on volume (mL/kg) | Collected samples | Mode of administrati on |
| G1 | 3 | Example compounds | 5 | 1 | 5 | Plasma | Intravenous administrati on |
| G2 | 3 | | 10 | 1 | 10 | Plasma | Intragastric administrati on |

| | | | | | | | |
|---|---|---|---|---|---|---|---|
| Note: solvent for intravenous administration: 10% DMA+10% Solutol+80% Saline; vehicle for intragastric administration: 5% DMA + 5% Solutol + 90% Saline (DMA: dimethylacetamide; Solutol: polyethylene glycol-15-hydroxystearate; Saline: physiological saline) | | | | | | | |

Before and after the administration, 0.06 mL of blood was taken from the orbit of the animals under isoflurane anesthesia and placed in an EDTAK2 centrifuge tube. The blood was centrifuged at 5000 rpm and 4°C for 10 min to collect the plasma. The blood collection time points for the intravenous group and intragastric administration group were: 0 min, 5 min, 15 min, 30 min, 1 h, 2 h, 4 h, 7 h and 24 h. Before analysis and detection, all samples were stored at -80°C, and a quantitative analysis of samples was performed using LC-MS/MS.

**Table 4.2 Pharmacokinetic parameters of test compounds in mouse plasma**

| Test compound | Mode of administration | Cₘₐₓ (ng/mL) | AUC₀₋ₜ (h·ng/mL) | T_{1/2} (h) |
|---|---|---|---|---|
| Compound 128 | i.g. (10 mg/kg) | 1091 | 8320 | - |
| Compound 160 | i.g. (10 mg/kg) | 1382 | 8171 | - |
| Compound 162 | i.g. (10 mg/kg) | 688 | 6176 | - |
| Compound 88 | i.g. (10 mg/kg) | 324 | 2318 | 102 |
| Comparative Example 1 | i.g. (10 mg/kg) | 267 | 2214 | 18.9 |

Conclusion: The compounds of the present invention, such as the compounds in the examples, have good pharmacokinetic profiles in mice. For example, T_{1/2} ( i.g., 10 mg/kg) results in mouse plasma: the T_{1/2} of compound 129 is 41.9 h, the T_{1/2} of compound 131 is 91.1 h, the T_{1/2} of compound 135 is 66.8 h, the T_{1/2} of compound 149 is 52.3 h, and the T_{1/2} of compound 150 is 40.7 h.

### V. Pharmacokinetic experiment in rats

1. Experimental animals: male SD rats, about 220 g, 6-8 weeks old, 6 rats/compound. Purchased from Chengdu Ddossy Experimental Animals Co., Ltd.
2. Experimental design: On the day of the test, (6×number of test compounds (n)) SD rats were randomly divided into groups according to body weight. The animals were fasted with water available for 12 to 14 h one day before the administration and were fed 4 h after the administration.

**Table 5 Administration information**

| Group | Number | Administration information | | | | | |
|---|---|---|---|---|---|---|---|
| | Male | Test compound | Administratio n dosage (mg/kg) | Administratio n concentration (mg/mL) | Administrat ion volume (mL/kg) | Collecte d samples | Mode of administr ation |
| G1 | 3 | Example compounds | 2.5 | 0.5 | 5 | Plasma | Intraveno us administr ation |
| G2 | 3 | | 10 | 1 | 10 | Plasma | Intragastr ic administr ation |

| | | | | | | | |
|---|---|---|---|---|---|---|---|
| Note: Solvent for intravenous administration: 5%DMA+5%Solutol+90%Saline; vehicle for intragastric administration: 0.5% MC (MC: methylcellulose) | | | | | | | |

Before and after the administration, 0.15 mL of blood was taken from the orbits under isoflurane anesthesia and placed in an EDTAK2 centrifuge tube. The blood was centrifuged at 5000 rpm at 4°C for 10 min, and plasma was collected. The blood collection time points for the intravenous administration group and intragastric administration group were: 0, 5 min, 15 min, 30 min, 1 h, 2 h, 4 h, 6 h, 8 h, and 24 h. Before analysis and detection, all samples were stored at -80°C, and a quantitative analysis of samples was performed using LC-MS/MS.

Conclusion: The compounds of the present invention, such as the compounds in the examples, have good pharmacokinetic profiles in rats.

### VI. Pharmacokinetic experiment of beagle dogs

1. Experimental animals: male beagle dogs, about 8-11 kg, 6 beagle dogs/compound, purchased from Beijing Marshall Biotechnology Co., Ltd.
2. Experimental method: On the day of the test, (6×number of test compounds (n)) beagles were randomly divided into groups according to body weight. The animals were fasted with water available for 12 to 14 h one day before the administration and were fed 4 h after the administration. Administration was performed as per Table 6.

**Table 6 Administration information**

| Group | Number | Administration information | | | | | |
|---|---|---|---|---|---|---|---|
| | Male | Test compound | Administrati on dosage (mg/kg) | Administratio n concentration (mg/mL) | Administrat ion volume (mL/kg) | Collecte d samples | Mode of administ ration |
| G1 | 3 | Example compounds | 1 | 0.5 | 2 | Plasma | Intraven ous administ ration |
| G2 | 3 | | 3 | 0.6 | 5 | Plasma | Intragast ric administ ration |

| | | | | | | | |
|---|---|---|---|---|---|---|---|
| Note: Vehicle for intravenous administration: 5% DMA+5% Solutol+90% Saline; vehicle for intragastric administration: 0.5% MC | | | | | | | |

Before and after the administration, 1 mL of blood was taken from the jugular veins or limb veins, and placed in an EDTAK2 centrifuge tube. Centrifugation was performed at 5000 rpm at 4°C for 10 min, and plasma was collected. The blood collection time points for the intravenous group and intragastric administration group were: 0 min, 5 min, 15 min, 30 min, 1 h, 2 h, 4 h, 6 h, 8 h, 10 h, 12 h, 24 h, 48 h, 72 h, and 96 h. Before analysis and detection, all samples were stored at - 80°C. The samples were analyzed quantitatively by LC-MS/MS.

Conclusion: The compounds of the present invention, such as the compounds in the examples, have good pharmacokinetic profiles in beagles, for example, the bioavailability (F) of compound 131 was 112%.

### VII. Pharmacokinetic experiment in monkey

1. Experimental animals: Male cynomolgus monkeys, 3-5 kg, 3-6 years old, 4 monkeys/compound. purchased from Suzhou Xishan Biotechnology Inc.
2. Experimental method: On the day of the test, (4×number of test compounds (n)) monkeys were randomly divided into groups according to body weight. The animals were fasted with water available for 14 to 18 h one day before the administration, and were fed 4 h after the administration.

**Table 7 Administration information**

| Group | Number | Administration information | | | | | |
|---|---|---|---|---|---|---|---|
| | Male | Test compound | Administrati on dosage (mg/kg) | Administration concentration (mg/mL) | Administrat ion volume (mL/kg) | Collected samples | Mode of administr ation |
| G1 | 2 | Example compounds | 1 | 1 | 1 | Plasma | Intraven ous administr ation |
| G2 | 2 | | 5 | 1 | 5 | Plasma | Intragast ric administr ation |

| | | | | | | | |
|---|---|---|---|---|---|---|---|
| Note: Vehicle for intravenous administration: 5% DMA+5% Solutol+90% Saline; vehicle for intragastric administration: 0.5% MC | | | | | | | |

Before and after the administration, 1.0 mL of blood samples were drawn from the limb veins and placed in an EDTAK2 centrifuge tube. Centrifugation was carried out at 5000 rpm at 4°C for 10 min, and the plasma was collected. The blood collection time points for the intravenous group and intragastric administration group were: 0 min, 5 min, 15 min, 30 min, 1 h, 2 h, 4 h, 6 h, 8 h, 10 h, 12 h, 24 h, 48 h, 72 h, and 96 h. Before analysis and detection, all samples were stored at - 80°C, and a quantitative analysis of samples was performed using LC-MS/MS.

Conclusion: The compounds of the present invention, such as the compounds in the examples, have good pharmacokinetic profiles in monkeys.

### VIII. Experiment of effect on hERG potassium ion channel

1. Experimental platform: electrophysiological manual patch-clamp system
2. Cell line: Chinese hamster ovary (CHO) cell lines stably expressing hERG potassium ion channel
3. Experimental method: in CHO (Chinese Hamster Ovary) cells stably expressing hERG potassium channel, whole cell patch-clamp technique was used to record hERG potassium channel current at room temperature. The glass microelectrode was made of a glass electrode blank (BF150-86-10, Sutter) by a puller. The tip resistance after filling the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current (*I* _{hERG}) was depolarized from -80 mV to +20 mV for 2 s, then repolarized to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells (n ≥ 2) were tested at each concentration.
4. Data processing: Data analysis processing was carried out by using pClamp 10, GraphPad Prism 5 and Excel software. The inhibition degree of hERG potassium current (peak value of hERG tail current induced at -50 mV) at different compound concentrations was calculated by the following formula:$Inhibition % = \left[1-\left(I/Io\right)\right] \times 100 %$ where Inhibition% represents the percentage of inhibition of hERG potassium current by the compound, and *I* and *I*o represent the amplitude of hERG potassium current after and before the administration, respectively.

Compound IC₅₀ was calculated using GraphPad Prism 5 software by fitting according to the following equation:$Y = Bottom + \left(Top-Bottom\right) / \left(1+{10}^{∧}\left(\left(LogIC50-X\right)*HillSlope\right)\right)$

Among the equation, X represents the Log value of the tested concentration of the test sample, Y represents the inhibition percentage at the corresponding concentration, and Bottom and Top represent the minimum and maximum inhibition percentage, respectively.

**Table 8 IC₅₀ value of the inhibitory effect of compounds on hERG potassium channel current**

| Compound No. | IC₅₀ (µM) |
|---|---|
| 57 | >40 |
| 92 | >40 |
| 124 | >40 |
| 145 | >40 |
| 150 | >40 |
| 157 | >40 |
| 158 | >40 |
| 164 | >40 |
| 165 | >40 |
| Comparative example 1 | 2.5 |

Conclusion: The compounds of the present invention, such as the compounds in the examples, have no obvious inhibitory effect on the hERG potassium channel current.

### IX. CYP450 enzyme inhibition experiment

The purpose of this study was to evaluate the effect of the test compound on the activity of five isoenzymes (CYP1A2, CYP2C9, CYP2C19, CYP2D6 and CYP3A4) of human liver microsomal cytochrome P450 (CYP) by using an *in vitro* testing system. The specific probe substrates of CYP450 isoenzymes were incubated with human liver microsomes and test compounds of different concentrations, and reduced nicotinamide adenine dinucleotide phosphate (NADPH) was added to initiate the reaction. After the completion of the reaction, the sample was treated and liquid chromatography-tandem mass spectrometry (LC-MS/MS) was used to quantitatively detect metabolites produced by specific substrates, changes in CYP enzyme activity were determined, and IC₅₀ value was calculated to evaluate the inhibitory potential of the test compound on each CYP enzyme subtype.

**Table 9 IC₅₀ value of the inhibitory effect of compounds on CYP enzyme**

| Compound No. | IC₅₀ (µM) | | | | |
|---|---|---|---|---|---|
| | CYP1A2 | CYP2C9 | CYP2C19 | CYP2D6 | CYP3A4 |
| 72 | >30 | >30 | >30 | >30 | >30 |
| 88 | >30 | >30 | >30 | >30 | >30 |
| 106 | >30 | >30 | >30 | >30 | >30 |
| 131 | >30 | >30 | >30 | >30 | >30 |
| 141 | >30 | >30 | >30 | >30 | >30 |
| 149 | >30 | >30 | >30 | >30 | >30 |
| 151 | >30 | >30 | >30 | >30 | >30 |
| 153 | >30 | >30 | >30 | >30 | >30 |
| 158 | >30 | >30 | >30 | >30 | >30 |
| 163 | >30 | >30 | >30 | >30 | >30 |
| 164 | >30 | >30 | >30 | >30 | >30 |
| 165 | >30 | >30 | >30 | >30 | >30 |
| 167 | >30 | >30 | >30 | >30 | >30 |
| 169 | >30 | >30 | >30 | >30 | >30 |
| 170 | >30 | >30 | >30 | >30 | >30 |
| 171 | >30 | >30 | >30 | >30 | >30 |
| 172 | >30 | >30 | >30 | >30 | >30 |
| 176 | >30 | >30 | >30 | >30 | >30 |
| Comparative example 1 | >30 | 4.44 | 1.85 | 8.81 | >30 |

Conclusion: The compounds of the present invention, such as the compounds in the examples, have no obvious inhibitory activity against the 5 isozymes of human liver microsomal cytochrome P450 (CYP).

### X. Pharmacokinetic experiment in mouse muscle tissue

1. Experimental animals: Male C57 mice, 22-25 g, 6 mice/compound. Purchased from Chengdu Ddossy Experimental Animals Co., Ltd.
2. Experimental design: On the day of the experiment, C57 mice were randomly grouped according to their body weight. The animals were fasted with water available for 12 to 14 h one day before the administration and were fed 4 h after the administration. Administration was performed as per Table 10.1.

**Table 10.1 Administration information**

| Group | Number | Administration information | | | | | |
|---|---|---|---|---|---|---|---|
| | Male | Test compound | Administra tion dosage (mg/kg) | Administration concentration (mg/mL) | Administr ation volume (mL/kg) | Collected samples | Mode of adminis tration |
| G1 | 6 | Example compounds | 10 | 1 | 10 | Plasma and muscle tissue | Intragas tric adminis tration |

| | | | | | | | |
|---|---|---|---|---|---|---|---|
| Note: vehicle for intragastric administration: 5% DMA + 5% Solutol + 90% Saline | | | | | | | |

After administration, 0.06 mL of blood was taken from the orbits and placed in an EDTAK2 centrifuge tube. The blood was centrifuged at 5000 rpm at 4°C for 10 min, and plasma was collected. At the same time, mouse tibialis anterior muscle tissue was taken, had the surface of which rinsed with physiological saline to clean the remaining blood, then sucked to dryness and homogenized. The time points for collecting blood and tibialis anterior muscle tissue were both at 6 h and 24 h. Before analysis and detection, all samples were stored at -80°C, and a quantitative analysis of samples was performed using LC-MS/MS.

**Table 10.2 Distribution ratio of compounds in mouse tibialis anterior muscle**

| Test compound | Ratio = Tibialis anterior Conc. /Plasma Conc. | |
|---|---|---|
| | 6 h | 24 h |
| Compound 56 | 175 | 316 |
| Compound 88 | 63.5 | 81.1 |
| Compound 129 | 146 | 178 |
| Compound 131 | 136 | 158 |
| Compound 135 | 133 | 165 |
| Compound 139 | 63.5 | 85.5 |
| Compound 141 | 89.9 | 116 |
| Compound 149 | 113 | 171 |
| Compound 150 | 82.2 | 140 |
| Compound 153 | 73.3 | 96.7 |
| Compound 171 | 90.1 | 161 |
| Compound 172 | 67 | 129 |
| Compound 172 | 67 | 129 |

| | | |
|---|---|---|
| Remark: Conc. denotes concentration, unit: ng/g. | | |

Conclusion: The compounds of the present invention, such as the compounds in the examples, have good tissue distribution selectivity and exposure in mouse tibialis anterior muscle, that is, the compounds in the examples have good targeting characteristics on target organ.

### XI. PK/PD detection of mouse model with Dmd (Mdx) gene mutation

Experimental objective: B10-Dmd-KO (Mdx) mice are DMD function-deficient mice caused by frameshift mutations in the Dmd gene. Phenotypic analysis shows that B10-Dmd-KO mice have a series of characteristics of DMD patients. This study has investigated the effects on CK and TNNI2 in the plasma of Mdx mice upon administration of compounds.

Experimental animal: B10-Dmd-KO (C57BL/10ScSnJGpt-Dmdem3Cd4/Gpt), male, 5-7 weeks old, weight 28-30 g, supplier: Gempharmatech Co., Ltd.;

### Experimental procedure:

1) Running training (D-5): Mdx mice underwent running training in batches. The training parameters were set as three time periods, i.e., the initial speed of 4 m/min for 2 min, then 8 m/min for 8 min, and finally 12 m/min for 30 min, a total of 40 min;
2) Gripping strength test (D-2): each round was measured three times, and three rounds were performed;
3) Grouping (D0): the animals were anesthetized by inhalation of isoflurane (concentration: 5%, gas flow rate: 1 L/min), and then a capillary tube was used to collect blood (about 150 µL) via orbital venous sinus into a 0.6 mL centrifuge tube containing EDTA anticoagulant, the plasma wad separated, and the CK value was detected, which was used as the basic value for grouping;
4) Administration, grasping, and blood collection (D1): The compounds were dissolved in the vehicle 5% DMA+5%HS-15+90% Saline, then orally administered to the mice, and then the grip strength test was conducted (three tests per round for three rounds). The animals were subjected to respiratory anesthesia with isoflurane (concentration: 5%, gas flow rate: 1 L/min); 1 h after the completion of the tests, then a capillary tube was used to collect blood (about 300 µL) via orbital venous sinus into a 0.6 mL centrifuge tube containing EDTA anticoagulant (ensuring that the blood was collected 6 h after administration);
5) Running, blood collection, and sample collection (D2): 22 h and 20 min after administration, a 40-min run was performed (the running parameters were the same as above). 1 h after running, mice were anesthetized by inhalation of isoflurane (concentration: 5%, gas flow rate: 1 L/min). A capillary tube was used to collect blood (≥ 300 µL) via orbital venous sinus into a 0.6 mL centrifuge tube containing EDTA anticoagulant, and then the animals were euthanized to collect the tibialis anterior muscle (left leg), soleus muscle (both legs) and cardiac muscle, which were weighed and recorded, placed in a homogenization tube and quickly frozen in liquid nitrogen, then stored at -80°C.

Sample processing: After the whole blood anticoagulated with EDTA was centrifuged at 3000 rpm and 4°C for 10 min, the serum was collected and subpackaged. The separated serum was subjected to a biochemical analyzer (Fully automatic biochemical analyzer Roche C311) to detect the CK value and TNNI2 value (Mouse TNNI2 Elisa Kit, ABBEXA, E2207370Y); The serum concentration of the compound and the drug concentration in tibialis anterior muscle, soleus muscle and cardiac muscle were measured by LC/MS instrument.

**Table 11 Inhibition rate of compound Mdx on CK value in mouse plasma**

| Test compound | Administration regimen | Inhibition rate of CK values (sampled 24 h after (last) dose) |
|---|---|---|
| Compound 56 | 10 mpk single administration | 88.4% |
| Compound 57 | 10 mpk single administration | 108.2% |
| Compound 88 | 10 mpk, QD administration for 7 days | 91.6% |
| Compound 131 | 10 mpk, QD administration for 7 days | 94.1% |

Conclusion: The compounds of the present invention, such as the compounds of the examples, have a good inhibitory effect on the CK value in plasma in mouse model with Dmd (Mdx) gene mutation.

## Claims

1. A compound represented by formula (I), a stereoisomer, a deuterated substance, a solvate, or a pharmaceutically acceptable salt or a eutectic crystal thereof, 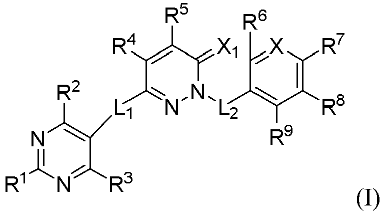 **characterized in that**
each of R¹, R² and R³ is independently selected from H, deuterium, R^{A1}, -O-haloC₁₋₄ alkyl, -NH-haloC₁₋₄ alkyl, -OC₁₋₄ alkyl, C₁₋₆ alkoxy, halogen, cyano, nitro, C₁₋₄ alkyl, -NH-C₁₋₄ alkyl, -N(C₁₋₄ alkyl)₂, -NH-C₃₋₁₀ cycloalkyl, -NHC(O)C₁₋₄ alkyl, or 4- to 10-membered heterocycloalkyl containing 1 to 3 heteroatoms selected from N, O, and S, and the alkyl, alkoxy, cycloalkyl, or heterocycloalkyl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, C₃₋₁₀ cycloalkyl, 4- to 6-membered heterocycloalkyl containing 1 to 3 heteroatoms selected from N, O, or S, phenyl, and NH₂, provided that R¹, R² and R³ are not simultaneously selected from H;
R^{A1} is selected from haloC₂₋₆ alkenyl, C₂₋₆ alkynyl, -O-(CH₂)ᵣ-R^{a}, C₃₋₁₀ cycloalkyl, 5- to 10-membered heteroaryl containing 1 to 3 heteroatoms selected from N, O, or S, -C₁₋₄ alkyl-R^{a}, -C₁₋₄ alkyl-OR^{a}, -C₁₋₄ alkyl-NR^{b}R^{a}, -NR^{b}-S(O)₂-R^{a}, -NR^{b}-S(O)₂-NR^{b}R^{a}, -O-NR^{b}R^{a}, -NH-OR^{b}, or -Se-(CH₂)ᵣ-R^{a}, and the alkynyl, heteroaryl, alkyl, or cycloalkyl is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{a} is selected from CN, haloC₁₋₆ alkyl, C₃₋₁₀ cycloalkyl, 4- to 10-membered heterocycloalkyl containing 1 to 3 heteroatoms selected from N, O, or S, 6- to 10-membered aryl, 5- to 10-membered heteroaryl containing 1 to 3 heteroatoms selected from N, O, or S, or -C(O)-R^{a1}, and the alkyl, cycloalkyl, heterocycloalkyl, aryl, or heteroaryl is optionally further substituted with 1 to 3 groups selected from halogen, =O, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each R^{b} is selected from H, deuterium, C₁₋₄ alkyl, C₃₋₆ cycloalkyl, haloC₁₋₄ alkyl, or deuterated C₁₋₄ alkyl;
R^{a1} is selected from OH, NH₂, -NHC₁₋₄ alkyl, -N(C₁₋₄ alkyl)₂, -NHC₃₋₁₀ cycloalkyl, C₁₋₄ alkoxy, C₁₋₆ alkyl, C₃₋₁₀ cycloalkyl, 6- to 10-membered aryl, 5- to 10-membered heteroaryl containing 1 to 3 heteroatoms selected from N, O, or S;
X is selected from CR¹⁰ or N;
X₁ is selected from O or S;
each of R⁴ and R⁵ is independently selected from H, deuterium, C₃₋₁₀ cycloalkyl, C₂₋₆ alkenyl, C₂₋₆ alkynyl, halogen, amino,-NH-C₁₋₄ alkyl, -N(C₁₋₄ alkyl)₂, C₁₋₄ alkyl, haloC₁₋₄ alkyl, 4- to 10-membered heterocycloalkyl containing 1 to 3 heteroatoms selected from N, O, or S, 5- to 10-membered heteroaryl containing 1 to 3 heteroatoms selected from N, O, or S, or 6- to 10-membered aryl;
each of R⁶, R⁷, R⁸, R⁹, and R¹⁰ is independently selected from H, deuterium, R^{A2}, halogen, OH, CN, amino, C₁₋₄ alkyl, C₂₋₆ alkenyl, C₂₋₆ alkynyl, C₁₋₄ alkoxy, haloC₁₋₄ alkyl, -NH-C₁₋₄ alkyl, -N(C₁₋₄ alkyl)₂, -S(O)R^{c}, -S(O)²R^{c}, -C(O)R^{c}, - C(O)OR^{c}, -C(O)N(R^{c})₂, or -OC(O)R^{c}, and the alkyl, alkenyl, alkynyl, or alkoxy is optionally further substituted with 1 to 3 groups selected from halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
R^{A2} is selected from -(CH₂)ᵣ-(C₃₋₁₀ cycloalkyl), -(CH₂)ᵣ-(4- to 10-membered heterocycloalkyl containing 1 to 3 heteroatoms selected from N, O, S, or Si), or - (CH₂)ᵣ-(5- to 10-membered heteroaryl containing 1 to 3 heteroatoms selected from N, O, or S), -P(O)(R^{c})₂, -Si(R^{c})₃, -SF₅, N₃, B(OH)₂, or -S(O)(=NH)R^{c}, and the cycloalkyl, heterocycloalkyl, or heteroaryl is optionally further substituted with 1 to 3 groups selected from halogen, =O, D, CN, OH, C₁₋₄ alkyl, haloC₁₋₄ alkyl, deuterated C₁₋₄ alkyl, C₁₋₄ alkoxy, haloC₁₋₄ alkoxy, C₃₋₆ cycloalkyl, 4- to 7-membered heterocycloalkyl containing 1 to 3 heteroatoms selected from N, O, or S, deuterated C₁₋₄ alkoxy and NH₂;
each R^{c} is independently selected from H, OH, C₁₋₄ alkyl, C₃₋₇ cycloalkyl, haloC₁₋₄ alkyl, deuterated C₁₋₄ alkyl, C₁₋₄ alkoxy, haloC₁₋₄ alkoxy, deuterated C₁₋₄ alkoxy, or NH₂;
each r is independently selected from 0, 1, 2 or 3;
L₁ is selected from a bond, O, NH, S, -CD₂-, -CHD-, -CR^{L1}R^{L2}-, C₁₋₄ alkyl, - C(O)-, S(O), or S(O)₂, and the alkyl is optionally further substituted with R^{L1};
L₂ is selected from a bond, O, NH, S, -CH₂-, -CD₂-, -CHD-, -CR^{L1}R^{L2}-, - CHR^{L2}-, -CDR^{L2}-, C₂₋₄ alkyl, -C(O)-, S(O), or S(O)₂, and the alkyl is optionally further substituted with 1 to 3 R^{L1};
each of R^{L1} and R^{L2} is independently selected from halogen, OH, CN, amino, C₁₋₄ alkyl, C₁₋₄ alkoxy, C₃₋₆ cycloalkyl, or 4- to 6-membered heterocycloalkyl containing 1 to 3 heteroatoms selected from N, O, or S;
alternatively, R², R⁴ and L₁ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
alternatively, R⁴ and R⁵ together with the atoms to which they are attached form a 5- to 7-membered monocyclic carbocyclic ring, or a 5- to 7-membered heterocyclic ring containing 1 to 3 heteroatoms selected from N, O, or S, and the carbocyclic ring or heterocyclic ring is optionally substituted with 1 to 3 groups selected from R^{A3};
alternatively, R^{L2} and R⁹ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
each R^{A3} is independently selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂ group substitution;
provided that: one of the combinations of R⁶ and R¹⁰, R¹⁰ and R⁷, R⁷ and R⁸, and R⁸ and R⁹ together with the atoms to which they are attached form a 4- to 8-membered carbocyclic ring or 5- to 7-membered heterocycloalkyl containing 1 to 3 heteroatoms selected from N, O, S, B, or Si, and the carbocyclic ring or heterocycloalkyl is optionally further substituted with 1 to 3 groups selected from =O, 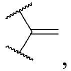 halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, 5- to 7-membered heterocycloalkyl containing 1 to 3 heteroatoms selected from N, O, S, B, or Si, and 5- to 7-membered heteroaryl containing 1 to 3 heteroatoms selected from N, O, S, B, or Si and NH₂.

2. The compound, and the stereoisomer, deuterated substance, solvate, or pharmaceutically acceptable salt or eutectic crystal thereof according to claim 1, **characterized in that**
each of R⁶, R⁷, R⁸, R⁹, and R¹⁰ is independently selected from H, deuterium, - SF₅, N₃, halogen, OH, CN, amino, C₁₋₂ alkyl, C₂₋₄ alkenyl, C₂₋₄ alkynyl, C₁₋₂ alkoxy, haloC₁₋₂ alkyl, -NH-C₁₋₂ alkyl, or -N(C₁₋₂ alkyl)₂, and the alkyl, alkenyl, alkynyl, or alkoxy is optionally further substituted with 1 to 3 groups selected from halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂.

3. The compound, and the stereoisomer, deuterated substance, solvate, or pharmaceutically acceptable salt or eutectic crystal thereof according to claim 1, **characterized in** having a structure of formula (II) or (II-a): 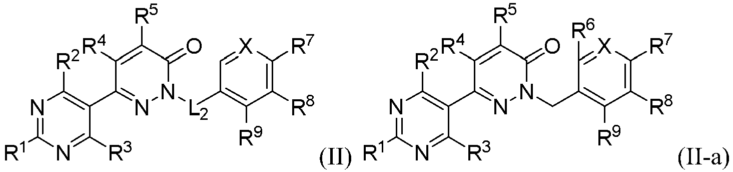
L₂ is selected from a bond, -CH₂-, -CR^{L1}R^{L2}- , -CHR^{L2}- , -CDR^{L2}-, or -C(O)-;
each of R^{L1} and R^{L2} is independently selected from halogen, OH, CN, amino, C₁₋₂ alkyl, C₁₋₂ alkoxy, C₃₋₄ cycloalkyl, or 4-membered heterocycloalkyl containing 1 to 3 heteroatoms selected from N, O, or S;
each of R² and R³ is independently selected from H, deuterium, C₁₋₂ alkoxy, halogen, cyano, nitro, or C₁₋₂ alkyl, and the alkyl or alkoxy is optionally further substituted with 1 to 3 groups selected from halogen, D, CN, OH and NH₂;
R¹ is selected from R^{A1}, -O-haloC₁₋₄ alkyl, -NH-haloC₁₋₄ alkyl, C₁₋₄ alkoxy, halogen, cyano, nitro, C₁₋₄ alkyl, -NH-C₁₋₄ alkyl, -N(C₁₋₄ alkyl)₂, -NH-C₃₋₁₀ cycloalkyl, -NHC(O)C₁₋₄ alkyl, or 4- to 10-membered heterocycloalkyl containing 1 to 3 heteroatoms selected from N, O, and S, and the alkyl, alkoxy, cycloalkyl, or heterocycloalkyl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, C₃₋₁₀ cycloalkyl, 4- to 6-membered heterocycloalkyl containing 1 to 3 heteroatoms selected from N, O, or S, phenyl, and NH₂;
X is selected from CR¹⁰ or N;
each of R⁴ and R⁵ is independently selected from H, deuterium, C₃₋₇ cycloalkyl, C₂₋₄ alkenyl, C₂₋₄ alkynyl, F, Cl, amino,-NH-C₁₋₂ alkyl, -N(C₁₋₂ alkyl)₂, C₁₋₂ alkyl, or 4- to 7-membered heterocycloalkyl containing 1 to 3 heteroatoms selected from N, O, or S;
each of R⁷ and R⁸ is independently selected from H, deuterium, F, Cl, OH, CN, amino, C₁₋₄ alkyl, C₂₋₄ alkenyl, C₂₋₄ alkynyl, C₁₋₄ alkoxy, haloC₁₋₄ alkyl, -NH-C₁₋₂ alkyl, -N(C₁₋₂ alkyl)₂, -S(O)R^{c}, -S(O)²R^{c}, -C(O)R^{c}, -C(O)OR^{c}, -C(O)N(R^{c})₂, or -OC(O)R^{c};
each of R⁶, R⁹, and R¹⁰ is independently selected from H, deuterium, F, Cl, OH, CN, amino, or C₁₋₂ alkyl;
alternatively, R², R⁴ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
each R^{A3} is independently selected from =O, halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
provided that: one of the combinations of R¹⁰ and R⁷, R⁷ and R⁸, and R⁸ and R⁹ together with the atoms to which they are attached form 4- to 8-membered cycloalkyl or 5- to 7-membered heterocycloalkyl containing 1 to 3 heteroatoms selected from N, O, S, B, or Si, and the cycloalkyl or heterocycloalkyl is optionally further substituted with 1 to 3 groups selected from =O, 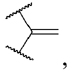 halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, 5- to 7-membered heterocycloalkyl containing 1 to 3 heteroatoms selected from N, O, S, B, or Si, 5- to 7-membered heteroaryl containing 1 to 3 heteroatoms selected from N, O, S, B, or Si and NH₂.

4. The compound, and the stereoisomer, deuterated substance, solvate, or pharmaceutically acceptable salt or eutectic crystal thereof according to claim 1, **characterized in** having a structure of formula (III) or (III-a): 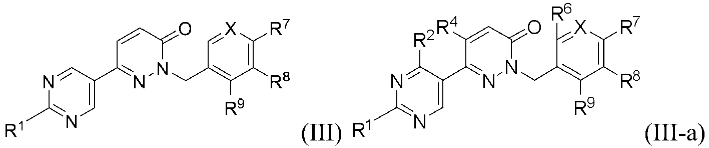
X is selected from CR¹⁰ or N;
each of R⁶, R⁷, R⁸, R⁹ and R¹⁰ is independently selected from H, deuterium, F, Cl, OH, CN, amino, or C₁₋₂ alkyl;
alternatively, R², R⁴ together with the atoms to which they are attached form a 5- to 7-membered carbocyclic ring, or a 5- to 7-membered heterocyclic ring containing 1 to 3 heteroatoms selected from N, O, or S, and the carbocyclic ring or heterocyclic ring is optionally further substituted with 1 to 3 groups selected from R^{A3};
provided that: one of the combinations of R¹⁰ and R⁷, R⁷ and R⁸, and R⁸ and R⁹ together with the atoms to which they are attached form 4- to 8-membered cycloalkyl or 5- to 7-membered heterocycloalkyl containing 1 to 3 heteroatoms selected from N, O, S, B, or Si, and the cycloalkyl or heterocycloalkyl is optionally further substituted with 1 to 3 groups selected from =O, 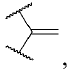 halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, 5- to 7-membered heterocycloalkyl containing 1 to 3 heteroatoms selected from N, O, S, B, or Si, 5- to 7-membered heteroaryl containing 1 to 3 heteroatoms selected from N, O, S, B, or Si and NH₂.

5. The compound, and the stereoisomer, deuterated substance, solvate, or pharmaceutically acceptable salt or eutectic crystal thereof according to any one of claims 1-4, **characterized in that**
R¹ is selected from R^{A1}, -O-haloC₁₋₄ alkyl or C₁₋₄ alkyl, and the alkyl is optionally further substituted with 1 to 3 groups selected from halogen, D, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
each of R² and R³ is independently selected from H, deuterium, C₂₋₄ alkenyl, C₂₋₄ alkynyl, halogen, cyano, nitro, OH, C₁₋₄ alkyl, haloC₁₋₄ alkyl, or deuterated C₁₋₄ alkyl;
R^{A1} is selected from haloC₂₋₄ alkenyl, C₂₋₄ alkynyl, -O-(CH₂)ᵣ-R^{a}, C₃₋₆ monocyclic cycloalkyl, C₅₋₈ bicyclic bridged cycloalkyl, C₆₋₁₀ bicyclic spirocyclic cycloalkyl, 5- to 6-membered monocyclic heteroaryl containing 1 to 3 heteroatoms selected from N, O, or S, 8- to 10-membered bicyclic heteroaryl containing 1 to 3 heteroatoms selected from N, O, or S, -C₁₋₂ alkyl-R^{a}, -C₁₋₂ alkyl-OR^{a}, -C₁₋₂ alkyl-NR^{b}R^{a}, -NR^{b}-S(O)₂-R^{a}, -NR^{b}-S(O)₂-NR^{b}R^{a}, -O-NR^{b}R^{a}, -NH-OR^{b}, or -Se-(CH₂)ᵣ-R^{a}, and the alkynyl, heteroaryl, alkyl, or cycloalkyl is optionally further substituted with 1 to 3 groups selected from halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
each R^{a} is selected from CN, haloC₁₋₄ alkyl, C₃₋₆ monocyclic cycloalkyl, C₇₋₁₀ spirocyclic cycloalkyl, C₄₋₈ bridged cycloalkyl, C₄₋₉ fused cycloalkyl, 4- to 6-membered monocyclic heterocycloalkyl containing 1 to 3 heteroatoms selected from N, O, or S, 7- to 10-membered spirocyclic heterocycloalkyl containing 1 to 3 heteroatoms selected from N, O, or S, 4- to 8-membered bridged heterocycloalkyl containing 1 to 3 heteroatoms selected from N, O, or S, 4- to 6-membered fused heterocycloalkyl containing 1 to 3 heteroatoms selected from N, O, or S, phenyl, 8-to 10- membered aryl, 5- to 6-membered monocyclic heteroaryl containing 1 to 3 heteroatoms selected from N, O, or S, 8- to 10-membered bicyclic heteroaryl containing 1 to 3 heteroatoms selected from N, O, or S, or -C(O)-R^{a1}, and the alkyl, cycloalkyl, heterocycloalkyl, aryl, or heteroaryl is optionally further substituted with 1 to 3 groups selected from halogen, =O, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
each R^{b} is selected from H, deuterium, C₁₋₂ alkyl, or C₃₋₄ cycloalkyl.

6. The compound, and the stereoisomer, deuterated substance, solvate, or pharmaceutically acceptable salt or eutectic crystal thereof according to claim 5, **characterized in that**
R¹ is selected from R^{A1}, -O-haloC₁₋₄ alkyl or C₁₋₄ alkyl, and the alkyl is optionally further substituted with 1 to 3 groups selected from halogen, D, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
each of R² and R³ is independently selected from H, deuterium, F, Cl, cyano, OH, C₁₋₂ alkyl, haloC₁₋₂ alkyl, or deuterated C₁₋₂ alkyl;
R^{A1} is selected from -O-(CH₂)ᵣ-R^{a}, C₃₋₆ monocyclic cycloalkyl, C₅₋₈ bicyclic bridged cycloalkyl, C₆₋₁₀ bicyclic spirocyclic cycloalkyl, 5- to 6-membered monocyclic heteroaryl containing 1 to 3 heteroatoms selected from N, O, or S, 8-to 10-membered bicyclic heteroaryl containing 1 to 3 heteroatoms selected from N, O, or S, -C₁₋₂ alkyl-R^{a}, -C₁₋₂ alkyl-OR^{a}, or -Se-(CH₂)ᵣ-R^{a}, and the heteroaryl, alkyl, or cycloalkyl is optionally further substituted with 1 to 3 groups selected from halogen, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂;
each R^{a} is selected from haloC₁₋₄ alkyl, C₃₋₆ monocyclic cycloalkyl, C₇₋₁₀ spirocyclic cycloalkyl, C₄₋₈ bridged cycloalkyl, C₄₋₉ fused cycloalkyl, 4- to 6-membered monocyclic heterocycloalkyl containing 1 to 3 heteroatoms selected from N, O, or S, 7- to 10-membered spirocyclic heterocycloalkyl containing 1 to 3 heteroatoms selected from N, O, or S, 4- to 8-membered bridged heterocycloalkyl containing 1 to 3 heteroatoms selected from N, O, or S, 4- to 6-membered fused heterocycloalkyl containing 1 to 3 heteroatoms selected from N, O, or S, phenyl, 8-to 10- membered aryl, 5- to 6-membered monocyclic heteroaryl containing 1 to 3 heteroatoms selected from N, O, or S, or 8- to 10-membered bicyclic heteroaryl containing 1 to 3 heteroatoms selected from N, O, or S, and the alkyl, cycloalkyl, heterocycloalkyl, aryl, or heteroaryl is optionally further substituted with 1 to 3 groups selected from halogen, =O, deuterium, CN, OH, C₁₋₂ alkyl, haloC₁₋₂ alkyl, deuterated C₁₋₂ alkyl, C₁₋₂ alkoxy, haloC₁₋₂ alkoxy, deuterated C₁₋₂ alkoxy and NH₂.

7. The compound, and the stereoisomer, deuterated substance, solvate, or pharmaceutically acceptable salt or eutectic crystal thereof according to claim 1, **characterized in** having a structure of formula (IV) or (V): 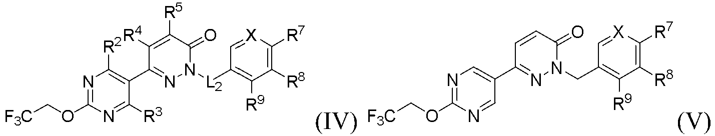
X is selected from CR¹⁰ or N;
each of R⁹ and R¹⁰ is independently selected from H, deuterium, F, Cl, OH, CN, amino, or C₁₋₂ alkyl;
L₂ is selected from a bond, -CH₂-, -CD₂-, -CHD-, -CR^{L1}R^{L2}-, -CHR^{L2}-, - CDR^{L2}-, C₂₋₄ alkyl, or -C(O)-, and the alkyl is optionally further substituted with 1 to 3 R^{L1};
provided that: one of the combinations of R¹⁰ and R⁷, R⁷ and R⁸, and R⁸ and R⁹ together with the atoms to which they are attached form 4- to 8-membered cycloalkyl, or 5- to 7-membered heterocycloalkyl containing 1 to 3 heteroatoms selected from N, O, S, B, or Si, and the cycloalkyl or heterocycloalkyl is optionally further substituted with 1 to 3 groups selected from =O, 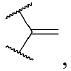 halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂.

8. The compound, and the stereoisomer, deuterated substance, solvate, or pharmaceutically acceptable salt or eutectic crystal thereof according to claim 1, **characterized in that**
L₁ is selected from a bond;
L₂ is selected from a bond, -CH₂-, -CD₂-, -CHD-, -C(O)-, -CHF-, -CDF-, - CF₂-, CH(CH₃)-, CD(CH₃)-, or C(CH₃)₂-;
each of R⁶, R⁹, and R¹⁰ is independently selected from H, deuterium, F, Cl, OH, CN, or methyl;
each of R⁷ and R⁸ is independently selected from H, deuterium, F, Cl, OH, CN, or methyl;
each of R² and R³ is independently selected from H, deuterium, vinyl, or ethynyl;
each of R⁴ and R⁵ is independently selected from H, deuterium, cyclopropyl, vinyl, or ethynyl;
alternatively, R⁴, R⁵ together with the atoms to which they are attached form phenyl, cyclohexyl, cyclopentyl, thienyl, furyl, pyrrolyl, pyrazolyl, or isoxazolyl;
alternatively, R², R⁴, L₁ together with the atoms to which they are attached form cyclohexyl, cycloheptyl, phenyl or cyclopentyl;
alternatively, R^{L1} and R⁹ together with the atoms to which they are attached form cyclopentyl or cyclohexyl;
R¹ is selected from -CF₃, -CH₂CH₃, -CH₂CH₂CH₃, 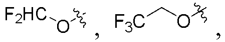 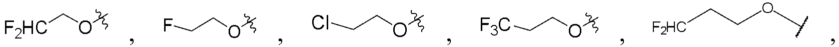 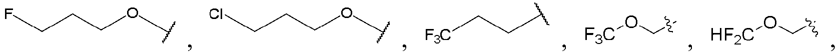 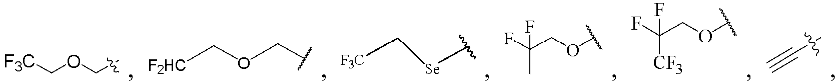 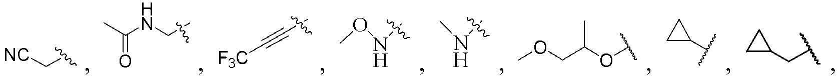 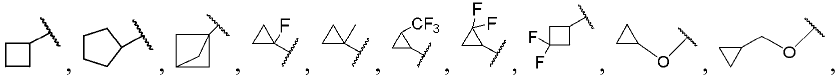 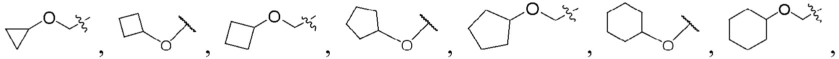 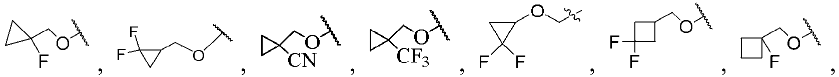 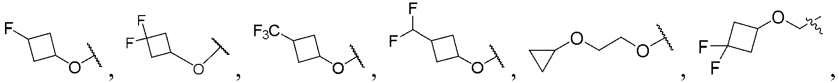 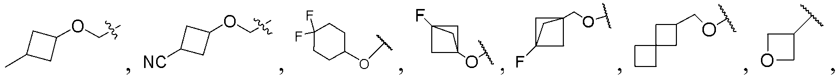 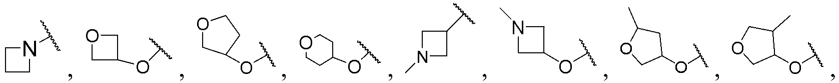 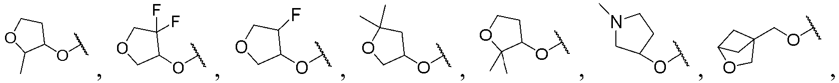 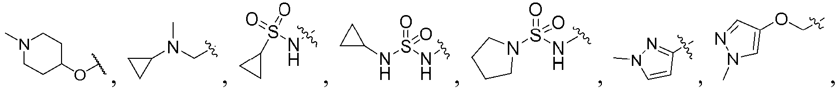 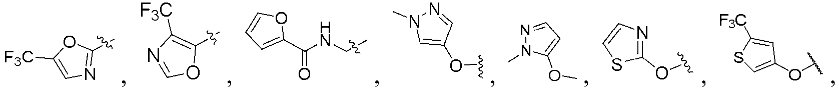 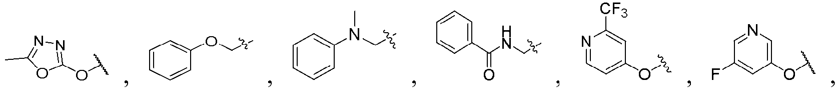 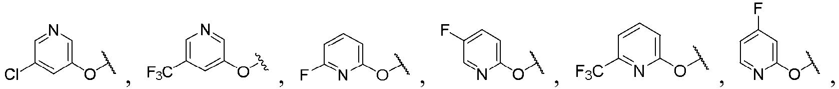 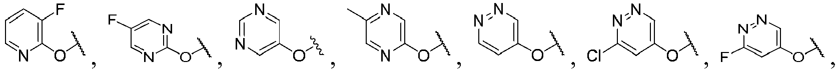 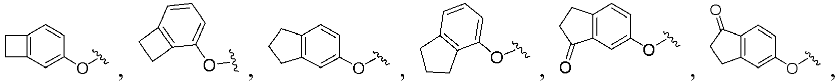 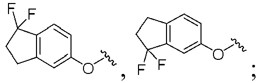
provided that: one of the combinations of R⁶ and R¹⁰, R¹⁰ and R⁷, R⁷ and R⁸, and R⁸ and R⁹ together with the atoms to which they are attached form 4- to 8-membered cycloalkyl or 5- to 7-membered heterocycloalkyl containing 1 Si atom, and the cycloalkyl or heterocycloalkyl is optionally further substituted with 1 to 3 groups selected from =O, 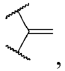 F, Cl, deuterium, OH, C₁₋₂ alkyl, haloC₁₋₂ alkyl, 5-to 6-membered heteroaryl containing 1 to 3 heteroatoms selected from N, O, S, B, or Si.

9. The compound, and the stereoisomer, deuterated substance, solvate, or pharmaceutically acceptable salt or eutectic crystal thereof according to claim 1, **characterized in that** the compound is selected from one of the structures in Table I and Table II.

10. A pharmaceutical composition or pharmaceutical preparation, **characterized in that** the pharmaceutical composition or pharmaceutical preparation comprises the compound, and the stereoisomer, deuterated substance, solvate, or pharmaceutically acceptable salt or eutectic crystal thereof according to any one of claims 1 to 9, and a pharmaceutically acceptable carrier and/or excipient.

11. The pharmaceutical composition or pharmaceutical preparation according to claim 10, **characterized in that** the pharmaceutical composition or pharmaceutical preparation comprises 1 mg to 1500 mg of the compound, and the stereoisomer, deuterated substance, solvate, or pharmaceutically acceptable salt or eutectic crystal thereof according to any one of claims 1 to 9, and the pharmaceutically acceptable carrier and/or excipient.

12. The compound, and the stereoisomer, deuterated substance, solvate, or pharmaceutically acceptable salt or eutectic crystal thereof according to any one of claims 1 to 9, or the composition according to claim 10-11 for use in the preparation of a drug for treating/preventing a Myosin II-mediated disease.

13. The use according to claim 12, **characterized in that** the Myosin II-mediated disease is selected from muscular dystrophy.

14. A method for treating a disease in a mammal, **characterized in that** the method comprises administering a therapeutically effective amount of the compound, and the stereoisomer, deuterated substance, solvate, or pharmaceutically acceptable salt or eutectic crystal thereof according to any one of claims 1 to 9 to a subject, the therapeutically effective amount is preferably 1 mg-1500 mg, and the disease is preferably muscular dystrophy.
